# EUROPEAN PATENT SPECIFICATION

(11) **EP 4 463 458 B1**
(45) Date of publication and mention of the grant of the patent: **25.11.2026**
(21) Application number: 23702959.0
(22) Date of filing: 06.01.2023
(51) Int. Cl.: C07D 487/04, A61K 31/53, A61P 35/00

(54) **BICYCLIC TRIAZINE DERIVATIVES FOR THE TREATMENT OF CANCER**
BIZYCLISCHE TRIAZINDERIVATIVE ZUR BEHANDLUNG VON KREBS
DERIVES BICYCLIQUES DE TRIAZINE COMME DES AGENTS ANTICANCEREUX

(30) Priority: 11.01.2022 EP 22150829
(43) Date of publication of application: 20.11.2024
(73) Proprietor: Deutsches Krebsforschungszentrum, 69120 Heidelberg (DE)
(72) Inventor: THEDE, Kai, 10437 Berlin (DE); BUCHGRABER, Philipp, 10435 Berlin (DE); SIEMEISTER, Gerhard, 13503 Berlin (DE); STEIGEMANN, Patrick, 13156 Berlin (DE); WENGNER, Antje Margret, 13086 Berlin (DE); BÖMER, Ulf, 16548 Glienicke (DE); LIENAU, Philip, 10967 Berlin (DE); WESTERMANN, Frank, 69120 Heidelberg (DE); GLIMM, Hanno, 69120 Heidelberg (DE); HERBST-NOWROUZI, Friederike, 69120 Heidelberg (DE); DIETER, Sebastian, 69120 Heidelberg (DE); KRETH, Sina, 69120 Heidelberg (DE)
(74) Representative: Ullrich & Naumann PartG mbB
(86) International application number: PCT/EP2023/050208
(87) International publication number: WO 2023/135057

(56) References cited:
- WO-A1-2021/116178

## Description

### BACKGROUND

The present invention provides compounds of general formula **(I)** which impair the activity of CDK12. In particular, the present invention provides compositions and methods for the treatment of cancer and other CDK12-dependant diseases. Thus, the present invention provides compounds capable of inhibiting the kinase activity of CDK12/Cyclin K for the treatment of breast cancer, liver cancer, lung cancer, ovarian cancer, endometrial cancer, cervical cancer, colorectal cancer, gastric cancer, esophageal cancer, bladder cancer, prostate cancer, Ewing sarcoma, glioblastoma and acute myeloid leukemia. Even more particularly, the present invention provides compounds capable of inhibiting CDK12/Cyclin K for the treatment of lung cancer, breast cancer, liver cancer, colorectal cancer, gastric cancer, prostate cancer and leukemia.

Cyclin-dependent kinase (CDK) 12 (CDK12, gene id 51755) is a member of the subset of the CDK serine/threonine kinase family that phosphorylates the C-terminal domain (CTD) of RNA polymerase II. CDK12 in complex with Cyclin K (CCNK, gene id 8812) regulates transcriptional, co- and posttranscriptional processes by phosphorylation of Ser2 and Ser5 of the CTD of RNA polymerase II complexes which are important in the elongation phase of pre-mRNA synthesis. CDK12/Cyclin K has been reported to regulate transcriptional elongation and mRNA processing, in particular co- and post-transcriptional pre-mRNA splicing, alternative splicing, 3'end processing, and suppression of intronic polyadenlyation. CDK13 (CDK13, gene id 8621), a kinase which is closely related to CDK12, also forms a complex with Cyclin K and regulates the transcription of a different set of genes (Bartkowiak et al. Genes Dev. 2010;24:2303-16. Dubbury et al. Nature. 2018;564:141-5. Greenleaf, Transcription. 2018;10:91-110. Greifenberg et al. Cell Rep. 2016;14:320-31. Liang et al. Mol. Cell. Biol. 2015;35:928-38. Lui et al. J. Clin. Pathol. 2018;71:957-62. Tien et al. Nuc. Acids Res. 2017;45:6698-716). The transcription of genes encoding components of DNA damage signaling and repair pathways, such as the homologous recombination and replication stress response genes BRCA1, FANCD2, FANCI, and ATR, as well as encoding components of other stress response pathways, such as NF-κB and oxidative stress response, has been reported to be specifically regulated by CDK12/Cyclin K as demonstrated by gene knock-down and chemoproteomics studies (Blazek et al. Genes Dev. 2011;25:2158-72. Henry et al. Sci. Signal. 2018;11:eaam8216. Li et al. Sci. Rep. 2016;6:21455.). In addition, CDK12/Cyclin K has been reported to control the translation of a subset of mRNAs, including the CHK1 mRNA, by directly phosphorylating the mRNA 5' cap-binding translational repressor 4E-BP1 leading to its release from the mRNA cap (Choi et al. Genes Dev. 2019;33:418-35). The recent discovery of rare bi-allelic CDK12 inactivating mutations in high-grade serous ovarian cancer and in primary and castration-resistant prostate cancer leading to a special type of genomic instability which is characterized by the occurance of numerous tandem duplications, indicating gross defects in DNA repair, underscores the role of CDK12 in DNA damage response and the maintenance of the genome (Ekumi et al. Nucl. Acids Res. 2015;43:2575-89. Grasso et al. Nature. 2012;487:239-43. Joshi et al. J. Biol. Chem. 2014;289:9247-53. Menghi et al. Cancer Cell. 2018;34:197-210.e5. Popova et al. Cancer Res. 2016;76:1882-91. Quigley et al. Cell. 2018;174:758-69.e9. Robinson et al. 2015;162:454. Viswanathan et al. Cell. 2018;174:433-47.e19. Wu et al. Cell. 2018;173:1770-82.e14). The CDK12 gene is located on chromosome 17 about 200 kb proximal to the ERBB2 gene and is often coamplified in breast cancer. Furthermore, CDK12 gene amplification has been observed in other cancer types such as stomach cancer, esophageal cancer, pancreatic cancer, uterine cancer, endometrial cancer, prostate cancer, and bladder cancer (Lui et al. J Clin Pathol. 2018;71:957-62. Gupta et al. Clin. Cancer Res. 2017;23:1346-57). CDK12 amplification and high expression levels suggest a tumor promoting role of CDK12 which is, at least partially, based on alterantively spliced mRNAs, increased DNA repair capabilities and increased stress tolerance (Lui et al. J Clin Pathol. 2018;71:957-62. Tien et al. Nucl. Acids Res. 2017;45:6698-716). Taken together these data validated CDK12 as a potential target to develop drugs for the treatment of cancer and other diseases such as myotonic dystrophy type 1.

Some inhibitors of CDK12 kinase activity are known:
Flavopiridol, a micromolar non-selective inhibitor of CDK12 which inhibits other kinases such as CDK9, CDK1, CDK4 etc. (Bösken et al. Nat. Comm. 2014;5:3505). Dinaciclib, a pan CDK inhibitor (Johnson et al. Cell Rep. 2016;17:2367-81). THZ531, a dual inhibitor of CDK12 and CDK13 (Zhang et al. Nat. Chem. Biol. 2016;12:876-84). SR-3029 and related purine compounds (Johannes et al. Chem. Med. Chem. 2018;13:231-5). SR-4835, a dual inhibitor of CDK12 and CDK13 (Quereda et al. Cancer Cell 2019; 36:1-14). Compound 919278, a micromolar CDK12 inhibitor (Henry et al. Science Signal. 2018;11:eaam8216). Arylurea derivatives (Ito et al. J. Med. Chem. 2018;61:7710-28).

In addition compounds have been described which induce the proteolytic degradation of CDK12 and/or CCNK in a cell (Slabicki et al. Nature 2020;585:293-297. Lv et al. eLife 2020;9:e59994. Jiang et al. Nat. Chem. Biol. 2021; 17:675-683. Dieter et al. Cell Reports 2021;36: 109394. WO 2021/116178. WO 2021/176045. WO 2021/176049).

There is a need for development of compounds selectively impairing the function of CDK12/Cyclin K for the treatment of cancer and other diseases. Covalent inhibitors of CDK12 and CDK13 kinase function as well as CDK12/Cyclin K degrader compounds have been described to induce tumor cell variants which are resistant towards such inhibitors or degrader compounds and thereby limiting their potential therapeutic use (Jiang et al. Nat. Chem. Biol. 2021;17:675-683). Surprisingly, the compounds described in the present invention overcome cellular resistance against CDK12/Cyclin K degrader compounds and show comparable anti-tumor activity against resistant cells and against the corresponding parental cells. CDK12 inhibitors with high kinase inhibition potential at physiological ATP concentrations but weak or absent CDK12 degrading potency are selective against other kinases. Thus, there is a need to provide compounds which impair the activity of CDK12/Cyclin K in the cell and which exhibit a good degree of selectivity towards the targeting of other CDKs and other kinases,.

### SUMMARY

The present invention provides compounds of general formula (I): 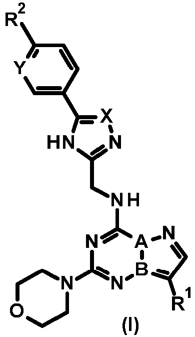 in which A, B, X, Y, R¹, R² and R³ are as described and defined herein, methods of preparing said compounds, intermediate compounds useful for preparing said compounds, pharmaceutical compositions and combinations comprising said compounds, and the use of said compounds for manufacturing pharmaceutical compositions for the treatment and/or prophylaxis of diseases, in particular of hyperproliferative disorders such as cancer disorders, as a sole agent or in combination with other active ingredients.

### DESCRIPTION OF THE INVENTION

It has now been found that the compounds of the present invention effectively impair the activity of CDK12/Cyclin K for which data are given in the biological experimental section and may therefore be used for the treatment and/or prophylaxis of hyperproliferative disorders, such as cancer disorders. In particular, the compounds of the present invention are CDK12 inhibitors with high kinase inhibition potential at physiological ATP concentrations but weak or absent proteolytic CDK12 and/or Cyclin K degrading potency in cells and which are selective against other kinases.

In accordance with a first aspect, the present invention provides compounds of general formula (I): 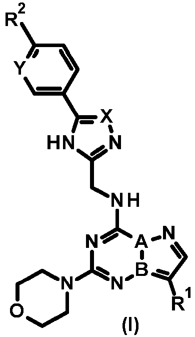 wherein
- A and B: are independently from each other, selected from a nitrogen atom or a carbon atom, wherein when A is a nitrogen atom, B is a carbon atom and wherein when A is a carbon atom, B is a nitrogen atom;
- R¹: is selected from a halogen atom and a C₁-C₃-haloalkyl group;
- R²: is selected from a C₁-C₃-alkyl group, a C₁-C₃-alkoxy group, a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

### DETAILED DESCRIPTION

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1, 2 or 3, more particularly 1 or 2, and even more particularly 1.

As used herein, the term "one or more", *e.g*. in the definition of the substituents of the compounds of general formula **(I)** of the present invention, means "1, 2, 3, 4 or 5, particularly 1, 2, 3 or 4, more particularly 1, 2 or 3, even more particularly 1 or 2".

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one, two or three substituents, more particularly with one substituent.

The terms "oxo", "an oxo group" or "an oxo substituent" mean a doubly bonded oxygen atom =O. Oxo may be attached to atoms of suitable valency, for example to a saturated carbon atom or to a sulfur atom. For example, but without limitation, one oxo group can be attached to a carbon atom, resulting in the formation of a carbonyl group C(=O), or two oxo groups can be attached to one sulfur atom, resulting in the formation of a sulfonyl group -S(=O)₂.

The term "ring substituent" means a substituent attached to an aromatic or nonaromatic ring which replaces an available hydrogen atom on the ring.

Should a composite substituent be composed of more than one parts, e.g. (C₁-C₄-alkoxy)-(C₁-C₄-alkyl)-, it is possible for the position of a given part to be at any suitable position of said composite substituent, *i.e.* the C₁-C₄-alkoxy part can be attached to any carbon atom of the C₁-C₄-alkyl part of said (C₁-C₄-alkoxy)-(C₁-C₄-alkyl)- group. A hyphen at the beginning or at the end of such a composite substituent indicates the point of attachment of said composite substituent to the rest of the molecule. Should a ring, comprising carbon atoms and optionally one or more heteroatoms, such as nitrogen, oxygen or sulfur atoms for example, be substituted with a substituent, it is possible for said substituent to be bound at any suitable position of said ring, be it bound to a suitable carbon atom and/or to a suitable heteroatom.

The term "comprising" when used in the specification includes "consisting of".

If within the present text any item is referred to as "as mentioned herein", it means that it may be mentioned anywhere in the present text.

If within the present text any item is referred to as *"supra"* within the description it indicates any of the respective disclosures made within the specification in any of the preceding pages, or above on the same page.

If within the present text any item is referred to as *"infra"* within the description it indicates any of the respective disclosures made within the specification in any of the subsequent pages, or below on the same page.

The terms as mentioned in the present text have the following meanings:
The term "halogen atom" means a fluorine, chlorine, bromine or iodine atom, particularly a fluorine, chlorine or bromine atom, more particularly a fluorine atom.

The term "C₁-C₆-alkyl" means a linear or branched, saturated, monovalent hydrocarbon group having 1, 2, 3, 4, 5 or 6 carbon atoms, *e.g*. a methyl-, ethyl-, propyl-, isopropyl-, butyl-, *sec*-butyl-, isobutyl-, *tert-*butyl*-,* pentyl-, isopentyl-, 2-methylbutyl-, 1-methylbutyl-, 1-ethylpropyl-, 1,2-dimethylpropyl-, *neo*-pentyl-, 1,1-dimethylpropyl-, hexyl-, 1-methylpentyl-, 2-methylpentyl-, 3-methylpentyl-, 4-methylpentyl-, 1-ethylbutyl-, 2-ethylbutyl-, 1,1-dimethylbutyl-, 2,2-dimethylbutyl-, 3,3-dimethylbutyl-, 2,3-dimethylbutyl-, 1,2-dimethylbutyl- or a 1,3-dimethylbutyl- group, or an isomer thereof. Particularly, said group has 1, 2, 3 or 4 carbon atoms ("C₁-C₄-alkyl"), *e.g*. a methyl-, ethyl-, propyl-, isopropyl-, butyl-, sec-butyl-, isobutyl- or a *tert-*butyl group, more particularly 1, 2 or 3 carbon atoms ("C₁-C₃-alkyl"), *e.g.* a methyl-, ethyl-, *n*-propyl- or an isopropyl group.

The term "C₁-C₆-hydroxyalkyl" means a linear or branched, saturated, monovalent hydrocarbon group in which the term "C₁-C₆-alkyl" is defined *supra,* and in which one or more hydrogen atoms are replaced with a hydroxy group, *e.g*. a hydroxymethyl-, 1-hydroxyethyl-, 2-hydroxyethyl-, 1,2-dihydroxyethyl-, 3-hydroxypropyl-, 2-hydroxypropyl-, 1-hydroxypropyl-, 1-hydroxypropan-2-yl-, 2-hydroxypropan-2-yl-, 2,3-dihydroxypropyl-, 1,3-dihydroxypropan-2-yl-, 3-hydroxy-2-methyl-propyl-, 2-hydroxy-2-methyl-propyl- or a 1-hydroxy-2-methyl-propyl- group.

The term "C₁-C₆-alkylsulfanyl" means a linear or branched, saturated, monovalent group of formula (C₁-C₆-alkyl)-S-, in which the term "C₁-C₆-alkyl" is as defined *supra, e.g.* a methylsulfanyl-, ethylsulfanyl-, propylsulfanyl-, isopropylsulfanyl-, butylsulfanyl-, *sec-*butylsulfanyl-, isobutylsulfanyl-, *tert*-butylsulfanyl-, pentylsulfanyl-, isopentylsulfanyl- or a hexylsulfanyl- group.

The term "C₁-C₆-haloalkyl" means a linear or branched, saturated, monovalent hydrocarbon group in which the term "C₁-C₆-alkyl" is as defined *supra* and in which one or more of the hydrogen atoms are replaced, identically or differently, with a halogen atom. Preferably, said halogen atom is a fluorine atom. Said C₁-C₆-haloalkyl, particularly a C₁-C₃-haloalkyl group is, for example, fluoromethyl-, difluoromethyl-, trifluoromethyl-, 2-fluoroethyl-, 2,2-difluoroethyl-, 2,2,2-trifluoroethyl-, pentafluoroethyl-, 3,3,3-trifluoropropyl- or a 1,3-difluoropropan-2-yl group.

The term "C₁-C₆-alkoxy" means a linear or branched, saturated, monovalent group of formula (C₁-C₆-alkyl)-O-, in which the term "C₁-C₆-alkyl" group is as defined *supra, e.g.* methoxy-, ethoxy-, *n*-propoxy-, isopropoxy-, *n*-butoxy-, *sec*-butoxy-, isobutoxy-, *tert-*butoxy*-,* pentyloxy-, isopentyloxy- or a *n*-hexyloxy group, or an isomer thereof.

The term "C₁-C₆-haloalkoxy" means a linear or branched, saturated, monovalent C₁-C₆-alkoxy group, as defined *supra,* in which one or more of the hydrogen atoms is replaced, identically or differently, with a halogen atom. Preferably, said halogen atom in "C₁-C₆-haloalkoxy-" is fluorine, resulting in a group referred herein as "C₁-C₆-fluoroalkoxy-". Representative C₁-C₆-fluoroalkoxy- groups include, for example, -OCF₃, -OCHF₂, - OCH₂F, -OCF₂CF₃ and -OCH₂CF₃.

The term "C₂-C₆-alkenyl-" means a linear or branched, monovalent hydrocarbon group, which contains one or more double bonds and which has 2, 3, 4, 5 or 6 carbon atoms, preferably 2, 3 or 4 carbon atoms ("C₂-C₄-alkenyl-") or 2 or 3 carbon atoms ("C₂-C₃-alkenyl-"), it being understood that in the case in which said alkenyl- group contains more than one double bond, then said double bonds may be isolated from, or conjugated with, each other. Representative alkenyl groups include, for example, an ethenyl-, prop-2-enyl-, (*E*)-prop-1-enyl-, (*Z*)-prop-1-enyl-, *iso*-propenyl-, but-3-enyl-, (*E*)-but-2-enyl-, (*Z*)-but-2-enyl-, (*E*)-but-1-enyl-, (*Z*)-but-1-enyl-, 2-methylprop-2-enyl-, 1-methylprop-2-enyl-, 2-methylprop-1-enyl-, (*E*)-1-methylprop-1-enyl-, (*Z*)-1-methylprop-1-enyl-, buta-1,3-dienyl-, pent-4-enyl-, (*E*)-pent-3-enyl-, (*Z*)-pent-3-enyl-, (*E*)-pent-2-enyl-, (*Z*)-pent-2-enyl-, (*E*)-pent-1-enyl-, (*Z*)-pent-1-enyl-, 3-methylbut-3-enyl-, 2-methylbut-3-enyl-, 1-methylbut-3-enyl-, 3-methylbut-2-enyl-, (*E*)-2-methylbut-2-enyl-, (*Z*)-2-methylbut-2-enyl-, (*E*)-1-methylbut-2-enyl-, (*Z*)-1-methylbut-2-enyl-, (*E*)-3-methylbut-1-enyl-, (*Z*)-3-methylbut-1-enyl-, (*E*)-2-methylbut-1-enyl-, (*Z*)-2-methylbut-1-enyl-, (*E*)-1-methylbut-1-enyl-, (*Z*)-1-methylbut-1-enyl-, 1,1-dimethylprop-2-enyl-, 1-ethylprop-1-enyl-, 1-propylvinyl-, 1-isopropylvinyl-, (*E*)-3,3-dimethylprop-1-enyl-, (*Z*)-3,3-dimethylprop-1-enyl-, penta-1,4-dienyl-, hex-5-enyl-, (*E*)-hex-4-enyl-, (*Z*)-hex-4-enyl-, (*E*)-hex-3-enyl-, (*Z*)-hex-3-enyl-, (*E*)-hex-2-enyl-, (*Z*)-hex-2-enyl-, (*E*)-hex-1-enyl-, (*Z*)-hex-1-enyl-, 4-methylpent-4-enyl-, 3-methylpent-4-enyl-, 2-methylpent-4-enyl-, 1-methylpent-4-enyl-, 4-methylpent-3-enyl-, (*E*)-3-methylpent-3-enyl-, (*Z*)-3-methylpent-3-enyl-, (*E*)-2-methylpent-3-enyl-, (Z*)*-2-methylpent-3-enyl-, (*E*)-1-methylpent-3-enyl-, (*Z*)-1-methylpent-3-enyl-, (*E*)-4-methylpent-2-enyl-, (*Z*)-4-methylpent-2-enyl-, (*E*)*-3-methylpent-2-enyl-,* (*Z*)-3-methylpent-2-enyl-, (*E*)-2-methylpent-2-enyl-, (*Z*)-2-methylpent-2-enyl-, (*E*)-1-methylpent-2-enyl-, (*Z*)-1-methylpent-2-enyl-, (*E*)-4-methylpent-1-enyl-, (*Z*)-4-methylpent-1-enyl-, (*E*)-3-methylpent-1-enyl-, (*Z*)-3-methylpent-1-enyl-, (*E*)-2-methylpent-1-enyl-, (*Z*)-2-methylpent-1-enyl-, (*E*)-1-methylpent-1-enyl-, (*Z*)-1-methylpent-1-enyl-, 3-ethylbut-3-enyl-, 2-ethylbut-3-enyl-, 1-ethylbut-3-enyl-, (*E*)-3-ethylbut-2-enyl-, (*Z*)-3-ethylbut-2-enyl-, (*E*)-2-ethylbut-2-enyl-, (*Z*)-2-ethylbut-2-enyl-, (*E*)-1-ethylbut-2-enyl-, (*Z*)-1-ethylbut-2-enyl-, (*E*)-3-ethylbut-1-enyl-, (*Z*)-3-ethylbut-1-enyl-, 2-ethylbut-1-enyl-, (*E*)-1-ethylbut-1-enyl-, (*Z*)-1-ethylbut-1-enyl-, 2-propylprop-2-enyl-, 1-propylprop-2-enyl-, 2-isopropylprop-2-enyl-, 1-isopropylprop-2-enyl-, (*E*)-2-propylprop-1-enyl-, (*Z*)-2-propylprop-1-enyl-, (*E*)-1-propylprop-1-enyl-, (*Z*)-1-propylprop-1-enyl-, (*E*)-2-isopropylprop-1-enyl-, (*Z*)-2-isopropylprop-1-enyl-, (*E*)-1-isopropylprop-1-enyl-, (*Z*)-1-isopropylprop-1-enyl-, hexa-1,5-dienyl- and a 1-(1,1-dimethylethyl-)ethenyl group. Particularly, said group is an ethenyl- or a prop-2-enyl group.

The same definitions can be applied should the alkenyl group be placed within a chain as a bivalent "C₂-C₆-alkenylene" moiety. All names as mentioned above then will bear a "ene" added to their end, thus *e.g*., a "pentenyl" becomes a bivalent "pentenylene" group.

The term "C₂-C₆-haloalkenyl-" means a linear or branched hydrocarbon group in which one or more of the hydrogen atoms of a "C₂-C₆-alkenyl-" as defined *supra* are each replaced, identically or differently, by a halogen atom. Preferably, said halogen atom is fluorine, resulting in a group referred herein as "C₂-C₆-fluoroalkenyl-". Representative C₂-C₆-fluoroalkenyl- groups include, for example, -CH=CF₂, -CF=CH₂, -CF=CF₂, - C(CH₃)=CF₂, -CH=C(F)-CH₃, -CH₂-CF=CF₂ and -CF₂-CH=CH₂.

The term "C₂-C₆-alkynyl-" means a linear or branched, monovalent hydrocarbon group which contains one or more triple bonds, and which contains 2, 3, 4, 5 or 6 carbon atoms, preferably 2, 3 or 4 carbon atoms ("C₂-C₄-alkynyl-") or 2 or 3 carbon atoms ("C₂-C₃-alkynyl-"). Representative C₂-C₆-alkynyl- groups include, for example, an ethynyl-, prop-1-ynyl-, prop-2-ynyl-, but-1-ynyl-, but-2-ynyl-, but-3-ynyl-, pent-1-ynyl-, pent-2-ynyl, pent-3-ynyl-, pent-4-ynyl-, hex-1-ynyl-, hex-2-ynyl-, hex-3-ynyl-, hex-4-ynyl-, hex-5-ynyl-, 1-methylprop-2-ynyl-, 2-methylbut-3-ynyl-, 1-methylbut-3-ynyl-, 1-methylbut-2-ynyl-, 3-methylbut-1-ynyl-, 1-ethylprop-2-ynyl-, 3-methylpent-4-ynyl-, 2-methylpent-4-ynyl-, 1-methylpent-4-ynyl-, 2-methylpent-3-ynyl-, 1-methylpent-3-ynyl-, 4-methylpent-2-ynyl-, 1-methylpent-2-ynyl-, 4-methylpent-1-ynyl-, 3-methylpent-1-ynyl-, 2-ethylbut-3-ynyl-, 1-ethylbut-3-ynyl-, 1-ethylbut-2-ynyl-, 1-propylprop-2-ynyl-, 1-isopropylprop-2-ynyl-, 2,2-dimethylbut-3-ynyl-, 1,1-dimethylbut-3-ynyl-, 1,1-dimethylbut-2-ynyl- and a 3,3-dimethylbut-1-ynyl- group. Particularly, said alkynyl- group is an ethynyl-, a prop-1-ynyl- or a prop-2-ynyl group.

The term "C₃-C₈-cycloalkyl" means a saturated, monovalent, mono- or bicyclic hydrocarbon ring which contains 3, 4, 5, 6, 7 or 8 carbon atoms ("C₃-C₈-cycloalkyl"). Analogously, the term "C₃-C₆-cycloalkyl" means a saturated, monovalent, mono- or bicyclic hydrocarbon ring which contains 3, 4, 5 or 6 carbon atoms ("C₃-C₆-cycloalkyl"). Said C₃-C₈-cycloalkyl or C₃-C₆-cycloalkyl group is for example, a monocyclic hydrocarbon ring, e.g. a cyclopropyl-, cyclobutyl-, cyclopentyl-, cyclohexyl-, cycloheptyl- or cyclooctyl-group, or a bicyclic hydrocarbon ring, e.g. a bicyclo[4.2.0]octyl- or a octahydropentalenyl-group.

The term "C₃-C₆-halocycloalkyl" means a saturated, monovalent hydrocarbon ring which contains 3, 4, 5 or 6 carbon atoms in which the term "C₃-C₆-cycloalkyl" is as defined *supra* and in which one or more of the hydrogen atoms of the hydrocarbon ring are replaced, identically or differently, with a halogen atom. Preferably, said halogen atom is a fluorine atom. The "C₃-C₆-cycloalkyl" group as defined *supra* in which one or more of the hydrogen atoms are replaced, identically or differently, with a halogen atom, preferably a fluorine atom, is for example and preferably, a monocyclic hydrocarbon ring, *e.g.* a cyclopropyl-, cyclobutyl-, cyclopentyl-, cyclohexyl- group.

The term "C₄-C₈-cycloalkenyl" means a monovalent, mono- or bicyclic hydrocarbon ring which contains 4, 5, 6, 7 or 8 carbon atoms and one double bond. Particularly, said ring contains 4, 5 or 6 carbon atoms ("C₄-C₆-cycloalkenyl"). Said C₄-C₈-cycloalkenyl group is for example, a monocyclic hydrocarbon ring, *e.g.*, a cyclobutenyl-, cyclopentenyl-, cyclohexenyl-, cycloheptenyl- or a cyclooctenyl group, or a bicyclic hydrocarbon ring, *e.g.,* a bicyclo[2.2.1]hept-2-enyl- or a bicyclo[2.2.2]oct-2-enyl group.

The term "C₃-C₈-cycloalkoxy" means a saturated, monovalent, mono- or bicyclic group of formula (C₃-C₈-cycloalkyl)-O-, which contains 3, 4, 5, 6, 7 or 8 carbon atoms, in which the term "C₃-C₈-cycloalkyl" is defined *supra, e.g.* a cyclopropyloxy-, cyclobutyloxy-, cyclopentyloxy-, cyclohexyloxy-, cycloheptyloxy- or a cyclooctyloxy- group.

If the term "heterocycloalkyl" is used without specifying a number of atoms it is meant to be a "4- to 10-membered heterocycloalkyl-" group, more particularly a 5- to 6-membered heterocycloalkyl group. The terms "4- to 7-membered heterocycloalkyl", "4- to 6-membered heterocycloalkyl" and "5- to 7-membered heterocycloalkyl" mean a monocyclic, saturated heterocycle with "4, 5, 6 or 7" or, respectively, "4, 5 or 6" or "5, 6 or 7" ring atoms in total, which are saturated or partially unsaturated monocycles, bicycles or polycycles that contain one or two identical or different ring heteroatoms selected from nitrogen, oxygen and sulfur or one group selected from -S(=O)-, -S(=O)₂- and -S(=O)(=NH)-. It is possible for said heterocycloalkyl group to be attached to the rest of the molecule via any one of the carbon atoms or, if present, a nitrogen atom.

Exemplarily, without being limited thereto, said "4- to 7-membered heterocycloalkyl", can be a 4-membered ring, a "4-membered heterocycloalkyl-" group, such as an azetidinyl- or an oxetanyl group; or a 5-membered ring, a "5-membered heterocycloalkyl-" group, such as a tetrahydrofuranyl-, dioxolinyl-, pyrrolidinyl-, imidazolidinyl-, pyrazolidinyl- or a pyrrolinyl group; or a 6-membered ring, a "6-membered heterocycloalkyl-" group, such as a tetrahydropyranyl-, piperidinyl-, morpholinyl-, 3-oxomorpholin-4-yl, dithianyl-, thiomorpholinyl- or a piperazinyl group; or a 7-membered ring, a "7-membered heterocycloalkyl-" group, such as an azepanyl-, diazepanyl- or an oxazepanyl group, for example. The heterocycloalkyl groups may be substituted one or more times independently with C₁-C₃-alkyl, C₁-C₃-alkoxy, hydroxy, halogen or a carbonyl group.

Particularly, "4- to 6-membered heterocycloalkyl" means a 4- to 6-membered heterocycloalkyl as defined *supra* containing one ring nitrogen atom and optionally one further ring heteroatom selected from nitrogen, oxygen and sulfur. Particularly, "5- to 7-membered heterocycloalkyl" means a 5- to 7-membered heterocycloalkyl as defined *supra* containing one ring nitrogen atom and optionally one further ring heteroatom selected from nitrogen, oxygen and sulfur. More particularly, "5- or 6-membered heterocycloalkyl" means a monocyclic, saturated heterocycle with 5 or 6 ring atoms in total, containing one ring nitrogen atom and optionally one further ring heteroatom selected from nitrogen and oxygen.

The term "heteroaryl-" means a monocyclic, bicyclic or tricyclic aromatic ring system having 5, 6, 7, 8, 9, 10, 11, 12, 13 or 14 ring atoms (a "5- to 14-membered heteroaryl-" group), preferably 5, 6, 9 or 10 ring atoms and which contains 1, 2, 3 or 4 heteroatoms which may be identical or different, said heteroatoms being selected from oxygen, nitrogen and sulfur. Said heteroaryl- group can be a 5-membered heteroaryl group, such as, for example, a thienyl-, furanyl-, pyrrolyl-, oxazolyl-, thiazolyl-, imidazolyl-, pyrazolyl-, isoxazolyl-, isothiazolyl-, oxadiazolyl-, triazolyl-, thiadiazolyl- or a tetrazolyl group; or a 6-membered heteroaryl group, such as, for example, a pyridyl-, pyridazinyl-, pyrimidyl-, pyrazinyl- or a triazinyl group; or a benzo-fused 5-membered heteroaryl- group, such as, for example, a benzofuranyl-, benzothienyl-, benzoxazolyl-, benzisoxazolyl-, benzimidazolyl-, benzothiazolyl-, benzotriazolyl-, indazolyl-, indolyl- or a isoindolyl group; or a benzo-fused 6-membered heteroaryl group, such as, for example, a quinolinyl-, quinazolinyl-, isoquinolinyl-, cinnolinyl-, phthalazinyl- or quinoxalinyl-; or another bicyclic group, such as, for example, indolizinyl-, purinyl- or a pteridinyl group.

Preferably, "heteroaryl-" is a monocyclic aromatic ring system having 5 or 6 ring atoms and which contains at least one heteroatom, if more than one, they may be identical or different, said heteroatom being selected from oxygen, nitrogen and sulfur, a ("5- to 6-membered monocyclic heteroaryl-") group, such as, for example, a thienyl-, furanyl-, pyrrolyl-, oxazolyl-, thiazolyl-, imidazolyl-, pyrazolyl-, isoxazolyl-, isothiazolyl-, oxadiazolyl-, triazolyl-, thiadiazolyl-, tetrazolyl-, pyridyl-, pyridazinyl-, pyrimidyl-, pyrazinyl- or a triazinyl group.

In particular, in the context of the present invention, when applied to any of the substituents of the compounds of general formula **(I),** the term "heteroaryl" is to be understood as meaning preferably a monocyclic aromatic ring system having 5 or 6 ring atoms and which contains one, two or three heteroatoms, preferably one or two heteroatoms, which may be identical or different, said heteroatom(s) being independently selected from oxygen, sulphur and nitrogen, preferably from oxygen and nitrogen, *i.e.* a ("5- to 6-membered monocyclic heteroaryl-") group.

In general, and unless otherwise mentioned, said heteroaryl- groups include all the possible isomeric forms thereof, *e.g*., the positional isomers thereof. Thus, for some illustrative non-restricting example, the term pyridyl- includes pyridin-2-yl-, pyridin-3-yl- and pyridin-4-yl-; the term thienyl- includes thien-2-yl- and thien-3-yl-, and a heteroarylene group may be inserted into a chain also in the inverse way such as e.g. a 2,3-pyridinylene includes pyridine-2,3-yl as well as pyridine-3,2-yl. Furthermore, said heteroaryl- groups can be attached to the rest of the molecule via any one of the carbon atoms, or, if applicable, a nitrogen atom, *e.g*., a pyrrol-1-yl-, a pyrazol-1-yl- or an imidazol-1-yl- group.

Particularly, the heteroaryl group is a pyridyl- or pyrimidyl group or a imidazolyl group, including a hydroxy substitution of the pyridyl group leading, e.g., to a 2-hydroxy-pyridine which is the tautomeric form to a 2-oxo-2(1H)-pyridine. In some embodiments, the heteroaryl group is an oxazolyl group.

Further, as used herein, the term "C₃-C₈", as used throughout this text, *e.g*., in the context of the definition of "C₃-C₈-cycloalkyl-", is to be understood as meaning *e.g*. a cycloalkyl-group having a whole number of carbon atoms of 3 to 8, *i.e.,* 3, 4, 5, 6, 7 or 8 carbon atoms. It is to be understood further that said term "C₃-C₈" is to be interpreted as disclosing any sub-range comprised therein, *e.g*., C₃-C₆, C₄-C₅, C₃-C₅, C₃-C₄, C₄-C₆, C₅-C₇; preferably C₃-C₆.

Similarly, as used herein, the term "C₂-C₆", as used throughout this text, *e.g*., in the context of the definitions of "C₂-C₆-alkenyl-" and "C₂-C₆-alkynyl-", is to be understood as meaning an alkenyl- group or an alkynyl- group having a whole number of carbon atoms from 2 to 6, *i.e.,* 2, 3, 4, 5 or 6 carbon atoms. It is to be understood further that said term "C₂-C₆" is to be interpreted as disclosing any sub-range comprised therein, *e.g*., C₂-C₆, C₃-C₅, C₃-C₄, C₂-C₃, C₂-C₄, C₂-C₅; preferably C₂-C₃.

The term "C₁-C₆", as used throughout this text, *e.g.*, in the context of the definition of "C₁-C₆-alkyl-", "C₁-C₆-haloalkyl-", "C₁-C₆-alkoxy-" or "C₁-C₆-haloalkoxy-" is to be understood as meaning an alkyl group having a whole number of carbon atoms from 1 to *6, i.e.,* 1, 2, 3, 4, 5 or 6 carbon atoms. It is to be understood further that said term "C₁-C₆" is to be interpreted as disclosing any sub-range comprised therein, *e.g*. C₁-C₆, C₂-C₅, C₃-C₄, C₁-C₂, C₁-C₃, C₁-C₄, C₁-C₅, C₁-C_{6;} preferably C₁-C₂, C₁-C₃, C₁-C₄, C₁-C₅, C₁-C_{6;} more preferably C₁-C_{4;} in the case of "C₁-C₆-haloalkyl-" or "C₁-C₆-haloalkoxy-" even more preferably C₁-C₂.

When a range of values is given, said range encompasses each value and sub-range within said range.

For example:
"C₁-C₆" encompasses C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆;
"C₂-C₆" encompasses C₂, C₃, C₄, C₅, C₆, C₂-C₆, C₂-C₅, C₂-C₄, C₂-C₃, C₃-C₆, C₃-C₅, C₃-C₄, C₄-C₆, C₄-C₅, and C₅-C₆;
"C₃-C₁₀" encompasses C₃, C₄, C₅, C₆, C₇, C₈, C₉, C₁₀, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₃-C₈" encompasses C₃, C₄, C₅, C₆, C₇, C₈, C₃-C₈, C₃-C₇, C₃-C₆, C₃-C₅, C₃-C₄, C₄-C₈, C₄-C₇, C₄-C₆, C₄-C₅, C₅-C₈, C₅-C₇, C₅-C₆, C₆-C₈, C₆-C₇ and C₇-C₈;
"C₃-C₆" encompasses C₃, C₄, C₅, C₆, C₃-C₆, C₃-C₅, C₃-C₄, C₄-C₆, C₄-C₅, and C₅-C₆;
"C₄-C₈" encompasses C₄, C₅, C₆, C₇, C₈, C₄-C₈, C₄-C₇, C₄-C₆, C₄-C₅, C₅-C₈, C₅-C₇, C₅-C₆, C₆-C₈, C₆-C₇ and C₇-C₈;
"C₄-C₇" encompasses C₄, C₅, C₆, C₇, C₄-C₇, C₄-C₆, C₄-C₅, C₅-C₇, C₅-C₆ and C₆-C₇;
"C₄-C₆" encompasses C₄, C₅, C₆, C₄-C₆, C₄-C₅ and C₅-C₆;
"C₅-C₁₀" encompasses C₅, C₆, C₇, C₈, C₉, C₁₀, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₆-C₁₀" encompasses C₆, C₇, C₈, C₉, C₁₀, C₆-C₁₀, C₆-C₉, C₆-C₈, C₆-C₇, C₇-C₁₀, C₇-C₉, C₇-C₈, C₈-C₁₀, C₈-C₉ and C₉-C₁₀.

As used herein, the term "leaving group" refers to an atom or a group of atoms that is displaced in a chemical reaction as stable species taking with it the bonding electrons, e.g., typically forming an anion. Preferably, a leaving group is selected from the group comprising: halo, in particular a chloro, bromo or iodo, (methylsulfonyl)oxy-, [(4-methylphenyl)sulfonyl]oxy-, [(trifluoromethyl)sulfonyl]oxy-, [(nonafluorobutyl)sulfonyl]oxy-, [(4-bromophenyl)sulfonyl]oxy-, [(4-nitrophenyl)sulfonyl]oxy-, [(2-nitrophenyl)sulfonyl]oxy-, [(4-isopropylphenyl)sulfonyl]oxy-, [(2,4,6-triisopropylphenyl)sulfonyl]oxy-, [(2,4,6-trimethylphenyl)sulfonyl]oxy-, [(4*-tert-*butylphenyl)sulfonyl]oxy-, (phenylsulfonyl)oxy-, and a [(4-methoxyphenyl)sulfonyl]oxy group.

As used herein, the term "protective group" is a protective group attached to an oxygen or nitrogen atom in intermediates used for the preparation of compounds of the general formula **(I).** Such groups are introduced *e.g*., by chemical modification of the respective hydroxy or amino group in order to obtain chemoselectivity in a subsequent chemical reaction. Protective groups for hydroxy and amino groups are described for example in T.W. Greene and P.G.M. Wuts in Protective Groups in Organic Synthesis, 4th edition, Wiley 2006; more specifically, protective groups for amino groups can be selected from substituted sulfonyl groups, such as a mesyl-, tosyl- or a phenylsulfonyl group, acyl groups such as a benzoyl-, acetyl- or a tetrahydropyranoyl group, or carbamate based groups, such as a *tert*-butoxycarbonyl group (Boc). Protective groups for hydroxy groups can be selected from acyl groups such as a benzoyl-, acetyl-, pivaloyl- or a tetrahydropyranoyl group, or can include silicon, as in *e.g*., a tert-butyldimethylsilyl-, tert-butyldiphenylsilyl-, triethylsilyl- or a triisopropylsilyl group.

The term "substituent" refers to a group "substituted" on, e.g., an alkyl-, haloalkyl-, cycloalkyl-, heterocyclyl-, heterocycloalkenyl-, cycloalkenyl-, aryl-, or a heteroaryl group at any atom of that group, replacing one or more hydrogen atoms therein. In one aspect, the substituent(s) on a group are independently any one single, or any combination of two or more of the permissible atoms or groups of atoms delineated for that substituent. In another aspect, a substituent may itself be substituted with any one of the above substituents. Further, as used herein, the phrase "optionally substituted" means unsubstituted (e.g., substituted with an H) or substituted.

It will be understood that the description of compounds herein is limited by principles of chemical bonding known to those skilled in the art. Accordingly, where a group may be substituted by one or more of a number of substituents, such substitutions are selected so as to comply with principles of chemical bonding with regard to valencies, etc., and to give compounds which are not inherently unstable. For example, any carbon atom will be bonded to two, three, or four other atoms, consistent with the four valence electrons of carbon.

By "subject" is meant a mammal, including, but not limited to, a human or non-human mammal, such as a bovine, equine, canine, ovine, rodent, or feline.

It is possible for the compounds of general formula **(I)** to exist as isotopic variants. The invention therefore includes one or more isotopic variant(s) of the compounds of general formula **(I),** particularly deuterium-containing compounds of general formula **(I).**

The invention also includes all suitable isotopic variations of a compound of the invention.

The term "isotopic variant" of a compound or a reagent is defined as a compound exhibiting an unnatural proportion of one or more of the isotopes that constitute such a compound.

The expression "unnatural proportion" in relation to an isotope means a proportion of such isotope which is higher than its natural abundance. The natural abundances of isotopes to be applied in this context are described in "Isotopic Compositions of the Elements 1997", Pure Appl. Chem., 70(1), 217-235, 1998.

An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I, respectively. Accordingly, recitation of "hydrogen" or "H" should be understood to encompass ¹H (protium), ²H (deuterium), and ³H (tritium) unless otherwise specified. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

With respect to the treatment and/or prophylaxis of the disorders specified herein, the isotopic variant(s) of the compounds of general formula **(I)** preferably contain deuterium ("deuterium-containing compounds of general formula **(I)").** Isotopic variants of the compounds of general formula **(I)** in which one or more radioactive isotopes, such as ³H or ¹⁴C, are incorporated are useful, *e.g*., in drug and/or substrate tissue distribution studies. These isotopes are particularly preferred for the ease of their incorporation and detectability. Positron-emitting isotopes such as ¹⁸F or ¹¹C may be incorporated into a compound of general formula **(I).** These isotopic variants of the compounds of general formula **(I)** are useful for in vivo imaging applications. Deuterium-containing and ¹³C-containing compounds of general formula **(I)** can be used in mass spectrometry analyses in the context of preclinical or clinical studies.

Isotopic variants of the compounds of general formula **(I)** can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from D₂O can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.

The term "deuterium-containing compound of general formula **(I)"** is defined as a compound of general formula **(I),** in which one or more hydrogen atom(s) is/are replaced by one or more deuterium atom(s) and in which the abundance of deuterium at each deuterated position of the compound of general formula **(I)** is higher than the natural abundance of deuterium, which is about 0.015%. Particularly, in a deuterium-containing compound of general formula **(I)** the abundance of deuterium at each deuterated position of the compound of general formula **(I)** is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula **(I)** may alter the physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula **(I).** In some cases deuterium substitution reduces or eliminates the formation of an undesired or toxic metabolite and enhances the formation of a desired metabolite (*e.g*., Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. mL-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (*e.g.*, Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (*e.g*., lower number of doses or lower dosage to achieve the desired effect) and/or may produce lower metabolite loads.

A compound of general formula **(I)** may have multiple potential sites of attack for metabolism. To optimize the above-described effects on physicochemical properties and metabolic profile, deuterium-containing compounds of general formula **(I)** having a certain pattern of one or more deuterium-hydrogen exchange(s) can be selected. Particularly, the deuterium atom(s) of deuterium-containing compound(s) of general formula **(I)** is/are attached to a carbon atom and/or is/are located at those positions of the compound of general formula **(I),** which are sites of attack for metabolizing enzymes such as e.g. cytochrome P₄₅₀.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.

Preferred compounds are those which produce the more desirable biological activity. Separated, pure or partially purified stereoisomers or racemic or diastereomeric mixtures of the compounds of the present invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g*., HPLC columns using a chiral phase), with or without conventional derivatisation, optimally chosen to maximise the separation of the enantiomers. Suitable HPLC columns using a chiral phase are commercially available, such as those manufactured by Daicel, *e.g*., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.

In order to distinguish different types of isomers from each other reference is made to IUPAC Rules Section E (Pure Appl Chem 45, 11-30, 1976).

In the context of the present invention, A and B are independently from each other, selected from a nitrogen atom or a carbon atom, wherein when A is a nitrogen atom, B is a carbon atom and wherein when A is a carbon atom, B is a nitrogen atom. Thus, when A is a nitrogen atom and B is a carbon atom, the compound of formula (I) is a compound of the formula

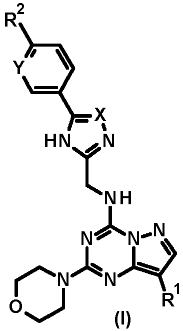

Similarly, in the context of the present invention, when A is a carbon atom and B is a nitrogen atom, the compound of formula (I) is a compound of the formula

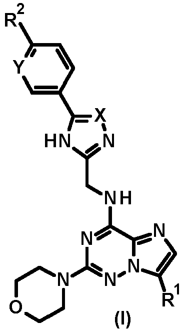

The present invention includes all possible stereoisomers of the compounds of the present invention as single stereoisomers, or as any mixture of said stereoisomers, *e.g*. (R)- or (S)- isomers, in any ratio. Isolation of a single stereoisomer, *e.g.* a single enantiomer or a single diastereomer, of a compound of the present invention is achieved by any suitable state of the art method, such as chromatography, especially chiral chromatography, for example.

Further, it may be possible for the compounds of the present invention to exist as tautomers. For example, any compound of the present invention which contains an imidazopyridine moiety as a heteroaryl group for example can exist as a 1H tautomer, or a 3H tautomer, or even a mixture in any amount of the two tautomers, namely :

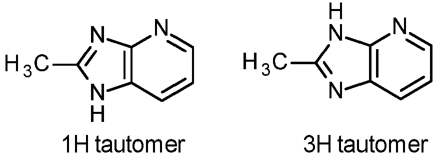

The present invention includes all possible tautomers of the compounds of the present invention as single tautomers, or as any mixture of said tautomers, in any ratio.

Further, in the context of the present invention, it may be possible for the compounds of formula (I) to exist as tautomers. For example, as depicted below, the compounds of formula (I) according to the present invention can exist as a 1H tautomer, or a 3H tautomer, or even a mixture in any amount of two or more of the possible tautomers:

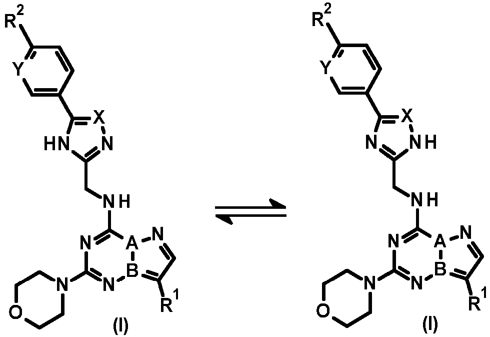

The present invention includes all possible tautomers of the compounds of formula (I) of the present invention as single tautomers, or as any mixture of any two or more of any possible tautomers, in any ratio.

Further, in the context of the present invention, it may be possible for the compounds of formula (I) where X is a nitrogen atom to exist as tautomers. For example, as depicted below, the compounds of formula (I) according to the present invention where X is a nitrogen atom can exist as a 1H tautomer, or a 4H tautomer, or even a mixture in any amount of two or more of the possible tautomers:

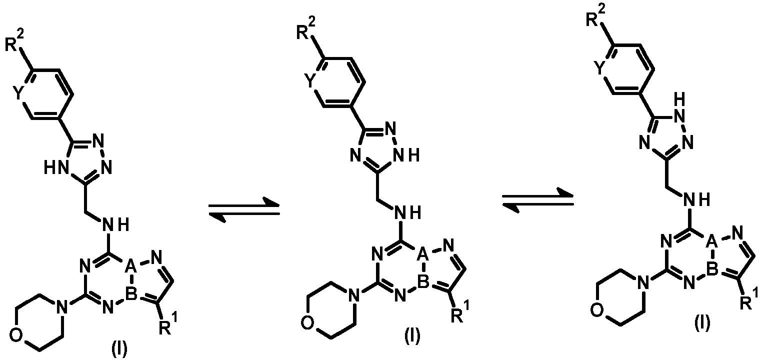

The present invention includes all possible tautomers of the compounds of formula (I) of the present invention where X is a nitrogen atom as single tautomers, or as any mixture of any two or more possible tautomers, in any ratio.

Further, in the context of the present invention, it may be possible for the compounds of formula (I) where X is a CR³ group to exist as tautomers. For example, as depicted below, the compounds of formula (I) according to the present invention where X is a CR³ group can exist as two different 1H tautomers, or even a mixture in any amount of two or more of the possible tautomers:

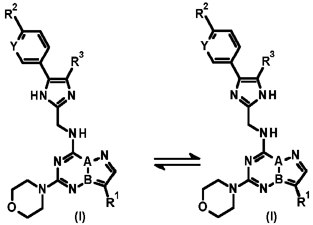

The present invention includes all possible tautomers of the compounds of formula (I) of the present invention where X is a CR³ group as single tautomers, or as any mixture of any two or more possible tautomers, in any ratio.

Further, in the context of the present invention, it may be possible for the triazine core of the compounds of formula (I) to exhibit tautomerism and for said compounds to exist as single tautomers or even as a mixture in any amount of two or more of the possible tautomers:

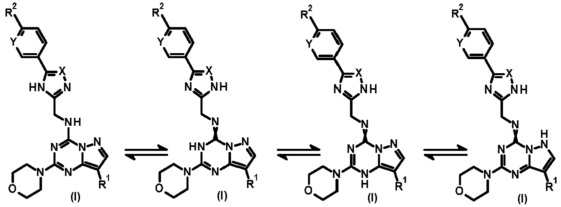

Further, the compounds of the present invention can exist as N-oxides, which are defined in that at least one nitrogen of the compounds of the present invention is oxidised. The present invention includes all such possible N-oxides.

The present invention also provides useful forms of the compounds of the present invention, such as hydrates, solvates, , salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, *e.g.* as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

Physiologically acceptable salts of the compounds according to the invention encompass acid addition salts of mineral acids, carboxylic acids and sulfonic acids, for example salts of hydrochloric acid, hydrobromic acid, hydroiodic acid, sulfuric acid, bisulfuric acid, phosphoric acid, nitric acid or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, paratoluenesulfonic, methansulfonic, 2-naphthalenesulfonic, naphthalenedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

A "pharmaceutically acceptable anion" refers to the deprotonated form of a conventional acid, such as, for example, a hydroxide, a carboxylate, a sulfate, a halide, a phosphate, or a nitrate.

Physiologically acceptable salts of the compounds according to the invention also comprise salts of conventional bases, such as, by way of example and by preference, alkali metal salts (for example lithium, sodium and potassium salts), alkaline earth metal salts (for example calcium, strontium and magnesium salts) and ammonium salts derived from ammonia or organic amines with 1 to 16 C atoms, such as, by way of example and by preference, ethylamine, diethylamine, triethylamine, ethyldiisopropylamine, monoethanolamine, diethanolamine, triethanolamine, dicyclohexylamine, dimethylaminoethanol, procaine, dibenzylamine, *N*-methylmorpholine, arginine, lysine, ethylenediamine, *N*-methylpiperidine, *N*-methylglucamine, dimethylglucamine, ethylglucamine, 1,6-hexadiamine, glucosamine, sarcosine, serinol, tris(hydroxymethyl)aminomethane, aminopropanediol, Sovak base, and 1-amino-2,3,4-butanetriol.

Additionally, the compounds according to the invention may form salts with a quaternary ammonium ion obtainable, *e.g.,* by quaternisation of a basic nitrogen-containing group with agents such as lower alkylhalides such as methyl-, ethyl-, propyl-, and butylchlorides, -bromides and -iodides; dialkylsulfates such as dimethyl-, diethyl-, dibutyl- and diamylsulfates, long chain halides such as decyl-, lauryl-, myristyl- and stearylchlorides, -bromides and -iodides, aralkylhalides such as benzyl- and phenethylbromides and others. Examples of suitable quaternary ammonium ions are tetramethylammonium, tetraethylammonium, tetra(*n*-propyl)ammonium, tetra (*n-*butyl)ammonium, or *N*-benzyl-*N,N,N*-trimethylammonium.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

Solvates and hydrates of disclosed intermediates or example compounds, or salts thereof, which have been obtained, by the preparation and/or purification processes described herein, may be formed in any ratio.

Furthermore, the present invention includes all possible crystalline forms, or polymorphs, of the compounds of the present invention, either as a single polymorph, or as a mixture of more than one polymorph, in any ratio.

Moreover, the in the present disclosure prodrugs of the compounds according to the invention are described but not claimed. The term "prodrugs" designates compounds which themselves can be biologically active or inactive, but are converted (for example metabolically or hydrolytically) into compounds according to the invention during their residence time in the body. For example, a prodrug may be in the form of an in vivo hydrolysable ester of the specified compound. Derivatives of the compounds of formula **(I)** and the salts thereof which are converted into a compound of formula **(I)** or a salt thereof in a biological system (bioprecursors or pro-drugs) are described in the context of this disclosure but not claimed. Said biological system may be, for example, a mammalian organism, particularly a human subject. The bioprecursor is, for example, converted into the compound of formula **(I)** or a salt thereof by metabolic processes.

Further, in the context of the present invention, when the inhibitory and/or degradatory activity of the compounds of formula (I) according to the present invention is referred to, the following terms are defined as follows:
As used herein and in the context of the present invention, the term "IC50 CDK12 hATP" refers to the IC₅₀ values obtained according to the assay described in section 2.2 of the Experimental Section herein below, i.e. the IC₅₀ values for the inhibition of CDK12 at high ATP.

As used herein and in the context of the present invention, the term "DC50 CDK12" refers to the DC₅₀ values obtained according to the assay described in section 7 of the Experimental Section herein below, i.e. the DC₅₀ values for the degradation of CDK12.

### DESCRIPTION

### Further embodiments of the first aspect of the present invention

In accordance with further embodiments, the present invention provides compounds of general formula (I), *supra,* wherein:
wherein
- A and B: are independently from each other, selected from a nitrogen atom or a carbon atom, wherein when A is a nitrogen atom, B is a carbon atom and wherein when A is a carbon atom, B is a nitrogen atom;
- R¹: is a halogen atom;
- R²: is selected from a C₁-C₃-alkyl group, a C₁-C₃-alkoxy group, a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
wherein
- A and B: are independently from each other, selected from a nitrogen atom or a carbon atom, wherein when A is a nitrogen atom, B is a carbon atom and wherein when A is a carbon atom, B is a nitrogen atom;
- R¹: is a C₁-C₃-haloalkyl group;
- R²: is selected from a C₁-C₃-alkyl group, a C₁-C₃-alkoxy group, a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A and B: are independently from each other, selected from a nitrogen atom or a carbon atom, wherein when A is a nitrogen atom, B is a carbon atom and wherein when A is a carbon atom, B is a nitrogen atom;
- R¹: is a halogen atom;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
wherein
- A and B: are independently from each other, selected from a nitrogen atom or a carbon atom, wherein when A is a nitrogen atom, B is a carbon atom and wherein when A is a carbon atom, B is a nitrogen atom;
- R¹: is a C₁-C₃-haloalkyl group;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
wherein
- A and B: are independently from each other, selected from a nitrogen atom or a carbon atom, wherein when A is a nitrogen atom, B is a carbon atom and wherein when A is a carbon atom, B is a nitrogen atom;
- R¹: is a halogen atom;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A and B: are independently from each other, selected from a nitrogen atom or a carbon atom, wherein when A is a nitrogen atom, B is a carbon atom and wherein when A is a carbon atom, B is a nitrogen atom;
- R¹: is C₁-C₃-haloalkyl group;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A: is a nitrogen atom and B is a carbon atom;
- R¹: is a halogen atom;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A: is a nitrogen atom and B is a carbon atom;
- R¹: is a C₁-C₃-haloalkyl group;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A: is a carbon atom and B is a nitrogen atom;
- R¹: is a halogen atom;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- A: is a carbon atom and B is a nitrogen atom;
- R¹: is a C₁-C₃-haloalkyl group;
- R²: is selected from a C₁-C₃-haloalkyl group and a C₁-C₃-haloalkoxy group;
- X: is selected from a nitrogen atom and a CR³ group;
- R³: is selected from a hydrogen atom, a C₁-C₃-alkyl group and a C₁-C₃-haloalkyl group;
- Y: is selected from a nitrogen atom or a carbon atom;
or a tautomer, or an N-oxide, or a salt thereof, or a salt of a tautomer, or a salt of an N-oxide, or a mixture of same.

The present invention provides the compounds of general formula **(I)** which are disclosed in the Example Section of this text, *infra.*

In some embodiments, the present invention includes compounds of general formula **(I)** selected from:
8-bromo-2-(morpholin-4-yl)-N-({5-[4-(trifluoromethyl)phenyl]-4H-1,2,4-triazol-3-yl}methyl)pyrazolo[1,5-a][1,3,5]triazin-4-amine,
8-bromo-2-(morpholin-4-yl)-N-({5-[4-(trifluoromethoxy)phenyl]-4H-1,2,4-triazol-3-yl}methyl)pyrazolo[1,5-a][1,3,5]triazin-4-amine,
8-bromo-2-(morpholin-4-yl)-N-({5-[4-(trifluoromethyl)phenyl]-1H-imidazol-2-yl}methyl)pyrazolo[1,5-a][1,3,5]triazin-4-amine,
8-bromo-2-(morpholin-4-yl)-N-({5-[4-(trifluoromethoxy)phenyl]-1H-imidazol-2-yl}methyl)pyrazolo[1,5-a][1,3,5]triazin-4-amine,
2-(morpholin-4-yl)-N-({5-[4-(trifluoromethoxy)phenyl]-4H-1,2,4-triazol-3-yl}methyl)-8-(trifluoromethyl)pyrazolo[1,5-a][1,3,5]triazin-4-amine,
2-(morpholin-4-yl)-8-(trifluoromethyl)-N-({5-[4-(trifluoromethyl)phenyl]-4H-1,2,4-triazol-3-yl}methyl)pyrazolo[1,5-a][1,3,5]triazin-4-amine,
2-(morpholin-4-yl)-7-(trifluoromethyl)-N-({5-[4-(trifluoromethyl)phenyl]-1H-imidazol-2-yl}methyl)imidazo[2, 1-f][1 ,2,4]triazin-4-amine,
2-(morpholin-4-yl)-N-({5-[4-(trifluoromethoxy)phenyl]-1H-imidazol-2-yl}methyl)-7-(trifluoromethyl)imidazo[2,1-f][1,2,4]triazin-4-amine,
2-(morpholin-4-yl)-8-(trifluoromethyl)-N-({5-[4-(trifluoromethyl)phenyl]-1H-imidazol-2-yl}methyl)pyrazolo[1,5-a][1,3,5]triazin-4-amine,
2-(morpholin-4-yl)-N-({5-[4-(trifluoromethoxy)phenyl]-1H-imidazol-2-yl}methyl)-8-(trifluoromethyl)pyrazolo[1,5-a][1,3,5]triazin-4-amine,
2-(morpholin-4-yl)-7-(trifluoromethyl)-N-({5-[4-(trifluoromethyl)phenyl]-4H-1,2,4-triazol-3-yl}methyl)imidazo[2, 1-f][1 ,2,4]triazin-4-amine,
2-(morpholin-4-yl)-7-(trifluoromethyl)-N-({5-[6-(trifluoromethyl)pyridin-3-yl]-1H-imidazol-2-yl}methyl)imidazo[2, 1-f][1 ,2,4]triazin-4-amine,
N-{[5-(4-methoxyphenyl)-1H-imidazol-2-yl]methyl}-2-(morpholin-4-yl)-7-(trifluoromethyl)imidazo[2,1-f][1,2,4]triazin-4-amine,
2-(morpholin-4-yl)-N-({5-[4-(trifluoromethoxy)phenyl]-4H-1,2,4-triazol-3-yl}methyl)-7-(trifluoromethyl)imidazo[2,1-f][1,2,4]triazin-4-amine,
N-{[5-(6-methoxypyridin-3-yl)-4H-1,2,4-triazol-3-yl]methyl}-2-(morpholin-4-yl)-8-(trifluoromethyl)pyrazolo[1,5-a][1,3,5]triazin-4-amine,
2-(morpholin-4-yl)-8-(trifluoromethyl)-N-({5-[6-(trifluoromethyl)pyridin-3-yl]-4H-1,2,4-triazol-3-yl}methyl)pyrazolo[1,5-a][1,3,5]triazin-4-amine,
2-(morpholin-4-yl)-N-({5-[6-(trifluoromethoxy)pyridin-3-yl]-4H-1,2,4-triazol-3-yl}methyl)-8-(trifluoromethyl)pyrazolo[1,5-a][1,3,5]triazin-4-amine,
N-{[5-(6-methoxypyridin-3-yl)-4H-1,2,4-triazol-3-yl]methyl}-2-(morpholin-4-yl)-7-(trifluoromethyl)imidazo[2,1-f][1,2,4]triazin-4-amine,
2-(morpholin-4-yl)-N-({5-[6-(trifluoromethoxy)pyridin-3-yl]-4H-1,2,4-triazol-3-yl}methyl)-7-(trifluoromethyl)imidazo[2,1-f][1,2,4]triazin-4-amine,
2-(morpholin-4-yl)-7-(trifluoromethyl)-N-({5-[6-(trifluoromethyl)pyridin-3-yl]-4H-1,2,4-triazol-3-yl}methyl)imidazo[2,1-f][1,2,4]triazin-4-amine,
N-{[5-(6-methoxypyridin-3-yl)-1H-imidazol-2-yl]methyl}-2-(morpholin-4-yl)-8-(trifluoromethyl)pyrazolo[1,5-a][1,3,5]triazin-4-amine,
N-{[5-(6-methylpyridin-3-yl)-4H-1,2,4-triazol-3-yl]methyl}-2-(morpholin-4-yl)-8-(trifluoromethyl)pyrazolo[1,5-a][1,3,5]triazin-4-amine,
N-{[5-(6-methylpyridin-3-yl)-4H-1,2,4-triazol-3-yl]methyl}-2-(morpholin-4-yl)-7-(trifluoromethyl)imidazo[2,1-f][1,2,4]triazin-4-amine and
2-(morpholin-4-yl)-8-(trifluoromethyl)-N-({5-[6-(trifluoromethyl)pyridin-3-yl]-1H-imidazol-2-yl}methyl)pyrazolo[1,5-a][1,3,5]triazin-4-amine.

### Further embodiments of the first aspect of the present invention:

In some embodiments, the present invention provides compounds of formula **(I),** *supra,* in which
- A and B: are independently from each other, selected from a nitrogen atom or a carbon atom, wherein when A is a nitrogen atom, B is a carbon atom and wherein when A is a carbon atom, B is a nitrogen atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
A is a nitrogen atom and B is a carbon atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
A is a carbon atom and B is a nitrogen atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R¹: is selected from a halogen atom and a C₁-C₃-haloalkyl group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R¹: is a halogen atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R¹: is a C₁-C₃-haloalkyl group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R²: is selected from a C₁-C₃-alkyl group, a C₁-C₃-alkoxy group, a C₁-C₃-haloalkyl group and a C₁-C₃-haloalkoxy group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R²: is selected from a C₁-C₃-haloalkyl group and a C₁-C₃-haloalkoxy group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- R²: is a C₁-C₃-haloalkyl group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- X: is selected from a nitrogen atom and a CR³ group;
- R³: is selected from a hydrogen atom, a C₁-C₃-alkyl group and a C₁-C₃-haloalkyl group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- X: is a nitrogen atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- X: is a CR³ group;
- R³: is selected from a hydrogen atom, a C₁-C₃-alkyl group and a C₁-C₃-haloalkyl group;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- Y: is selected from a nitrogen atom or a carbon atom;
or a tautomer, or an N-oxide, or a salt thereof, or a salt of a tautomer, or a salt of an N-oxide, or a mixture of same.

In some embodiments, the present invention provides compounds of formula **(I),** *supra,* in which
- Y: is a carbon atom;
or a tautomer, or an N-oxide, or a salt thereof, or a salt of a tautomer, or a salt of an N-oxide, or a mixture of same.

In further embodiments, the present invention includes compounds of formula **(I),** or a tautomer, an N-oxide, or a salt thereof, or a salt of a tautomer or an N-oxide, or a mixture of same.

In further embodiments, the present invention includes compounds of formula **(I),** or a salt thereof.

In further embodiments, the present invention includes compounds of formula **(I),** or a tautomer, or a salt thereof, or a salt of a tautomer, or a mixture of same.

In further embodiments, the present invention includes compounds of formula **(I),** which are a salt.

In further embodiments, the present invention includes compounds of formula **(I),** which are a tautomer or a salt thereof, or a salt of a tautomer, or a mixture of same.

In further embodiments, the present invention includes compounds of formula **(I),** which are an N-oxide, or a salt thereof, or a salt of an N-oxide, or a mixture of same.

In further embodiments of the first aspect, the present invention provides combinations of two or more of the above mentioned embodiments under the heading "further embodiments of the first aspect of the present invention".

Furthermore it is understood that the invention includes any subcombination of the disclosed single embodiments herein for certain residues or subcombination of residues of formula **(I).**

The present invention includes any sub-combination within any embodiments or aspects of the present invention of compounds of general formula **(I),** *supra.*

The present invention includes any sub-combination within any embodiments or aspects of the present invention of compounds of general formula **(I)** or intermediate compounds. The present invention includes the compounds of general formula **(I)** which are disclosed in the Example Section of this text, *infra.*

### General synthesis of compounds of general formula (I) of the present invention

The following paragraphs outline a variety of synthetic approaches suitable to prepare compounds of the general formula (I), and intermediates useful for their synthesis.

In addition to the routes described below, also other routes may be used to synthesise the target compounds, in accordance with common general knowledge of a person skilled in the art of organic synthesis. The order of transformations exemplified in the following schemes is therefore not intended to be limiting, and suitable synthesis steps from various schemes can be combined to form additional synthesis sequences. In addition, interconversion of any of the substituents, in particular R¹, R², R³ or R⁴ can be achieved before and/or after the exemplified transformations. These modifications can be such as the introduction of protective groups, cleavage of protective groups, reduction or oxidation of functional groups, halogenation, metallation, metal catalysed coupling reactions, exemplified by but not limited to Suzuki, Sonogashira and Ullmann coupling, ester saponifications, amide coupling reactions, and/or substitution or other reactions known to a person skilled in the art. These transformations include those which introduce a functionality allowing for further interconversion of substituents. Appropriate protective groups and their introduction and cleavage are well-known to a person skilled in the art (see for example T.W. Greene and P.G.M. Wuts in Protective Groups in Organic Synthesis, 3rd edition, Wiley 1999).

### Pyrazolotriazines

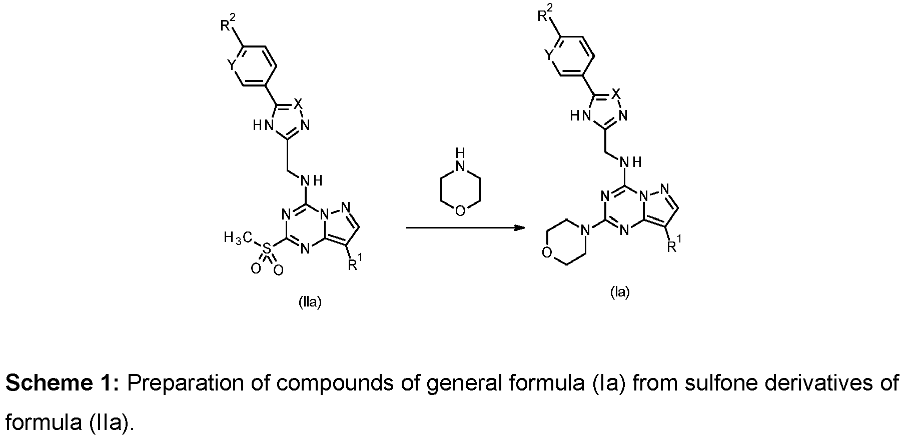

Pyrazolotriazines of general formula (Ia), in which R¹, R² X and Y are as defined for the compounds of general formula (I) can be assembled from sulfone derivatives of formula (IIa), in which R¹, R² X and Y are as defined for the compounds of general formula (I), and an amine such as morpholine by means of an aromatic nucleophilic substitution well known to the person skilled in the art, according to Scheme 1. Said nucleophilic reaction can be performed in the presence of a suitable base, such as sodium hydroxide, sodium hydride, sodium carbonate, potassium carbonate or cesium carbonate, N,N-diisopropylethylamine, triethylamine or 1,8-diazabicyclo(5.4.0)undec-7-ene (DBU), and in the case of aromatic amines in the presence of an acid such as 4-methylbenzenesulfonic acid in an appropriate solvent.

Preferred herein is the performance of said nucleophilic reaction in the case of amines using *N,N*-diisopropylethylamine as a base in acetonitrile as a solvent, within a temperature range from 20 °C to 80 °C.

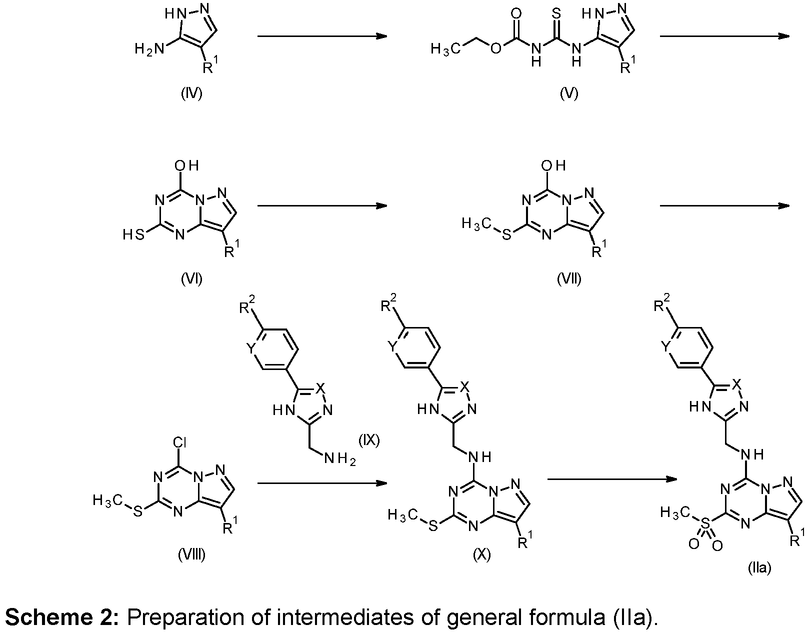

Intermediate sulfone derivatives of formula (IIa) are available for example by the sequence depicted in Scheme 2. This approach started with commercially available or synthesized (according to e.g. WO2018/195397) amino-pyrazole derivatives of the formula (IV), in which R¹ are as defined for the compounds of general formula (I), with ethyl carbonisothiocyanatidate in ethyl acetate to give intermediates (V), which under basic condition such as aqueous sodium hydroxide form the pyrazolotriazin derivatives of the formula (VI). Using methyliodide under basic conditions such as sodium hydroxide the methylsulfanyl derivatives (VII) are formed. In the case of R¹=H in compound (VII) it is possible to introduce halogens such as bromo, chloro or iodo using the corresponding N-halo-succinimide reagent. Reaction of said derivatives (VII) with phosphorus oxychloride resulted in chloro intermediates (VIII). Reaction of (VIII) with commercially available or prepared amines of the general formula (IX) in which R³ and X are as defined for the compounds of general formula (I) under basic conditions such as *N,N-*diisopropylethylamine in an appropriate solvent such as acetonitrile within a temperature range from 20 °C to 80 °C and the subsequent oxidation of the sulfur atom with meta-chloroperoxybenzoic acid (mCPBA) yields the sulfones (IIa).

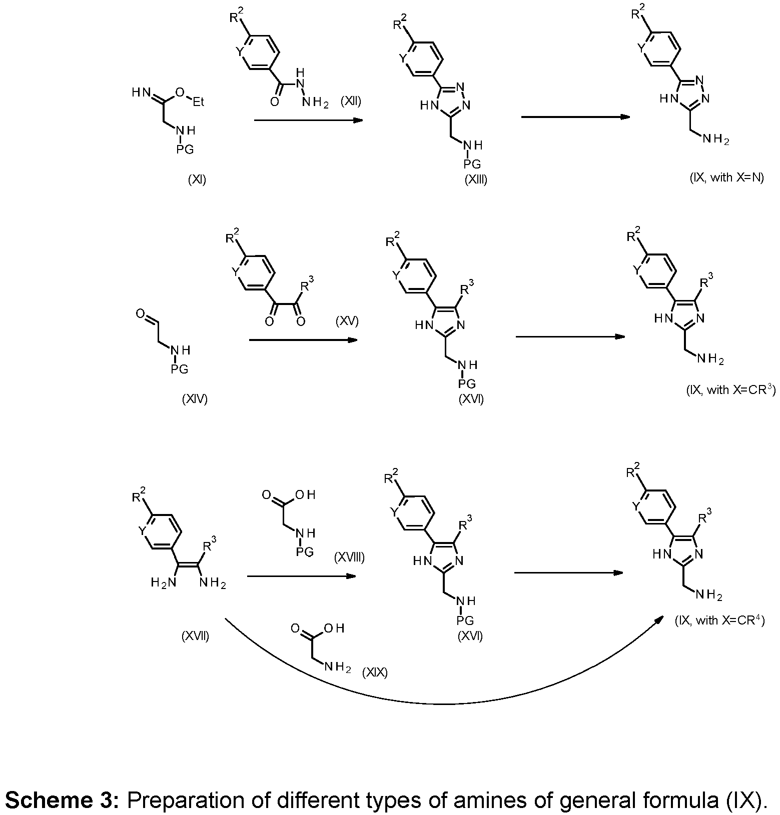

The synthesis of different types of amines of the general formula (IX) is depicted in Scheme 3. In the case of amines with X=N and R² is as defined for the compounds of general formula (I) in the first step commercially available protected ethyl 2-aminoethanimidate (XI) reacts with acylhydrazides of the gerenal formula (XII) in which R² is as defined for the compounds of general formula (I) according to US2010/22599 under basic conditions such as sodium bicarbonate or potassium carbonate to yield the protected amines (XIII) which in the subsequent step are deprotected using conditions known by the person skilled in the art to give amines of formula (IX) with X=N. The used acylhydrazides (XII) are commercially available or can be easily prepared using the corresponding acid or ester via known procedures for person skilled in the art.

For amines (IX) with X=CR³ and R³ as defined for the compounds of general formula (I) commercially available protected aminoacetaldehydes (XIV) react with 1,2-diketones (XV) (for preparation see Landais, Y.; Vincent, J. M., Science of Synthesis, (2005) 26, 647) in the presence of ammonium acetate in methanol/tetrahydrofurane according to Bioorganic and Medicinal Chemistry, 2012, 7128 to yield the protected amines (XVI) which in the subsequent step are deprotected unsing conditions known by the person skilled in the art to give amines of formula (IX) with X=CR³.

These amines can be also prepared starting with 1,2-diamino compounds (XVII) by the reaction with commercially available protected glycine derivatives of formula (XVIII) using 1-ethyl-3-(3-dimethylaminopropyl)carbodiimide hydrochloride and hydroxybenzotriazole mono hydrate followed by acetic acid according to Bioorganic and Medicinal Chemistry Letters, 2013, 4374 to yield the protected amines (XVI) which in the subsequent step are deprotected unsing conditions known by the person skilled in the art to give amines of formula (IX) with X= CR³.

Alternatively, the 1,2-diamino compounds (XVII) can react with glycine (XIX) using acid condition such as aqueous HCl according to EP1135374 (2006) to give amines of formula (IX) with X=CR³.

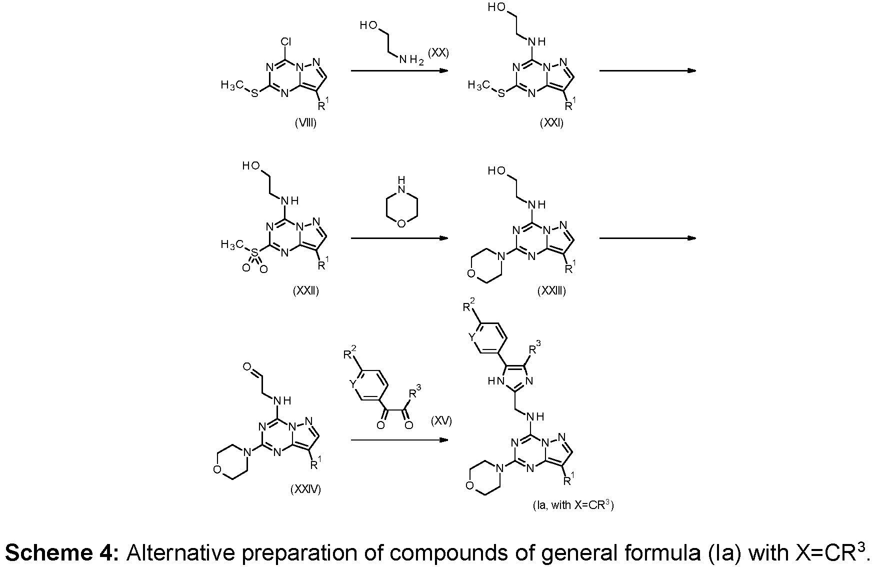

Alternatively, compounds of the formula (VIII) can react with 2-aminoethanol (XX) to give compounds of formula (XXI) which can be oxidized with meta-chloroperoxybenzoic acid (mCPBA) to sulfones of the formula (XXII). These sulfones of formula (XXII) and an amine can react in an aromatic nucleophilic substitution well known to the person skilled in the art and as described for Scheme 1 to give compounds of the formula (XXIII) which can be oxidized to the corresponding aldehydes of formula (XXIV) with methods well known to the person skilled in the art. Compounds of the formula (la) with X=CR³ and R³ as defined for the compounds of general formula (I) can be assembled by the reaction of aldehydes of formula (XXIV) with 1,2-diketones of formula (XV) as described for scheme 3.

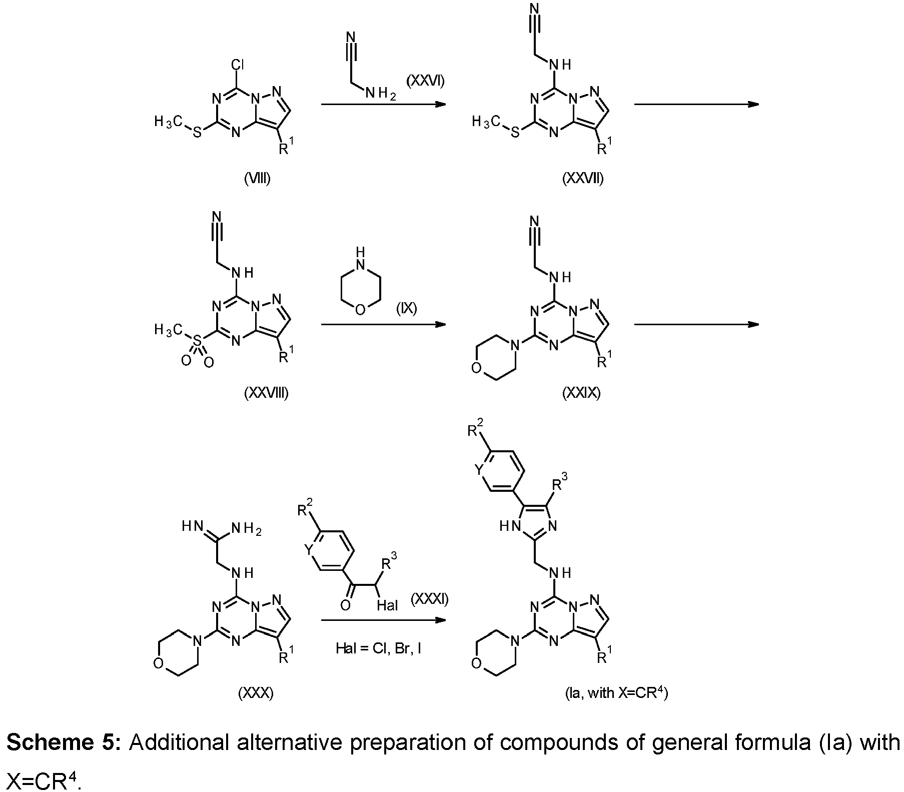

Alternatively, compounds of the formula (VIII) can react with aminoacetonitrile (XXVI) to give compounds of formula (XXVII) which can be oxidized with meta-chloroperoxybenzoic acid (mCPBA) to sulfones of the formula (XXVIII). These sulfones of formula (XXVIII) and an amine can react in an aromatic nucleophilic substitution well known to the person skilled in the art and as described for Scheme 1 to give compounds of the formula (XXXIX) which can be reacted to the corresponding imidamides of formula (XXX) with methods well known to the person skilled in the art. Compounds of the formula (la) with X=CR³ and R³ as defined for the compounds of general formula (I) can be assembled by the reaction of imidamides of formula (XXX) with alpha-halogenated ketones of formula (XXXI) with methods well known to the person skilled in the art.

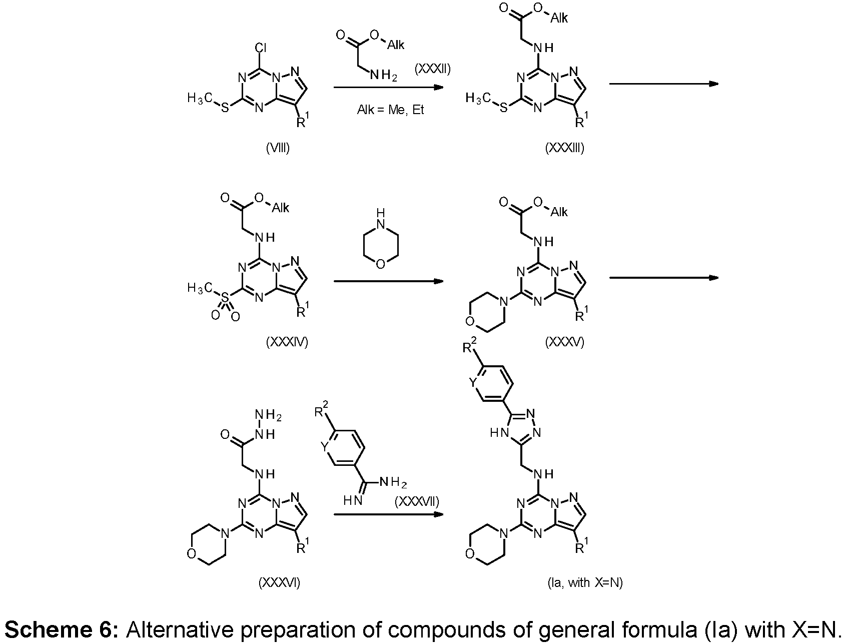

Alternatively, compounds of the formula (VIII) can react with glycinates of formula (XXXII) to give compounds of formula (XXXIII) which can be oxidized with meta-chloroperoxybenzoic acid (mCPBA) to sulfones of the formula (XXXIV). These sulfones of formula (XXXIV) and an amine can react in an aromatic nucleophilic substitution well known to the person skilled in the art and as described for Scheme 1 to give compounds of the formula (XXXV) which can be reacted to the corresponding hydrazides of formula (XXXVI) with methods well known to the person skilled in the art. Alternatively, compounds of the formula (XXXV) can first be hydrolized to the corresponding carbonic acid which can be reacted to the corresponding hydrazides of formula (XXXVI) with methods well known to the person skilled in the art. Compounds of the formula (Ia) with X=N and R³ as defined for the compounds of general formula (I) can be assembled by the reaction of hydrazides of formula (XXXVI) with imidamides of formula (XXXVII) with methods well known to the person skilled in the art.

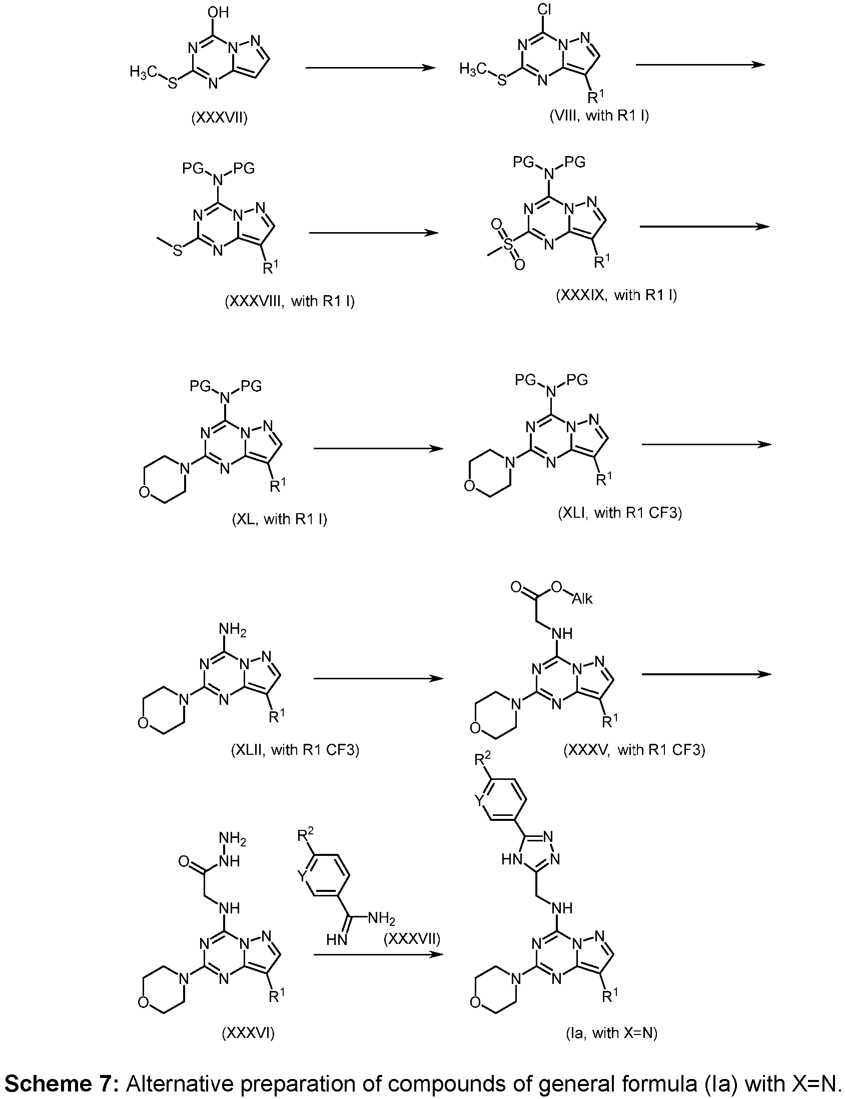

Alternatively, compounds of the formula (XXXVII) can be halogenated and reacted with phosphorus oxychloride to give compounds of formula (VIII) which can react with an amine with two protecting groups (PG) as for example para-methoxybenzyl and can be oxidized with meta-chloroperoxybenzoic acid (mCPBA) to sulfones of the formula (XXXIX). These sulfones of formula (XXXIX) and an amine can react in an aromatic nucleophilic substitution well known to the person skilled in the art and as described for Scheme 1 to give compounds of the formula (XL) which can be reacted to the corresponding CF3 derivative of formula (XLI) with methods well known to the person skilled in the art. After deprotection compounds of the formula (XLII) can be alkylated with chloroacetic esters to give compounds of the formula (XXXV) which can be hydrolized to the corresponding carbonic acids and then be reacted to the corresponding hydrazides of formula (XXXVI) with methods well known to the person skilled in the art. Compounds of the formula (la) with X=N and R³ as defined for the compounds of general formula (I) can be assembled by the reaction of hydrazides of formula (XXXVI) with imidamides of formula (XXXVII) with methods well known to the person skilled in the art.

### Imidazotriazines

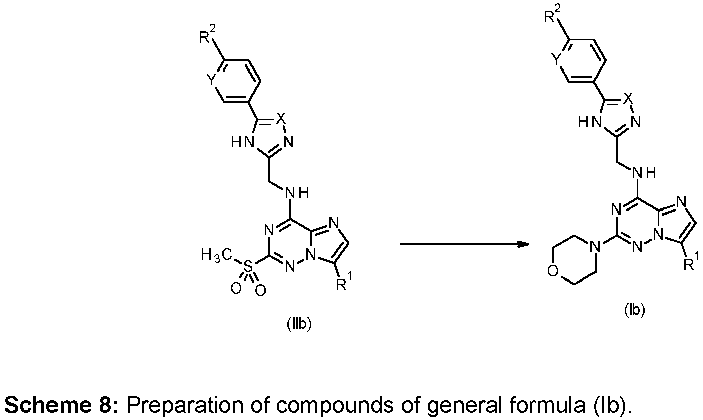

Imidazotriazines of general formula (Ib), in which R¹, R^{2,} R³, X and Y are as defined for the compounds of general formula (I) can be assembled from sulfone derivatives of formula (Ilb), in which R¹, R^{2,} R³, X and Y are as defined for the compounds of general formula (I), and an amine by means of an aromatic nucleophilic substitution well known to the person skilled in the art, according to Scheme 8. Said nucleophilic reaction can be performed by reaction of compounds of the formulae (Ilb) and (III) in the presence of a suitable base, such as sodium hydroxide, sodium hydride, sodium carbonate, potassium carbonate or cesium carbonate, *N,N*-diisopropylethylamine, triethylamine or 1,8-diazabicyclo(5.4.0)undec-7-ene (DBU), and in the case of aromatic amines in the presence of an acid such as 4-methylbenzenesulfonic acid in an appropriate solvent.

Preferred herein is the performance of said nucleophilic reaction in the case of amines using N,N-diisopropylethylamine as a base in acetonitrile as a solvent, within a temperature range from 20 °C to 80 °C.

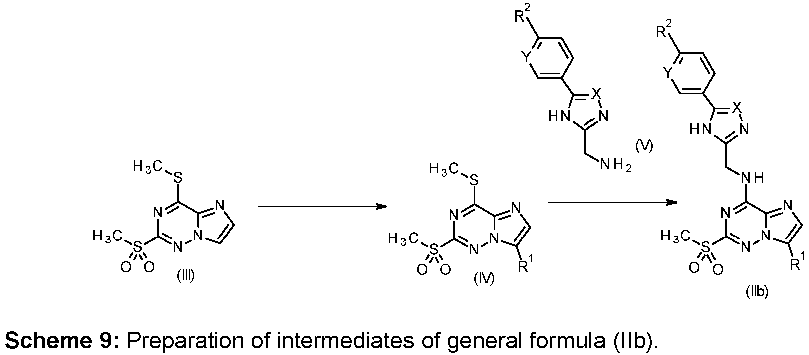

Intermediate sulfone derivatives of formula (llb) are available for example by the sequence depicted in Scheme 9. This approach started with 2-(methanesulfonyl)-4-(methylsulfanyl)imidazo[2,1-f][1,2,4]triazine (see Dudfield, Philip J.; Le, Van-Due; Lindell, Stephen D.; Rees, Charles W. Journal of the Chemical Society. Perkin transactions I, 1999, # 20, p. 2929 - 2936) of the formula (III). It is possible to introduce halogens such as bromo, chloro or iodo using the corresponding *N*-halo-succinimide reagent to obtain compounds of the formula (IV) with R¹ being bromo, chloro or iodo. Reaction of said derivatives (IV) with compounds of the formula (V) resulted in sulfones of the formula (IIb).

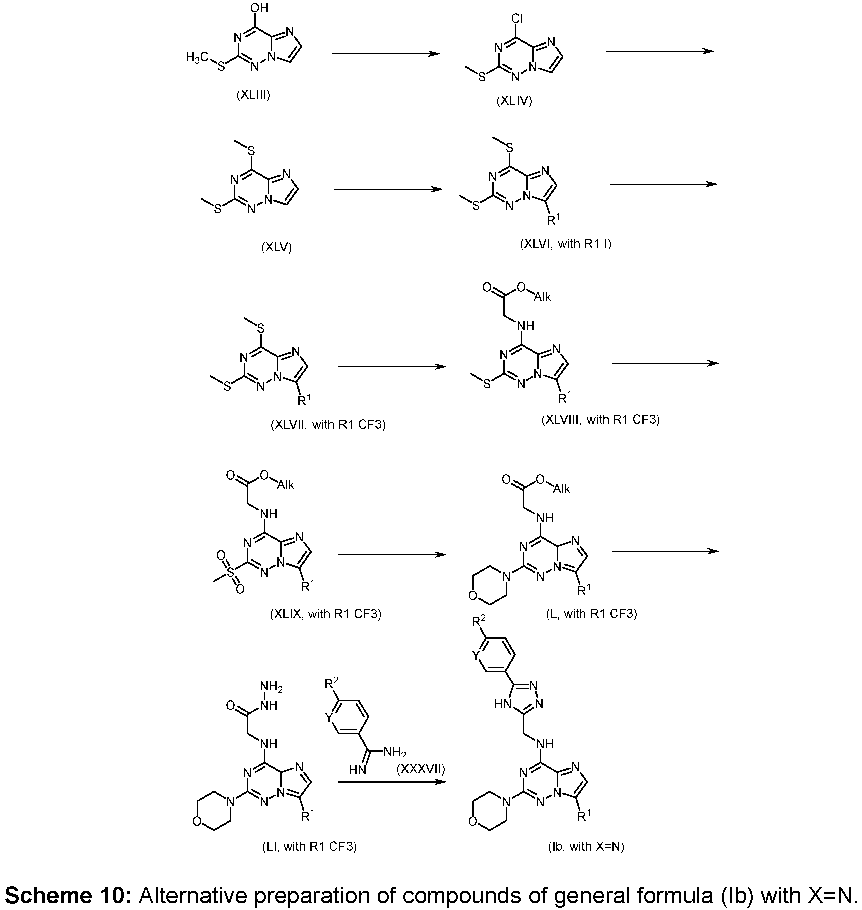

Alternatively, compounds of the formula (XLIII) can be reacted with phosphorus oxychloride to give compounds of formula (XLIV) which can react with methanethiol to compounds of the formula (XLV). These disulfanes of the formula (XLV) can be halogenated to compounds of the formula (XLVI) which can be reacted to the corresponding CF₃ derivative of formula (XLVI) with methods well known to the person skilled in the art. These compounds of the formula (XLVI) and an amine can react in an aromatic nucleophilic substitution well known to the person skilled in the art to give compounds of the formula (XLVIII). After oxydation sulfones of the formula (XLIX) can react in an aromatic nucleophilic substitution well known to the person skilled in the art to give compounds of the formula (L) which can be hydrolized to the corresponding carbonic acids and then be reacted to the corresponding hydrazides of formula (LI) with methods well known to the person skilled in the art. Compounds of the formula (Ib) with X=N and R³ as defined for the compounds of general formula (I) can be assembled by the reaction of hydrazides of formula (LI) with imidamides of formula (XXXVII) with methods well known to the person skilled in the art.

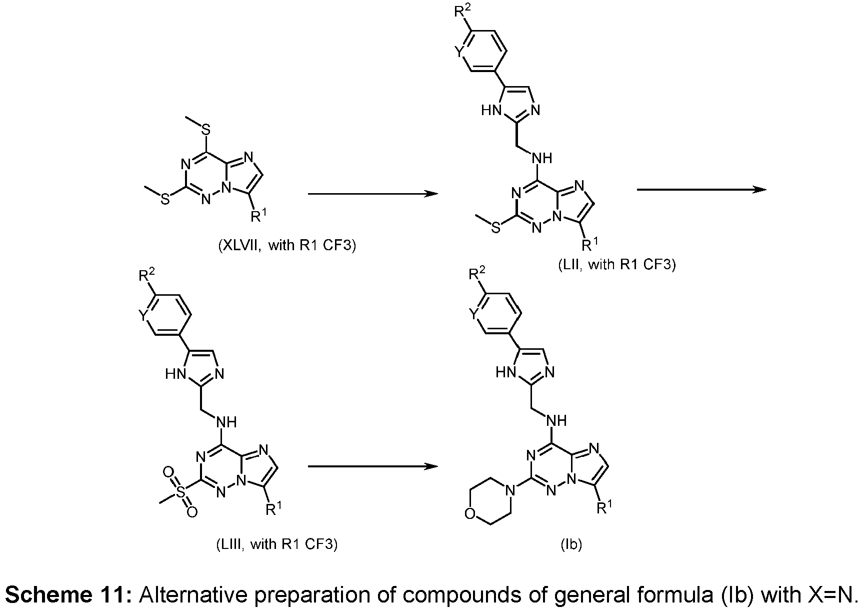

Alternatively, compounds of the formula (XLVI) and an amine can react in an aromatic nucleophilic substitution well known to the person skilled in the art to give compounds of the formula (LII). After oxidation, sulfones of the formula (LIII) can react in an aromatic nucleophilic substitution well known to the person skilled in the art with an amine as morpholine to give compounds of the formula (Ib).

The present invention includes the intermediate compounds which are disclosed in the Example Section of this text, *infra.*

The compounds of general formula **(I)** of the present invention can be converted to any salt, preferably pharmaceutically acceptable salts, by any method which is known to the person skilled in the art. Similarly, any salt of a compound of general formula **(I)** of the present invention can be converted into the free compound, by any method which is known to the person skilled in the art.

Compounds of general formula **(I)** of the present invention demonstrate a valuable pharmacological spectrum of action which could not have been predicted. The compounds of the present invention effectively inhibit the activity of CDK12 for which data are given in the biological experimental section and may therefore be used for the treatment and/or prophylaxis of hyperproliferative disorders, such as cancer disorders in humans and animals.

### Methods and administration

Compounds of general formula **(I)** of the present invention demonstrate a valuable pharmacological spectrum of action and pharmacokinetic profile, both of which could not have been predicted. Compounds of the present invention have surprisingly been found to effectively impair the activity of CDK12, showing a strong CDK12 degrading potency which induce the proteolytic degradation of CDK12 protein in the cell resulting in an increased selectivity against other kinases.Therefore, it is possible that said compounds can be used for the treatment and/or prophylaxis of diseases, preferably hyperproliferative disorders in humans and animals.

Further, CDK12 has been identified as a druggable target for addressing the RNA-based disease myotonic dystrophy type 1 (DM1) (Ketley et al., Sci. Transl. Med. 12, eaaz2415 (2020)). Thus, it is possible that compounds of general formula **(I)** of the present invention can be used for the treatment and/or prophylaxis of diseases in which CDK12 is involved, such as myotonic dystrophy type 1 (DM1).

As used herein, "prophylaxis" includes a use of the compound that, in a statistical sample, reduces the occurrence of the disorder or condition in the treated sample relative to an untreated control sample, or delays the onset or reduces the severity of one or more symptoms of the disorder or condition relative to the untreated control sample, when administered to prior to the onset of the disorder or condition.

Compounds of the present invention can be utilized to inhibit, block, reduce, decrease, *etc.,* cell proliferation and/or cell division, and/or produce apoptosis, which are all types of "treatment". This method comprises administering to a mammal in need thereof, including a human, an amount of a compound of general formula **(I)** of the present invention, or a pharmaceutically acceptable salt, polymorph, hydrate, solvate or ester thereof, which is effective to treat the disorder.

Hyperproliferative disorders include, but are not limited to, for example: psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancers include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypothalamic glioma, cerebellar and cerebral astrocytoma, medulloblastoma, ependymoma, as well as neuroectodermal and pineal tumour.

Tumours of the male reproductive organs include, but are not limited to, prostate and testicular cancer.

Tumours of the female reproductive organs include, but are not limited to, endometrial, cervical, ovarian, vaginal, and vulvar cancer, as well as sarcoma of the uterus.

Tumours of the digestive tract include, but are not limited to, anal, colon, colorectal, oesophageal, gallbladder, gastric, pancreatic, rectal, small-intestine, and salivary gland cancers.

Tumours of the urinary tract include, but are not limited to, bladder, penile, kidney, renal pelvis, ureter, urethral and human papillary renal cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, basal cell carcinoma, squamous cell carcinoma, Kaposi's sarcoma, malignant melanoma, Merkel cell skin cancer and non-melanoma skin cancer.

Head-and-neck cancers include, but are not limited to, laryngeal, hypopharyngeal, nasopharyngeal, oropharyngeal cancer, lip and oral cavity cancer and squamous cell.

Lymphomas include, but are not limited to, AIDS-related lymphoma, chronic lymphocytic lymphoma (CLL), non-Hodgkin's lymphoma (NHL), T-non-Hodgkin lymphoma (T-NHL), subtypes of NHL such as Diffuse Large Cell Lymphoma (DLBCL), activated B-cell DLBCL, germinal center B-cell lymphoma DLBCL, double-hit lymphoma and double-expressor lymphoma; anaplastic large cell lymphoma, B-cell lymphoma, cutaneous T-cell lymphoma, Burkitt's lymphoma, follicular lymphoma, hairy cell lymphoma, Hodgkin's disease, mantle cell lymphoma (MCL), lymphoma of the central nervous system, small lymphocytic lymphoma and chronic lymphocytic lymphoma and Sezary syndrome.

Sarcomas include, but are not limited to, sarcoma of the soft tissue, osteosarcoma, malignant fibrous histiocytoma, lymphosarcoma, and rhabdomyosarcoma.

Leukemias include, but are not limited to acute lymphoblastic leukemia, acute myeloid leukemia, (acute) T-cell leukemia, acute lymphoblastic leukemia, acute lymphocytic leukemia (ALL) , acute monocytic leukemia (AML), acute promyelocytic leukemia (APL), bisphenotypic B myelomonocytic leukemia, chronic lymphocytic leukemia (CLL), chronic myelogenous leukemia, chronic myeloid leukemia (CML), chronic myelomonocytic leukemia (CMML), large granular lymphocytic leukemia, plasma cell leukemia and also myelodysplastic syndrome (MDS), which can develop into an acute myeloid leukemia.

The present invention also provides methods of treating angiogenic disorders including diseases associated with excessive and/or abnormal angiogenesis.

Inappropriate and ectopic expression of angiogenesis can be deleterious to an organism. A number of pathological conditions are associated with the growth of extraneous blood vessels. These include, for example, diabetic retinopathy, ischemic retinal-vein occlusion, and retinopathy of prematurity [Aiello et al., New Engl. J. Med., 1994, 331, 1480; Peer et al., Lab. Invest., 1995, 72, 638], age-related macular degeneration (AMD) [Lopez et al., Invest. Ophthalmol. Vis. Sci., 1996, 37, 855], neovascular glaucoma, psoriasis, retrolental fibroplasias, angiofibroma, inflammation, rheumatoid arthritis (RA), restenosis, in-stent restenosis, vascular graft restenosis, *etc.* In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of general formula **(I)** of the present invention can be utilized to treat and/or prevent any of the aforementioned angiogenesis disorders, for example by inhibiting and/or reducing blood vessel formation; by inhibiting, blocking, reducing, decreasing, *etc.* endothelial cell proliferation, or other types involved in angiogenesis, as well as causing cell death or apoptosis of such cell types.

These disorders have been well characterized in humans, but also exist with a similar etiology in other mammals, and can be treated by administering pharmaceutical compositions of the present invention.

The term "treating" or "treatment" as stated throughout this document is used conventionally, for example the management or care of a subject for the purpose of combating, alleviating, reducing, relieving and/or improving the condition of a disease or disorder, such as a carcinoma.

The compounds of the present invention can be used in particular in therapy and prevention, *i.e.* prophylaxis, of tumour growth and metastases, especially in solid tumours of all indications and stages with or without pre-treatment of the tumour growth.

Generally, the use of chemotherapeutic agents and/or anti-cancer agents in combination with a compound or pharmaceutical composition of the present invention will serve to:
1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of general formula **(I)** of the present invention can also be used in combination with radiotherapy and/or surgical intervention.

In a further embodiment of the present invention, the compounds of general formula **(I)** of the present invention may be used to sensitize a cell to radiation, *i.e.* treatment of a cell with a compound of the present invention prior to radiation treatment of the cell renders the cell more susceptible to DNA damage and cell death than the cell would be in the absence of any treatment with a compound of the present invention. In one aspect, the cell is treated with at least one compound of general formula **(I)** of the present invention.

Thus, the present invention also provides a method of killing a cell, wherein a cell is administered one or more compounds of the present invention in combination with conventional radiation therapy.

The present invention also provides a method of rendering a cell more susceptible to cell death, wherein the cell is treated with one or more compounds of general formula **(I) of** the present invention prior to the treatment of the cell to cause or induce cell death. In one aspect, after the cell is treated with one or more compounds of general formula **(I) of** the present invention, the cell is treated with at least one compound, or at least one method, or a combination thereof, in order to cause DNA damage for the purpose of inhibiting the function of the cell or killing the cell.

In other embodiments of the present invention, a cell is killed by treating the cell with at least one DNA damaging agent, *i.e.* after treating a cell with one or more compounds of general formula **(I)** of the present invention to sensitize the cell to cell death, the cell is treated with at least one DNA damaging agent to kill the cell. DNA damaging agents useful in the present invention include, but are not limited to, chemotherapeutic agents (*e.g*. cis platin), ionizing radiation (X-rays, ultraviolet radiation), carcinogenic agents, and mutagenic agents.

In other embodiments, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In some instances, a compound of general formula **(I)** as disclosed herein may be administered to a cell prior to the radiation or other induction of DNA damage in the cell. Typically, a compound of general formula **(I)** of the present invention may be administered to a cell concomitantly with the radiation or other induction of DNA damage in the cell. In the alternative, a compound of general formula **(I)** may be administered to a cell after radiation or other induction of DNA damage in the cell has begun. Again alternatively, a compound of general formula **(I)** may be administered to a cell immediately after radiation or other induction of DNA damage in the cell has begun. In some embodiments, the cell is *in vitro.*

Thus in some embodiments, the present invention includes a method of inhibiting proliferation of a cell and/or the induction of apoptosis in a cell, comprising contacting the cell with a compound of formula **(I).**

In the context of this disclosure, a method for treating, preventing or prophylaxing cancer (i.e. a method for the treatment, prevention or prophylaxis of cancer) in a subject (e.g., human, other mammal, such as rat, etc.) by administering an effective amount of at least one compound of general formula **(I),** or a pharmaceutically acceptable salt, polymorph, hydrate, solvate or ester thereof to the subject is described herein but not claimed.

In such instances, the subject may be administered a medicament, comprising at least one compound of general formula **(I)** and one or more pharmaceutically acceptable carriers, excipients and/or diluents.

Furthermore, in the context of the present disclosure a method of using a compound of general formula **(I)** for the treatment of diseases is described herein but not claimed.

Particularly, in the context of the present disclosure a method of treating a hyperproliferative disease, more particularly cancer, comprising administering an effective amount of at least one compound of general formula **(I)** to a subject in need thereof is described herein but not claimed.

In some instances disclosed herein but not claimed, the method of treatment and/or prophylaxis of a hyperproliferative disorder in a subject may comprise administering to the subject an effective amount of a compound of general formula **(I).** The hyperproliferative disorder may be, for example, cancer (e.g., lung cancer, breast cancer, acute myeloid leukemia, lymphoma, glioblastoma, prostate cancer, etc.).

Furthermore in some instances described herein but not claimed, the present invention includes a method of treating cancer, particularly lymphoma, non-Hodgkin-lymphoma type, diffuse large B-cell lymphoma subtype, acute leukemia, acute myeloid leukemia type, multiple myeloma, ovarian cancer, comprising administering an effective amount of at least one compound of formula **(I)** to a subject in need thereof.

Furthermore in some embodiments, in the context of the present disclosurea method of treating cancer, particularly multiple myeloma, ovarian carcinoma, acute monocytic leukemia, melanoma and lung cancer, comprising administering an effective amount of at least one compound of formula **(I)** to a subject in need thereof is described herein but not claimed.

Furthermore in some instanced disclosed herein but not claimed, a method of treating cancer, particularly breast cancer; lung cancer; lymphoma including non-Hodgkin-lymphoma type, diffuse large B-cell lymphoma subtype including GC-DLBCL* and ABC-DLBCL** subtypes, and mantle cell lymphoma; acute leukemia, acute myeloid leukemia type, acute monocytic leukemia; melanoma; multiple myeloma; ovarian cancer; and pancreas cancer, comprising administering an effective amount of at least one compound of formula **(I)** to a subject in need thereof according to any one of claims 1-8 is described. GC-DLBCL means Germinal B-cell Diffuse Large B-Cell Lymphoma and ** ABC-DLBCL means Activated B-cell Diffuse Large B-Cell Lymphoma.

Furthermore in some instances disclosed herein but not claimed, a method of treating cancer, particularly breast cancer, lung cancer, diffuse large B-cell lymphoma subtype including GC-DLBCL* and ABC-DLBCL** subtypes, mantle cell lymphoma, acute monocytic leukemia, melanoma, ovarian cancer, and pancreas cancer comprising administering an effective amount of at least one compound of formula **(I)** to a subject in need thereof according to any one of claims 1-8 is described. Furthermore in some embodiments, the present invention provides a compound of formula **(I)** for use of treating diseases.

Furthermore in some instances disclosed herein but not claimed, a method of treating cancer, particularly breast cancer; lymphoma, leukemia, multiple myeloma; and ovarian cancer, comprising administering an effective amount of at least one compound of formula **(I)** to a subject in need thereof is described.

Furthermore in some instances disclosed herein but not claimed, a method of treating cancer, particularly lymphoma, non-Hodgkin-lymphoma type, diffuse large B-cell lymphoma subtype, acute leukemia, acute myeloid leukemia type, multiple myeloma, and ovarian cancer, comprising administering an effective amount of at least one compound of formula **(I)** to a subject in need thereof is described.

Furthermore in some instances disclosed herein but not claimed, a method of treating cancer, particularly breast cancer, lymphoma (including non-Hodgkin-lymphoma type, diffuse large B-cell lymphoma subtype, mantle cell lymphoma), leukemia (including acute monocytic leukemia), liver cancer, multiple myeloma, melanoma, non-small cell lung cancer, small cell lung cancer, ovarian cancer, ovarian carcinoma, stomach cancer, and squamous cell carcinoma, comprising administering an effective amount of at least one compound of formula **(I)** to a subject in need thereof is described.

Furthermore in some instances disclosed herein but not claimed, a method of treating cancer, particularly breast cancer, diffuse large B-cell lymphoma subtype, mantle cell lymphoma, acute monocytic leukemia, liver cancer, multiple myeloma, melanoma, non-small cell lung cancer, small cell lung cancer, ovarian cancer, ovarian carcinoma, prostate cancer, stomach cancer, and squamous cell carcinoma, comprising administering an effective amount of at least one compound of formula **(I)** to a subject in need thereof is described.

Furthermore in some instances disclosed herein but not claimed, a method of treating cancer, particularly bladder cancer, bone cancer, brain cancer, breast cancer, colon cancer (colorectal cancer), endometrial (uterine) cancer, gastric cancer, head and neck cancer, kidney cancer, leukemia, liver cancer, lung cancer, lymphoma, lung cancer, myeloma, neuroblastoma, ovarian cancer, pancreatic cancer, rhabdoid tumor, sarcoma and skin cancer, comprising administering an effective amount of at least one compound of formula (I) to a subject in need thereof is described.

Furthermore in some instances disclosed herein but not claimed , a method of treating cancer, particularly breast cancer, liver cancer, lung cancer, ovarian cancer, endometrial cancer, cervical cancer, colorectal cancer, gastric cancer, esophageal cancer, bladder cancer, prostate cancer, sarcoma, glioblastoma and acute myeloid leukemia comprising administering an effective amount of at least one compound of formula **(I)** to a subject in need thereof is described.

Furthermore in some instances disclosed herein but not claimed, a method of treating cancer, particularly lung cancer, breast cancer, liver cancer, colorectal cancer, gastric cancer, prostate cancer and leukemia comprising administering an effective amount of at least one compound of formula **(I)** to a subject in need thereof is described.

Furthermore in some instances disclosed herein but not claimed, a method of treating myotonic dystrophy type 1 (DM1) comprising administering an effective amount of at least one compound of general formula **(I)** to a subject in need thereof is described.In accordance with some embodiments, the present invention provides compounds of general formula **(I),** as described *supra,* or stereoisomers, tautomers, N-oxides, hydrates, solvates, and salts thereof, particularly pharmaceutically acceptable salts thereof, or mixtures of same, for use in the treatment and/or prophylaxis of diseases, in particular hyperproliferative disorders.

Furthermore in accordance with a further aspect, the present invention provides a compound of formula **(I)** for use of treating diseases.In in accordance with a further aspect, the present invention includes a compound of general formula **(I)** for use in a method of inhibiting proliferation of a cell and/or the induction of apoptosis in a cell, comprising contacting the cell with a compound of formula **(I).**

Particularly in some embodiments, the present invention includes compounds of general formula **(I)** for use in a method of treating a hyperproliferative disease, more particularly wherein the hyperproliferative disease is cancer, and yet even more particularly wherein the cancer disease is selected from lymphoma, non-Hodgkin-lymphoma type, diffuse large B-cell lymphoma subtype, ovarian cancer, multiple myeloma, acute leukemia, and acute myeloid leukemia.

More particularly in some embodiments, the present invention includes compounds of general formula **(I)** for use in a method of treating a hyperproliferative disease, more particularly wherein the hyperproliferative disease is cancer, and yet even more particularly wherein the cancer disease is selected from breast cancer; lymphoma, leukemia, multiple myeloma; and ovarian cancer.

Particularly in some embodiments, the present invention includes compounds of general formula **(I)** for use in a method of treating a hyperproliferative disease, more particularly wherein the hyperproliferative disease is cancer, and yet even more particularly wherein the cancer is selected from breast cancer; esophageal cancer; liver cancer; lung cancer; lymphoma including non-Hodgkin-lymphoma type, diffuse large B-cell lymphoma subtype including GC-DLBCL* and ABC-DLBCL** subtypes, and mantle cell lymphoma; acute leukemia, acute myeloid leukemia type, acute monocytic leukemia; melanoma; multiple myeloma; melanoma; ovarian cancer; or pancreas cancer.

More particularly in some embodiments, the present invention includes compounds of general formula **(I)** for use in a method of treating cancer wherein the cancer disease is selected from breast cancer; lymphoma, leukemia, multiple myeloma; and ovarian cancer.

More particularly in some embodiments, the present invention includes compounds of general formula **(I)** for use in a method of treating cancer wherein the cancer disease is selected from breast cancer, liver cancer, lung cancer, ovarian cancer, endometrial cancer, cervical cancer, colorectal cancer, gastric cancer, esophageal cancer, bladder cancer, prostate cancer, sarcoma, glioblastoma, and acute myeloid leukemia.

More particularly in some embodiments, the present invention includes compounds of general formula **(I)** for use in a method of treating cancer wherein the cancer disease is selected from lung cancer, breast cancer, liver cancer, colorectal cancer, gastric cancer, prostate cancer, and leukemia.

Furthermore in some embodiments, the present invention includes compounds of general formula **(I)** for use in a method of treating myotonic dystrophy type 1 (DM1).

In some instances disclosed herein but not claimed, the use of the compounds of general formula **(I)** for the manufacture of a medicament for the treatment and/or prophylaxis of a hyperproliferative disease is described.

In some instances disclosed herein but not claimed, the use of the compounds of general formula **(I)** for the manufacture of a medicament for the treatment and/or prophylaxis of a hyperproliferative disease, wherein the hyperproliferative disease is cancer is described.

In some instances disclosed herein but not claimed, the use of the compounds of general formula **(I)** for the manufacture of a medicament for the treatment of a hyperproliferative disease, particularly cancer and more particularly lymphoma, non-Hodgkin-lymphoma type, diffuse large B-cell lymphoma subtype, ovarian cancer, multiple myeloma, acute leukemia, and acute myeloid leukemia type is described.

In some instances disclosed herein but not claimed, the use of the compounds of general formula **(I)** for the manufacture of a medicament for the treatment of a hyperproliferative disease, particularly cancer and more particularly breast cancer, liver cancer, lung cancer, ovarian cancer, endometrial cancer, cervical cancer, colorectal cancer, gastric cancer, esophageal cancer, bladder cancer, prostate cancer, sarcoma, glioblastoma, and acute myeloid leukemia is described.

In some instances disclosed herein but not claimed, the use of the compounds of general formula **(I)** for the manufacture of a medicament for the treatment of a hyperproliferative disease, particularly cancer and more particularly lung cancer, breast cancer, liver cancer, colorectal cancer, gastric cancer, prostate cancer, and leukemia is described.

In some instances disclosed herein but not claimed, the use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer is described.

In some instances disclosed herein but not claimed, the use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly breast cancer, liver cancer, lung cancer, ovarian cancer, endometrial cancer, cervical cancer, colorectal cancer, gastric cancer, esophageal cancer, bladder cancer, prostate cancer, sarcoma, glioblastoma, and acute myeloid leukemia is described.

Furthermore in some instances disclosed herein but not claimed, the use of the compounds of general formula **(I)** for the manufacture of a medicament for the treatment of myotonic dystrophy type 1 (DM1) is described.

In some instances disclosed herein but not claimed, a method of treatment and/or prophylaxis of diseases, in particular hyperproliferative disorders, particularly cancer, comprising administering an effective amount of a compound of general formula **(I), as** described *supra,* or stereoisomers, tautomers, N-oxides, hydrates, solvates, and salts thereof, particularly pharmaceutically acceptable salts thereof, or mixtures of same to a subject in need thereof is described.

In some instances disclosed herein but not claimed, a method of treatment and/or prophylaxis of diseases, in particular hyperproliferative disorders, particularly cancer, more particularly breast cancer, liver cancer, lung cancer, ovarian cancer, endometrial cancer, cervical cancer, colorectal cancer, gastric cancer, esophageal cancer, bladder cancer, prostate cancer, sarcoma, glioblastoma, and acute myeloid leukemia comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof is described.

Furthermore in some instances disclosed herein but not claimed, a method of treatment of myotonic dystrophy type 1 (DM1) comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof is described.

In some embodiments, the present invention provides pharmaceutical compositions, in particular a medicament, comprising a compound of general formula **(I),** as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore provides pharmaceutical compositions, in particular medicaments, which comprise at least one compound according to the invention, conventionally together with one or more pharmaceutically suitable excipients, and to their use for the above mentioned purposes.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®}),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention.

In some embodiments, the present invention provides pharmaceutical combinations, in particular medicaments, comprising at least one compound of general formula **(I)** of the present invention and at least one or more further active ingredients, in particular for the treatment and/or prophylaxis of a hyperproliferative disorder, particularly cancer.

Particularly, the present invention provides a pharmaceutical combination, which comprises:
- one or more first active ingredients, in particular compounds of general formula **(I)** as defined *supra,* and
- one or more further active ingredients, in particular for the treatment and/or prophylaxis of a hyperproliferative disorder, particularly cancer.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula **(I)** of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

The compounds of the present invention can be administered as the sole pharmaceutical agent or in combination with one or more other pharmaceutically active ingredients where the combination causes no unacceptable adverse effects. The present invention also provides such pharmaceutical combinations. For example, the compounds of the present invention can be combined with known anti-cancer agents.

Examples of anti-cancer agents include:
131I-chTNT, abarelix, abemaciclib, abiraterone, acalabrutinib, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, apalutamide, aprepitant, arcitumomab, arglabin, arsenic trioxide, asparaginase, atezolizumab, avelumab, axicabtagene ciloleucel, axitinib, azacitidine, basiliximab, belotecan, bendamustine, besilesomab, belinostat, bevacizumab, bexarotene, bicalutamide, bisantrene, bleomycin, blinatumomab, bortezomib, bosutinib, buserelin, brentuximab vedotin, brigatinib, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, durvalumab, eculizumab, edrecolomab, elliptinium acetate, elotuzumab, eltrombopag, enasidenib, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, inotuzumab ozogamicin, interferon alfa, interferon beta, interferon gamma, iobitridol, iobenguane (123I), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, lutetium Lu 177 dotatate, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, midostaurin, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, mvasi, nabilone, nabiximols, nafarelin, naloxone + pentazocine, naltrexone, nartograstim, necitumumab, nedaplatin, nelarabine, neratinib, neridronic acid, netupitant/palonosetron, nivolumab, pentetreotide, nilotinib, nilutamide, nimorazole, nimotuzumab, nimustine, nintedanib, niraparib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, ribociclib, risedronic acid, rhenium-186 etidronate, rituximab, rolapitant, romidepsin, romiplostim, romurtide, rucaparib, samarium (153Sm) lexidronam, sargramostim, sarilumab, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid and zorubicin.

Based upon standard laboratory techniques known to evaluate compounds useful for the treatment of hyperproliferative disorders, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### EXPERIMENTAL SECTION

### EXPERIMENTAL SECTION - NMR SPECTRA

To the extent NMR peak forms and multiplicities are specified, they are stated as they appear in the spectra, possible higher order effects have not been considered.

The ¹H-NMR data of selected examples are listed in the form of ¹H-NMR peaklists. For each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of target compounds (also the subject of the invention), and/or peaks of impurities. The peaks of stereoisomers, and/or peaks of impurities are typically displayed with a lower intensity compared to the peaks of the target compounds (e.g., with a purity of >90%). Such stereoisomers and/or impurities may be typical for the particular manufacturing process, and therefore their peaks may help to identify the reproduction of our manufacturing process on the basis of "by-product fingerprints". An expert who calculates the peaks of the target compounds by known methods (MestReC, ACD simulation, or by use of empirically evaluated expectation values), can isolate the peaks of target compounds as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. Research Disclosure Database Number 605005, 2014, 01 Aug 2014, or http://www.researchdisclosure.com/searching-disclosures). In the peak picking routine, as described in the Research Disclosure Database Number 605005, the parameter "MinimumHeight" can be adjusted between 1% and 4%. Depending on the chemical structure and/or depending on the concentration of the measured compound it may be reasonable to set the parameter "MinimumHeight" <1%.

### EXPERIMENTAL SECTION - ABBREVIATIONS

The following table lists the abbreviations used in this paragraph and in the Intermediates and Examples section as far as they are not explained within the text body. Other abbreviations have their meanings customary *per se* to the skilled person. A comprehensive list of the abbreviations utilized by organic chemists of ordinary skill in the art appears presented in the first issue of each volume of the Journal of Organic Chemistry; this list is typically presented in a table titled "Standard List of Abbreviations". In case of doubt, the abbreviations and/or their meaning according to the following table shall prevail.

**Table 1: Abbreviations**

| **Abbreviation** | **Meaning** |
|---|---|
| DMF | N,N-Dimethylformamide |
| DMSO | dimethylsulfoxide |
| ESI | electrospray (ES) ionisation |
| h, hr (hrs) | hour(s) |
| HPLC | high performance liquid chromatography |
| LC-MS | liquid chromatography-mass spectrometry |
| m | multiplet (NMR) |
| Min | minute(s) |
| MS | mass spectrometry |
| NMR | nuclear magnetic resonance spectroscopy : chemical shifts (δ) are given in ppm. The chemical shifts were corrected by setting the DMSO signal to 2.50 ppm using dmso-d6 unless otherwise stated. |
| rt, RT | room temperature |
| Rₜ, Rt | retention time |
| THF | tetrahydrofuran |
| UPLC | ultra performance liquid chromatography |
| UV | ultraviolet |
| δ | chemical shift |

Other abbreviations have their meanings customary *per se* to the skilled person.

The various aspects of the invention described in this application are illustrated by the following examples which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art. Reactions were set up and started, e.g. by the addition of reagents, at temperatures as specified in the protocols; if no temperature is specified, the respective working step was performed at ambient temperature, *i.e.* between 18 and 25 °C.

"Silicone filter" or "water resistant filter" refers to filter papers which are made hydrophobic (impermeable to water) by impregnation with a silicone. With the aid of these filters, water can be separated from water-immiscible organic solvents by means of a filtration (i.e. filter paper type MN 617 WA, Macherey-Nagel).

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent or solvent mixture. In some cases, the compounds may be purified by chromatography, particularly flash column chromatography, using for example prepacked silica gel cartridges, e.g. Biotage SNAP cartridges KP-Sil^{®} or KP-NH^{®} in combination with a Biotage autopurifier system (SP4^{®} or Isolera Four^{®}) and eluents such as gradients of hexane/ethyl acetate or dichloromethane/ethanol. In flash column chromatography, unmodified ("regular") silica gel may be used as well as aminophase functionalized silica gel. As used herein, "Biotage SNAP cartridge silica" refers to the use of regular silica gel; "Biotage SNAP cartridge NH₂ silica" refers to the use of aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid, diethylamine or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### UPLC-MS Standard Procedures

Analytical UPLC-MS was performed as described below. The masses (m/z) are reported from the positive mode electrospray ionisation unless the negative mode is indicated (ESI-).

### Analytical UPLC methods:

### Method 1:

Instrument: Waters Acquity UPLCMS SingleQuad; Column: Acquity UPLC BEH C18 1.7 µm, 50x2.1 mm; eluent A: water + 0.1 vol % formic acid (99 %), eluent B: acetonitrile; gradient: 0-1.6 min 1-99 % B, 1.6-2.0 min 99 % B; flow 0.8 mL/min; temperature: 60 °C; DAD scan: 210-400 nm.

### Method 2:

Instrument: Waters Acquity UPLCMS SingleQuad; Column: Acquity UPLC BEH C18 1.7 µm, 50x2.1 mm; eluent A: water + 0.2 vol % aqueous ammonia (32 %), eluent B: acetonitrile; gradient: 0-1.6 min 1-99 % B, 1.6-2.0 min 99 % B; flow 0.8 mL/min; temperature: 60 °C; DAD scan: 210-400 nm.

### Method 3:

Instrument: Waters Acquity UPLCMS SingleQuad; Column: Acquity UPLC BEH C18 1.7 µm, 50x2.1 mm; eluent A: water + 0.2 vol % aqueous ammonia (32 %), eluent B: acetonitrile; gradient: 0-1.6 min 1-99 % B, 1.6-2.0 min 99 % B; flow 0.8 mL/min; temperature: 60 °C; DAD scan: 210-400 nm.

### Method C:5-95AB, Shimadzu

Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min, 5-95% B, 0.8-1.2 min 95% B; flow 1.5 mL/min; temperature: 50 °C; PDA: 220 nm & 254 nm.

### Method D:5-95AB, Agilent

Instrument: Agilent 1100\G1956A SingleQuad; Column: Kinetex@ 5 µm EVO C18 30*2.1 mm; eluent A: water + 0.0375 vol % trifluoroacetic acid, eluent B: acetonitrile + 0.01875 vol % trifluoroacetic acid; gradient: 0-0.8 min 5-95% B, 0.8-1.2 min 95% B; flow 1.5 mL/min; temperature: 50 °C; PDA: 220 nm & 254 nm.

### Preparative HPLC methods:

### Method HT acidic:

Instrument: Waters Autopurificationsystem; Column: Waters XBrigde C18 5 µ 100x30 mm; eluent A: water + 0.1 vol % formic acid (99 %), eluent B: acetonitrile; gradient; DAD scan: 210-400 nm.

### Method HT basic:

Instrument: Waters Autopurificationsystem; Colum: Waters XBrigde C18 5 µ 100x30 mm; eluent A: water + 0.2 vol % aqueous ammonia (32 %), eluent B: acetonitrile; gradient; DAD scan: 210-400 nm.

### Specific Optial Rotation Methods:

Method O1: Instrument: JASCO P2000 Polarimeter; wavelength 589 nm; temperature: 20 °C; integration time 10 s; path length 100 mm.

### Intermediate 1

### ethyl [(1H-pyrazol-5-yl)carbamothioyl]carbamate

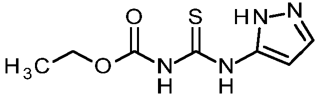

1H-pyrazol-5-amine (58.7 g, 706 mmol; CAS 1820-80-0) was dissolved in ethyl acetate (420 mL), under nitrogen, and stirred at 75°C. Ethyl carbonisothiocyanatidate (88 mL, 750 mmol; CAS 16182-04-0) was added dropwise at 75°C and the mixture was stirred for 1h at 75°C. The mixture was cooled to 0°C, filtered, washed with ethyl acetate and the solid was dried under reduced pressure at 50°C to give 124 g (77 % yield) of the title compound.

¹H-NMR (400 MHz, DMSO-d6) δ [ppm]: 1.232 (7.32), 1.250 (16.00), 1.267 (7.44), 2.518 (0.40), 4.184 (2.18), 4.201 (6.76), 4.219 (6.67), 4.237 (2.07), 5.889 (0.89), 5.893 (0.82), 6.998 (1.81), 7.003 (2.83), 7.008 (1.72), 7.697 (2.70), 7.867 (0.72), 7.872 (0.75), 11.317 (2.61), 12.036 (2.75), 12.709 (1.55).

### Intermediate 2

### 2-sulfanylpyrazolo[1,5-a][1,3,5]triazin-4-ol

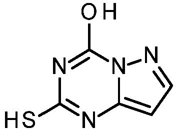

Ethyl [(1H-pyrazol-5-yl)carbamothioyl]carbamate **(Intermediate 1,** 124 g, 580 mmol) was stirred in sodium hydroxide (550 mL, 2.0 M, 1.1 mol) for 3h at rt. The mixture was cooled to 0°C and sulfuric acid (580 mL, 2.0 M, 1.2 mol) was added dropwise. The suspension was filtered, washed with water and the solid was dried under reduced pressure at 50°C to give 85.2 g (87 % yield) of the title compound.

¹H-NMR (400 MHz, DMSO-d6) δ [ppm]: 2.518 (0.54), 3.349 (0.66), 5.888 (14.42), 5.892 (16.00), 7.866 (14.93), 7.870 (13.98), 12.730 (0.83), 13.450 (0.66).

### Intermediate 3

### 2-(methylsulfanyl)pyrazolo[1,5-a][1,3,5]triazin-4-ol

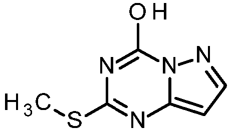

2-sulfanylpyrazolo[1,5-a][1,3,5]triazin-4-ol **(Intermediate 2,** 85.2 g, 507 mmol) was dissolved in ethanol (2.0 I) and sodium hydroxide (580 mL, 1.7 M, 1.0 mol). lodomethane (32 mL, 510 mmol; CAS 74-88-4) was added dropwise at rt and the mixture was stirred for 2h at rt. The mixture was cooled to 0°C, sulfuric acid (510 mL, 1.0 M, 510 mmol) was added dropwise and the mixture was stirred for 1h at rt. The precipitate was collected by filtration, washed with water dried under reduced pressure at 50°C. The solid was stirred 2 times in acetonitrile, liquid phases were filtered off and the soid was washed with hexane and dried to give 60.5 g (65 % yield) of the title compound.

¹H-NMR (400 MHz, DMSO-d6) δ [ppm]: 2.530 (16.00), 6.351 (3.35), 6.355 (3.08), 7.970 (2.67), 7.976 (3.22).

### Intermediate 4

### 8-bromo-2-(methylsulfanyl)pyrazolo[1,5-a][1,3,5]triazin-4-ol

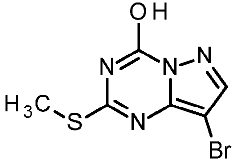

2-(methylsulfanyl)pyrazolo[1,5-a][1,3,5]triazin-4-ol **(Intermediate 3,** 59.0 g, 324 mmol) was dissolved in DMF (690 mL), cooled to 0°C, NBS (63.4 g, 356 mmol; CAS 128-08-5) dissolved in DMF (200 mL) was added dropwise and the mixture was stirred for 1 h at 0°C. The mixture was poured into water, stirred for 15 min, filtered and washed with water, acetonitrile and hexane. The solid was dried under reduced pressure at 50°C to give 71.7 g (85 % yield) of the title compound.

¹H-NMR (400 MHz, DMSO-d6) δ [ppm]: 8.113 (16.00).

### Intermediate 5

### 8-iodo-2-(methylsulfanyl)pyrazolo[1,5-a][1,3,5]triazin-4-ol

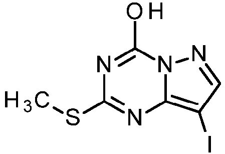

2-(methylsulfanyl)pyrazolo[1,5-a][1,3,5]triazin-4-ol **(Intermediate 3,** 25.0 g, 137 mmol) was dissolved in N,N-dimethylformamide (200 mL), cooled to 0 °C, N-lodosuccinimid (32.4 g, 144 mmol; CAS-RN:[516-12-1]) was added within 10 min and the mixture was stirred for 1 h at 0 °C. The reaction mixture was poured into water. The precipitate was collected by filtration, washed with water and dried over night to give 41.5 g (98 % yield) of the title compound.
LC-MS (Method 1): Rt = 0.86 min; MS (ESlpos): m/z = 309 [M+H]⁺
¹H-NMR (400 MHz, DMSO-d6) δ [ppm]: 2.523 (0.53), 2.575 (16.00), 8.048 (5.89).

### Intermediate 6

### 8-bromo-4-chloro-2-(methylsulfanyl)pyrazolo[1,5-a][1,3,5]triazine

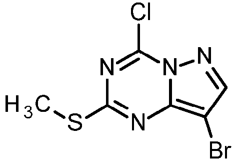

8-bromo-2-(methylsulfanyl)pyrazolo[1,5-a][1,3,5]triazin-4-ol **(Intermediate 4,** 33.3 g, 128 mmol) was dissolved in phosphorus oxychloride (170 mL, 1.8 mol; CAS 10025-87-3) and N,N-dimethylaniline (16 mL, 130 mmol; CAS 121-69-7) was added. The mixture was stirred for 3h at 105°C. The mixture was poured carefully into ice water and neutralized with sodium bicarbonate. The suspension was filtered and washed with water and hexane to give 24.0 g (67 % yield) of the title compound.

¹H-NMR (400 MHz, DMSO-d6) δ [ppm]: 2.518 (0.49), 2.567 (16.00), 8.116 (6.88).

### Intermediate 7

### methyl N-[8-bromo-2-(methylsulfanyl)pyrazolo[1,5-a][1,3,5]triazin-4-yl]glycinate

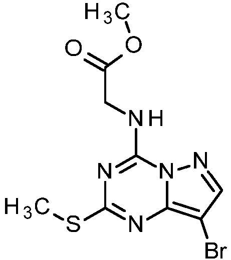

### 8-bromo-4-chloro-2-(methylsulfanyl)pyrazolo[1,5-a][1,3,5]triazine (

**Intermediate 6, 3.53** g, 12.6 mmol) and methyl glycinate hydrochloride (1/1) (2.38 g, 18.9 mmol) were dissolved in n-butanol (71 mL), N,N-diisopropylethylamine (8.8 mL, 51 mmol; CAS-RN:[7087-68-5]) was added and the mixture was stirred for 30 min at 90 °C. The mixture was diluted with ethanol and water and the precipitate was collected by filtration, washed with water and ethanol and dried under reduced pressure at 50 °C to give 3.18 g (72 % yield) of the title compound.
LC-MS (Method 2): Rₜ = 1.09 min; MS (ESlpos): m/z = 332 [M+H]⁺
¹H-NMR (400 MHz, CHLOROFORM-d) δ [ppm]: 1.537 (5.12), 2.524 (16.00), 3.764 (12.91), 4.330 (3.05), 4.344 (3.18), 7.821 (4.46).

### Intermediate 8

### methyl N-[8-bromo-2-(methanesulfonyl)pyrazolo[1,5-a][1,3,5]triazin-4-yl]glycinate

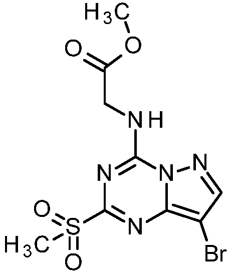

Methyl N-[8-bromo-2-(methylsulfanyl)pyrazolo[1,5-a][1,3,5]triazin-4-yl]glycinate **(Intermediate 7,** 3.18 g, 9.56 mmol) was dissolved in dichloromethane (60 mL), cooled to 0 °C, mCPBA (4.95 g, 70 % purity, 28.7 mmol; CAS-RN:[937-14-4]) was added and the mixture was stirred over weekend at rt. The mixture was diluted with dichlormethane and washed with saturated aqueous sodium bicarbonate solution.The organic layer was dried over sodium sulfate, filtred and concentrated under reduce pressure to give 3.33 g (86 % yield) of the title compound, which was used without further purification.
LC-MS (Method 2): Rₜ = 0.61 min; MS (ESlpos): m/z = 364 [M+H]⁺
¹H-NMR (400 MHz, DMSO-d6) δ [ppm]: 2.084 (0.68), 2.323 (0.42), 2.327 (0.60), 2.332 (0.44), 2.523 (1.92), 2.665 (0.44), 2.669 (0.59), 3.695 (16.00), 4.366 (3.18), 4.381 (3.18), 5.758 (1.65), 8.576 (7.06), 10.062 (0.59), 10.077 (1.20), 10.091 (0.59).

### Intermediate 9

### methyl N-[8-bromo-2-(morpholin-4-yl)pyrazolo[1,5-a][1,3,5]triazin-4-yl]glycinate

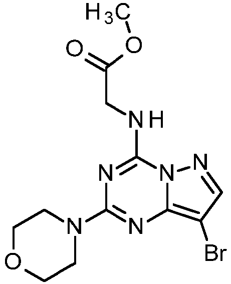

Methyl N-[8-bromo-2-(methanesulfonyl)pyrazolo[1,5-a][1,3,5]triazin-4-yl]glycinate **(Intermediate 8,** 1.00 g, 2.75 mmol) and morpholine (720 µL, 8.2 mmol; CAS-RN:[110-91-8]) were dissolved in acetonitrile (10 mL, 190 mmol; CAS-RN:[75-05-8]). N,N-diisopropylethylamine (1.4 mL, 8.2 mmol; CAS-RN:[7087-68-5]) was added and the mixture was stirred at 70 °C over night. The reaction mixture was poured into water and extracted with dichlormethane. The combined organic layers were dried over sodium sulfat, filtered and evaporated to dryness. The residue was stirred with ethanol and the precipitate was collected by filtration and dried to give 647 mg (55 % yield) of the title compound.
LC-MS (Method 2): Rₜ = 1.01 min; MS (ESlneg): m/z = 371 [M+H]⁺
¹H-NMR (400 MHz, DMSO-d6) δ [ppm]: 2.075 (0.96), 2.518 (1.88), 2.523 (1.22), 3.613 (1.63), 3.623 (3.49), 3.636 (3.26), 3.659 (16.00), 3.677 (2.97), 3.689 (3.10), 3.700 (1.55), 4.170 (2.77), 5.758 (0.68), 8.028 (6.59), 8.977 (0.75).

### Intermediate 10

### N-[8-bromo-2-(morpholin-4-yl)pyrazolo[1,5-a][1,3,5]triazin-4-yl]glycine

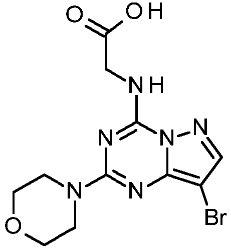

Methyl N-[8-bromo-2-(morpholin-4-yl)pyrazolo[1,5-a][1,3,5]triazin-4-yl]glycinate **(Intermediate 9**, 647 mg, 1.74 mmol) was provided in a mixture of ethanol (8 mL) and tetrahydrofurane (4.0 mL). An aqueous solution of lithium hydroxide (4.4 mL, 1.0 M, 4.4 mmol; CAS-RN:[1310-65-2]) was added and the mixture was stirred for 48 h at rt. The reaction mixture was diluted with water and citric acid (837 mg, 4.36 mmol; CAS-RN:[77-92-9]) and stirred for 15 min. The mixture was concentrated and the precipitate was collected by filtration, washed with water and dried under reduced pressure at 50 °C to give 585 mg (90 % yield) of the title compound.
LC-MS (Method 2): Rₜ = 0.57 min; MS (ESlpos): m/z = 357 [M+H]⁺
¹H-NMR (400 MHz, DMSO-d6) δ [ppm]: 0.951 (10.46), 1.052 (0.76), 2.323 (0.81), 2.327 (1.10), 2.331 (0.85), 2.665 (0.83), 2.669 (1.10), 2.673 (0.83), 3.616 (6.66), 3.627 (12.47), 3.638 (11.31), 3.695 (11.20), 3.707 (12.43), 3.717 (6.79), 4.069 (8.67), 4.084 (8.62), 6.553 (0.83), 7.374 (1.39), 7.379 (1.54), 7.387 (2.82), 7.391 (2.50), 7.671 (1.27), 7.683 (1.18), 7.690 (1.38), 7.695 (1.03), 8.019 (16.00), 8.033 (0.62), 8.786 (1.85), 8.801 (3.84), 8.816 (1.86), 12.840 (0.71).

### Intermediate 11

### 2-{[8-bromo-2-(morpholin-4-yl)pyrazolo[1,5-a][1,3,5]triazin-4-yl]amino}acetohydrazide

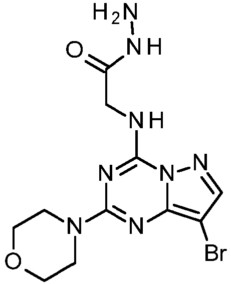

N-[8-bromo-2-(morpholin-4-yl)pyrazolo[1,5-a][1,3,5]triazin-4-yl]glycine **(Intermediate 10,** 1.63 g, 4.56 mmol) was provided in tetrahydrofuran (60 mL), di(1H-imidazol-1-yl)methanone (1.48 g, 9.13 mmol; CAS 530-62-1) was added and the mixture was stirred for 6 h at reflux. A solution of hydrazine in tetrahydrofuran (23 mL, 1.0 M, 23 mmol) was added at room temperature and the mixture was stirred for 24 h at room temperature. The precipitate was collected by filtration, washed with ethanol and water and dried under reduced pressure at 50 °C to give 1.46 g (84 % yield) of the title compound.
LC-MS (Method 1): Rt = 0.76 min; MS (ESlpos): m/z = 371 [M+H]⁺
¹H-NMR (400 MHz, DMSO-d6) δ [ppm]: 1.758 (1.19), 1.774 (0.41), 2.518 (3.54), 2.523 (2.59), 3.582 (0.56), 3.593 (0.48), 3.599 (1.08), 3.606 (0.70), 3.616 (3.57), 3.627 (6.63), 3.638 (5.87), 3.693 (5.53), 3.705 (6.63), 3.715 (3.66), 3.974 (7.92), 4.232 (2.21), 4.345 (0.50), 4.491 (0.59), 7.989 (16.00), 7.994 (1.45), 8.003 (0.43), 8.658 (0.43), 9.199 (1.81).

### Intermediate 12

### {[8-bromo-2-(methylsulfanyl)pyrazolo[1,5-a][1,3,5]triazin-4-yl]amino}acetonitrile

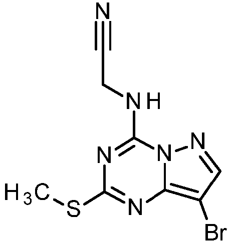

### 8-bromo-4-chloro-2-(methylsulfanyl)pyrazolo[1,5-a][1,3,5]triazine (

**Intermediate 6,** 9.20 g, 32.9 mmol) was provided in n-butanol (200 mL). Aminoacetonitrile hydrogen chloride (1/1) (4.57 g, 49.4 mmol) and N,N-diisopropylethylamine (23.0 mL, 130 mmol; CAS-RN:[7087-68-5])) were added and the mixture was stirred for 2.5 h at 100 °C. The mixture was concentrated, the residue was stirred with water and the precipitate was collected by filtration and dried under reduce pressure at 50 °C to give 9.67 g (88 % yield) of the title compound.
LC-MS (Method 1): Rt = 1.00 min; MS (ESlpos): m/z = 299 [M+H]⁺
¹H-NMR (400 MHz, DMSO-d6) δ [ppm]: 1.238 (0.45), 2.518 (1.20), 2.522 (0.83), 2.561 (16.00), 4.539 (2.34), 8.287 (6.18), 8.300 (1.06), 9.651 (0.55).

### Intermediate 13

### {[8-bromo-2-(methanesulfonyl)pyrazolo[1,5-a][1,3,5]triazin-4-yl]amino}acetonitrile

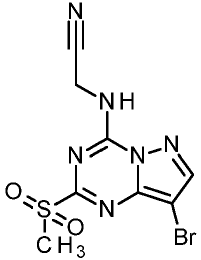

### {[8-bromo-2-(methylsulfanyl)pyrazolo[1,5-a][1,3,5]triazin-4-yl]amino}acetonitrile

**(Intermediate 12,** 722 mg, 2.41 mmol) was dissolved in acetonitrile (50 mL), cooled to 0°C, mCPBA (1.11 g, 75 % purity, 4.83 mmol; CAS-RN:[937-14-4]) was added and the mixture was stirred for 16 h at rt. The mixture was diluted with dichloromethane, the layers were separated and the aqueous phase was extracted 3 times with dichloromethane. The combined organic layers were dried and evaporated to give 1.54 g (193 % yield) of the title compound, which was used without further purification.

LC-MS (Method 1): Rt = 0.75 min; MS (ESlpos): m/z = 331 [M+H]⁺

### Intermediate 14

### {[8-bromo-2-(morpholin-4-yl)pyrazolo[1,5-a][1,3,5]triazin-4-yl]amino}acetonitrile

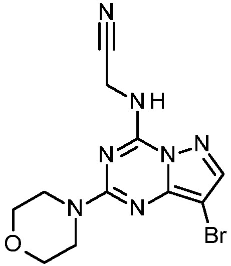

### {[8-bromo-2-(methanesulfonyl)pyrazolo[1,5-a][1,3,5]triazin-4-yl]amino}acetonitrile

**(Intermediate 13,** 3.80 g, 11.5 mmol) and morpholine (3.0 mL, 34 mmol; CAS-RN:[110-91-8]) were provided in acetonitrile (100 mL). N,N-diisopropylethylamine (6.0 mL, 34 mmol; CAS-RN:[7087-68-5]) was added and the mixture was stirred over night at 70 °C. The reaction mixture was poured into water and the precipitate was collected by filtration, washed with water and dried under reduce pressure at 50 °C to give 3.26 g (82 % yield) of the title compound.
LC-MS (Method 2): Rt = 0.95 min; MS (ESlneg): m/z = 338 [M+H]⁺
¹H-NMR (400 MHz, DMSO-d6) δ [ppm]: 2.075 (3.84), 2.518 (3.54), 2.523 (2.39), 3.651 (4.82), 3.662 (8.33), 3.675 (7.03), 3.788 (4.97), 4.505 (15.43), 8.043 (16.00), 8.057 (2.60), 9.215 (1.09).

### Intermediate 15

### {[8-bromo-2-(morpholin-4-yl)pyrazolo[1,5-a][1,3,5]triazin-4-yl]amino}ethanimidamide hydrogen chloride

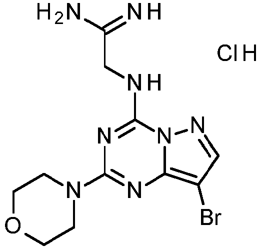

Ammonium chloride (1.11 g, 20.8 mmol) was suspended in toluene (100 mL), trimethylaluminium (10 mL, 2.0 M in toluene, 20.8 mmol) was added and the mixture was stirred for 20 min at room temperature. {[8-bromo-2-(morpholin-4-yl)pyrazolo[1,5-a][1,3,5]triazin-4-yl]amino}acetonitrile **(Intermediate 14,** 2.34 g, 6.92 mmol) was added and the mixture was stirred for 16 h at 80 °C. Ammonium chloride (1.11 g, 20.8 mmol) and trimethylaluminium (10 mL, 2.0 M in toluene, 20.8 mmol) were added and the mixture was stirred for 6 h at 80 °C. After the reaction mixture cooled down, 10 g of silica gel and 20 mL of methanol were added and the mixture was stirred for 1 h at rt. The solids were removed by filtration and washed with methanol. The filtrate was concentrated to give 2.80 g (95 % yield) of the title compound.
LC-MS (Method 1): Rt = 0.65 min; MS (ESlpos): m/z = 355 [M+H]⁺
¹H-NMR (400 MHz, DMSO-d6) δ [ppm]: 2.109 (2.94), 2.331 (0.80), 2.518 (3.71), 2.522 (2.59), 2.669 (1.15), 2.673 (0.82), 3.153 (7.04), 3.166 (7.08), 3.622 (4.14), 3.631 (7.31), 3.644 (6.43), 3.660 (1.28), 3.673 (0.86), 3.695 (1.05), 3.711 (6.57), 3.723 (6.77), 3.734 (3.62), 3.785 (0.43), 4.111 (0.52), 4.125 (1.28), 4.138 (1.22), 4.151 (0.43), 4.413 (10.30), 4.476 (0.76), 4.490 (0.74), 4.509 (1.21), 7.210 (1.13), 7.216 (1.26), 7.228 (1.42), 7.238 (1.42), 7.303 (5.54), 7.313 (3.27), 7.316 (3.46), 7.354 (2.62), 7.955 (0.66), 8.033 (16.00), 8.041 (0.45), 8.045 (1.71), 8.049 (3.25), 8.059 (0.41).

### Intermediate 16

### 4-chloro-8-iodo-2-(methylsulfanyl)pyrazolo[1,5-a][1,3,5]triazine

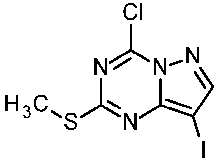

To a mixture of 8-iodo-2-(methylsulfanyl)pyrazolo[1,5-a][1,3,5]triazin-4-ol **(Intermediate** 5, 7.00 g, 22.7 mmol) in phosphorus oxychloride (70 mL) was added N,N-dimethylaniline (8.6 mL, 68 mmol; CAS-RN:[121-69-7]) in one portion. The reaction mixture was stirred at 100 °C for 2 h. The reaction mixture was concentrated under reduced pressure to give a residue. The residue was diluted with water and extracted with ethyl acetate. The organic phase was washed with brine, dried over anhydrous sodium sulfate and filtered. The filtrate was concentrated and purified by column chromatography on silica gel (200-300 mesh, petroleum ether: ethyl acetate = 40: 1) to give 6.00 g (81 % yield) of the title compound as a yellow solid.

### Intermediate 17

### 8-iodo-N,N-bis[(4-methoxyphenyl)methyl]-2-(methylsulfanyl)pyrazolo[1,5-a][1,3,5]triazin-4-amine

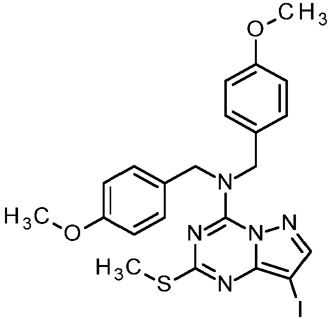

To a mixture of 4-chloro-8-iodo-2-(methylsulfanyl)pyrazolo[1,5-a][1,3,5]triazine **(Intermediate 16,** 3.00 g, 9.19 mmol) and 1-(4-methoxyphenyl)-N-[(4-methoxyphenyl)methyl]methanamine (2.84 g, 11.0 mmol, CAS-RN:[17061-62-0]) in tetrahydrofuran (20 mL) was added N,N-diisopropylethylamine (4.8 mL, 28 mmol; CAS-RN:[7087-68-5]) at rt. The mixture was stirred at rt for 2 h. The reaction mixture was concentrated under reduced pressure to give a residue. The residue was diluted with water and extracted with ethyl acetate. The organic phase was washed with brine, dried with anhydrous sodium sulfate and filtered. The filtrate was concentrated in reduced pressure to give 2.00 g (40 % yield) of the title compound as a white solid.

LC-MS (Method D): Rt = 1.075 min; MS (ESlpos): m/z = 548.1 [M+H]⁺.

### Intermediate 18

### 8-iodo-2-(methanesulfonyl)-N,N-bis[(4-methoxyphenyl)methyl]pyrazolo[1,5-a][1,3,5]triazin-4-amine

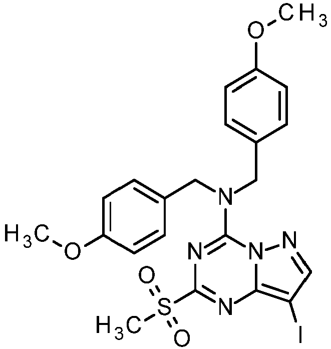

To a solution of 8-iodo-N,N-bis[(4-methoxyphenyl)methyl]-2-(methylsulfanyl)pyrazolo[1,5-a][1,3,5]triazin-4-amine **(Intermediate 17,** 2.00 g, 3.65 mmol) in dichloromethane (15 mL) was added meta-Chloroperoxybenzoic acid (1.89 g, 11.0 mmol; CAS-RN:[937-14-4]) at 0 °C. The reaction mixture was stirred at rt for 12 h. The mixture was diluted with dichloromethane and washed with saturated sodium bicarbonate and brine. The organic phase was dried over anhydrous sodium sulfate, filtered and the filtrate was concentrated to give 3.30 g (crude) of the title compound as a white solid.

LC-MS (Method D): Rt = 1.033min; MS (ESlpos): m/z = 580.3 [M+H]⁺.

### Intermediate 19

### 8-iodo-N,N-bis[(4-methoxyphenyl)methyl]-2-(morpholin-4-yl)pyrazolo[1,5-a][1,3,5]triazin-4-amine

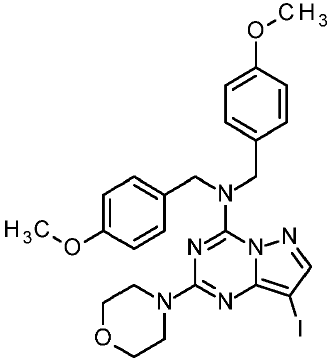

To a solution of 8-iodo-2-(methanesulfonyl)-N,N-bis[(4-methoxyphenyl)methyl]pyrazolo[1,5-a][1,3,5]triazin-4-amine **(Intermediate 18, 3.30** g, 60 % purity, 3.42 mmol) and morpholine (893 mg, 10.3 mmol) in acetonitrile (20 mL) was added N,N-diisopropylethylamine (1.8 mL, 10 mmol; CAS-RN:[7087-68-5]) at rt. The reaction mixture was stirred at 70 °C for 16 h. The reaction mixture was filtered and the filter cake was dried to give 2.40 g (crude) of the title compound as am off-white solid.

LC-MS (Method D): Rt = 1.142min; MS (ESlpos): m/z = 587.3 [M+H]⁺.

### Intermediate 20

### N,N-bis[(4-methoxyphenyl)methyl]-2-(morpholin-4-yl)-8-(trifluoromethyl)pyrazolo[1,5-a][1,3,5]triazin-4-amine

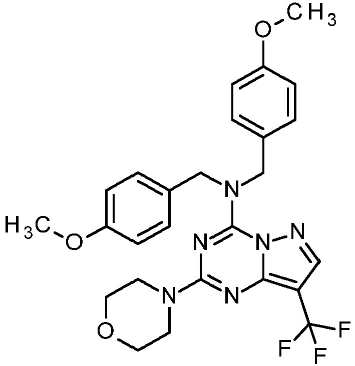

To a solution of 8-iodo-N,N-bis[(4-methoxyphenyl)methyl]-2-(morpholin-4-yl)pyrazolo[1,5-a][1,3,5]triazin-4-amine **(Intermediate 19,** 820 mg, 1.40 mmol) in N,N-dimethylformamide (40 mL) were added methyl difluoro(fluorosulfonyl)acetate (1.07 g, 5.59 mmol) and copper(I) iodide (1.07 g, 5.59 mmol) at rt. The reaction mixture was stirred at 80 °C for 16 h. The reaction mixture was filtered. The filtrate was concentrated and purified by column chromatography on silica gel (200-300 mesh, petroleum ether: ethyl acetate = 50: 1) to give 650 mg (88 % yield) of the title compound as a white solid.

LC-MS (Method D): Rt = 1.139min; MS (ESlpos): m/z = 529.4 [M+H]⁺.

### Intermediate 21

### 2-(morpholin-4-yl)-8-(trifluoromethyl)pyrazolo[1,5-a][1,3,5]triazin-4-amine

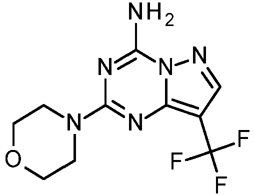

A solution of N,N-bis[(4-methoxyphenyl)methyl]-2-(morpholin-4-yl)-8-(trifluoromethyl)pyrazolo[1,5-a][1,3,5]triazin-4-amine **(Intermediate 20,** 650 mg, 1.23 mmol) in trifluoromethanesulfonic acid (23 mL) was stirred at 70 °C for 16 h. The reaction mixture was poured into ice water and basified to pH = 8 by sodium carbonate. The mixture was extracted with ethyl acetate. The organic phase was washed with brine, dried over anhydrous sodium sulfate and filtered. The filtrate was concentrated and purified by column chromatography on silica gel (200-300 mesh, petroleum ether: ethyl acetate = 5: 1) to give 400 mg (crude) of the title compound as a yellow solid.

LC-MS (Method D): Rt = 0.856min; MS (ESlpos): m/z = 289.5 [M+H]⁺.

### Intermediate 22

### methyl N-[2-(morpholin-4-yl)-8-(trifluoromethyl)pyrazolo[1,5-a][1,3,5]triazin-4-yl]glycinate

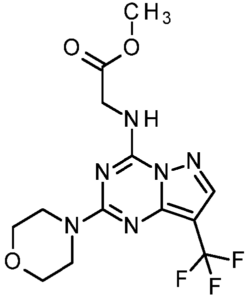

To a solution of 2-(morpholin-4-yl)-8-(trifluoromethyl)pyrazolo[1,5-a][1,3,5]triazin-4-amine **(Intermediate** 21, 900 mg, 3.12 mmol) in 5 mL of DMF was added sodium hydride (74.9 mg, 60 % in mineral oil, 1.87 mmol; CAS-RN:[7646-69-7]) and the mixture was stirred for 20 min at 60 °C. Then methyl chloroacetate (300 µL, 3.44 mmol) was added and the mixture was stirred for 20 hours at room temperature. The reaction mixture was quenched with water and extracted with ethyl acetate. The combined organic layers were washed with brine, filtered over a hydrophobic filter and concentrated. The residue was purified by flash chromatography (silica gel, dichloromethane/ ethanol gradient) and the product was stirred with methyltertbutylether. The precipitate was filtered off and dried to give 383 mg (34 % yield) of the title compound.
LC-MS (Method 2): Rt = 1.15 min; MS (ESlpos): m/z = 361 [M+H]⁺
¹H-NMR (400 MHz, DMSO-d6) δ [ppm]: 3.618 (2.08), 3.628 (4.14), 3.639 (3.58), 3.664 (16.00), 3.695 (3.32), 3.706 (3.73), 4.184 (4.60), 8.252 (4.23), 9.131 (1.34).

### Intermediate 23

### N-[2-(morpholin-4-yl)-8-(trifluoromethyl)pyrazolo[1,5-a][1,3,5]triazin-4-yl]glycine

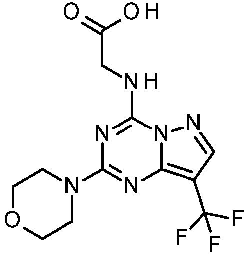

To a solution of methyl N-[2-(morpholin-4-yl)-8-(trifluoromethyl)pyrazolo[1,5-a][1,3,5]triazin-4-yl]glycinate **(Intermediate 22,** 380 mg, 1.05 mmol) in 5.0 mL of tetrahydrofuran and 2.0 mL of ethanol an aqueous solution of lithium hydroxide (2.6 mL, 1.0 M, 2.60 mmol; CAS-RN:[1310-65-2]) was added. The mixture was stirred for 72 hours at room temperature. Water was added and the mixture was acidified with an aqueous solution of citric acid (10%) till pH 3-4 was achieved. The precipitate was filtered off, washed with water and dried to give 319 mg (79 % yield) of the title compound.
LC-MS (Method 2): Rₜ = 0.63 min; MS (ESlpos): m/z = 347 [M+H]⁺
¹H-NMR (400 MHz, DMSO-d6) δ [ppm]: 1.102 (14.43), 1.154 (0.44), 1.166 (0.41), 1.171 (0.89), 1.189 (0.46), 1.230 (0.48), 1.250 (0.43), 1.268 (0.87), 1.907 (0.43), 1.987 (1.15), 2.322 (0.74), 2.327 (1.02), 2.332 (0.78), 2.522 (3.88), 2.664 (0.78), 2.669 (1.07), 2.673 (0.80), 3.072 (4.57), 3.620 (10.43), 3.630 (15.37), 3.642 (14.34), 3.711 (14.32), 3.724 (16.00), 3.734 (8.88), 4.084 (10.40), 4.099 (10.43), 5.757 (1.02), 8.173 (3.03), 8.184 (0.70), 8.221 (0.76), 8.243 (15.02), 8.948 (2.13), 8.963 (4.66), 8.978 (2.18), 12.860 (0.52).

### Intermediate 24

### 2-{[2-(morpholin-4-yl)-8-(trifluoromethyl)pyrazolo[1,5-a][1,3,5]triazin-4-yl]amino}acetohydrazide

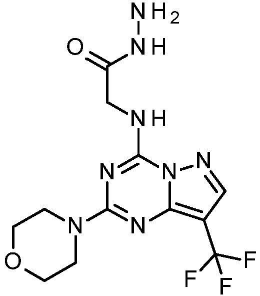

### N-[2-(morpholin-4-yl)-8-(trifluoromethyl)pyrazolo[1,5-a][1,3,5]triazin-4-yl]glycine

**(Intermediate** 23, 315 mg, 819 µmol) was dissolved in 8.0 mL of THF, di-1H-imidazol-1-ylmethanone (266 mg, 1.64 mmol; CAS-RN:[530-62-1]) was added and the reaction mixture was stirred under reflux for 6 h. The solution was cooled to rt and hydrazine in THF (4.1 mL, 1.0 M, 4.1 mmol) was added dropwise. The reaction mixture was stirred for 18 h at room temperature. Water was added and the mixture was extracted with ethyl acetate. The combined organic layers were washed with brine, filtered and concentrated. The residue was stirred in methyltertbutylether, the precipitate was filtered off, washed with methyltertbutylether and dried to give 244 mg of the title compound (66 % yield).
LC-MS (Method 2): Rt = 0.90 min; MS (ESlpos): m/z = 361 [M+H]⁺
¹H-NMR (400 MHz, DMSO-d6) δ [ppm]: 1.154 (2.21), 1.172 (4.53), 1.189 (2.21), 1.266 (0.40), 1.757 (0.65), 1.987 (7.98), 2.322 (0.88), 2.326 (1.20), 2.331 (0.88), 2.518 (7.94), 2.522 (5.37), 2.664 (0.95), 2.668 (1.26), 2.673 (0.91), 3.599 (0.99), 3.620 (7.83), 3.629 (15.47), 3.642 (14.97), 3.707 (13.09), 3.719 (15.12), 3.730 (8.97), 3.986 (11.87), 4.016 (2.00), 4.034 (1.77), 4.052 (0.55), 4.240 (5.03), 4.357 (0.67), 4.499 (1.24), 7.633 (0.42), 8.141 (0.46), 8.174 (0.88), 8.194 (0.51), 8.209 (16.00), 8.217 (1.94), 8.669 (0.63), 8.811 (1.71), 9.204 (4.74).

### Intermediate 25

### ethyl 1-amino-1H-imidazole-2-carboxylate

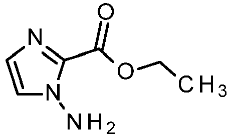

Ethyl 1H-imidazole-2-carboxylate (100 g, 678 mmol) was dissolved in N,N-dimethylformamide (1500 mL), cooled to 0 °C and Lithium bis(trimethylsilyl)amide (810 mL, 1.0 M, 810 mmol; CAS-RN:[4039-32-1]) was added dropwise. The mixture was stirred for 10 min and O-(Diphenylphosphinoyl)hydroxylamine (213 g, 915 mmol; CAS-RN:[72804-96-7]) was added. The reaction mixture was stirred for 1 h at room temperature. The mixture was cooled with an ice-bath, diluted with water and extracted with ethyl acetate and dichlormethane. The combined organic layers were dried, filtered and evaporated. The aqueous layer was extracted further 3 times with ethyl acetate. The combined organic layers were dried and concentrated under reduced pressure to give 92.2 g (88 % yield) of the title compound, which was used without further purification.
LC-MS (Method 1): Rt = 0.53 min; MS (ESlpos): m/z = 156 [M+H]⁺
¹H-NMR (400 MHz, DMSO-d6) δ [ppm]: 1.285 (7.37), 1.303 (16.00), 1.320 (7.52), 2.728 (5.51), 2.888 (6.51), 3.823 (0.91), 4.270 (2.20), 4.288 (6.95), 4.305 (6.91), 4.323 (2.14), 6.575 (5.16), 6.966 (4.72), 6.968 (4.95), 7.375 (4.92), 7.377 (4.90), 7.951 (0.84).

### Intermediate 26

### ethyl 1-[(benzoylcarbamothioyl)amino]-1H-imidazole-2-carboxylate

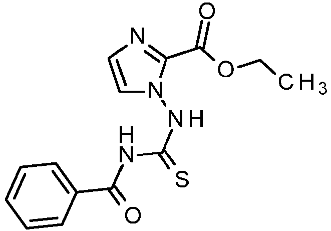

To ethyl 1-amino-1H-imidazole-2-carboxylate **(Intermediate 25,** 92.0 g, 593 mmol) in tetrahydrofuran (2 L) was added a solution of benzoyl isothiocyanate (80 mL, 590 mmol; CAS-RN:[532-55-8]) in tetrahydrofuran (2.5 L) under cooling. The reaction mixture was stirred for 1 h at rt and concentrated under reduce pressure. The crude product was purified by flash chromatography (silica gel, hexane / ethyl acetate gradient) to give 162 g (86 % yield) of the title compound as an orange solid.
LC-MS (Method 2): Rt = 0.50 min; MS (ESlpos): m/z = 319 [M+H]⁺
¹H-NMR (400 MHz, DMSO-d6) δ [ppm]: 1.202 (7.05), 1.220 (16.00), 1.238 (7.34), 3.772 (0.72), 4.194 (2.17), 4.211 (7.40), 4.229 (7.32), 4.247 (2.10), 7.151 (6.55), 7.154 (6.82), 7.541 (2.55), 7.546 (1.00), 7.559 (5.33), 7.573 (8.93), 7.576 (9.95), 7.580 (4.06), 7.664 (1.11), 7.667 (2.08), 7.670 (1.22), 7.682 (0.96), 7.686 (2.78), 7.691 (0.83), 7.701 (0.69), 7.705 (1.15), 7.708 (0.60), 7.975 (3.96), 7.979 (5.11), 7.983 (1.36), 7.996 (4.49), 7.999 (3.66), 12.072 (0.85), 13.041 (0.87).

### Intermediate 27

### 2-sulfanylimidazo[2,1-f][1,2,4]triazin-4-ol

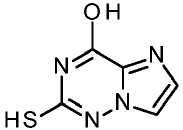

Ethyl 1-[(benzoylcarbamothioyl)amino]-1H-imidazole-2-carboxylate (**Intermediate 26,** 110 g, 346 mmol) was provided in an aqueous solution of NaOH (860 mL, 1.0 M, 860 mmol). The mixture was stirred for 2 h at 85 °C. The reaction mixture was diluted with ethanol (280 mL), cooled to 0 °C and acetic acid (79 mL) was added. The suspension was stirred for 30 min at 0 °C. The precipitate was collected by filtration, washed with water and cold ethanol, dried under reduced pressure at 40 °C to give 53.2 g (92 % yield) of the title compound as a white solid.

¹H-NMR (400 MHz, DMSO-d6) δ [ppm]: 1.906 (0.65), 7.543 (12.57), 7.546 (13.17), 7.679 (15.73), 7.682 (16.00), 11.376 (0.88).

### Intermediate 28

### 2-(methylsulfanyl)imidazo[2,1-f][1,2,4]triazin-4-ol

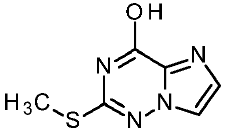

2-sulfanylimidazo[2,1-f][1,2,4]triazin-4-ol **(Intermediate 27,** 53.2 g, 316 mmol) was suspended in tetrahydrofuran (800 mL). lodomethane (26 mL, 410 mmol; CAS-RN:[74-88-4]) was added and the mixture was stirred for 2 h at 45 °C. The reaction mixture was concentrated to give 55.9 g (97 % yield) of the title compound, which was used without further purification.
LC-MS (Method 1): Rt = 0.60 min; MS (ESlpos): m/z = 183 [M+H]⁺
¹H-NMR (400 MHz, DMSO-d6) δ [ppm]: 2.548 (16.00), 3.722 (0.67), 7.594 (2.71), 7.597 (2.74), 8.020 (3.15), 8.023 (3.15).

### Intermediate 29

### 4-chloro-2-(methylsulfanyl)imidazo[2,1-f][1,2,4]triazine

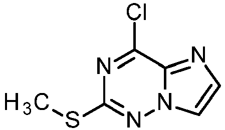

2-(methylsulfanyl)imidazo[2,1-f][1,2,4]triazin-4-ol **(Intermediate 28,** 18.0 g, 98.8 mmol) was provided in toluene (340 mL). N,N-diisopropylethylamine (22 mL, 130 mmol; CAS-RN:[7087-68-5]) and phosphoroxychloride (28 mL, 300 mmol; CAS-RN:[10025-87-3]) were added and the mixture was stirred for 2 h at 120 °C. The reaction mixture was concentrated. The residue was dissolved in ethyl acetat, poured into a solution of sodium bicarbonate and stirred for 15 min. The phases were separated and the aqueous phase was extracted with ethyl acetate. The combined organic layers were dried, filtered and concentrated. The residue was purified by flash chromatography (silica gel, hexane / ethyl acetate gradient) to give 17.7 g (89 % yield) of the title compound.

¹H-NMR (400 MHz, CHLOROFORM-d) δ [ppm]: 2.609 (16.00), 7.839 (2.25), 7.841 (2.36), 7.960 (2.80), 7.963 (2.73).

### Intermediate 30

### 2,4-bis(methylsulfanyl)imidazo[2,1-f][1,2,4]triazine

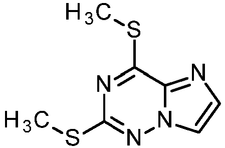

4-chloro-2-(methylsulfanyl)imidazo[2,1-f][1,2,4]triazine **(Intermediate 29,** 17.7 g, 88.2 mmol) was dissolved in tetrahydrofuran (400 mL). Sodium methanethiolate (9.76 g, 95 % purity, 132 mmol; CAS-RN:[5188-07-8]) was added and the mixture was stirred for 1 h at room temperature. The reaction mixture was poured into water and extracted with ethyl acetate. The combined organic layers were washed, dried, filtered and concentrated to give 18.6 g (99 % yield) of the title compound.
LC-MS (Methode 1): Rₜ = 1.05 min; MS (ESlpos): m/z = 213 [M+H]⁺
¹H-NMR (400 MHz, CHLOROFORM-d) δ [ppm]: 2.591 (16.00), 2.679 (14.38), 7.624 (2.41), 7.627 (2.43), 7.765 (2.80), 7.767 (2.87).

### Intermediate 31

### 7-iodo-2,4-bis(methylsulfanyl)imidazo[2,1-f][1,2,4]triazine

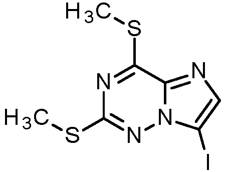

2,4-bis(methylsulfanyl)imidazo[2,1-f][1,2,4]triazine **(Intermediate** 30, 150 mg, 707 µmol) was dissolved in N,N-dimethylformamide (3 mL). N-lodsuccinimid (397 mg, 1.77 mmol; CAS-RN:[516-12-1]) was added and the mixture was stirred over night at 85 °C. The reaction mixture was poured into ice water and stirred for 30 min. The precipitate was collected by filtration and dried under reduce pressure at 40 °C to give 216 mg (90 % yield) of the title compound
LC-MS (Method 1): Rt = 1.30 min; MS (ESlpos): m/z = 339 [M+H]⁺
¹H-NMR (400 MHz, DMSO-d6) δ [ppm]: 2.619 (15.41), 2.637 (16.00), 2.646 (0.47), 7.814 (7.28).

### Intermediate 32

### 2,4-bis(methylsulfanyl)-7-(trifluoromethyl)imidazo[2,1-f][1,2,4]triazine

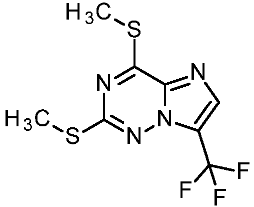

To a suspension of 7-iodo-2,4-bis(methylsulfanyl)imidazo[2,1-f][1,2,4]triazine **(Intermediate 31,** 2.93 g, 8.68 mmol) and Copper(I) iodide (4.96 g, 26.0 mmol; CAS-RN:[7681-65-4]) in N,N-dimethylformamide (55 mL) was added at 110 °C methyl difluoro(fluorosulfonyl)acetate (3.3 mL, 26 mmol; CAS-RN:[680-15-9]). The mixture was stirred for 75 min at 110 °C. The reaction mixture was concentrated. The residue was purified by flash chromatography (silica gel, dichlormethane / ethyl acetate gradient) to give 2.22 g (83 % yield) of the title compound.
LC-MS (Method 2): Rt = 1.31 min; MS (ESlpos): m/z = 281 [M+H]⁺
¹H-NMR (400 MHz, DMSO-d6) δ [ppm]: 2.518 (0.71), 2.523 (0.47), 2.580 (15.12), 2.668 (16.00), 2.876 (2.08), 3.173 (2.02), 8.221 (2.12), 8.224 (2.25).

### Intermediate 33

### 2-(methanesulfonyl)-4-(methylsulfanyl)-7-(trifluoromethyl)imidazo[2,1-f][1,2,4]triazine

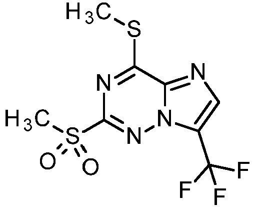

2,4-bis(methylsulfanyl)-7-(trifluoromethyl)imidazo[2,1-f][1,2,4]triazine **(Intermediate 32,** 568 mg, 2.03 mmol) was provided in dichlormethane, cooled with a water bath, mCPBA (2.27 g, 77 % purity, 10.1 mmol; CAS-RN:[937-14-4]) was added in 45 min and the mixture was stirred for 3 h at rt. The reaction mixture was diluted with dichlormethane, stirred with sodium thiosulfate and filtered over a hydrophobic filter. The organic phase was purified by flash chromatography (silica gel, dichloromethane/ ethanol gradient). The product was stirred in ethanol and the precipitate was filtered off. The filtrate was concentrated and the residue was diluted with water and 1 % acetonitrile. The precipitate was collected by filtration, washed with water and dried to give 108 mg (15 % yield) of the title compound.

LC-MS (Method 2): Rt = 0.97 min; MS (ESlpos): m/z = 313 [M+H]⁺

### Intermediate 34

### tert-butyl (2-amino-2-iminoethyl)carbamate hydrogen chloride

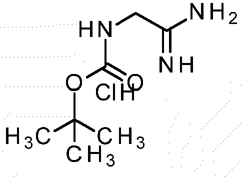

To a solution of tert-butyl (cyanomethyl)carbamate (25.0 g, 160 mmol) in methanol (300 mL) was added a suspension of sodium methoxide (1.73 g, 32.0 mmol; CAS-RN:[124-41-4]) in methanol (50 mL) at 50 °C. The reaction mixture was stirred at 50 °C for 12 h. Ammounium chloride (11.1 g, 208 mmol) was added to the reaction mixture at 50 °C. The reaction mixture was stirred at 50 °C for 16 hours. The reaction mixture was concentrated and diluted with ethyl acetate. The suspension was concentrated and diluted with ethyl acetate. The suspension was filtered and the solid was dried under reduced pressure to give 20.0 g (95 % purity, 57 % yield) of the title compound as a white solid.

### Intermediate 35

### tert-butyl ({5-[4-(trifluoromethyl)phenyl]-1H-imidazol-2-yl}methyl)carbamate

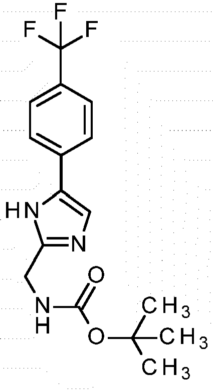

tert-butyl (2-amino-2-iminoethyl)carbamate hydrogen chloride **(Intermediate 34,** 750 mg, 3.58 mmol) and 2-bromo-1-[4-(trifluoromethyl)phenyl]ethan-1-one (955 mg, 3.58 mmol; CAS-RN:[383-53-9]) were provided in acetonitrile (22 mL). Potassium carbonate (1.98 g, 14.3 mmol; CAS-RN:[584-08-7]) was added and the mixture was stirred for 1 h at 50 °C. The reaction mixture was concentrated and the residue was purified by flash chromatography (silica gel, dichlormethane / ethanol gradient) to give 219 mg (18 % yield) of the title compound.
LC-MS (Method 2): Rt = 1.17 min; MS (ESlpos): m/z = 342 [M+H]⁺
¹H-NMR (400 MHz, DMSO-d6) δ [ppm]: 1.311 (0.63), 1.322 (0.60), 1.353 (0.66), 1.401 (16.00), 2.518 (2.20), 2.523 (1.45), 2.674 (0.43), 4.178 (1.90), 4.193 (1.92), 7.313 (0.62), 7.652 (2.02), 7.673 (2.38), 7.692 (1.83), 7.697 (1.83), 7.830 (0.47), 7.850 (0.62), 7.936 (2.22), 7.957 (1.88), 8.049 (0.50), 8.070 (0.43), 12.045 (0.59).

### Intermediate 36

### 1-{5-[4-(trifluoromethyl)phenyl]-1H-imidazol-2-yl}methanamine

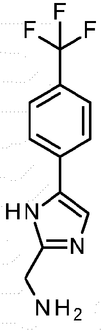

tert-butyl ({5-[4-(trifluoromethyl)phenyl]-1H-imidazol-2-yl}methyl)carbamate **(Intermediate 35,** 218 mg, 639 µmol) was provided in methanol (2.6 mL). A solution of HCl in dioxane (1.6 mL, 4.0 M, 6.4 mmol; CAS-RN:[7647-01-0]) was added and the mixture was stirred at rt over night. The reaction mixture was diluted with water and adjusted to pH 8-9 with a saturated aqueous solution of sodium bicarbonate and then extracted with ethyl acetate. The combined organic layers were filtered over a hydrophobic filter and concentrated to give 64.0 mg (42 % yield) of the title compound.
LC-MS (Method 2): Rₜ = 0.91 min; MS (ESlpos): m/z = 242 [M+H]⁺
¹H-NMR (400 MHz, CHLOROFORM-d) δ [ppm]: 1.172 (0.57), 1.183 (1.64), 1.189 (1.07), 1.208 (0.48), 1.977 (0.84), 3.636 (16.00), 3.890 (2.26), 4.014 (7.77), 7.265 (5.98), 7.522 (3.58), 7.543 (4.36), 7.647 (0.85), 7.667 (0.94), 7.732 (3.45), 7.752 (2.85), 7.848 (0.78), 7.868 (0.65).

### Intermediate 37

### tert-butyl ({5-[4-(trifluoromethoxy)phenyl]-1H-imidazol-2-yl}methyl)carbamate

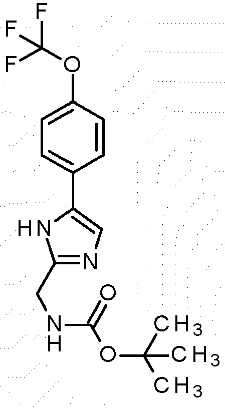

tert-butyl (2-amino-2-iminoethyl)carbamate hydrogen chloride (**Intermediate 34**, 500 mg, 2.38 mmol) and 2-bromo-1-[4-(trifluoromethoxy)phenyl]ethan-1-one (675 mg, 2.38 mmol; CAS-RN:[103962-10-3]) were provided in acetonitrile (15 mL). Potassium carbonate (1.32 g, 9.54 mmol; CAS-RN:[584-08-7]) was added and the mixture was stirred for 90 min at 100 °C. The reaction mixture was concentrated and the residue was purified by flash chromatography (silica gel, dichlormethane / ethanol gradient). The product was stirred in methyl-tert-butylether. The precipitate was collected by filtration and dried to give 44.0 mg (5 % yield) of the title compound.
LC-MS (Method 2): Rt = 1.21 min; MS (ESlpos): m/z = 358 [M+H]⁺
¹H-NMR (400 MHz, DMSO-d6) δ [ppm]: 1.104 (0.52), 1.233 (0.46), 1.400 (16.00), 2.518 (2.92), 2.523 (1.95), 4.164 (2.04), 4.178 (2.08), 7.295 (2.58), 7.315 (2.37), 7.556 (1.93), 7.821 (0.46), 7.828 (3.92), 7.833 (1.20), 7.845 (1.14), 7.850 (3.48), 11.934 (0.64).

### Intermediate 38

### 1-{5-[4-(trifluoromethoxy)phenyl]-1H-imidazol-2-yl}methanamine

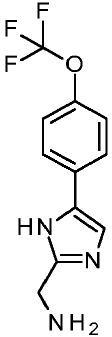

### First batch

tert-butyl ({5-[4-(trifluoromethoxy)phenyl]-1H-imidazol-2-yl}methyl)carbamate **(Intermediate 37,** 218.0 mg, 0.6 mmol) was provided in methanol (2.4 mL). HCl in dioxane (1.5 mL, 4.0 M, 61.0 mmol; CAS-RN:[7647-01-0]) was added and the mixture was stirred over night at rt. The reaction mixture was diluted with water, basified by an aqueous solution of saturated sodium bicarbonate and extracted with ethyl actetate. The combined organic layers were dried over a hydrophobic filter and concentrated.

### Second batch

tert-butyl ({5-[4-(trifluoromethoxy)phenyl]-1H-imidazol-2-yl}methyl)carbamate **(Intermediate 37**, 60.0 mg, 168 µmol) was provided in methanol (680 µL). HCl in dioxane (420 µL, 4.0 M, 1.7 mmol; CAS-RN:[7647-01-0]) was added and the mixture was stirred over night at rt. The reaction mixture was concentrated.

The first and the second batch were combined and purified by flash chromatography (silica gel, dichlormethane / ethyl acetate gradient) to give 142 mg of the title compound.
LC-MS (Method 2): Rₜ = 0.94 min; MS (ESlpos): m/z = 258 [M+H]⁺
¹H-NMR (400 MHz, DMSO-d6) δ [ppm]: 0.798 (0.47), 0.814 (0.96), 0.821 (0.63), 0.830 (0.70), 0.840 (0.47), 0.846 (0.72), 0.852 (0.95), 0.868 (0.80), 0.892 (0.84), 0.901 (0.43), 0.904 (0.61), 0.910 (0.44), 1.005 (0.47), 1.024 (1.02), 1.044 (1.06), 1.052 (0.66), 1.064 (0.46), 1.232 (1.20), 1.895 (2.45), 1.917 (0.64), 2.336 (0.71), 2.518 (9.20), 2.523 (6.57), 2.678 (0.69), 3.385 (0.50), 3.763 (16.00), 3.828 (1.97), 4.496 (0.69), 7.298 (2.89), 7.318 (2.80), 7.466 (0.62), 7.486 (0.67), 7.564 (1.23), 7.593 (0.57), 7.647 (1.19), 7.812 (1.91), 7.822 (2.45), 7.834 (2.62), 7.842 (2.31).

### Intermediate 39

### 2-(methanesulfonyl)-N-({5-[4-(trifluoromethoxy)phenyl]-1H-imidazol-2-yl}methyl)-7-(trifluoromethyl)imidazo[2,1-f][1,2,4]triazin-4-amine

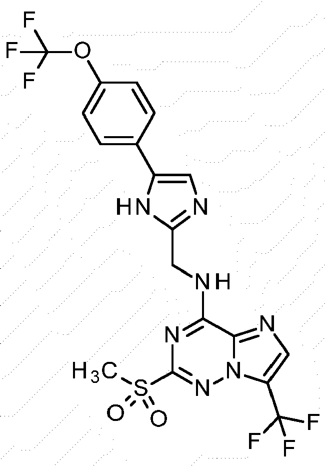

### 2-(methanesulfonyl)-4-(methylsulfanyl)-7-(trifluoromethyl)imidazo[2,1-f][1,2,4]triazine

**(Intermediate 33,** 96.6 mg, 247 µmol) and 1-{5-[4-(trifluoromethoxy)phenyl]-1H-imidazol-2-yl}methanamine **(Intermediate 38,** 70.0 mg, 272 µmol) were provided in acetonitrile (4.6 mL). N,N-diisopropylethylamine (170 µL, 990 µmol; CAS-RN:[7087-68-5]) was added and the mixture was stirred for 90 min at 50 °C. The reaction mixture was concentrated under reduce pressure. The residue was purified by flash chromatography (silica gel, dichlormethane / ethyl acetate gradient) to give 34.0 mg (26 % yield) of the title compound.
LC-MS (Method 2): Rₜ = 1.16 min; MS (ESlpos): m/z = 522 [M+H]⁺
¹H-NMR (400 MHz, DMSO-d6) δ [ppm]: 1.153 (0.44), 1.171 (0.85), 1.189 (0.41), 1.986 (1.49), 2.518 (1.94), 2.523 (1.29), 3.332 (16.00), 4.890 (3.00), 7.304 (0.99), 7.325 (1.09), 7.642 (0.93), 7.836 (1.16), 7.858 (1.03), 8.347 (1.94), 8.350 (2.07), 11.993 (0.43).

### Intermediate 40

### 4-chloro-8-iodo-2-(methylsulfanyl)pyrazolo[1,5-a][1,3,5]triazine

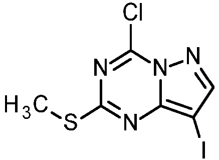

To a mixture of 8-iodo-2-(methylsulfanyl)pyrazolo[1,5-a][1,3,5]triazin-4-ol **(Intermediate 5, 7.00** g, 22.7 mmol) in phosphorus oxychloride (70 mL) was added N,N-dimethylaniline (8.6 mL, 68 mmol; CAS-RN:[121-69-7]) in one portion. The reaction mixture was stirred at 100 °C for 2 h. The reaction mixture was concentrated under reduced pressure to give a residue. The residue was diluted with water and extracted with ethyl acetate. The organic phase was washed with brine, dried over anhydrous sodium sulfate and filtered. The filtrate was concentrated and purified by column chromatography on silica gel (200-300 mesh, petroleum ether: ethyl acetate = 40: 1) to give 6.00 g (81 % yield) of the title compound as a yellow solid.

### Intermediate 41

### 8-iodo-2,4-bis(methylsulfanyl)pyrazolo[1,5-a][1,3,5]triazine

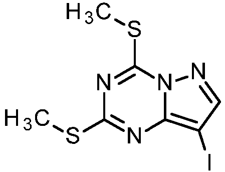

To a solution of 4-chloro-8-iodo-2-(methylsulfanyl)pyrazolo[1,5-a][1,3,5]triazine **(Intermediate 40,** 9.98 g, 30.6 mmol) in tetrahydrofuran (200 mL) was added sodium methanethiolate (2.62 g, 90 % purity, 33.6 mmol; CAS-RN:[5188-07-8]). The mixture was stirred for 16 h at room temperature. Sodium methanethiolate (952 mg, 90 % purity, 12.2 mmol; CAS-RN:[5188-07-8]) was added and the mixture was stirred for 3 days at room temperature. The mixture was concentrated and water was added. The precipitate was isolated by filtration to give 7.01 g (66 % yield) of the title compound.
LC-MS (Method 1): Rt = 1.33 min; MS (ESlpos): m/z = 339 [M+H]⁺
¹H-NMR (400 MHz, DMSO-d6) δ [ppm]: 2.518 (1.43), 2.523 (0.97), 2.603 (16.00), 2.668 (15.72), 8.307 (6.49).

### Intermediate 42

### 2,4-bis(methylsulfanyl)-8-(trifluoromethyl)pyrazolo[1,5-a][1,3,5]triazine

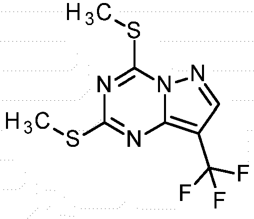

To a suspension of 8-iodo-2,4-bis(methylsulfanyl)pyrazolo[1,5-a][1,3,5]triazine **(Intermediate 41,** 4.65 g, 13.8 mmol) and copper(I) iodide (10.6 g, 55.0 mmol; CAS-RN:[7681-65-4]) in N,N-dimethylformamide (87 mL) was added methyl difluoro(fluorosulfonyl)acetate (7.0 mL, 55.0 mmol; CAS-RN:[680-15-9]) at rt under an argon atmosphere. The reaction mixture was stirred at 80 °C for 20 h. Methyl difluoro(fluorosulfonyl)acetate (3.5 mL, 22.5 mmol; CAS-RN:[680-15-9]) was added and the mixture was stirred for 24 h at 80 °C and 3 days at room temperature. The reaction mixture was concentrated and the residue was purified by column chromatography (silica gel, hexane/ dichloromethane/ ethyl acetate gradient) to give 2.85 g (72 % yield) of the title compound.
LC-MS (Method 2): Rt = 1.35 min; MS (ESlpos): m/z = 281 [M+H]⁺
¹H-NMR (400 MHz, DMSO-d6) δ [ppm]: 2.518 (0.74), 2.523 (0.50), 2.603 (16.00), 2.693 (15.54), 8.622 (2.65).

### Intermediate 43

### benzyl ({5-[4-(trifluoromethyl)phenyl]-1H-imidazol-2-yl}methyl)carbamate

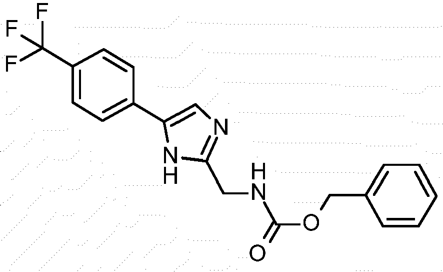

N-[(benzyloxy)carbonyl]glycine (1.00 g, 4.78 mmol) and caesiumcarbonat (779 mg, 2.39 mmol; CAS-RN:[534-17-8]) were provided in N-methyl-pyrrolidinone (10 mL) and stirred for 1 h at rt. 2-Bromo-1-[4-(trifluoromethyl)phenyl]ethan-1-one (1.28 g, 4.78 mmol) was added and the mixture was stirred for 30 min at rt. Afterwards xylene (30 mL) and ammonium acetate (9.21 g, 120 mmol; CAS-RN:[631-61-8]) were added and the mixture was stirred for 9 h at 120 °C. The reaction mixture was diluted with ethyl acetate and washed with a saturated aqueous solution of sodium carbonate. The organic phase was dried over anhydrous sodium sulfate, filtered and concentrated to give a crude product. The residue was stirred in dichlormethane. The precipitate was collected by filtration and dried to give 284 mg (16 % yield) of the title compound.
LC-MS (Method 1): Rt = 1.00 min; MS (ESlneg): m/z = 374 [M-H]⁻
¹H-NMR (400 MHz, DMSO-d6) δ [ppm]: 2.518 (4.78), 2.523 (3.22), 4.269 (6.00), 4.283 (6.27), 5.056 (2.04), 5.065 (16.00), 7.257 (0.63), 7.304 (0.88), 7.315 (1.56), 7.326 (1.78), 7.336 (1.43), 7.345 (1.28), 7.366 (11.50), 7.377 (8.92), 7.465 (0.42), 7.656 (4.85), 7.677 (5.70), 7.713 (4.36), 7.719 (4.51), 7.731 (0.63), 7.752 (0.68), 7.792 (0.98), 7.806 (1.89), 7.820 (0.98), 7.848 (0.52), 7.941 (4.98), 7.962 (4.33), 12.148 (1.48).

### Intermediate 44

### 1-{5-[4-(trifluoromethyl)phenyl]-1H-imidazol-2-yl}methanamine hydrogen bromide

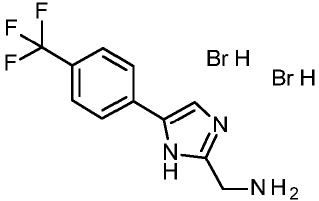

benzyl ({5-[4-(trifluoromethyl)phenyl]-1H-imidazol-2-yl}methyl)carbamate **(Intermediate 43,** 284 mg, 757 µmol) was stirred in a solution of hydrogen bromide (32 % purity; CAS-RN:[10035-10-6]) in acetic acid over night at rt. The reaction mixture was concentrated, toluene was added and the mixture was concentrated. The residue was stirred in toluene and the precipitate was collected by filtration and dried under reduce pressure at 50 °C to give 277 mg (91 % yield) of the title compound.
LC-MS (Method 1): Rₜ = 0.76 min; MS (ESlpos): m/z = 242 [M+H]⁺
¹H-NMR (400 MHz, DMSO-d6) δ [ppm]: 1.905 (0.52), 2.295 (1.25), 2.336 (0.74), 2.518 (11.19), 2.522 (7.52), 2.678 (0.77), 4.040 (3.45), 4.177 (8.60), 4.705 (0.40), 7.770 (10.82), 7.790 (13.15), 7.966 (16.00), 7.991 (14.25), 8.011 (11.50), 8.411 (4.83).

### Intermediate 45

### 2-(methylsulfanyl)-8-(trifluoromethyl)-N-({5-[4-(trifluoromethyl)phenyl]-1H-imidazol-2-yl}methyl)pyrazolo[1,5-a][1,3,5]triazin-4-amine

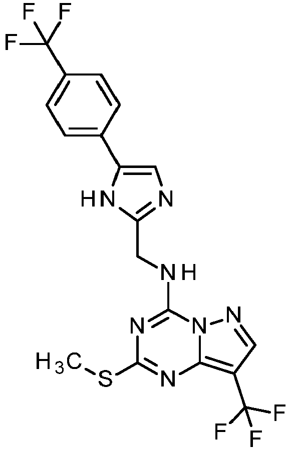

2,4-bis(methylsulfanyl)-8-(trifluoromethyl)pyrazolo[1,5-a][1,3,5]triazine **(Intermediate 42,** 159 mg, 569 µmol) was dissolved in acetonitrile (5 mL). 1-{5-[4-(trifluoromethyl)phenyl]-1H-imidazol-2-yl}methanamine hydrogen bromide **(Intermediate 44,** 275 mg, 682 µmol) and N,N-diisopropylethylamine (400 µL, 2.3 mmol; CAS-RN:[7087-68-5]) were added and the mixture was stirred for 3 h at reflux. 2,4-bis(methylsulfanyl)-8-(trifluoromethyl)pyrazolo[1,5-a][1,3,5]triazine **(Intermediate 42,** 20 mg, 71 µmol) was added and the mixture was stirred for 3 h at reflux. The reaction mixture was diluted with water and extracted with ethyl acetate. The combined organic layers were concentrated to give a crude product. The residue was purified by column chromatography (silica gel, dichloromethane/ ethanol gradient) to give 310 mg of the title compound.
LC-MS (Method 2): Rt = 1.41 min; MS (ESlpos): m/z = 474 [M+H]⁺
¹H-NMR (400 MHz, DMSO-d6) δ [ppm]: 0.884 (0.41), 0.902 (0.82), 1.035 (1.06), 1.052 (2.55), 1.070 (1.35), 2.065 (1.12), 2.084 (1.40), 2.454 (0.72), 2.469 (16.00), 2.518 (2.00), 2.523 (1.28), 4.810 (6.26), 5.758 (0.40), 7.665 (1.69), 7.685 (1.97), 7.765 (1.54), 7.941 (1.68), 7.961 (1.42), 8.505 (4.32), 12.128 (0.44).

### Intermediate 46

### 2-(methanesulfonyl)-8-(trifluoromethyl)-N-({5-[4-(trifluoromethyl)phenyl]-1H-imidazol-2-yl}methyl)pyrazolo[1,5-a][1,3,5]triazin-4-amine

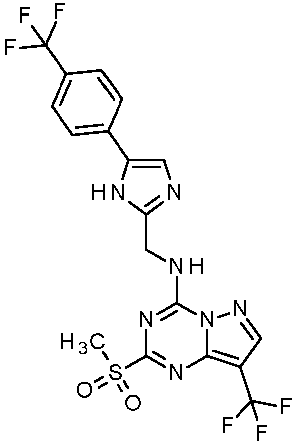

2-(methylsulfanyl)-8-(trifluoromethyl)-N-({5-[4-(trifluoromethyl)phenyl]-1H-imidazol-2-yl}methyl)pyrazolo[1,5-a][1,3,5]triazin-4-amine **(Intermediate 45,** 300 mg, 634 µmol) was provided in dichlormethane (5 mL), cooled to 2 °C, mCPBA (426 mg, 77 % purity, 1.90 mmol; CAS-RN:[937-14-4]) was added and the mixture was stirrd for 20 h at rt. The reaction mixture was diluted with a saturated aqueous sodium thiosulfate solution and stirred for 1 h at rt. The phases were separated and the aqueous phase was extracted with dichlormethane. The combined organic layers were extracted with a saturated aqueous sodium thiosulfate solution, with a saturated sodium hydrogen carbonat solution and washed with a saturated aqueous sodium chlorid solution. The organic phase was filtered over a hydrophobic filter and concentrated. The residue was purified by column chromatography (silica gel, dichloromethane/ ethanol gradient) to give 205 mg (38 % yield) of the title compound.
LC-MS (Method 2): Rt = 1.05 min; MS (ESlpos): m/z = 506 [M+H]⁺
¹H-NMR (400 MHz, DMSO-d6) δ [ppm]: 1.103 (16.00), 2.518 (0.64), 2.523 (0.42), 3.073 (5.25), 3.345 (1.04), 7.547 (0.63), 7.695 (0.47), 7.698 (0.41), 7.892 (0.48), 7.896 (0.45), 7.902 (0.64), 8.821 (0.40).

### Intermediate 47

### benzyl ({5-[4-(trifluoromethoxy)phenyl]-1H-imidazol-2-yl}methyl)carbamate

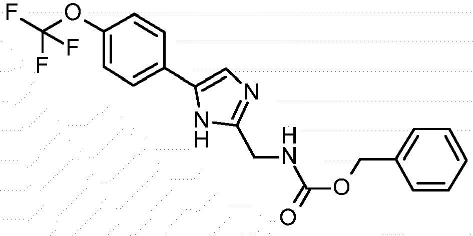

N-[(benzyloxy)carbonyl]glycine (1.00 g, 4.78 mmol) and caesiumcarbonat (779 mg, 2.39 mmol; CAS-RN:[534-17-8]) were dissolved in N-methyl-pyrrolidinone (10 mL) and stirred for 1 h at rt. 2-bromo-1-[4-(trifluoromethoxy)phenyl]ethan-1-one (1.35 g, 4.78 mmol) was added and the mixture was stirred for 30 min at rt. Xylene (30 mL) and ammonium acetate (9.21 g, 120 mmol; CAS-RN:[631-61-8]) were added and the mixture was stirred for 18 h at 120 °C. The reaction mixture was diluted with ethyl acetate and washed with a saturated aqueous sodium carbonate solution. The organic phase was washed with a saturated aqueous sodium chloride solution, dried over a hydrophobic filter and concentrated to give a crude product. The residue was stirred in methyl-tert-butylether. The precipitate was collected by filtration and dried to give 954 mg (48 % yield) of the title compound.
LC-MS (Method 2): Rt = 1.23 min; MS (ESlpos): m/z = 392 [M+H]⁺
¹H-NMR (400 MHz, DMSO-d6) δ [ppm]: 2.206 (0.74), 2.327 (0.98), 2.332 (0.72), 2.518 (3.70), 2.523 (2.27), 2.669 (0.99), 2.673 (0.72), 2.729 (0.42), 2.822 (0.41), 2.888 (0.53), 3.535 (3.66), 3.551 (3.67), 4.255 (6.38), 4.270 (6.54), 5.023 (7.43), 5.061 (15.25), 7.003 (0.76), 7.259 (0.78), 7.298 (6.11), 7.319 (7.04), 7.343 (2.77), 7.355 (11.59), 7.364 (16.00), 7.374 (10.86), 7.578 (5.30), 7.778 (1.12), 7.793 (1.89), 7.807 (1.08), 7.833 (5.51), 7.855 (5.05), 12.049 (1.51).

### Intermediate 48

### 1-{5-[4-(trifluoromethoxy)phenyl]-1H-imidazol-2-yl}methanamine hydrogen bromide

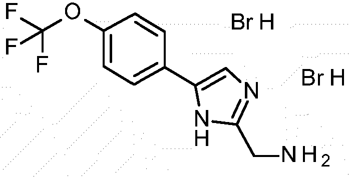

benzyl ({5-[4-(trifluoromethoxy)phenyl]-1H-imidazol-2-yl}methyl)carbamate **(Intermediate 47,** 950 mg, 2.43 mmol) was stirred with a solution of hydrogen bromide (6.0 mL, 32 % purity, 35 mmol; CAS-RN:[10035-10-6]) in acetic acid at rt for 22 h. The reaction mixture was concentrated, toluene was added and the mixture was concentrated. The residue was stirred in methyl-tert-butylether. The precipitate was collected by filtration, washed with methyl-tert-butylether and dried under reduce pressure to give 950 mg (91 % yield) of the title compound.
LC-MS (Method 2): Rₜ = 0.95 min; MS (ESlneg): m/z = 256 [M-H]⁻
¹H-NMR (400 MHz, DMSO-d6) δ [ppm]: 1.101 (9.83), 1.905 (0.45), 1.938 (0.52), 2.518 (3.76), 2.523 (2.31), 2.848 (0.56), 3.071 (3.25), 3.482 (1.48), 3.496 (3.88), 3.511 (3.85), 3.525 (1.55), 4.221 (5.85), 7.453 (6.45), 7.474 (7.09), 7.752 (0.92), 7.888 (2.42), 7.895 (16.00), 7.900 (5.61), 7.912 (5.92), 7.917 (14.80), 7.930 (7.51), 8.454 (2.95).

### Intermediate 49

### 2-(methylsulfanyl)-N-({5-[4-(trifluoromethoxy)phenyl]-1H-imidazol-2-yl}methyl)-8-(trifluoromethyl)pyrazolo[1,5-a][1,3,5]triazin-4-amine

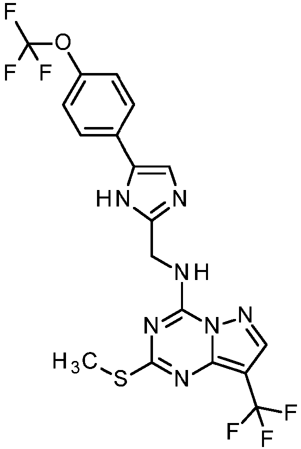

2,4-bis(methylsulfanyl)-8-(trifluoromethyl)pyrazolo[1,5-a][1,3,5]triazine **(Intermediate 42,** 265 mg, 945 µmol) was provided in acetonitrile (6 mL). 1-{5-[4-(trifluoromethoxy)phenyl]-1H-imidazol-2-yl}methanamine hydrogen bromide **(Intermediate 48, 475** mg, 1.13 mmol) and N,N-diisopropylethylamine (660 µL, 3.8 mmol; CAS-RN:[7087-68-5]) were added and the mixture was stirred for 21 h at reflux. 2,4-bis(methylsulfanyl)-8-(trifluoromethyl)pyrazolo[1,5-a][1,3,5]triazine **(Intermediate 42,** 53 mg, 189 µmol) was added and stirred for 5 h at reflux. The reaction mixture was dilluted with water and extracted with ethyl acetate. The combined organic layers were washed with a saturated aqueous sodium chloride solution, dried over a hydrophobic filter and concentrated to give a crude product. The residue was purified by column chromatography (silica gel, dichloromethane / ethanol gradient). The combined fractions were stirred in methyl-tert-butylether. The precipitate was removed by filtration and the filtrate was concentrated and dried under reduce pressure to give 403 mg (73 % yield) of the title compound.
LC-MS (Method 2): Rₜ = 1.38 min; MS (ESlpos): m/z = 490 [M+H]⁺
¹H-NMR (400 MHz, DMSO-d6) δ [ppm]: 1.103 (16.00), 2.456 (0.36), 2.470 (8.62), 2.518 (1.05), 2.523 (0.68), 3.073 (5.39), 4.016 (0.20), 4.797 (3.07), 7.305 (1.30), 7.325 (1.42), 7.627 (1.34), 7.835 (2.07), 7.857 (1.85), 8.482 (0.20), 8.506 (2.53), 9.661 (0.47), 12.019 (0.56).

### Intermediate 50

### 2-(methanesulfonyl)-N-({5-[4-(trifluoromethoxy)phenyl]-1H-imidazol-2-yl}methyl)-8-(trifluoromethyl)pyrazolo[1,5-a][1,3,5]triazin-4-amine

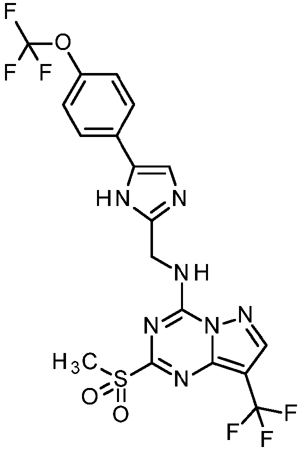

2-(methylsulfanyl)-N-({5-[4-(trifluoromethoxy)phenyl]-1H-imidazol-2-yl}methyl)-8-(trifluoromethyl)pyrazolo[1,5-a][1,3,5]triazin-4-amine **(Intermediate 49,** 400 mg, 687 µmol) was dissolved in dichlormethane (6 mL), cooled to 2 °C, mCPBA (462 mg, 77 % purity, 2.06 mmol; CAS-RN:[937-14-4]) was added and the mixture was stirred for 23 h at rt. The reaction mixture was diluted with a saturated aqueous sodium thiosulfate solution and stirred for 1 h at rt. The precipitate was removed by filtration. The phases of the filtrate were separated and the aqueous phase was extracted with dichlormethane. The combinated organic layers were extracted with a saturated aqueous sodium thiosulfate solution, with a saturated aqueous sodium hydrogen carbonate solution and washed with a saturated aqueous sodium chloride solution. The organic phase was filtered over a hydrophobic filter and concentrated. The residue was purified by column chromatography (silica gel, dichloromethane/ ethanol gradient) to give 186 mg (66 % purity, 34 % yield) of the title compound.
LC-MS (Method 2): Rt = 1.03 min; MS (ESlpos): m/z = 522 [M+H]⁺

### Intermediate 51

### ethyl 4-(trifluoromethyl)benzene-1-carboximidate

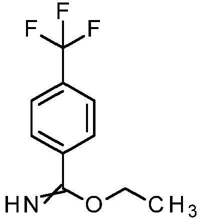

4-(trifluoromethyl)benzonitrile (1.00 g, 5.84 mmol; CAS-RN:[455-18-5]) was provided in ethanol (5 mL), cooled with an ice-bath, acetylchloride (2.9 mL, 41 mmol; CAS-RN:[75-36-5]) was added dropwise. The mixture was stirred over night at rt and 90 min at 50 °C. The reaction mixture was poured into ice-water and the precipitate was removed by filtration. The filtrate was neutralized with sodium bicarbonate and extracted with dichlormethane. The organic layer was dried over sodium sulfate, filtered and concentrated. The residue was stirred in hexane and the precipitate was removed by filtration. The filtrate was purified by column chromatography (silica gel, dichloromethane/ ethyl acetate gradient) to give 533 mg (42 % yield) of the title compound.
LC-MS (Method 1): Rt = 0.86 min; MS (ESlpos): m/z = 218 [M+H]⁺
¹H-NMR (400 MHz, DMSO-d6) δ [ppm]: 1.312 (7.33), 1.320 (1.77), 1.330 (16.00), 1.337 (3.67), 1.347 (7.38), 1.355 (1.71), 2.518 (1.06), 2.523 (0.76), 4.084 (0.40), 4.102 (1.25), 4.119 (1.23), 4.240 (2.17), 4.258 (6.84), 4.275 (6.76), 4.292 (2.07), 7.789 (0.81), 7.810 (0.95), 7.823 (3.04), 7.825 (3.53), 7.845 (4.33), 8.019 (3.52), 8.021 (4.07), 8.041 (3.36), 8.043 (2.79), 8.116 (0.87), 8.136 (0.75), 8.347 (0.49), 9.241 (3.90).

### Intermediate 52

### tert-butyl ({5-[4-(trifluoromethyl)phenyl]-4H-1,2,4-triazol-3-yl}methyl)carbamate

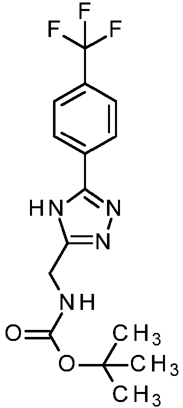

ethyl 4-(trifluoromethyl)benzene-1-carboximidate (**Intermediate 51,** 100 mg, 373 µmol) was dissolved in acetonitrile (3 mL), tert-butyl (2-hydrazinyl-2-oxoethyl)carbamate (70.6 mg, 373 µmol) was added and the mixture was stirred over night at 50 °C and for 16 h at 105 °C. The reaction mixture was evaporated and purified by column chromatography (silica gel, dichloromethane/ ethanol gradient) to give 119 mg (93 % yield) of the title compound.
LC-MS (Method 2): Rt = 0.88 min; MS (ESlpos): m/z = 343 [M+H]⁺
¹H-NMR (400 MHz, DMSO-d6) δ [ppm]: 1.035 (0.94), 1.052 (2.01), 1.070 (1.04), 1.285 (0.46), 1.400 (16.00), 2.518 (2.67), 2.523 (1.77), 2.673 (0.43), 4.282 (1.58), 4.296 (1.52), 5.759 (0.44), 7.454 (0.44), 7.828 (1.72), 7.848 (1.91), 8.173 (2.38), 8.194 (2.06).

### Intermediate 53

### 1-{5-[4-(trifluoromethyl)phenyl]-4H-1,2,4-triazol-3-yl}methanamine

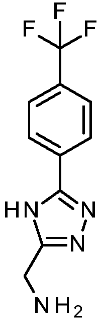

tert-butyl ({5-[4-(trifluoromethyl)phenyl]-4H-1,2,4-triazol-3-yl}methyl)carbamate **(Intermediate 52,** 117 mg, 342 µmol) was dissolved in methanol (1.4 mL). A solution of HCl in dioxane (850 µL, 8 mmol 4M; CAS-RN:[7647-01-0]) was added and the mixture was stirred over night at rt. The reaction mixture was concentrated, the residue was provided in dichlormethane, triethylamine (1 mL) was added, and the mixture was concentrated. The residue was purified by column chromatography (silica gel, amino phase, dichloromethane / ethanol gradient) to give 77.0 mg (93 % yield) of the title compound.
LC-MS (Method 2): Rt = 0.63 min; MS (ESlpos): m/z = 243 [M+H]⁺
¹H-NMR (400 MHz, DMSO-d6) δ [ppm]: 1.232 (0.42), 2.518 (5.36), 2.522 (3.49), 2.673 (0.89), 3.874 (16.00), 7.807 (3.11), 7.827 (3.43), 8.179 (3.34), 8.199 (2.96), 8.201 (2.50).

### Intermediate 54

### 2-(methylsulfanyl)-7-(trifluoromethyl)-N-({5-[4-(trifluoromethyl)phenyl]-4H-1,2,4-triazol-3-yl}methyl)imidazo[2, 1-f][1 ,2,4]triazin-4-amine

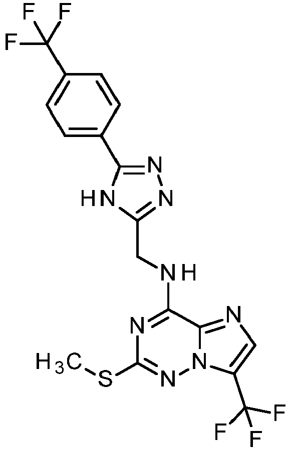

2,4-bis(methylsulfanyl)-7-(trifluoromethyl)imidazo[2,1-f][1,2,4]triazine (**Intermediate 32,** 75.0 mg, 268 µmol) and 1-{5-[4-(trifluoromethyl)phenyl]-4H-1,2,4-triazol-3-yl}methanamine (**Intermediate 53,** 71.3 mg, 294 µmol) were provided in acetonitrile (5 mL). N,N-diisopropylethylamine (190 µL, 1.1 mmol; CAS-RN:[7087-68-5]) was added and the mixture was stirred for 2 h at 150 °C, for 2 h at 160 °C and for 1 h at 180 °C in a microwave. The precipitate was collected by filtration and washed with acetonitrile to give 65.0 mg (51 % yield) of the title compound.
LC-MS (Method 2): Rt = 1.02 min; MS (ESlpos): m/z = 475 [M+H]⁺
¹H-NMR (400 MHz, DMSO-d6) δ [ppm]: 2.332 (0.40), 2.416 (16.00), 2.518 (2.28), 2.522 (1.49), 2.673 (0.41), 4.886 (4.44), 7.823 (2.32), 7.844 (2.57), 8.118 (2.86), 8.121 (2.97), 8.173 (2.88), 8.193 (2.47).

### Intermediate 55

### 2-(methanesulfonyl)-7-(trifluoromethyl)-N-({5-[4-(trifluoromethyl)phenyl]-4H-1,2,4-triazol-3-yl}methyl)imidazo[2,1-f][1,2,4]triazin-4-amine

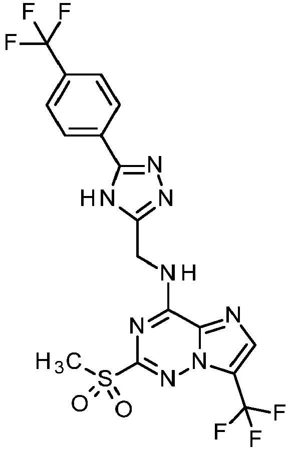

2-(methylsulfanyl)-7-(trifluoromethyl)-N-({5-[4-(trifluoromethyl)phenyl]-4H-1,2,4-triazol-3-yl}methyl)imidazo[2,1-f][1,2,4]triazin-4-amine **(Intermediate 54, 60.0** mg, 126 µmol) was provided in dichloromethane (3.0 mL), cooled with a water-bath, mCPBA (113 mg, 77 % purity, 506 µmol; CAS-RN:[937-14-4]) was added and the mixture was stirred over night at rt. The reaction mixture was diluted with ethyl acetate and extracted with a saturated aqueous sodium bicarbonate solution. The organic layer was dried over a hydrophobic filter and evaporated. The crude product was purified by column chromatography (silica gel, amino Phase, dichloromethane / ethanol gradient) to give 62.0 mg (97 % yield) of the title compound.
LC-MS (Method 2): Rt = 0.84 min; MS (ESlpos): m/z = 507 [M+H]⁺
¹H-NMR (400 MHz, DMSO-d6) δ [ppm]: 0.904 (0.41), 1.052 (0.52), 2.518 (3.63), 2.522 (2.40), 3.313 (16.00), 3.330 (10.73), 3.390 (0.61), 4.986 (3.13), 5.758 (0.56), 7.830 (1.71), 7.851 (1.94), 8.173 (2.32), 8.193 (2.01), 8.360 (2.02), 8.362 (2.14).

### Intermediate 56

### benzyl ({5-[6-(trifluoromethyl)pyridin-3-yl]-1H-imidazol-2-yl}methyl)carbamate

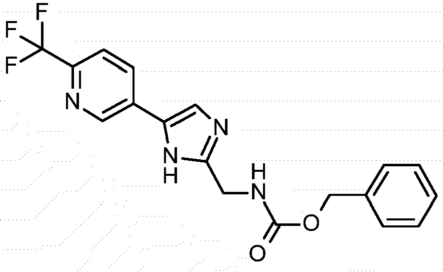

N-[(benzyloxy)carbonyl]glycine (1.50 g, 7.17 mmol) and caesiumcarbonat (1.17 g, 3.59 mmol; CAS-RN:[534-17-8]) were dissolved in N,N-dimethylformamide (13 mL) and stirred for 1 h at rt. 2-bromo-1-[6-(trifluoromethyl)pyridin-3-yl]ethan-1-one (1.92 g, 7.17 mmol) was added and the mixture was stirred for 30 min at rt. Afterwards xylene (45 mL) and ammonium acetate (13.8 g, 179 mmol; CAS-RN:[631-61-8]) were added and the mixture was stirrd for 19 h at 120 °C. The reaction mixture was diluted with a saturated aqueous sodium bicarbonate solution. The aqueous layer was extracted with ethyl acetate. The combined organic layers were washed with a saturated aqueous sodium bicarbonate solution and a saturated aqueous sodium chloride solution, filtered over a hydrophobic filter and concentrated to give a crude product. The residue was stirred in methyl-tert-butylether. The precipitate was removed by filtration. The filtrate was purified by column chromatography (silica gel, dichloromethane/ ethanol gradient) to give 278 mg (11 % yield) of the title compound.
LC-MS (Method 2): Rt = 1.07 min; MS (ESlpos): m/z = 377 [M+H]⁺

### Intermediate 57

### 1-{5-[6- (trifluoromethyl) pyridin-3-yl]- 1H-imidazol-2-yl}methanamine

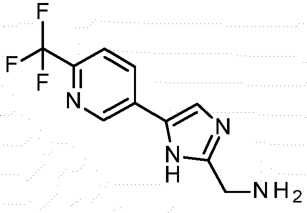

benzyl ({5-[6-(trifluoromethyl)pyridin-3-yl]-1H-imidazol-2-yl}methyl)carbamate **(Intermediate 56,** 275 mg, 658 µmol) was stirred with a solution of hydrogen bromide (5.0 mL, 32 % purity, 29 mmol; CAS-RN:[10035-10-6]) in acetic acid for 22 h at rt. The reaction mixture was concentrated, toluene was added and the mixture was concentrated. The residue was stirred in methyl-tert-butylether. The precipitate was collected by filtration, washed with methyl-tert-butylether, dried and purified purified by column chromatography (silica gel, amino phase, dichloromethane/ethanol gradient) to give 98.0 mg (57 % yield) of the title compound.
LC-MS (Method 2): Rₜ = 0.76 min; MS (ESlpos): m/z = 243 [M+H]⁺
¹H-NMR (400 MHz, DMSO-d6) δ [ppm]: 1.226 (0.44), 1.792 (0.45), 1.884 (1.47), 1.953 (2.27), 2.080 (9.69), 2.332 (1.14), 2.518 (6.94), 2.522 (4.26), 2.711 (1.16), 2.778 (2.12), 2.938 (3.01), 3.161 (2.07), 3.624 (6.36), 3.639 (7.52), 3.654 (6.65), 3.668 (3.80), 3.741 (2.08), 3.931 (0.90), 4.157 (4.95), 4.209 (0.75), 4.224 (0.71), 4.478 (3.46), 4.492 (3.47), 6.997 (1.44), 7.125 (1.48), 7.252 (1.41), 7.872 (0.41), 7.887 (2.24), 7.908 (3.59), 7.928 (5.35), 7.948 (5.75), 7.975 (2.32), 7.995 (2.53), 8.011 (2.32), 8.026 (1.79), 8.031 (1.94), 8.043 (16.00), 8.090 (0.42), 8.128 (0.87), 8.169 (0.54), 8.189 (0.48), 8.364 (3.81), 8.368 (3.86), 8.385 (4.18), 8.388 (4.29), 8.473 (0.63), 8.706 (2.53), 8.848 (0.59), 8.978 (0.77), 8.993 (1.48), 9.008 (0.78), 9.159 (0.50), 9.179 (5.36), 9.184 (5.22).

Alternatively, in a first batch tert-butyl [(5-bromo-1H-imidazol-2-yl)methyl]carbamate (**Intermediate 74,** 707 mg, 2.56 mmol), 5-(4,4,5,5-tetramethyl-1,3,2-dioxaborolan-2-yl)-2-(trifluoromethyl)pyridine (839 mg, 3.07 mmol; CAS-RN:[1218790-39-6]) and potassium carbonate (1.06 g, 7.67 mmol) were provided in 1,4-dioxane (15 mL) and water (5 mL). 1,1'-Bis(diphenylphosphino)ferrocenepalladium(II) chloride (187 mg, 256 µmol; CAS-RN:[72287-26-4]) was added. The mixture was flushed with argon and stirred for 1 h at 130 °C in a microwave. A second batch of tert-butyl [(5-bromo-1H-imidazol-2-yl)methyl]carbamate (**Intermediate 74,** 605 mg, 2.19 mmol), 5-(4,4,5,5-tetramethyl-1,3,2-dioxaborolan-2-yl)-2-(trifluoromethyl)pyridine (718 mg, 2.63 mmol; CAS-RN:[1218790-39-6]) and potassium carbonate (908 mg, 6.57 mmol) were dissolved in 1,4-dioxane (15 mL) and water (5 mL). 1,1'-Bis(diphenylphosphino)ferrocenepalladium(II) chloride (160 mg, 219 µmol; CAS-RN:[72287-26-4]) was added. The mixture was flushed with argon and stirred for 1 h at 130 °C in a microwave. The two reaction mixtures were combined, concentrated and stirred in a mixture of dichloromethane and ethanol. The precipitate was removed by filtration. The filtrate was concentrated and purified by column chromatography (silica gel, aminophase, dichlormethane / ethyl acetate gradient) to give 1340 mg of tert-butyl ({5-[6-(trifluoromethyl)pyridin-3-yl]-1H-imidazol-2-yl}methyl)carbamate.

LC-MS (Method 1): Rt = 0.89 min; MS (ESlpos): m/z = 343 [M+H]⁺
To tert-butyl ({5-[6-(trifluoromethyl)pyridin-3-yl]-1H-imidazol-2-yl}methyl)carbamate (1.33 g, 3.89 mmol) in methanol (16 L) was added a solution of HCl in 1,4-dioxane (9.7 mL, 4.0 M, 38.9 mmol CAS-RN:[7647-01-0]). The mixture was stirred for 16 h at rt.

The reaction mixture was concentrated and stirred with dichloromethane. The precipitate was collected by filtration to give 946 mg of the title compound as a crude product hydrogen chloride salt which was used without further purification.

### Intermediate 58

### 2-(methylsulfanyl)-7-(trifluoromethyl)-N-({5-[6-(trifluoromethyl)pyridin-3-yl]-1H-imidazol-2-yl}methyl)imidazo[2,1-f][1,2,4]triazin-4-amine

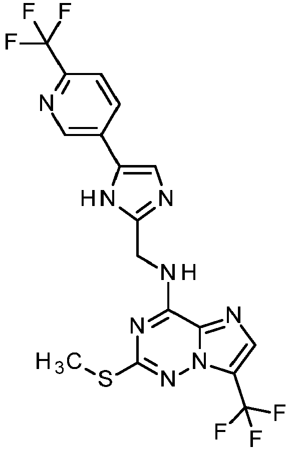

2,4-bis(methylsulfanyl)-7-(trifluoromethyl)imidazo[2,1-f][1,2,4]triazine (**Intermediate 32,** 99.9 mg, 357 µmol) and 1-{5-[6-(trifluoromethyl)pyridin-3-yl]-1H-imidazol-2-yl}methanamine (**Intermediate 57,** 95.0 mg, 392 µmol) were provided in acetonitrile (3 mL). N,N-diisopropylethylamine (250 µL, 1.4 mmol; CAS-RN:[7087-68-5]) was added and the mixture was stirred for 2 h at 150 °C in a microwave. The reaction mixture was diluted with water. The precipitate was collected by filtration, washed with water and stirred in methyl-tert-butylether at 50 °C. The precipitate was collected by fitration, washed with methyl-tert-butylether and dried give 64.0 mg (34 % yield) of the title compound.
LC-MS (Method 2): Rt = 1.25 min; MS (ESlpos): m/z = 475 [M+H]⁺
¹H-NMR (400 MHz, DMSO-d6) δ [ppm]: 1.102 (0.32), 2.073 (0.62), 2.323 (0.34), 2.327 (0.48), 2.331 (0.35), 2.440 (16.00), 2.452 (1.21), 2.461 (0.34), 2.518 (2.28), 2.523 (1.45), 2.579 (0.23), 2.665 (0.40), 2.669 (0.56), 2.673 (0.38), 4.816 (4.71), 5.094 (0.25), 5.931 (0.19), 7.844 (1.26), 7.865 (1.39), 7.901 (1.92), 7.933 (0.17), 8.104 (3.00), 8.106 (3.13), 8.146 (0.20), 8.314 (0.89), 8.334 (0.80), 9.124 (1.66), 9.731 (0.23), 12.231 (0.22).

### Intermediate 59

### 2-(methanesulfonyl)-7-(trifluoromethyl)-N-({5-[6-(trifluoromethyl)pyridin-3-yl]-1H-imidazol-2-yl}methyl)imidazo[2,1-f][1,2,4]triazin-4-amine

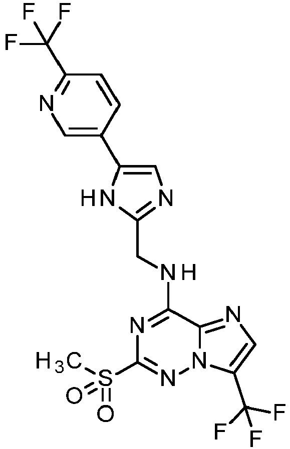

2-(methylsulfanyl)-7-(trifluoromethyl)-N-({5-[6-(trifluoromethyl)pyridin-3-yl]-1H-imidazol-2-yl}methyl)imidazo[2,1-f][1,2,4]triazin-4-amine **(Intermediate 58,** 60.0 mg, 126 µmol) was provided in dichlormethane (2 mL), cooled to 2 °C, mCPBA (65.5 mg, 77 % purity, 379 µmol; CAS-RN:[937-14-4]) was added and the mixture was stirrd for 20 h at rt. The reaction mixture was diluted with a sodium thiosulfate solution (50 %, aq) and stirred for 1 h at rt. The layers were separated and the aqueous phase was extracted with dichlormethane. The combinated organic layers were extracted with a sodium thiosulfate solution (50 %, aq), with a saturated aqueous sodium hydrogen carbonate solution and washed with a saturated aqueous sodium chloride solution. The organic phase was filtered over a hydrophobic filter and concentrated to give 62.5 mg of the title compound which was used without further purification.
LC-MS (Method 2): Rt = 1.07 min; MS (ESlpos): m/z = 507 [M+H]⁺

### Intermediate 60

### benzyl {[5-(4-methoxyphenyl)-1H-imidazol-2-yl]methyl}carbamate

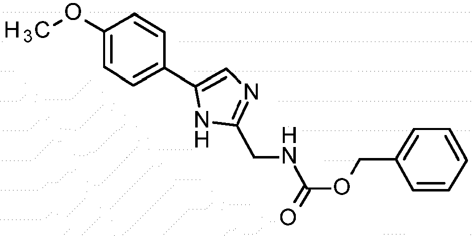

N-[(benzyloxy)carbonyl]glycine (1.50 g, 7.17 mmol) and caesium carbonate (1.17 g, 3.59 mmol; CAS-RN:[534-17-8]) were dissolved in N,N-dimethylformamide (15 mL) and stirred for 1 h at rt. 2-bromo-1-(4-methoxyphenyl)ethan-1-one (1.64 g, 7.17 mmol) was added and the mixture was stirred for 30 min at rt. Afterwards xylene (45 mL) and ammonium acetate (9.21 g, 120 mmol; CAS-RN:[631-61-8]) were added and the mixture was stirred for 19 h at 120 °C. The reaction mixture was diluted with a saturated aqueous sodium bicarbonate solution. The aqueous layer was extracted with ethyl acetate. The combinated organic layers were washed with saturated aqueous sodium bicarbonate solution and a saturated aqueous sodium chloride solution, filtered over a hydrophobic filter and concentrated to give a crude product. The residue was stirred in methyl-tert-butylether. The precipitate was collected by filtration, washed with methyl-tert-butylether and purified by column chromatography (silica gel, dichloromethane/ ethanol gradient) to give 1.28 g (48 % yield) of the title compound.
LC-MS (Method 2): Rt = 1.05 min; MS (ESlpos): m/z = 338 [M+H]⁺
¹H-NMR (400 MHz, DMSO-d6) δ [ppm]: 2.332 (0.22), 2.518 (0.99), 2.523 (0.64), 2.819 (0.31), 2.931 (0.24), 3.307 (0.24), 3.384 (0.19), 3.747 (16.00), 3.764 (2.00), 3.798 (0.23), 3.840 (0.83), 3.848 (0.30), 4.236 (2.90), 4.251 (3.02), 5.028 (0.26), 5.059 (6.02), 6.880 (2.99), 6.903 (3.17), 6.949 (0.50), 6.970 (0.51), 7.092 (0.19), 7.114 (0.31), 7.122 (0.42), 7.270 (0.37), 7.290 (0.36), 7.302 (0.57), 7.312 (0.92), 7.323 (1.06), 7.333 (0.86), 7.343 (0.82), 7.363 (8.48), 7.374 (5.26), 7.543 (0.48), 7.565 (0.47), 7.644 (2.52), 7.665 (2.46), 7.749 (0.47), 7.763 (0.87), 7.777 (0.45), 8.128 (0.16), 9.202 (0.27), 11.851 (0.72), 12.184 (0.18).

### Intermediate 61

### 1-[5-(4-methoxyphenyl)-1H-imidazol-2-yl]methanamine hydrogen bromide

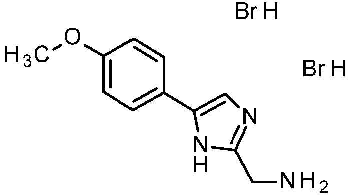

Benzyl {[5-(4-methoxyphenyl)-1H-imidazol-2-yl]methyl}carbamate (**Intermediate 60,** 1.28 g, 3.79 mmol) was stirred with a solution of hydrogen bromide (5.0 mL, 32 % purity, 29 mmol; CAS-RN:[10035-10-6]) in acetic acid for 23 h at rt. The reaction mixture was concentratet, toluene was added and the mixture was concentrated. The residue was stirred in methyl-tert-butylether. The precipitate was collected by filtration, washed with methyl-tert-butylether and dried under reduce pressure to give 1.37 g (93 % yield) of the title compound.
LC-MS (Method 2): Rₜ = 0.68 min; MS (ESlpos): m/z = 204 [M+H]⁺
¹H-NMR (400 MHz, DMSO-d6) δ [ppm]: 1.905 (6.34), 2.332 (0.74), 2.518 (4.59), 2.522 (2.87), 2.888 (0.96), 2.933 (1.39), 3.622 (2.15), 3.696 (2.20), 3.745 (2.18), 3.772 (2.07), 3.826 (1.92), 3.838 (3.46), 3.847 (1.73), 3.896 (1.02), 3.983 (0.84), 4.291 (7.41), 4.705 (0.39), 7.070 (9.40), 7.091 (10.13), 7.113 (0.74), 7.707 (1.85), 7.714 (16.00), 7.719 (4.92), 7.731 (4.80), 7.736 (14.47), 7.744 (1.63), 7.851 (0.19), 7.913 (3.62), 8.127 (0.44), 8.133 (0.44), 8.149 (0.43), 8.495 (2.55), 9.202 (0.68).

### Intermediate 62

### N-{[5-(4-methoxyphenyl)-1H-imidazol-2-yl]methyl}-2-(methylsulfanyl)-7-(trifluoromethyl)imidazo[2,1-f][1,2,4]triazin-4-amine

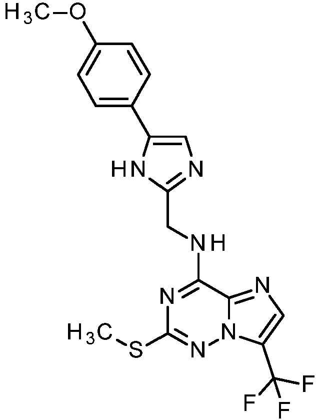

2,4-bis(methylsulfanyl)-7-(trifluoromethyl)imidazo[2,1-f][1,2,4]triazine (**Intermediate 32,** 454 mg, 1.62 mmol) and 1-[5-(4-methoxyphenyl)-1H-imidazol-2-yl]methanamine hydrogen bromide (**Intermediate 61,** 650 mg, 1.78 mmol) were provided in acetonitrile (12 mL). N,N-diisopropylethylamine (1.1 mL, 6.5 mmol; CAS-RN:[7087-68-5]) was added and the mixture was stirred for 2 h at 150 °C in a microwave. The reaction mixture was diluted with water and extracted with etyl acetate. The combined organic layers were washed with a saturated aqueous sodium chloride solution and filtered over a hydrophobic filter to give a crude product. The residue was purified by column chromatography (silica gel, dichloromethane/ ethanol gradient) to give 480 mg (57 % yield) of the title compound.
LC-MS (Method 2): Rₜ = 1.18 min; MS (ESlpos): m/z = 436 [M+H]⁺
¹H-NMR (400 MHz, DMSO-d6) δ [ppm]: 0.782 (0.31), 0.801 (0.79), 0.814 (0.19), 0.820 (0.44), 0.883 (0.46), 0.901 (1.02), 0.920 (0.48), 0.991 (0.22), 1.009 (0.24), 1.035 (0.35), 1.053 (1.32), 1.071 (1.87), 1.088 (0.79), 1.159 (1.03), 1.525 (0.20), 1.544 (0.20), 2.065 (1.25), 2.079 (0.46), 2.091 (0.17), 2.336 (0.18), 2.418 (0.30), 2.438 (0.55), 2.451 (16.00), 2.461 (0.61), 2.518 (1.90), 2.522 (1.23), 2.660 (0.19), 2.917 (0.21), 3.349 (1.14), 3.363 (0.51), 3.367 (0.28), 3.381 (0.17), 3.705 (0.23), 3.732 (2.22), 3.745 (14.66), 3.756 (1.74), 3.784 (0.47), 3.798 (0.41), 3.822 (0.49), 4.764 (2.35), 4.777 (2.28), 4.940 (0.20), 5.378 (0.35), 6.881 (3.21), 6.903 (3.28), 6.947 (0.41), 6.969 (0.41), 7.128 (0.33), 7.397 (2.14), 7.401 (2.01), 7.497 (0.61), 7.514 (0.37), 7.535 (0.38), 7.603 (0.48), 7.608 (0.17), 7.625 (0.47), 7.644 (3.19), 7.666 (2.81), 8.063 (0.25), 8.092 (3.42), 8.094 (3.49), 9.663 (0.76), 11.791 (0.85), 12.100 (0.19).

### Intermediate 63

### N-{[5-(4-methoxyphenyl)-1H-imidazol-2-yl]methyl}-2-(methylsulfinyl)-7-(trifluoromethyl)imidazo[2,1-f][1,2,4]triazin-4-amine

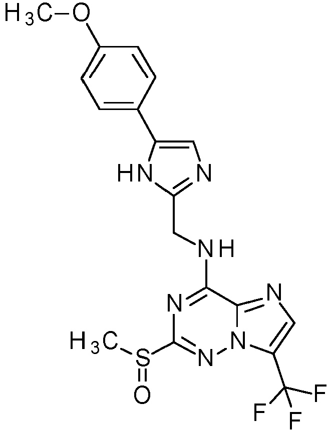

N-{[5-(4-methoxyphenyl)-1H-imidazol-2-yl]methyl}-2-(methylsulfanyl)-7-(trifluoromethyl)imidazo[2,1-f][1,2,4]triazin-4-amine (**Intermediate 62**, 480 mg, 882 µmol) was provided in dichlormethane (8 mL), cooled to 2 °C, mCPBA (457 mg, 77 % purity, 2.65 mmol; CAS-RN:[937-14-4]) was added and the mixture was stirred for 22 h at rt. The reaction mixture was diluted with a sodium thiosulfate solution (50 %, aq) and stirred for 30 min at rt. The layers were separated and the aqueous phase was extracted with dichlormethane. The combined organic layers were extracted with a sodium thiosulfate solution (50 %, aq), with a saturated aqueous sodium hydrogen carbonate solution and washed with a saturated aqueous sodium chloride solution. The organic phase was filtered over a hydrophobic filter and concentrated to give 494 mg of the title compound which was used without further purification.
LC-MS (Method 2): Rt = 1.01 min; MS (ESlneg): m/z = 450 [M-H]⁻
¹H-NMR (400 MHz, DMSO-d6) δ [ppm]: 1.231 (0.45), 2.518 (3.20), 2.523 (2.03), 2.673 (0.69), 2.869 (16.00), 2.888 (2.57), 2.895 (0.59), 2.918 (0.49), 3.164 (0.63), 3.646 (0.54), 3.730 (1.06), 3.744 (15.91), 3.797 (0.45), 3.811 (0.41), 3.863 (0.55), 4.819 (3.39), 4.824 (3.70), 4.862 (0.66), 6.882 (1.68), 6.903 (1.89), 7.408 (1.25), 7.641 (1.63), 7.662 (1.42), 8.271 (3.18), 11.824 (0.59).

### Intermediate 64

### ethyl 4-(trifluoromethoxy)benzene-1-carboximidate

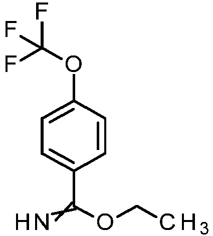

### First Batch

4-(trifluoromethoxy)benzonitrile (780 µL, 5.3 mmol; CAS-RN:[332-25-2]) was provided in ethanol (5 mL), cooled with an ice-bath, acetylchlorid (2.7 mL, 37 mmol; CAS-RN:[75-36-5]) was added dropwise and the mixture was stirred over night at rt and for 2 days at 50 °C. The reaction mixture was concentrated, dichlormethane and triethylamine were added and the mixture was concentrated again.

### Second batch

4-(trifluoromethoxy)benzonitrile (780 µL, 5.3 mmol; CAS-RN:[332-25-2]) was provided in ethanol (4.6 mL, cooled with an ice-bath, acetylchlorid (2.7 mL, 37 mmol; CAS-RN:[75-36-5]) was added dropwise and the mixture was stirred for 29 h at 50 °C. The reaction mixture was concentrated, dichlormethane and triethylamine were added and the mixture was concentrated again.

The first and the second batch were combined and purified by flash chromatography (silica gel, dichlormethane / ethyl acetate gradient) to give 1.75 g (70 % yield) of the title compound.
LC-MS (Method 1): Rt = 0.81 min; MS (ESlpos): m/z = 234 [M+H]⁺
¹H-NMR (400 MHz, DMSO-d6) δ [ppm]: 1.297 (7.12), 1.305 (1.43), 1.314 (16.00), 1.323 (2.97), 1.332 (7.27), 1.340 (1.32), 2.518 (0.89), 2.522 (0.58), 4.074 (0.95), 4.091 (0.96), 4.218 (2.01), 4.235 (6.40), 4.253 (6.21), 4.270 (1.88), 7.396 (0.51), 7.399 (0.58), 7.419 (0.63), 7.421 (0.59), 7.428 (0.41), 7.435 (2.70), 7.438 (2.98), 7.455 (2.11), 7.457 (3.38), 7.460 (2.99), 7.932 (0.74), 7.939 (7.34), 7.944 (1.98), 7.956 (2.01), 7.961 (6.42), 7.969 (0.65), 8.028 (1.21), 8.050 (1.18), 9.057 (3.85).

### Intermediate 65

### tert-butyl ({5-[4-(trifluoromethoxy)phenyl]-4H-1,2,4-triazol-3-yl}methyl)carbamate

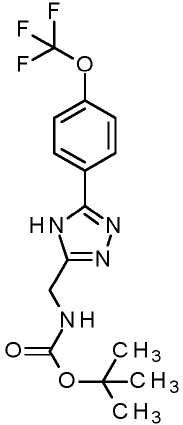

Ethyl 4-(trifluoromethoxy)benzene-1-carboximidate **(Intermediate 64,** 750 mg, 87 % purity, 2.80 mmol) was dissolved in acetonitrile (15 mL). Tert-butyl (2-hydrazinyl-2-oxoethyl)carbamate (529 mg, 2.80 mmol; CAS-RN:[6926-09-6]) was added and the mixture was stirred over night at 50 °C, for 32 h at 105 °C and over the weekend at rt. The precipitate was collected by filtration and washed with acetonitrile to give 546 mg (54 % yield) of the title compound.
LC-MS (Method 1): Rₜ = 1.17 min; MS (ESlneg): m/z = 357 [M-H]⁻
¹H-NMR (400 MHz, DMSO-d6) δ [ppm]: 1.287 (0.41), 1.397 (16.00), 2.518 (1.25), 2.522 (0.83), 4.260 (1.23), 4.275 (1.18), 7.457 (1.23), 7.478 (1.22), 8.065 (0.49), 8.072 (4.47), 8.077 (1.29), 8.089 (1.25), 8.094 (4.02), 8.101 (0.43).

### Intermediate 66

### 1-{5-[4-(trifluoromethoxy)phenyl]-4H-1,2,4-triazol-3-yl}methanamine

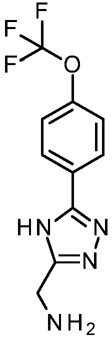

Tert-butyl ({5-[4-(trifluoromethoxy)phenyl]-4H-1,2,4-triazol-3-yl}methyl)carbamate **(Intermediate 65,** 483 mg, 1.35 mmol) was dissolved in methanol (5.5 mL). A solution of HCl in dioxane (3.4 mL, 13 mmol, 4M; CAS-RN:[7647-01-0]) was added and the mixture was stirred over night at rt. The reaction mixture was concentrated. Dichlormethane and triethylamine (1 mL) were added, the mixture was concentrated and the residue was purified by column chromatography (silica gel, aminophase, dichloromethane/ ethanol gradient) to give 328 mg (78 % yield) of the title compound.
LC-MS (Method 2): Rt = 0.70 min; MS (ESlneg): m/z = 257 [M-H]⁻
¹H-NMR (400 MHz, DMSO-d6) δ [ppm]: 0.883 (0.78), 0.901 (1.67), 0.920 (0.86), 1.013 (0.62), 1.022 (0.64), 1.032 (1.18), 1.035 (1.01), 1.040 (0.58), 1.052 (1.83), 1.058 (0.48), 1.070 (0.82), 1.171 (0.52), 1.227 (1.53), 1.798 (1.01), 1.808 (0.46), 1.826 (0.42), 1.930 (1.19), 2.064 (1.85), 2.244 (1.17), 2.419 (0.41), 2.437 (0.44), 2.518 (6.55), 2.523 (4.62), 3.349 (2.10), 3.367 (1.93), 3.386 (2.16), 3.411 (1.45), 3.428 (1.68), 3.446 (1.59), 3.464 (0.96), 4.284 (0.44), 4.298 (0.44), 4.382 (0.55), 4.451 (1.27), 7.386 (0.52), 7.406 (0.64), 7.437 (12.52), 7.457 (13.77), 8.080 (16.00), 8.090 (3.90), 8.101 (14.96), 8.131 (1.19), 8.179 (0.40).

### Intermediate 67

### 2-(methylsulfanyl)-N-({5-[4-(trifluoromethoxy)phenyl]-4H-1,2,4-triazol-3-yl}methyl)-7-(trifluoromethyl)imidazo[2,1-f][1,2,4]triazin-4-amine

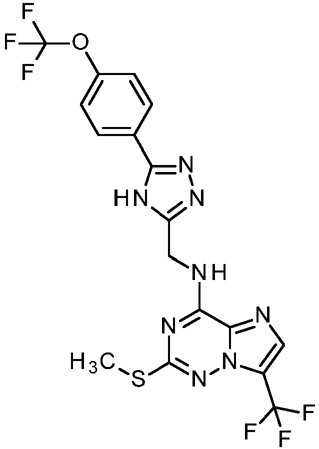

2,4-bis(methylsulfanyl)-7-(trifluoromethyl)imidazo[2,1-f][1,2,4]triazine **(Intermediate 32,** 150 mg, 535 µmol) and 1-{5-[4-(trifluoromethoxy)phenyl]-4H-1,2,4-triazol-3-yl}methanamine **(Intermediate 66,** 166 mg, 642 µmol) were dissolved in acetonitrile (10 mL). N,N-diisopropylethylamine (370 µL, 2.1 mmol; CAS-RN:[7087-68-5]) was added and the mixture was stirred for 3 h at 180 °C in a microwave. The reaction mixture was concentrated to give a crude product. The residue was purified by column chromatography (silica gel, dichloromethane/ ethyl acetate gradient). The resulting precipitate was collected by filtration to give 104 mg (39 % yield) of the title compound
LC-MS (Method 1): Rt = 1.37 min; MS (ESlpos): m/z = 491 [M+H]⁺
¹H-NMR (400 MHz, DMSO-d6) δ [ppm]: 0.840 (0.41), 0.852 (0.57), 0.857 (1.13), 1.237 (0.92), 2.318 (0.48), 2.419 (16.00), 2.518 (14.05), 2.523 (10.18), 2.678 (0.64), 4.865 (2.66), 5.759 (0.58), 7.456 (1.79), 7.476 (1.93), 8.066 (0.81), 8.073 (7.35), 8.078 (2.61), 8.089 (2.18), 8.095 (6.92), 8.102 (1.00), 8.117 (4.38), 9.808 (0.65).

### Intermediate 68

### 2-(methanesulfonyl)-N-({5-[4-(trifluoromethoxy)phenyl]-4H-1,2,4-triazol-3-yl}methyl)-7-(trifluoromethyl)imidazo[2,1-f][1,2,4]triazin-4-amine

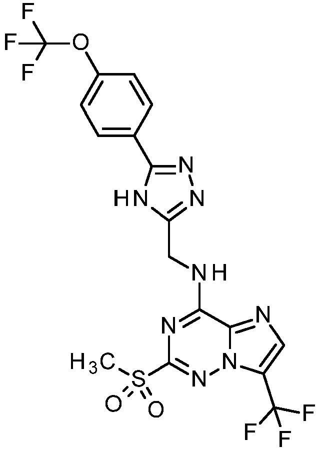

2-(methylsulfanyl)-N-({5-[4-(trifluoromethoxy)phenyl]-4H-1,2,4-triazol-3-yl}methyl)-7-(trifluoromethyl)imidazo[2,1-f][1,2,4]triazin-4-amine **(Intermediate 67,** 102 mg, 208 µmol) was provided in dichlormethane (5 mL), cooled with a water-bath, mCPBA (186 mg, 77 % purity, 832 µmol; CAS-RN:[937-14-4]) was added and the mixture was stirred over night at rt. The reaction mixture was diluted with ethyl acetate and extracted with a saturated aqueous sodium bicarbonate solution. The organic layer was dried over a hydrophobic filter and concentrated. The crude product was purified by column chromatography (silica gel, dichloromethane / ethyl acetate gradient) to give 94.0 mg (87 % yield) of the title compound.
LC-MS (Method 2): Rt = 0.89 min; MS (ESlpos): m/z = 523 [M+H]⁺
¹H-NMR (400 MHz, DMSO-d6) δ [ppm]: 1.154 (1.08), 1.172 (2.36), 1.190 (1.24), 1.987 (4.66), 2.518 (1.54), 2.522 (1.01), 3.316 (2.22), 3.330 (16.00), 3.345 (0.43), 4.017 (0.99), 4.034 (0.98), 5.758 (1.50), 8.071 (1.35), 8.093 (1.22), 8.368 (0.64).

### Intermediate 69

### ethyl N-[2-(methylsulfanyl)-7-(trifluoromethyl)imidazo[2,1-f][1,2,4]triazin-4-yl]glycinate

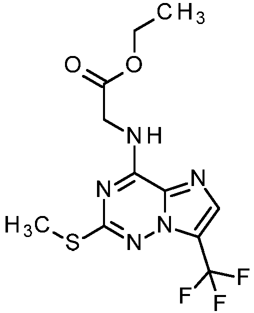

2,4-bis(methylsulfanyl)-7-(trifluoromethyl)imidazo[2,1-f][1,2,4]triazine **(Intermediate 32,** 2.00 g, 7.14 mmol) was dissolved in acetonitrile (40 mL). Ethyl glycinate hydrogen chloride (1/1) (1.49 g, 10.7 mmol) and N,N-diisopropylethylamine (5.0 mL, 29 mmol; CAS-RN:[7087-68-5]) were added and the mixture was stirred for 5 days at 70 °C. The reaction mixture was concentrated and the residue was diluted with water. The precipitate was collected by filtration and dried under reduce pressure at 60 °C to give 2.11 g (87 % yield) of the title compound.
LC-MS (Method 1): Rt = 1.26 min; MS (ESlpos): m/z = 336 [M+H]⁺
¹H-NMR (400 MHz, DMSO-d6) δ [ppm]: 1.183 (4.82), 1.189 (0.46), 1.201 (10.88), 1.218 (4.91), 2.459 (16.00), 2.468 (0.48), 2.482 (1.01), 2.518 (1.41), 2.523 (1.04), 4.117 (1.32), 4.135 (4.13), 4.152 (4.04), 4.170 (1.22), 4.209 (2.92), 4.223 (2.92), 8.110 (2.36), 8.112 (2.43), 9.604 (0.46), 9.620 (0.95), 9.634 (0.44).

### Intermediate 70

### ethyl N-[2-(methanesulfonyl)-7-(trifluoromethyl)imidazo[2,1-f][1,2,4]triazin-4-yl]glycinate

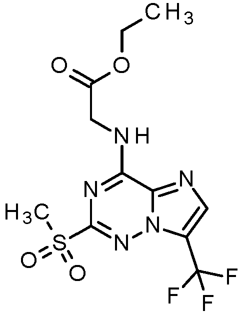

Ethyl N-[2-(methylsulfanyl)-7-(trifluoromethyl)imidazo[2,1-f][1,2,4]triazin-4-yl]glycinate **(Intermediate 69,** 2.11 g, 6.29 mmol) was dissolved in dichlormethane (50 mL), cooled with an ice-bath, 3-chlorobenzene-1-carboperoxoic acid (2.82 g, 77 % purity, 12.6 mmol; CAS-RN:[937-14-4]) was added and the mixture was stirred over night at rt. The reaction mixture was adjusted to a basic pH by the addition of a saturated aqueous sodium bicarbonate solution. The organic layer was washed with an aqueous sodium thiosulfate solution and brine, dried over a hydrophobic filter and concentrated to give 2.08 g (90 % yield) of the title compound.
LC-MS (Method 1): Rt = 0.97 min; MS (ESlpos): m/z = 368 [M+H]⁺
¹H-NMR (400 MHz, DMSO-d6) δ [ppm]: 1.184 (0.30), 1.189 (1.77), 1.201 (0.26), 1.207 (3.93), 1.225 (1.76), 2.518 (0.60), 2.523 (0.42), 2.879 (0.31), 3.331 (16.00), 3.347 (7.13), 3.380 (0.42), 4.127 (0.47), 4.144 (1.47), 4.162 (1.47), 4.180 (0.44), 4.343 (1.49), 5.758 (0.31), 8.366 (0.88), 8.369 (0.93), 10.257 (0.25).

### Intermediate 71

### ethyl N-[2-(morpholin-4-yl)-7-(trifluoromethyl)imidazo[2,1-f][1,2,4]triazin-4-yl]glycinate

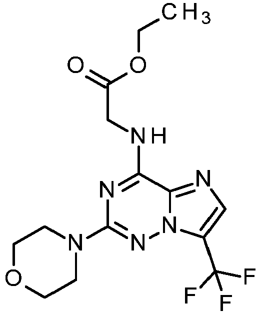

Ethyl N-[2-(methanesulfonyl)-7-(trifluoromethyl)imidazo[2,1-f][1,2,4]triazin-4-yl]glycinate **(Intermediate 70,** 2.08 g, 5.66 mmol) was dissolved in acetonitrile (40 mL). Morpholine (1.5 mL, 17 mmol; CAS-RN:[110-91-8]) and N,N-diisopropylethylamine (3.0 mL, 17 mmol; CAS-RN:[7087-68-5]) were added and the mixture was stirred for 7 days at 70 °C.The reaction mixture was concentrated, diluted with water and extracted with ethyl acetate. The organic phase was dried over a hydrophobic filter and concentrated to give a crude product. The residue was purified by column chromatography (silica gel, hexane / ethyl acetate gradient) to give 450 mg (21 % yield) of the title compound.
LC-MS (Method 1): Rt = 1.19 min; MS (ESlneg): m/z = 373 [M-H]⁻
¹H-NMR (400 MHz, DMSO-d6) δ [ppm]: 1.163 (7.21), 1.180 (16.00), 1.194 (0.95), 1.198 (7.26), 2.518 (2.66), 2.523 (1.93), 3.504 (3.12), 3.514 (5.18), 3.528 (4.63), 3.638 (4.65), 3.651 (5.48), 3.662 (3.34), 4.097 (2.08), 4.114 (6.76), 4.132 (6.70), 4.148 (5.00), 4.162 (4.49), 7.938 (3.97), 7.940 (4.10), 9.276 (0.76), 9.290 (1.65), 9.305 (0.77).

### Intermediate 72

### N-[2-(morpholin-4-yl)-7-(trifluoromethyl)imidazo[2,1-f][1,2,4]triazin-4-yl]glycine

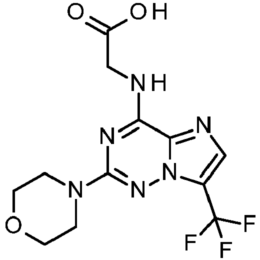

Ethyl N-[2-(morpholin-4-yl)-7-(trifluoromethyl)imidazo[2,1-f][1,2,4]triazin-4-yl]glycinate (**Intermediate 71,** 450 mg, 1.20 mmol) was dissolved in tetrahydrofuran (25 mL) and ethanol (10 mL). An aqueous solution of lithium hydroxide (3.0 mL, 1.0 M, 3.0 mmol; CAS-RN:[1310-65-2]) was added and the mixture was stirred over night at rt. The reaction mixture was concentrated and the residue was diluted with water. The aqueous phase was adjusted to a slightly acid pH with a solution of citric acid (50 %, aq). The resulting precipitate was filtered off, washed with water and the solid was dried to give 381 mg (90 % yield) of the title compound.
LC-MS (Method 1): Rt = 0.99 min; MS (ESlpos): m/z = 347 [M+H]⁺
¹H-NMR (500 MHz, DMSO-d6) δ [ppm]: 1.232 (0.44), 2.365 (1.56), 2.369 (0.70), 2.515 (7.16), 2.518 (7.04), 2.522 (5.54), 3.521 (9.69), 3.530 (15.21), 3.540 (13.44), 3.644 (13.74), 3.655 (16.00), 3.664 (10.34), 4.070 (11.53), 4.082 (11.51), 7.927 (11.79), 9.058 (1.63), 9.069 (2.98), 9.080 (1.57).

### Intermediate 73

### 2-{[2-(morpholin-4-yl)-7-(trifluoromethyl)imidazo[2,1-f][1,2,4]triazin-4-yl]amino}acetohydrazide

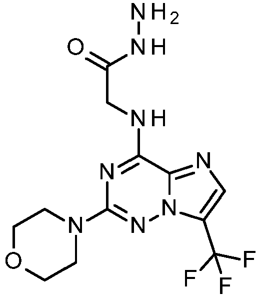

### N-[2-(morpholin-4-yl)-7-(trifluoromethyl)imidazo[2,1-f][1,2,4]triazin-4-yl]glycine

**(Intermediate 72,** 380 mg, 1.10 mmol) was dissolved in tetrahydrofuran (20 mL). Di(1H-imidazol-1-yl)methanone (356 mg, 2.19 mmol; CAS-RN:[530-62-1]) was added and the mixture was stirred for 6 h at reflux. The reaction mixture cooled to rt, a solution of hydrazine in tetrahydrofuran (5.5 mL, 1.0 M, 5.5 mmol; CAS-RN:[302-01-2]) was added and stirred for 18 h at rt. The reaction mixture was diluted with water and extracted with ethyl acetate. The combined organic layers were washed with brine, dried over a hydrophobic filter and concentrated. The crude product was stirred in methyl-tert-butylether. The precipitate was collected by filtration, washed with methyl-tert-butylether and dried to give 330 mg (79 % yield) of the title compound.
LC-MS (Method 1): Rt = 0.90 min; MS (ESlneg): m/z = 359 [M-H]⁻
¹H-NMR (400 MHz, DMSO-d6) δ [ppm]: 1.154 (0.80), 1.173 (1.59), 1.190 (0.84), 1.758 (0.40), 1.988 (3.18), 2.337 (0.76), 2.518 (10.46), 2.523 (7.42), 3.516 (8.95), 3.527 (15.32), 3.540 (13.47), 3.599 (0.55), 3.643 (13.36), 3.656 (16.00), 3.667 (9.74), 3.996 (9.49), 4.010 (9.36), 4.035 (0.87), 4.222 (5.55), 4.373 (0.78), 4.388 (0.76), 4.496 (1.16), 7.906 (11.75), 8.631 (0.55), 8.948 (1.85), 8.963 (3.75), 8.977 (1.80), 9.167 (4.32).

### Intermediate 74

### tert-butyl [(5-bromo-1H-imidazol-2-yl)methyl]carbamate

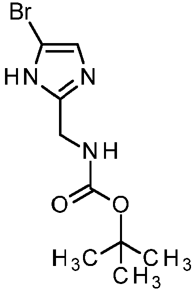

Tert-butyl [(1H-imidazol-2-yl)methyl]carbamate (654 mg, 3.32 mmol; CAS-RN:[203664-05-5]) was provided in N,N-dimethylformamide (30 mL), cooled to -50 °C, 1-bromopyrrolidine-2,5-dione (590 mg, 3.32 mmol; CAS-RN:[128-08-5]) was added and the mixture was allowed to warm up to rt. The reaction mixture was concentrated and the residue was purified by column chromatography twice (1. silica gel, dichlormethane / ethanol gradient; 2. silica gel aminophase, dichlormethane / ethanol gradient) to give 313 mg (34 % yield) of the title compound.
LC-MS (Method 1): Rt = 0.78 min; MS (ESlpos): m/z = 276 [M+H]⁺
¹H-NMR (400 MHz, DMSO-d6) δ [ppm]: 1.323 (0.41), 1.363 (0.92), 1.384 (16.00), 2.518 (0.90), 2.523 (0.71), 2.563 (0.78), 2.728 (0.40), 2.889 (0.47), 4.077 (2.12), 4.091 (2.05), 4.248 (0.16), 5.758 (0.19), 7.019 (0.20), 7.022 (0.20), 7.112 (2.09), 7.116 (2.09), 7.259 (0.32), 7.273 (0.56), 7.287 (0.30), 12.127 (0.39).

### Intermediate 75

### tert-butyl {[5-(6-methoxypyridin-3-yl)-1H-imidazol-2-yl]methyl}carbamate

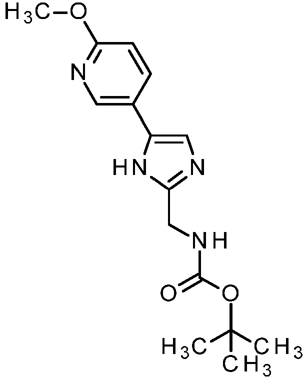

A first batch of tert-butyl [(5-bromo-1H-imidazol-2-yl)methyl]carbamate **(Intermediate 74,** 50.0 mg, 181 µmol), (6-methoxypyridin-3-yl)boronic acid (22.2 mg, 145 µmol; CAS-RN:[163105-89-3]) and potassium carbonate (62.6 mg, 435 µmol) were dissolved in 1,4-dioxane (3 mL) and water (1 mL). 1,1'-Bis(diphenylphosphino)ferrocenepalladium(II) chloride (6.6 mg, 9 µmol; CAS-RN:[72287-26-4]) was added. The mixture was flushed with argon and stirred over night at 80 °C. A second batch of tert-butyl [(5-bromo-1H-imidazol-2-yl)methyl]carbamate **(Intermediate 74,** 101 mg, 366 µmol), (6-methoxypyridin-3-yl)boronic acid (67.1 mg, 439 µmol; CAS-RN:[163105-89-3]) and potassium carbonate (153 mg, 1.10 mmol) were dissolved in 1,4-dioxane (6.1 mL) and water (2 mL). 1,1'-Bis(diphenylphosphino)ferrocenepalladium(II) chloride (13.4 mg, 18.3 µmol; CAS-RN:[72287-26-4]) was added. The mixture was flushed with argon and stirred for 3 h at 80 °C. The two reaction mixtures were combined, concentrated and purified by column chromatography (silica gel NH2, dichlormethane / ethyl acetate gradient) to give 116 mg of the title compound
LC-MS (Method 2): Rt = 0.93 min; MS (ESlpos): m/z = 305 [M+H]⁺
¹H-NMR (400 MHz, DMSO-d6) δ [ppm]: 1.066 (0.97), 1.154 (0.42), 1.172 (0.89), 1.190 (0.45), 1.233 (0.41), 1.385 (11.05), 1.399 (16.00), 1.987 (1.44), 2.336 (0.21), 2.518 (3.43), 2.522 (2.24), 2.660 (0.22), 3.842 (15.36), 3.857 (0.91), 4.017 (0.31), 4.035 (0.32), 4.077 (0.89), 4.091 (0.88), 4.114 (0.67), 4.128 (0.70), 4.157 (1.97), 4.171 (2.02), 4.209 (0.18), 4.214 (0.18), 6.780 (1.93), 6.801 (1.86), 6.855 (0.16), 6.875 (0.17), 6.976 (0.16), 7.114 (1.05), 7.190 (0.20), 7.208 (0.28), 7.274 (0.58), 7.289 (0.74), 7.302 (0.35), 7.461 (2.04), 7.466 (2.00), 7.480 (0.16), 7.552 (0.22), 7.987 (1.49), 7.993 (1.38), 8.009 (1.34), 8.014 (1.38), 8.515 (1.73), 8.519 (1.73), 11.870 (0.62), 12.132 (0.17).

### Intermediate 76

### 1-[5-(6-methoxypyridin-3-yl)-1H-imidazol-2-yl]methanamine

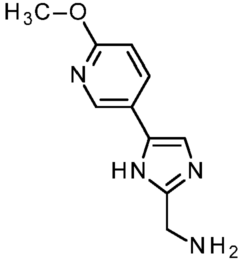

To tert-butyl {[5-(6-methoxypyridin-3-yl)-1H-imidazol-2-yl]methyl}carbamate **(Intermediate 75,** 110 mg, 361 µmol) was added a solution of HCl in 1,4-dioxane (1.8 mL, 4.0 M, 7.2 mmol CAS-RN:[7647-01-0]). 1,4-dioxane (5.0 mL) was added and the mixture was stirred for 90 min at rt. The reaction mixture was concentrated and diluted with ethyl acetate. The organic phase was washed with a saturated aqueous sodium bicarbonate solution, dried over a hydrophobic filter and concentrated to give a crude product. The residue was purified by column chromatography (silica gel, dichlormethane/ ethanol gradient) to give 44 mg (59 % yield) of the title compound.
LC-MS (Method 2): Rt = 0.62 min; MS (ESlpos): m/z = 205 [M+H]⁺
¹H-NMR (400 MHz, DMSO-d6) δ [ppm]: 1.052 (0.17), 1.231 (0.77), 1.840 (2.05), 2.332 (0.50), 2.336 (0.25), 2.518 (2.76), 2.523 (1.97), 2.678 (0.24), 3.333 (2.17), 3.428 (0.34), 3.445 (0.25), 3.461 (0.21), 3.641 (3.62), 3.657 (0.17), 3.679 (3.27), 3.727 (16.00), 3.860 (0.33), 5.760 (0.88), 6.782 (1.89), 6.804 (1.99), 6.867 (0.23), 7.101 (1.90), 7.430 (0.59), 7.978 (1.70), 7.984 (1.83), 8.000 (1.66), 8.006 (1.75), 8.505 (2.23), 8.509 (2.35).

### Intermediate 77

### N-{[5-(6-methoxypyridin-3-yl)-1H-imidazol-2-yl]methyl}-2-(methylsulfanyl)-8-(trifluoromethyl)pyrazolo[1,5-a][1,3,5]triazin-4-amine

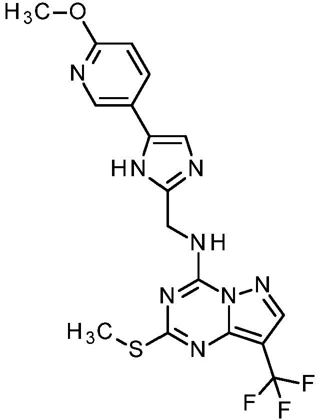

2,4-bis(methylsulfanyl)-8-(trifluoromethyl)pyrazolo[1,5-a][1,3,5]triazine **(Intermediate 42,** 69.2 mg, 247 µmol) and 1-[5-(6-methoxypyridin-3-yl)-1H-imidazol-2-yl]methanamine **(Intermediate 76,** 42.0 mg, 206 µmol) were dissolved in acetonitrile (2 mL). N,N-diisopropylethylamine (110 µL, 620 µmol; CAS-RN:[7087-68-5]) was added and the mixture was stirred for 1 h at 60 °C and over night at 50 °C. The reaction mixture was concentrated to give a crude product. The residue was purified by column chromatography (silica gel, dichlormethane / ethanol gradient) to give 54 mg (50 % yield) of the title compound.
LC-MS (Method 2): Rt = 1.16 min; MS (ESlpos): m/z = 437 [M+H]⁺
¹H-NMR (400 MHz, DMSO-d6) δ [ppm]: 1.035 (1.34), 1.053 (2.72), 1.070 (1.22), 1.232 (0.64), 2.318 (0.55), 2.337 (0.52), 2.464 (4.56), 2.474 (16.00), 2.518 (6.97), 2.523 (5.36), 2.660 (0.56), 2.679 (0.55), 3.422 (0.43), 3.435 (0.44), 3.845 (15.64), 4.355 (0.43), 4.739 (0.99), 4.791 (3.81), 5.759 (14.97), 6.791 (1.60), 6.812 (1.72), 7.526 (1.83), 7.530 (1.82), 7.993 (1.18), 8.000 (1.25), 8.015 (1.11), 8.021 (1.19), 8.489 (0.80), 8.502 (3.44), 8.523 (1.71), 8.528 (1.70), 9.651 (0.51), 11.953 (0.75).

### Intermediate 78

### {[2-(methylsulfanyl)-8-(trifluoromethyl)pyrazolo[1,5-a][1,3,5]triazin-4-yl]amino}acetonitrile

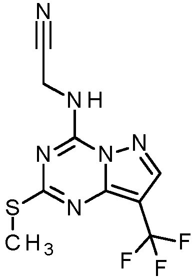

2,4-bis(methylsulfanyl)-8-(trifluoromethyl)pyrazolo[1,5-a][1,3,5]triazine **(Intermediate 42,** 1.93 g, 6.89 mmol) was provided in acetonitrile (50 mL). Aminoacetonitrile hydrogen chloride (956 mg, 10.3 mmol) and N,N-diisopropylethylamine (4.8 mL, 28 mmol; CAS-RN:[7087-68-5]) were added and the mixture was stirred for 24 h at 70 °C. Aminoacetonitrile hydrogen chloride (637 mg, 6.8 mmol) was added and the mixture was stirred for 3 h at 70 °C and 18 h at 80 °C. The reaction mixture was poured into water and extracted with ethyl acetate. The organic phase was dried over sodium sulfate, filtered and concentrated to give a crude product. The residue was purified by column chromatography (silica gel, dichlormethane / ethyl acetate gradient). The product was stirred in pentane. The precipitate was collected by filtration and dried to give 1.30 g (60 % yield) of the title compound.
LC-MS (Method 1): Rt = 1.12 min; MS (ESlpos): m/z = 289 [M+H]⁺
¹H-NMR (400 MHz, DMSO-d6) δ [ppm]: 1.102 (0.48), 1.349 (0.79), 1.366 (0.84), 2.518 (1.47), 2.523 (1.00), 2.547 (0.43), 2.567 (16.00), 4.564 (6.50), 8.437 (0.58), 8.527 (3.56), 9.819 (0.53).

### Intermediate 79

### {[2-(methylsulfinyl)-8-(trifluoromethyl)pyrazolo[1,5-a][1,3,5]triazin-4-yl]amino}acetonitrile

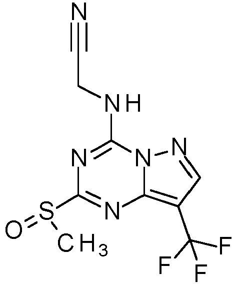

{[2-(methylsulfanyl)-8-(trifluoromethyl)pyrazolo[1,5-a][1,3,5]triazin-4-yl]amino}acetonitrile **(Intermediate 78,** 4.01 g, 13.9 mmol) was provided in dichlormethane (100 mL), cooled with an ice-bath, 3-chlorobenzene-1-carboperoxoic acid (4.68 g, 77 % purity, 20.9 mmol; CAS-RN:[937-14-4]) was added and the mixture stirred over night at rt. The precipitate was collected by filtration and washed with dichlormethane. The solid was dried and stirred in an aqueous solution of sodium bicarbonate. The precipitate was filtered and dried to give 1.91 g (43 % yield) of the title compound.
LC-MS (Method 1): Rt = 0.74 min; MS (ESlpos): m/z = 305 [M+H]⁺
¹H-NMR (400 MHz, DMSO-d6) δ [ppm]: 2.518 (0.96), 2.523 (0.67), 2.961 (16.00), 3.420 (0.64), 4.600 (0.40), 4.644 (3.43), 4.653 (3.34), 4.697 (0.44), 8.750 (3.35), 10.300 (0.74).

### Intermediate 80

### {[2-(morpholin-4-yl)-8-(trifluoromethyl)pyrazolo[1,5-a][1,3,5]triazin-4-yl]amino}acetonitrile

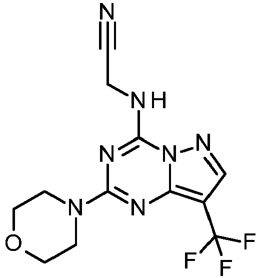

{[2-(methanesulfinyl)-8-(trifluoromethyl)pyrazolo[1,5-a][1,3,5]triazin-4-yl]amino}acetonitrile **(Intermediate 79**, 1.90 g, 6.24 mmol) and morpholine (1.6 mL, 19 mmol; CAS-RN:[110-91-8]) were provided in acetonitrile (75 mL). N,N-diisopropylethylamine (3.3 mL, 19 mmol; CAS-RN:[7087-68-5]) was added and the mixture stirred over night at 70 °C. The reaction mixture was poured into water. The precipitate was collected by filtration, washed with water and dried to give 1.89 g (92 % yield) of the title compound.
LC-MS (Method 1): Rt = 1.07 min; MS (ESlneg): m/z = 326 [M-H]⁻
¹H-NMR (400 MHz, DMSO-d6) δ [ppm]: 2.073 (4.21), 2.332 (0.70), 2.518 (3.63), 2.522 (2.49), 2.673 (0.71), 3.655 (4.78), 3.666 (8.17), 3.679 (6.81), 3.803 (2.80), 4.521 (16.00), 8.262 (9.35), 9.354 (1.01).

### Intermediate 81

### N-hydroxy{[2-(morpholin-4-yl)-8-(trifluoromethyl)pyrazolo[1,5-a][1,3,5]triazin-4-yl]amino}ethanimidamide

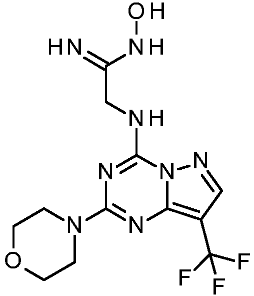

{[2-(morpholin-4-yl)-8-(trifluoromethyl)pyrazolo[1,5-a][1,3,5]triazin-4-yl]amino}acetonitrile **(Intermediate 80,** 1.89 g, 5.78 mmol) was dissolved in methanol (100 mL). Hydroxylamine hydrogen chloride (1.81 g, 26.0 mmol) and triethylamine (3.6 mL, 26 mmol; CAS-RN:[121-44-8]) were added and the mixture was stirred for 72 h at rt. The solid was collected by filtration, washed with methanol and dried to give 1.94 g (91 % yield) of the title compound.
LC-MS (Method 1): Rt = 0.80 min; MS (ESlneg): m/z = 359 [M-H]⁻
¹H-NMR (400 MHz, DMSO-d6) δ [ppm]: 2.331 (0.67), 2.518 (4.09), 2.523 (2.78), 2.673 (0.67), 3.632 (4.35), 3.642 (7.80), 3.655 (6.70), 3.706 (0.42), 3.750 (5.09), 4.063 (3.75), 4.070 (3.70), 5.475 (6.09), 8.205 (8.75), 8.668 (1.51), 9.144 (16.00).

### Intermediate 82

### {[2-(morpholin-4-yl)-8-(trifluoromethyl)pyrazolo[1,5-a][1,3,5]triazin-4-yl]amino}ethanimidamide hydrogen chloride

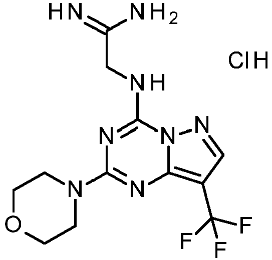

N-hydroxy{[2-(morpholin-4-yl)-8-(trifluoromethyl)pyrazolo[1,5-a][1,3,5]triazin-4-yl]amino}ethanimidamide (**Intermediate 81,** 264 mg, 733 µmol) was provided in a mixture of 10 mL of ethanol / water (1:1). Iron (246 mg, 4.40 mmol; CAS-RN:[7439-89-6]) was added and the mixture was heated to 100 °C. A solution of 1N HCl in 3 mL of ethanol / water (1:1) was added dropwise and the mixture was stirred for 1 h at 100 °C. The reaction mixture was filtered hot over celite and washed with ethanol. The filtrate was concentrated. The residue was diluted with ethanol and concentrated several times and dried to give a crude product. The crude product was purified preparative HPLC [Waters Autopurificationsystem; Column: Waters XBridge C18 100*30mm* 5 µm; eluent A: water (0.1% TFA (99%)), eluent B: acetonitrile; gradient: 0.0-0.5 min 14% B (25-70 mL/min), 0.51-5.5 min 14-34% B; flow 70 mL/min; Detector: DAD scan 210-400nm] to give 112 mg (39 % yield) of the title compound.
LC-MS (Method 1): Rt = 0.77 min; MS (ESlneg): m/z = 343 [M-H]⁻
¹H-NMR (400 MHz, DMSO-d6) δ [ppm]: 1.232 (0.67), 2.075 (1.36), 2.332 (2.81), 2.336 (1.20), 2.518 (16.00), 2.523 (11.27), 2.673 (2.82), 2.678 (1.24), 3.651 (13.08), 3.678 (5.48), 3.728 (13.39), 3.741 (13.95), 3.751 (9.46), 4.395 (7.41), 4.410 (6.68), 6.964 (2.00), 7.091 (2.31), 7.219 (2.01), 8.208 (3.24), 8.265 (12.16), 8.803 (7.71), 8.938 (7.68), 9.023 (1.72), 9.037 (3.67), 9.052 (1.66).

### Example 1

### 8-bromo-2-(morpholin-4-yl)-N-({5-[4-(trifluoromethyl)phenyl]-4H-1,2,4-triazol-3-yl}methyl)pyrazolo[1,5-a][1,3,5]triazin-4-amine

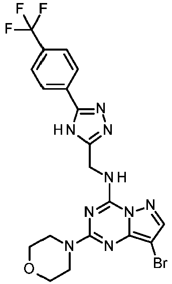

### 2-{[8-bromo-2-(morpholin-4-yl)pyrazolo[1,5-a][1,3,5]triazin-4-yl]amino}acetohydrazide

(**Intermediate 11,** 80.0 mg, 216 µmol), 4-(trifluoromethyl)benzene-1-carboximidamide hydrogen chloride (1/1) (58.1 mg, 259 µmol) and sodium ethylate (29.3 mg, 431 µmol; CAS-RN:[141-52-6]) were dissolved in N,N-dimethylformamide (2.5 mL). The mixture was stirred for 45 min at 180 °C in a microwave. The reaction mixture was filtered and the filtrate was purified by preparative HPLC (HT basic) to give 34.0 mg (29 % yield) of the title compound.
LC-MS (Method 2): Rₜ = 0.91 min; MS (ESlpos): m/z = 526 [M+H]⁺
¹H-NMR (400 MHz, DMSO-d6) δ [ppm]: 1.232 (0.62), 1.249 (0.43), 2.337 (1.11), 2.518 (13.96), 2.523 (9.58), 2.679 (1.11), 3.539 (8.34), 3.650 (8.96), 4.818 (3.09), 7.838 (3.09), 7.976 (0.80), 7.996 (1.11), 8.031 (16.00), 8.111 (0.99), 8.130 (0.74), 8.167 (7.41), 8.187 (6.42), 9.176 (1.30), 14.117 (0.62).

### Example 2

### 8-bromo-2-(morpholin-4-yl)-N-({5-[4-(trifluoromethoxy)phenyl]-4H-1,2,4-triazol-3-yl}methyl)pyrazolo[1,5-a][1,3,5]triazin-4-amine

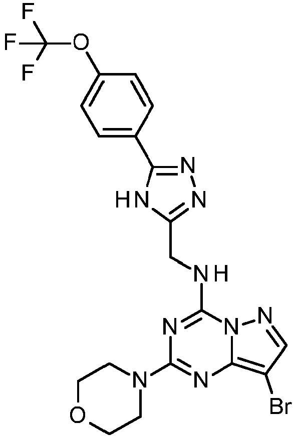

2-{[8-bromo-2-(morpholin-4-yl)pyrazolo[1,5-a][1,3,5]triazin-4-yl]amino}acetohydrazide **(Intermediate 11,** 80.0 mg, 216 µmol), 4-(trifluoromethoxy)benzene-1-carboximidamide hydrogen chloride (1/1) (62.2 mg, 259 µmol; CAS-RN:[121219-95-2]) and sodium ethylate (29.3 mg, 431 µmol; CAS-RN:[141-52-6]) were dissolved in N,N-dimethylformamide (2.5 mL). The mixture was stirred for 45 min at 180 °C in a microwave. The reaction mixture was filtered, and the filtrate was purified by preparative HPLC (HT basic) to give 38.7 mg (32 % yield) of the title compound.
LC-MS (Method 2): Rₜ = 0.97 min; MS (ESlpos): m/z = 540 [M+H]⁺
¹H-NMR (400 MHz, DMSO-d6) δ [ppm]: 2.332 (1.21), 2.336 (0.52), 2.518 (9.19), 2.523 (6.70), 2.540 (1.41), 2.673 (1.21), 3.542 (5.02), 3.642 (5.41), 3.654 (6.40), 4.787 (4.52), 7.453 (3.37), 7.473 (3.59), 8.025 (16.00), 8.039 (0.41), 8.058 (1.05), 8.064 (9.35), 8.070 (2.92), 8.081 (2.76), 8.087 (8.39), 8.093 (1.02), 9.141 (0.63).

### Example 3

### 8-bromo-2-(morpholin-4-yl)-N-({5-[4-(trifluoromethyl)phenyl]-1H-imidazol-2-yl}methyl)pyrazolo[1,5-a][1,3,5]triazin-4-amine

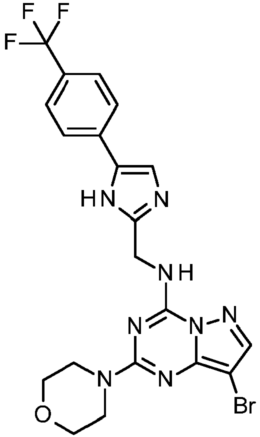

{[8-bromo-2-(morpholin-4-yl)pyrazolo[1,5-a][1,3,5]triazin-4-yl]amino}ethanimidamide hydrogen chloride (1/1) (**Intermediate 15,** 150 mg, 383 µmol) and 2-bromo-1-[4-(trifluoromethyl)phenyl]ethan-1-one (133 mg, 498 µmol) were dissolved in N,N-dimethylformamide (2.5 mL). Cesium carbonate (499 mg, 1.53 mmol; CAS-RN:[534-17-8]) was added and the reaction mixture was stirred over night at 90 °C. The mixture was filtered, and the filtrate was purified by preparative HPLC (HT basic) to give 20.5 mg (9 % yield) of the title compound.
LC-MS (Method 2): Rt = 1.26 min; MS (ESlpos): m/z = 523 [M+H]⁺
¹H-NMR (400 MHz, DMSO-d6) δ [ppm]: 2.331 (1.20), 2.336 (0.53), 2.518 (5.84), 2.522 (3.97), 2.539 (0.45), 2.669 (1.63), 2.673 (1.20), 3.561 (5.09), 3.669 (6.27), 3.682 (6.83), 3.693 (3.87), 3.903 (0.43), 4.716 (3.81), 4.729 (3.55), 7.656 (4.56), 7.676 (5.15), 7.729 (4.29), 7.733 (4.35), 7.755 (0.45), 7.815 (0.43), 7.931 (5.12), 7.952 (4.24), 8.013 (16.00), 8.026 (0.99), 9.024 (1.44), 12.125 (1.71).

### Example 4

### 8-bromo-2-(morpholin-4-yl)-N-({5-[4-(trifluoromethoxy)phenyl]-1H-imidazol-2-yl}methyl)pyrazolo[1,5-a][1,3,5]triazin-4-amine

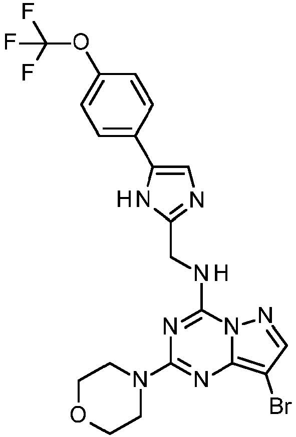

{[8-bromo-2-(morpholin-4-yl)pyrazolo[1,5-a][1,3,5]triazin-4-yl]amino}ethanimidamide hydrogen chloride (1/1) (**Intermediate 15,** 150 mg, 383 µmol) and 2-bromo-1-[4-(trifluoromethoxy)phenyl]ethan-1-one (141 mg, 498 µmol) were dissolved in N,N-dimethylformamide (2.5 mL). Cesium carbonate (499 mg, 1.53 mmol; CAS-RN:[534-17-8]) was added and the reaction mixture was stirred over night at 90 °C. The mixture was filtered, and the filtrate was purified by preparative HPLC (HT basic) to give 22.7 mg (10 % yield) of the title compound.
LC-MS (Method 2): Rt = 1.28 min; MS (ESlpos): m/z = 539 [M+H]⁺
¹H-NMR (400 MHz, DMSO-d6) δ [ppm]: 2.327 (1.81), 2.331 (1.33), 2.336 (0.60), 2.518 (6.27), 2.523 (4.19), 2.669 (1.87), 2.673 (1.33), 2.678 (0.63), 3.564 (4.91), 3.670 (6.03), 3.683 (6.54), 3.693 (3.62), 4.702 (3.68), 4.715 (3.40), 7.298 (3.95), 7.318 (4.43), 7.591 (4.25), 7.596 (4.19), 7.706 (0.51), 7.728 (0.45), 7.818 (0.90), 7.825 (7.44), 7.830 (2.38), 7.841 (2.23), 7.846 (6.51), 7.854 (0.72), 8.009 (16.00), 8.023 (1.11), 9.007 (1.42), 12.022 (1.66).

### Example 5

### 2-(morpholin-4-yl)-N-({5-[4-(trifluoromethoxy)phenyl]-4H-1,2,4-triazol-3-yl}methyl)-8-(trifluoromethyl)pyrazolo[1,5-a][1,3,5]triazin-4-amine

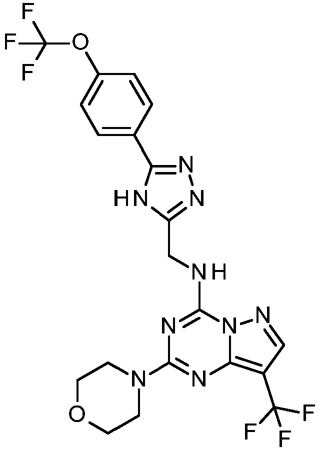

2-{[2-(morpholin-4-yl)-8-(trifluoromethyl)pyrazolo[1,5-a][1,3,5]triazin-4-yl]amino}acetohydrazide **(Intermediate 24,** 125 mg, 347 µmol) and 4-(trifluoromethoxy)benzene-1-carboximidamide hydrogen chloride (1/1) (100 mg, 416 µmol) were dissolved in N,N-dimethylformamide (3.0 mL). Sodium ethylate (47.2 mg, 694 µmol; CAS-RN:[141-52-6]) was added and the mixture was stirred for 45 min at 180 °C in a microwave. The reaction mixture was poured into water and extracted with ethyl acetate. The combined organic layers were concentrated and the residue was purified by preparative HPLC (HT acid) to give 87.2 mg (47 % yield) of the title compound.
LC-MS (Method 1): Rₜ = 1.33 min; MS (ESlpos): m/z = 530 [M+H]⁺
¹H-NMR (400 MHz, DMSO-d6) δ [ppm]: 2.336 (0.86), 2.518 (12.00), 2.523 (8.22), 2.674 (1.86), 2.678 (0.86), 3.548 (5.90), 3.669 (9.47), 4.804 (4.11), 7.455 (3.75), 7.474 (3.89), 8.061 (1.96), 8.068 (16.00), 8.073 (4.95), 8.085 (4.88), 8.090 (13.84), 8.097 (1.68), 8.247 (15.14), 9.300 (1.42).

### Example 6

### 2-(morpholin-4-yl)-8-(trifluoromethyl)-N-({5-[4-(trifluoromethyl)phenyl]-4H-1,2,4-triazol-3-yl}methyl)pyrazolo[1,5-a][1,3,5]triazin-4-amine

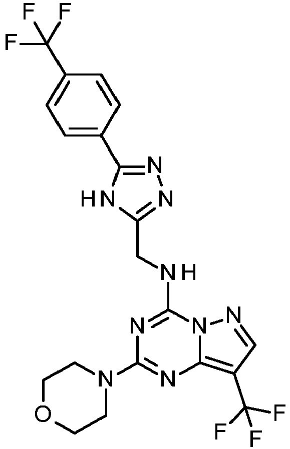

2-{[2-(morpholin-4-yl)-8-(trifluoromethyl)pyrazolo[1,5-a][1,3,5]triazin-4-yl]amino}acetohydrazide **(intermediate 24,** 125 mg, 347 µmol) and 4-(trifluoromethyl)benzene-1-carboximidamide hydrogen chloride (1/1) (93.5 mg, 416 µmol) were dissolved in N,N-dimethylformamide (3.0 mL). Sodium ethylate (47.2 mg, 694 µmol; CAS-RN:[141-52-6]) was added and the mixture was stirred for 45 min at 180 °C in a microwave. The reaction mixture was poured into water and extracted with ethyl acetate. The combined organic layers were concentrated and the residue was purified by preparative HPLC (HT acid) to give 49.6 mg (28 % yield) of the title compound.
LC-MS (Method 1): Rt = 1.31 min; MS (ESlpos): m/z = 514 [M+H]⁺
¹H-NMR (400 MHz, DMSO-d6) δ [ppm]: 2.331 (1.94), 2.518 (13.23), 2.523 (8.62), 2.673 (1.93), 3.546 (6.18), 3.668 (10.32), 4.829 (5.64), 7.825 (5.47), 7.845 (5.97), 8.170 (10.26), 8.190 (8.84), 8.252 (16.00), 9.321 (1.98), 14.158 (0.49).

### Example 7

### 2-(morpholin-4-yl)-7-(trifluoromethyl)-N-({5-[4-(trifluoromethyl)phenyl]-1H-imidazol-2-yl}methyl)imidazo[2, 1-f][1 ,2,4]triazin-4-amine

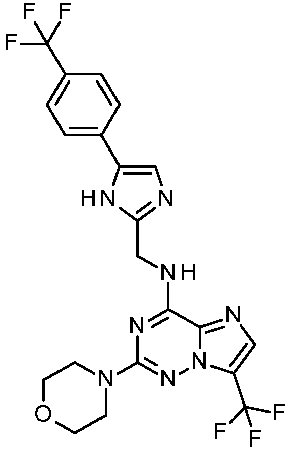

### 2-(methanesulfonyl)-4-(methylsulfanyl)-7-(trifluoromethyl)imidazo[2,1-f][1,2,4]triazine

(**Intermediate 33,** 50.0 mg, 160 µmol) and 1-{5-[4-(trifluoromethyl)phenyl]-1H-imidazol-2-yl}methanamine (**Intermediate 36,** 42.5 mg, 176 µmol) were dissolved in acetonitrile (3 mL). N,N-diisopropylethylamine (112 µL, 640 µmol; CAS-RN:[7087-68-5]) was added and the mixture was stirred for 2 h at 150 °C in a microwave. To the crude reaction mixture was added morpholine (9.5 mg, 109 µmol; CAS-RN:[110-91-8]) and the mixture was stirred for 1 h at 70 °C and over night at 60 °C. The reaction mixture was concentrated. The residue was purified by flash chromatography (silica gel, dichloromethane / ethyl acetate / ethanol gradient) to give a crude product. The crude product was purified by flash chromatography (silica gel, dichloromethane / ethanol gradient) to give 5.0 mg (9 % yield) of the title compound.
LC-MS (Method 2): Rt = 1.32 min; MS (ESlpos): m/z = 513 [M+H]⁺
¹H-NMR (400 MHz, DMSO-d6) δ [ppm]: 0.851 (0.83), 1.230 (4.70), 1.256 (1.35), 1.295 (0.49), 1.352 (0.45), 1.906 (0.59), 2.326 (3.56), 2.332 (2.48), 2.335 (1.12), 2.518 (16.00), 2.522 (10.40), 2.668 (3.58), 2.673 (2.51), 2.678 (1.15), 3.499 (5.56), 3.510 (5.16), 3.572 (5.44), 3.584 (5.57), 4.742 (3.76), 4.756 (3.64), 7.656 (3.21), 7.676 (3.81), 7.720 (3.05), 7.725 (3.02), 7.819 (0.53), 7.841 (0.84), 7.926 (5.84), 7.933 (3.80), 7.953 (2.99), 8.195 (0.44), 9.318 (0.87), 9.332 (1.66), 9.346 (0.78), 12.079 (1.38).

### Example 8

### 2-(morpholin-4-yl)-N-({5-[4-(trifluoromethoxy)phenyl]-1H-imidazol-2-yl}methyl)-7-(trifluoromethyl)imidazo[2,1-f][1,2,4]triazin-4-amine

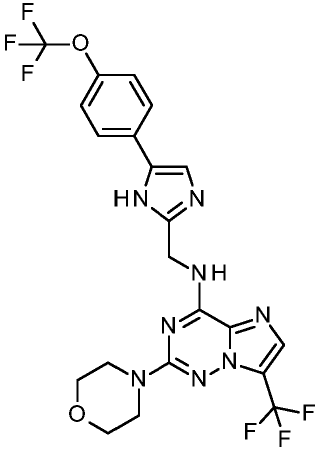

2-(methanesulfonyl)-N-({5-[4-(trifluoromethoxy)phenyl]-1H-imidazol-2-yl}methyl)-7-(trifluoromethyl)imidazo[2,1-f][1,2,4]triazin-4-amine (**Intermediate 39,** 32.0 mg, 61.4 µmol) was dissolved in acetonitrile (1.0 mL). Morpholine (7.0 µL, 80 µmol) and N,N-diisopropylethylamine (32 µL, 180 µmol; CAS-RN:[7087-68-5]) were added and the mixture was stirred for 23 h at 70 °C. Morpholin (100 µL, 562.5 µmol) was added and the reaction mixture was stirred further 48 h at 70 °C. The reaction mixture was poured into water and extracted with ethyl acetate. The combined organic layers were concentrated and the residue was purified by preparative HPLC (HT basic) to give 8.0 mg (23 % yield) of the title compound
LC-MS (Method 2): Rt = 1.38 min; MS (ESlpos): m/z = 529 [M+H]⁺
¹H-NMR (400 MHz, DMSO-d6) δ [ppm]: 0.833 (0.58), 0.850 (0.94), 1.232 (5.94), 2.540 (16.00), 3.500 (12.25), 3.512 (10.90), 3.575 (11.56), 3.587 (11.98), 4.730 (7.91), 4.744 (7.47), 7.297 (6.77), 7.317 (7.50), 7.383 (0.55), 7.403 (0.55), 7.584 (6.84), 7.588 (6.76), 7.706 (0.58), 7.727 (0.54), 7.827 (10.52), 7.849 (9.55), 7.923 (10.19), 9.303 (1.84), 9.317 (3.56), 9.331 (1.70), 11.972 (3.15).

### Example 9

### 2-(morpholin-4-yl)-8-(trifluoromethyl)-N-({5-[4-(trifluoromethyl)phenyl]-1H-imidazol-2-yl}methyl)pyrazolo[1,5-a][1,3,5]triazin-4-amine

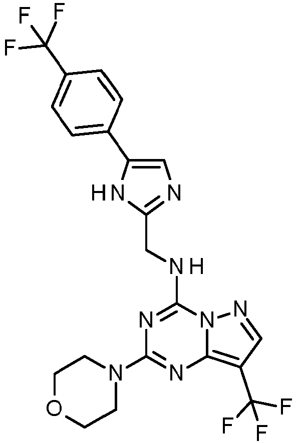

2-(methanesulfonyl)-8-(trifluoromethyl)-N-({5-[4-(trifluoromethyl)phenyl]-1H-imidazol-2-yl}methyl)pyrazolo[1,5-a][1,3,5]triazin-4-amine (**Intermediate 46,** 100 mg, 60 % purity, 119 µmol) was dissolved in acetonitrile (2.0 mL). Morpholine (13 µL, 150 µmol) and N,N-diisopropylethylamine (62 µL, 360 µmol; CAS-RN:[7087-68-5]) were added and the mixture was stirred for 72 h at 70 °C. The reaction mixture was diluted with water and extracted with ethyl acetate. The combined organic layers were concentrated and the residue was purified by preparative HPLC (HT basic) to give 19.6 mg (32 % yield) of the title compound
LC-MS (Method 2): Rt = 1.33 min; MS (ESlpos): m/z = 513 [M+H]⁺
¹H-NMR (400 MHz, DMSO-d6) δ [ppm]: 0.845 (0.47), 1.227 (2.65), 2.518 (4.44), 2.535 (1.40), 3.561 (7.54), 3.681 (11.16), 3.692 (13.17), 4.733 (8.43), 7.655 (6.47), 7.676 (7.54), 7.734 (8.15), 7.931 (7.29), 7.951 (6.13), 8.229 (16.00), 8.473 (0.41), 9.180 (2.09), 12.128 (2.08).

### Example 10

### 2-(morpholin-4-yl)-N-({5-[4-(trifluoromethoxy)phenyl]-1H-imidazol-2-yl}methyl)-8-(trifluoromethyl)pyrazolo[1,5-a][1,3,5]triazin-4-amine

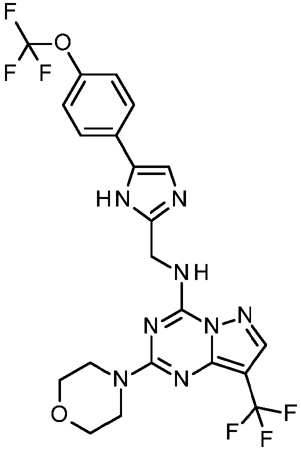

2-(methanesulfonyl)-N-({5-[4-(trifluoromethoxy)phenyl]-1H-imidazol-2-yl}methyl)-8-(trifluoromethyl)pyrazolo[1,5-a][1,3,5]triazin-4-amine (**Intermediate 50,** 80.0 mg, 66 % purity, 101 µmol) was dissolved in acetonitrile (2.0 mL). Morpholine (12 µL, 130 µmol) and N,N-diisopropylethylamine (53 µL, 300 µmol; CAS-RN:[7087-68-5]) were added and the mixture was stirred for 20 h at 70 °C. The reaction mixture was diluted with water and extracted with ethyl acetate. The combined organic layers were concentrated and the residue was purified by preparative HPLC (HT basic) to give 16.5 mg (30 % yield) of the title compound.
LC-MS (Method 2): Rt = 1.33 min; MS (ESlpos): m/z = 529 [M+H]⁺
¹H-NMR (400 MHz, DMSO-d6) δ [ppm]: 0.850 (0.51), 0.940 (0.59), 0.957 (0.58), 1.232 (2.65), 2.332 (1.63), 2.336 (0.72), 2.518 (8.13), 2.523 (5.17), 2.673 (1.65), 3.567 (6.91), 3.686 (10.74), 3.698 (12.18), 3.708 (7.00), 4.722 (7.07), 7.300 (6.81), 7.321 (7.50), 7.388 (0.48), 7.405 (0.44), 7.599 (6.88), 7.602 (6.71), 7.706 (0.48), 7.725 (0.44), 7.828 (11.73), 7.833 (4.12), 7.845 (3.84), 7.850 (10.35), 8.229 (16.00), 9.165 (1.85), 12.025 (2.88).

### Example 11

### 2-(morpholin-4-yl)-7-(trifluoromethyl)-N-({5-[4-(trifluoromethyl)phenyl]-4H-1,2,4-triazol-3-yl}methyl)imidazo[2, 1-f][1 ,2,4]triazin-4-amine

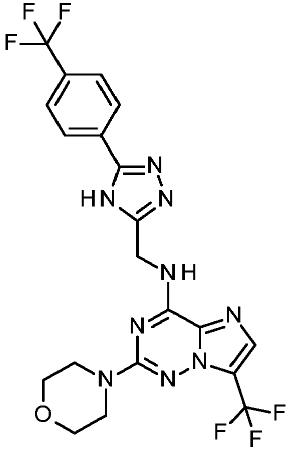

2-(methanesulfonyl)-7-(trifluoromethyl)-N-({5-[4-(trifluoromethyl)phenyl]-4H-1,2,4-triazol-3-yl}methyl)imidazo[2,1-f][1,2,4]triazin-4-amine (**Intermediate 55,** 60.0 mg, 118 µmol) was dissolved in acetonitrile (2.2 mL). Morpholine (41 µL, 470 µmol; CAS-RN:[110-91-8]) and N,N-diisopropylethylamine (100 µL, 590 µmol; CAS-RN:[7087-68-5]) were added and the mixture was stirred for 6 h at 70 °C. Morpholine (3 mL, 34.4 mmol; CAS-RN:[110-91-8]) was added and the mixture was stirred for 3 h at 100 °C and for 5 days at rt. The reaction mixture was concentrated and the residue was purified by column chromatography (silica gel, dichlormethane / ethyl acetate gradient) to give 29.0 mg (43 % yield) of the title compound.
LC-MS (Method 2): Rₜ = 0.99 min; MS (ESlpos): m/z = 514 [M+H]⁺
¹H-NMR (400 MHz, DMSO-d6) δ [ppm]: 0.851 (0.86), 1.232 (2.44), 1.983 (0.99), 2.005 (0.46), 2.337 (1.16), 2.518 (16.00), 2.523 (11.15), 2.571 (0.46), 2.635 (1.02), 2.647 (1.22), 2.678 (1.21), 3.357 (1.07), 3.371 (2.95), 3.381 (0.99), 3.392 (0.72), 3.416 (0.66), 3.454 (6.26), 3.465 (10.96), 3.477 (10.18), 3.499 (0.99), 3.506 (1.08), 3.519 (1.06), 3.530 (1.17), 3.549 (11.18), 3.561 (12.39), 3.572 (7.74), 3.659 (0.97), 3.672 (0.96), 4.829 (5.25), 4.840 (5.24), 7.822 (6.57), 7.843 (7.15), 7.885 (0.51), 7.942 (9.05), 7.943 (9.25), 8.166 (8.88), 8.186 (7.65), 9.470 (2.23), 14.150 (0.48).

### Example 12

### 2-(morpholin-4-yl)-7-(trifluoromethyl)-N-({5-[6-(trifluoromethyl)pyridin-3-yl]-1H-imidazol-2-yl}methyl)imidazo[2,1-f][1,2,4]triazin-4-amine

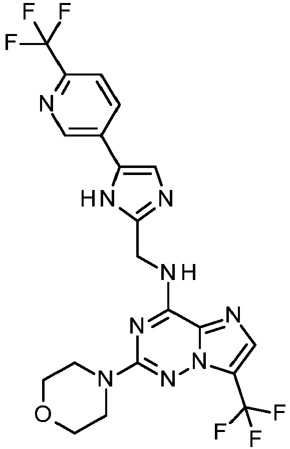

2-(methanesulfonyl)-7-(trifluoromethyl)-N-({5-[6-(trifluoromethyl)pyridin-3-yl]-1H-imidazol-2-yl}methylimidazo[2,1-f][1,2,4]triazin-4-amine **(Intermediate 59,** 62.0 mg, 20 % purity, 24.5 µmol) was dissolved in acetonitrile (1.0 mL). Morpholine (2.8 µL, 32 µmol) and N,N-diisopropylethylamine (13 µL, 73 µmol; CAS-RN:[7087-68-5]) were added and the mixture was stirred for 23 h at 70 °C. Morpholine (4.3 µL, 49.1 µmol) was added and the mixture was stirred for 24 h at 70 °C. The reaction mixture was diluted with water and extracted with ethyl acetate. The combined organic layers were concentrated and the residue was purified by preparative HPLC [Waters Autopurificationsystem; Column: Kinetex Evo C18 150*30mm* 5 µm; eluent A: water (0.2 % aqueous ammonia (32 %)), eluent B: acetonitrile; gradient: 0-0.5 min 35 % B (35-70 mL/min), 0.5-5.5 min 35-65 % B; flow 70 mL/min; temperature: 25 °C; Detector: DAD scan 210-400nm; analytical method: instrument: Waters Acquity UPLCMS SingleQuad; Column: Kinetex Evo C18 2.0µ, 100x2.1mm; eluent A: water + 0.2 vol % aqueous ammonia (32%); eluent B: acetonitril; gradient: 0-4.0 min 1-99% B, 4.0-5.0 min 99% B; flow: 0.8 ml/min; temperature: 40°C; DAD scan: 210-400 nm] to give 3.6 mg (27% yield) of the title compound.
LC-MS: Rt = 3.06 min; MS (ESlpos): m/z = 514 [M+H]⁺
¹H-NMR (400 MHz, DMSO-d6) δ [ppm]: 1.232 (1.72), 1.351 (0.52), 2.074 (0.42), 2.336 (1.21), 2.518 (16.00), 2.523 (10.72), 3.387 (1.17), 3.495 (4.10), 3.507 (3.72), 3.571 (3.90), 3.583 (4.16), 4.758 (2.21), 4.771 (2.12), 7.841 (1.72), 7.862 (1.89), 7.877 (2.80), 7.930 (3.35), 8.307 (1.05), 8.331 (0.95), 8.509 (0.51), 9.118 (1.92), 9.366 (0.82), 12.225 (0.87).

Alternatively, 2-(methylsulfanyl)-7-(trifluoromethyl)-N-({5-[6-(trifluoromethyl)pyridin-3-yl]-1H-imidazol-2-yl}methyl)imidazo[2,1-f][1,2,4]triazin-4-amine **(Intermediate 58,** 450 mg, 949 µmol) was provided in dichlormethane (25 mL) at 0 °C, mCPBA (818 mg, 4.74 mmol; CAS-RN:[937-14-4]) was added and the mixture was stirred for 4 h at rt. Morpholine (4.0 mL, 45.9 mmol) was added and dichloromethane was removed at 60 °C and a reduced pressure of 700 mbar. The remaining mixture was stirred over night at 70 °C. The reaction mixture was diluted with water and extracted with ethyl acetate. The combined organic layers were washed with a saturated aqueous sodium chloride solution, dried over a hydrophobic filter and concentrated. The residue was purified by column chromatography (silica gel, dichlormethane/ ethanol gradient). The product was stirred in dichloromethane. The precipitate was collected by filtration and dried to give 133 mg of the title compound.
LC-MS (Method 2): Rt = 1.22 min; MS (ESlpos): m/z = 514 [M+H]⁺

### Example 13

### N-{[5-(4-methoxyphenyl)-1H-imidazol-2-yl]methyl}-2-(morpholin-4-yl)-7-(trifluoromethyl)imidazo[2,1-f][1,2,4]triazin-4-amine

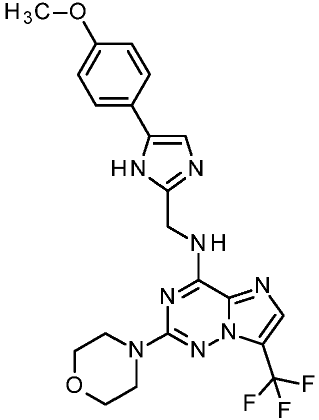

2-(methanesulfinyl)-N-{[5-(4-methoxyphenyl)-1H-imidazol-2-yl]methyl}-7-(trifluoromethyl)imidazo[2,1-f][1,2,4]triazin-4-amine **(Intermediate 63,** 47.2 mg, 88 % purity, 92.0 µmol) was dissolved in acetonitrile (1.0 mL). Morpholine (10 µL, 120 µmol) and N,N-diisopropylethylamine (48 µL, 280 µmol; CAS-RN:[7087-68-5]) were added and the mixture was stirred for 22 h at 70 °C. Morpholine (38 µL, 456 µmol) was added and the mixture was stirred for further 48 h at 70 °C. Morpholine (38 µL, 456 µmol) and N,N-diisopropylethylamine (80 µL, 466 µmol; CAS-RN:[7087-68-5]) were added and the mixture was stirred for 72 h at 70 °C and 1 h at 140 °C in a microwave. The reaction mixture was diluted with water and extracted with ethyl acetate. The combined organic layers were concentrated and the residue was purified by preparative HPLC (HT basic) to give 24.5 mg (51 % yield) of the title compound.
LC-MS (Method 2): Rₜ = 1.18 min; MS (ESlpos): m/z = 475 [M+H]⁺
¹H-NMR (400 MHz, DMSO-d6) δ [ppm]: 1.231 (1.23), 2.332 (0.80), 2.336 (0.50), 2.518 (3.88), 2.522 (2.51), 2.673 (0.68), 3.508 (6.32), 3.519 (5.12), 3.582 (5.57), 3.594 (5.68), 3.668 (0.43), 3.743 (16.00), 4.714 (3.16), 4.727 (2.73), 6.878 (3.51), 6.900 (3.70), 6.933 (0.44), 6.948 (0.47), 6.970 (0.46), 7.371 (2.56), 7.375 (2.50), 7.512 (0.44), 7.530 (0.44), 7.637 (3.73), 7.658 (3.44), 7.918 (5.21), 9.288 (1.08), 11.782 (1.34).

### Example 14

### 2-(morpholin-4-yl)-N-({5-[4-(trifluoromethoxy)phenyl]-4H-1,2,4-triazol-3-yl}methyl)-7-(trifluoromethyl)imidazo[2,1-f][1,2,4]triazin-4-amine

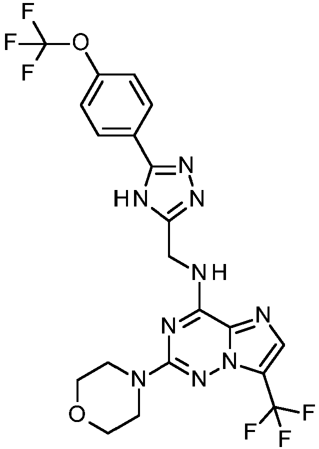

To 2-(methanesulfonyl)-N-({5-[4-(trifluoromethoxy)phenyl]-4H-1,2,4-triazol-3-yl}methyl)-7-(trifluoromethyl)imidazo[2,1-f][1,2,4]triazin-4-amine **(Intermediate 68,** 90.0 mg, 172 µmol) was added morpholine (1.0 mL, 11.5 mmol; CAS-RN:[110-91-8]) and the mixture was stirred at 70 °C for 3 h. The reaction mixture was concentrated and the residue was purified by preparative HPLC (HT acid) to give 33.0 mg (36 % yield) of the title compound.
LC-MS (Method 2): Rt = 1.12 min; MS (ESlpos): m/z = 530 [M+H]⁺
¹H-NMR (400 MHz, DMSO-d6) δ [ppm]: 1.232 (0.85), 1.353 (0.43), 2.327 (3.27), 2.331 (2.23), 2.337 (0.99), 2.518 (12.81), 2.523 (8.92), 2.669 (3.32), 2.674 (2.30), 2.678 (1.01), 3.457 (6.86), 3.468 (11.89), 3.480 (10.70), 3.502 (0.81), 3.552 (11.86), 3.564 (12.77), 3.575 (7.07), 4.806 (5.18), 4.816 (5.06), 7.452 (5.45), 7.473 (5.68), 7.937 (9.67), 7.939 (9.67), 8.057 (2.16), 8.064 (16.00), 8.070 (4.85), 8.082 (4.85), 8.087 (14.29), 8.094 (1.53), 9.449 (2.36), 14.042 (0.56).

### Example 15

### N-{[5-(6-methoxypyridin-3-yl)-4H-1,2,4-triazol-3-yl]methyl}-2-(morpholin-4-yl)-8-(trifluoromethyl)pyrazolo[1,5-a][1,3,5]triazin-4-amine

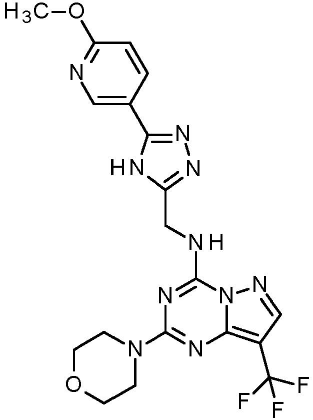

2-{[2-(morpholin-4-yl)-8-(trifluoromethyl)pyrazolo[1,5-a][1,3,5]triazin-4-yl]amino}acetohydrazide **(Intermediate 24,** 100 mg, 278 µmol) and 6-methoxypyridine-3-carboximidamide hydrogen chloride (1/1) (62.5 mg, 333 µmol) were dissolved in N,N-dimethylformamide (3.0 mL). Sodium ethylate (37.8 mg, 555 µmol; CAS-RN:[141-52-6]) was added and the mixture was stirred for 45 min at 180 °C in a microwave. The reaction mixture was filtered, and the filtrate was purified by preparative HPLC (HT acid) to give 38.4 mg (28 % yield) of the title compound.
LC-MS (Method 1): Rt = 1.10 min; MS (ESlpos): m/z = 477 [M+H]⁺
¹H-NMR (400 MHz, DMSO-d6) δ [ppm]: 2.074 (2.19), 2.327 (1.02), 2.331 (0.72), 2.518 (4.40), 2.523 (2.92), 2.669 (1.01), 2.673 (0.72), 3.554 (1.97), 3.677 (3.10), 3.896 (16.00), 4.788 (1.20), 6.915 (0.89), 6.936 (0.90), 8.182 (1.76), 8.187 (1.85), 8.203 (1.66), 8.209 (1.78), 8.245 (4.39), 8.732 (2.46), 8.736 (2.44), 9.287 (0.52).

### Example 16

### 2-(morpholin-4-yl)-8-(trifluoromethyl)-N-({5-[6-(trifluoromethyl)pyridin-3-yl]-4H-1,2,4-triazol-3-yl}methyl)pyrazolo[1,5-a][1,3,5]triazin-4-amine

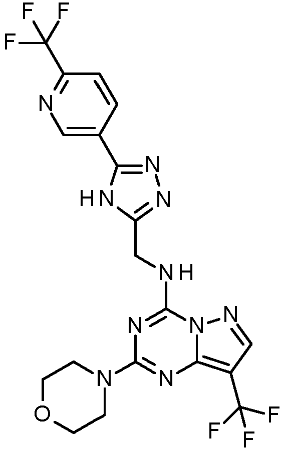

2-{[2-(morpholin-4-yl)-8-(trifluoromethyl)pyrazolo[1,5-a][1,3,5]triazin-4-yl]amino}acetohydrazide **(Intermediate 24,** 100 mg, 278 µmol) and 6-(trifluoromethyl)pyridine-3-carboximidamide hydrogen chloride (1/1) (75.1 mg, 333 µmol) were dissolved in N,N-dimethylformamide (3.0 mL). Sodium ethylate (37.8 mg, 555 µmol; CAS-RN:[141-52-6]) was added and the mixture was stirred for 45 min at 180 °C in a microwave. The reaction mixture was filtered, and the filtrate was purified by preparative HPLC (HT acid) to give 78.2 mg (55 % yield) of the title compound.
LC-MS (Method 1): Rt = 1.21 min; MS (ESlpos): m/z = 515 [M+H]⁺
¹H-NMR (400 MHz, DMSO-d6) δ [ppm]: 2.074 (1.00), 2.331 (2.52), 2.336 (1.16), 2.518 (16.00), 2.523 (10.83), 2.669 (3.52), 2.673 (2.47), 3.549 (5.79), 3.663 (9.01), 4.858 (6.94), 7.999 (5.86), 8.019 (6.30), 8.258 (15.79), 8.550 (4.08), 8.554 (3.85), 8.571 (3.55), 8.574 (3.64), 9.302 (7.08), 9.307 (7.00), 9.355 (2.10), 14.336 (0.63).

### Example 17

### 2-(morpholin-4-yl)-N-({5-[6-(trifluoromethoxy)pyridin-3-yl]-4H-1,2,4-triazol-3-yl}methyl)-8-(trifluoromethyl)pyrazolo[1,5-a][1,3,5]triazin-4-amine

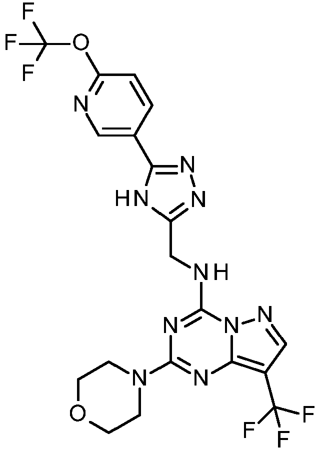

2-{[2-(morpholin-4-yl)-8-(trifluoromethyl)pyrazolo[1,5-a][1,3,5]triazin-4-yl]amino}acetohydrazide **(Intermediate 24,** 100 mg, 278 µmol) and 6-(trifluoromethoxy)pyridine-3-carboximidamide hydrogen chloride (1/1) (80.5 mg, 333 µmol) were dissolved in N,N-dimethylformamide (3.0 mL). Sodium ethylate (37.8 mg, 555 µmol; CAS-RN:[141-52-6]) was added and the mixture was stirred for 1 hour and 45 min at 180 °C in a microwave. The reaction mixture was filtered, and the filtrate was purified by preparative HPLC (HT acid) to give 29.9 mg (20 % yield) of the title compound.
LC-MS (Method 1): Rt = 1.26 min; MS (ESlpos): m/z = 531 [M+H]⁺
¹H-NMR (400 MHz, DMSO-d6) δ [ppm]: 2.331 (2.73), 2.336 (1.20), 2.428 (1.04), 2.518 (16.00), 2.523 (11.12), 2.673 (2.75), 2.678 (1.27), 3.551 (6.26), 3.663 (7.94), 4.842 (3.51), 7.378 (1.70), 7.398 (1.86), 8.253 (11.89), 8.477 (5.75), 8.483 (6.18), 8.498 (5.51), 8.505 (5.71), 8.891 (8.86), 8.895 (8.84), 9.346 (2.00), 14.147 (1.70).

### Example 18

### N-{[5-(6-methoxypyridin-3-yl)-4H-1,2,4-triazol-3-yl]methyl}-2-(morpholin-4-yl)-7-(trifluoromethyl)imidazo[2,1-f][1,2,4]triazin-4-amine

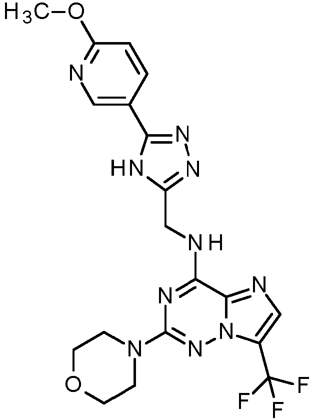

2-{[2-(morpholin-4-yl)-7-(trifluoromethyl)imidazo[2,1-f][1,2,4]triazin-4-yl]amino}acetohydrazide (**Intermediate 73,** 100 mg, 278 µmol) and 6-methoxypyridine-3-carboximidamide hydrogen chloride (1/1) (62.5 mg, 333 µmol) were dissolved in N,N-dimethylformamide (2.5 mL). Sodium ethylate (37.8 mg, 555 µmol; CAS-RN:[141-52-6]) was added and the mixture was stirred for 45 min at 180 °C in a microwave. The reaction mixture was filtered, and the filtrate was purified by preparative HPLC (HT acid) to give a first batch of 32.4 mg (25 % yield) and a second batch of 20.4 mg (14 % yield) of the title compound.
LC-MS (Method 2): Rₜ = 0.82 min; MS (ESlpos): m/z = 477 [M+H]⁺
¹H-NMR (400 MHz, DMSO-d6) δ [ppm]: 2.327 (0.60), 2.332 (0.43), 2.518 (2.75), 2.523 (1.88), 2.673 (0.43), 3.476 (3.01), 3.488 (2.71), 3.559 (3.15), 3.572 (3.43), 3.582 (1.91), 3.894 (16.00), 4.801 (1.14), 6.911 (0.89), 6.933 (0.92), 7.934 (2.53), 8.177 (1.72), 8.184 (1.80), 8.200 (1.53), 8.205 (1.72), 8.728 (2.18), 8.732 (2.21), 8.734 (1.99), 9.436 (0.55).
LC-MS (Method 2): Rt = 0.82 min; MS (ESlpos): m/z = 477 [M+H]⁺
¹H-NMR (400 MHz, DMSO-d6) δ [ppm]: 2.518 (2.88), 2.523 (2.15), 3.466 (1.67), 3.476 (2.88), 3.488 (2.57), 3.559 (2.93), 3.572 (3.16), 3.582 (1.76), 3.894 (16.00), 4.801 (1.16), 6.912 (1.06), 6.934 (1.09), 7.935 (2.40), 8.177 (1.62), 8.184 (1.79), 8.200 (1.52), 8.205 (1.72), 8.726 (1.95), 8.728 (2.10), 8.732 (2.03), 9.436 (0.49).

### Example 19

### 2-(morpholin-4-yl)-N-({5-[6-(trifluoromethoxy)pyridin-3-yl]-4H-1,2,4-triazol-3-yl}methyl)-7-(trifluoromethyl)imidazo[2,1-f][1,2,4]triazin-4-amine

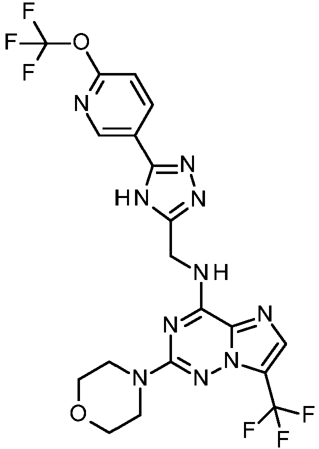

2-{[2-(morpholin-4-yl)-7-(trifluoromethyl)imidazo[2,1-f][1,2,4]triazin-4-yl]amino}acetohydrazide (**Intermediate 73,** 65.0 mg, 180 µmol) and 6-(trifluoromethoxy)pyridine-3-carboximidamide hydrogen chloride (1/1) (52.3 mg, 216 µmol) were dissolved in N,N-dimethylformamide (1.6 mL). Sodium ethylate (24.6 mg, 361 µmol; CAS-RN:[141-52-6]) was added and the mixture was stirred for 2 h at 180 °C in a microwave. The reaction mixture was filtered, and the filtrate was purified by preparative HPLC (HT acid) to give 21.4 mg (22 % yield) of the title compound.
LC-MS (Method 1): Rt = 1.26 min; MS (ESlpos): m/z = 531 [M+H]⁺
¹H-NMR (400 MHz, DMSO-d6) δ [ppm]: 2.332 (2.97), 2.336 (1.27), 2.518 (16.00), 2.523 (11.43), 2.678 (1.28), 3.150 (1.33), 3.451 (7.68), 3.462 (13.58), 3.475 (12.50), 3.504 (0.77), 3.554 (12.87), 3.566 (14.30), 3.577 (8.25), 4.832 (6.98), 4.844 (7.00), 7.385 (7.34), 7.406 (7.67), 7.941 (11.19), 7.943 (11.81), 8.472 (8.66), 8.478 (8.51), 8.494 (7.69), 8.499 (7.81), 8.884 (9.40), 8.890 (9.27), 8.892 (9.19), 9.476 (3.08), 14.167 (0.65).

### Example 20

### 2-(morpholin-4-yl)-7-(trifluoromethyl)-N-({5-[6-(trifluoromethyl)pyridin-3-yl]-4H-1,2 ,4-triazol-3-yl}methyl)imidazo[2,1-f][1,2,4]triazin-4-amine

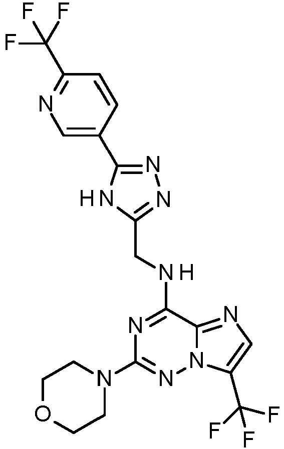

2-{[2-(morpholin-4-yl)-7-(trifluoromethyl)imidazo[2,1-f][1,2,4]triazin-4-yl]amino}acetohydrazide (**intermediate 73,** 100 mg, 278 µmol) and 6-(trifluoromethyl)pyridine-3-carboximidamide hydrogen chloride (1/1) (75.1 mg, 333 µmol) were dissolved in N,N-dimethylformamide (1.6 mL). Sodium ethylate (37.8 mg, 555 µmol; CAS-RN:[141-52-6]) was added and the mixture was stirred for 2 h at 180 °C in a microwave. The reaction mixture was filtered, and the filtrate was purified by preparative HPLC (HT acid). The crude product was stirred in dichlormethane and the precipitate was filtered off and dried at 60 °C under reduced pressure to give 67.3 mg (47 % yield) of the title compound.
LC-MS (Method 1): Rt = 1.21 min; MS (ESlpos): m/z = 515 [M+H]⁺
¹H-NMR (400 MHz, DMSO-d6) δ [ppm]: 2.074 (2.34), 2.331 (1.28), 2.518 (6.59), 2.523 (4.38), 3.448 (8.78), 3.459 (15.36), 3.471 (13.85), 3.500 (0.70), 3.550 (14.31), 3.563 (16.00), 3.573 (9.14), 4.859 (8.58), 4.873 (8.46), 7.946 (12.28), 7.948 (12.70), 7.995 (6.53), 8.016 (6.94), 8.545 (4.92), 8.549 (4.64), 8.565 (4.27), 8.569 (4.34), 9.297 (8.46), 9.302 (8.30), 9.489 (2.15), 9.503 (4.38), 9.516 (2.05), 14.296 (0.84).

### Example 21

### N-{[5-(6-methoxypyridin-3-yl)-1H-imidazol-2-yl]methyl}-2-(morpholin-4-yl)-8-(trifluoromethyl)pyrazolo[1,5-a][1,3,5]triazin-4-amine

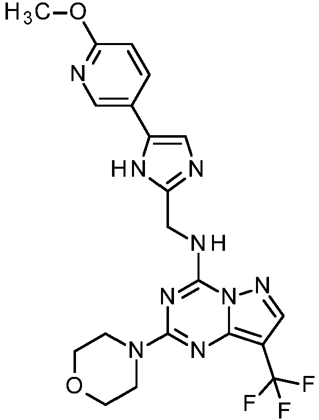

N-{[5-(6-methoxypyridin-3-yl)-1H-imidazol-2-yl]methyl}-2-(methylsulfanyl)-8-(trifluoromethyl)pyrazolo[1,5-a][1,3,5]triazin-4-amine (**Intermediate 77,** 53.0 mg, 121 µmol) was dissolved in dichlormethane (5 mL) at 0 °C, 3-chlorobenzene-1-carboperoxoic acid (83.8 mg, 486 µmol; CAS-RN:[937-14-4]) was added and the mixture was stirred for 1 h at rt. Morpholine (1.1 mL, 12 mmol; CAS-RN:[110-91-8]) was added and the solvent was evaporated under reduce pressure at 60 °C. The residue was purified by column chromatography (silica gel, dichlormethane / ethanol gradient) to give 17.0 mg (29 % yield) of the title compound.
LC-MS (Method 1): Rₜ = 0.94.min; MS (ESlpos): m/z = m/z = 476 [M+H]⁺
¹H-NMR (400 MHz, DMSO-d6) δ [ppm]: 1.232 (0.80), 2.327 (2.04), 2.332 (1.46), 2.336 (0.65), 2.518 (7.38), 2.523 (5.63), 2.669 (2.03), 2.673 (1.40), 2.678 (0.58), 3.573 (2.03), 3.691 (2.86), 3.703 (3.22), 3.714 (1.87), 3.843 (16.00), 4.714 (1.97), 4.727 (1.82), 5.759 (0.95), 6.788 (1.85), 6.807 (1.83), 6.809 (1.95), 7.502 (2.19), 7.507 (2.08), 7.989 (1.54), 7.995 (1.55), 8.010 (1.30), 8.016 (1.52), 8.228 (4.00), 8.515 (1.75), 8.517 (1.89), 8.522 (1.88), 9.149 (0.43), 9.164 (0.88), 9.178 (0.41), 11.950 (0.81).

### Example 22

### N-{[5-(6-methylpyridin-3-yl)-4H-1,2,4-triazol-3-yl]methyl}-2-(morpholin-4-yl)-8-(trifluoromethyl)pyrazolo[1,5-a][1,3,5]triazin-4-amine

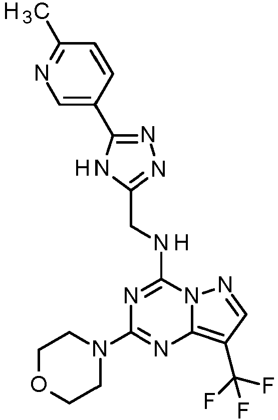

2-{[2-(morpholin-4-yl)-8-(trifluoromethyl)pyrazolo[1,5-a][1,3,5]triazin-4-yl]amino}acetohydrazide **(Intermediate 24,** 100 mg, 278 µmol) and 6-methylpyridine-3-carboximidamide hydrogen chloride (1/1) (57.2 mg, 333 µmol) were dissolved in N,N-dimethylformamide (2.5 mL). Sodium ethylate (37.8 mg, 555 µmol; CAS-RN:[141-52-6]) was added and the mixture was stirred for 2 h at 180 °C in a microwave. The reaction mixture was filtered, and the filtrate was purified by preparative HPLC (HT basic) to give 23.9 mg (18 % yield) of the title compound.
LC-MS (Method 2): Rt = 0.75 min; MS (ESlpos): m/z = 461 [M+H]⁺
¹H-NMR (400 MHz, DMSO-d6) δ [ppm]: 1.229 (0.45), 2.327 (2.61), 2.331 (1.91), 2.336 (1.07), 2.518 (11.32), 2.523 (7.48), 2.669 (2.60), 2.673 (1.85), 2.678 (0.86), 2.775 (0.43), 3.559 (7.59), 3.629 (1.45), 3.642 (1.34), 3.674 (10.04), 3.687 (11.71), 3.697 (7.13), 3.986 (0.55), 4.783 (16.00), 5.758 (9.06), 7.318 (5.54), 7.338 (5.73), 8.143 (4.78), 8.149 (4.93), 8.163 (4.46), 8.169 (4.63), 8.208 (0.41), 8.234 (13.51), 8.538 (1.66), 8.998 (6.48), 9.002 (6.61).

### Example 23

### N-{[5-(6-methylpyridin-3-yl)-4H-1,2,4-triazol-3-yl]methyl}-2-(morpholin-4-yl)-7-(trifluoromethyl)imidazo[2,1-f][1,2,4]triazin-4-amine

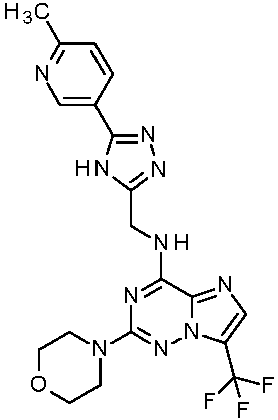

2-{[2-(morpholin-4-yl)-7-(trifluoromethyl)imidazo[2,1-f][1,2,4]triazin-4-yl]amino}acetohydrazide **(Intermediate 73,** 62.0 mg, 172 µmol) and 6-methylpyridine-3-carboximidamide hydrogen chloride (1/1) (35.4 mg, 206 µmol) were dissolved in N,N-dimethylformamide (2.5 mL). Sodium ethylate (23.4 mg, 344 µmol; CAS-RN:[141-52-6]) was added and the mixture was stirred for 2 h at 180 °C in a microwave. The reaction mixture was filtered, and the filtrate was purified by preparative HPLC (HT basic) to give 22.2 mg (25 % yield) of the title compound.
LC-MS (Method 2): Rt = 0.77 min; MS (ESlpos): m/z = 461 [M+H]⁺
¹H-NMR (400 MHz, DMSO-d6) δ [ppm]: 1.035 (0.47), 1.052 (0.91), 1.070 (0.44), 1.229 (0.51), 2.327 (3.11), 2.331 (2.23), 2.518 (16.00), 2.523 (10.14), 2.669 (3.11), 2.673 (2.24), 3.466 (8.15), 3.476 (13.94), 3.489 (13.10), 3.539 (1.82), 3.558 (13.19), 3.570 (14.30), 3.581 (8.21), 3.643 (0.75), 3.655 (0.82), 4.807 (14.63), 5.759 (3.44), 7.333 (6.39), 7.353 (6.73), 7.934 (10.61), 8.147 (5.47), 8.153 (5.46), 8.167 (5.03), 8.173 (5.22), 8.536 (1.99), 8.999 (7.59), 9.003 (7.57), 9.423 (0.72).

### Example 24

### 2-(morpholin-4-yl)-8-(trifluoromethyl)-N-({5-[6-(trifluoromethyl)pyridin-3-yl]-1H-imidazol-2-yl}methyl)pyrazolo[1,5-a][1,3,5]triazin-4-amine

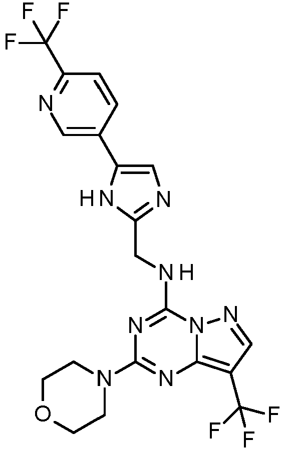

{[2-(morpholin-4-yl)-8-(trifluoromethyl)pyrazolo[1,5-a][1,3,5]triazin-4-yl]amino}ethanimidamide (intermediate **82, 50.0** mg, 145 µmol) and sodium bicarbonate (67.1 mg, 799 µmol; CAS-RN:[144-55-8]) were dissolved in tetrahydrofuran (2 mL) and water (0.75 mL) and stirred at 70 °C. 2-bromo-1-[6-(trifluoromethyl)pyridin-3-yl]ethan-1-one (38.9 mg, 145 µmol) was dissolved in tetrahydrofuran (1 mL) and added dropwise to the mixture. The reaction mixture was stirred at 70 °C over night. The mixture was concentrated and the residue was diluted with ethyl acetate and water. The phases were separated and the aqueous phase was extracted with ethyl acetate. The combined organic layers were dried over a hydrophobic filter and were concentrated to give a residue which was purified by preparative HPLC [Waters Autopurificationsystem; Column: Waters XBridge C18 100*30mm* 5 µm; eluent A: water (0.1% formic acid), eluent B: acetonitrile; gradient: 0.0-0.5 min 32 % B (25-70 mL/min), 0.51-5.5 min 32-52 % B; flow 70 mL/min; Detector: DAD scan 210-400nm] to give 1.3 mg (2 % yield) of the title compound.
LC-MS (Method 1): Rt = 1.17 min; MS (ESlpos): m/z = 514 [M+H]⁺
¹H-NMR (400 MHz, DMSO-d6) δ [ppm]: 1.233 (0.78), 2.327 (3.73), 2.331 (2.68), 2.336 (1.20), 2.518 (16.00), 2.523 (10.68), 2.669 (3.76), 2.673 (2.63), 2.678 (1.19), 3.566 (2.76), 3.682 (4.11), 3.694 (4.90), 4.748 (2.61), 4.759 (2.57), 7.845 (1.82), 7.866 (2.05), 7.891 (2.28), 8.177 (2.37), 8.237 (6.32), 8.311 (1.22), 8.331 (1.11), 9.121 (2.30), 9.216 (0.97), 12.265 (1.10).

### EXPERIMENTAL SECTION - BIOLOGICAL ASSAYS

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

An empty field in any of the following tables means that the respective compound has not been tested in that Assay.

### 1. Expression and Purification of the CDK12/CycK and CDK13/CycK used in the CDK12 and CDK13 kinase activity assays

### 1.1 Cloning of CDK 12/13, CycK and CAK1 in insect destination vectors

The cDNAs encoding the following protein sequences were codon optimized for expression in Sf9 / Hi-5 insect cells and synthesized by the GeneArt Technology at Thermo Fischer Scientific.

The human CDK12 wt/DN (Acc. Q9NYV4), CDK13 (Q14004), CycK (O75909) and the saccharomyces cerevisiae CAK1 (P43568) full length sequence were used for cloning. These cDNAs also encoded att-site sequences at the 5' and 3' ends for subcloning into the following destination vectors using the Gateway Technology.

By using of baculovirus vectors with a strong polyhedrin promoter provides an N-terminal fusion of a His-tag with a Tobacco Edge virus cleavage site to the integrated gene of interest. Only the saccharomyces cerevisiae CAK1 (P43568) full length sequence was cloned in an insect vector which provides a tag-free gene of interest.

### 1.2 Sequence

His-CDK12 (aa Q696-S1082) 
MTSHHHHHHS SMGSRTSLYK KAGSDYDIPT TENLYFQGQP YKKRPKICCP
RYGERRQTES DWGKRCVDKF DIIGIIGEGT YGQVYKAKDK DTGELVALKK
VRLDNEKEGF PITAIREIKI LRQLIHRSVV NMKEIVTDKQ DALDFKKDKG
AFYLVFEYMD HDLMGLLESG LVHFSEDHIK SFMKQLMEGL EYCHKKNFLH
RDIKCSNILL NNSGQIKLAD FGLARLYNSE ESRPYTNKVI TLWYRPPELL
LGEERYTPAI DVWSCGCILG ELFTKKPIFQ ANLELAQLEL ISRLCGSPCP
AVWPDVIKLP YFNTMKPKKQ YRRRLREEFS FIPSAALDLL DHMLTLDPSK
RCTAEQTLQS DFLKDVELSK MAPPDLPHWQ DCHELWSKKR RRQRQSGVVV
EEPPPSKTSR KETTSGTSTE PVKNS
His-CDK12-DN (aa Q696-S1082; K756A; D877N) 
MTSHHHHHHS SMGSRTSLYK KAGSDYDIPT TENLYFQGQP YKKRPKICCP
RYGERRQTES DWGKRCVDKF DIIGIIGEGT YGQVYKAKDK DTGELVALAK
VRLDNEKEGF PITAIREIKI LRQLIHRSVV NMKEIVTDKQ DALDFKKDKG
AFYLVFEYMD HDLMGLLESG LVHFSEDHIK SFMKQLMEGL EYCHKKNFLH
RDIKCSNILL NNSGQIKLAN FGLARLYNSE ESRPYTNKVI TLWYRPPELL
LGEERYTPAI DVWSCGCILG ELFTKKPIFQ ANLELAQLEL ISRLCGSPCP
AVWPDVIKLP YFNTMKPKKQ YRRRLREEFS FIPSAALDLL DHMLTLDPSK
RCTAEQTLQS DFLKDVELSK MAPPDLPHWQ DCHELWSKKR RRQRQSGVVV
EEPPPSKTSR KETTSGTSTE PVKNS
His-CDK13 (aa Q673-P1059) 
MTSHHHHHHS SMGSRTSLYK KAGSDYDIPT TENLYFQGQL HSKRRPKICG
PRYGETKEKD IDWGKRCVDK FDIIGIIGEG TYGQVYKARD KDTGEMVALK
KVRLDNEKEG FPITAIREIK ILRQLTHQSI INMKEIVTDK EDALDFKKDK GAFYLVFEYM
DHDLMGLLES GLVHFNENHI KSFMRQLMEG LDYCHKKNFL HRDIKCSNIL
LNNRGQIKLA DFGLARLYSS EESRPYTNKV ITLWYRPPEL LLGEERYTPA
IDVWSCGCIL GELFTKKPIF QANQELAQLE LISRICGSPC PAVWPDVIKL
PYFNTMKPKK QYRRKLREEF VFIPAAALDL FDYMLALDPS KRCTAEQALQ
CEFLRDVEPS KMPPPDLPLW QDCHELWSKK RRRQKQMGMT DDVSTIKAPR
KDLSLGLDDS RTNTP
His-CDK13-DN (aa Q673-P1059; K734A; D855N) 
MTSHHHHHHS SMGSRTSLYK KAGSDYDIPT TENLYFQGQL HSKRRPKICG
PRYGETKEKD IDWGKRCVDK FDIIGIIGEG TYGQVYKARD KDTGEMVALA
 
KVRLDNEKEG FPITAIREIK ILRQLTHQSI INMKEIVTDK EDALDFKKDK GAFYLVFEYM
DHDLMGLLES GLVHFNENHI KSFMRQLMEG LDYCHKKNFL HRDIKCSNIL
LNNRGQIKLA NFGLARLYSS EESRPYTNKV ITLWYRPPEL LLGEERYTPA
IDVWSCGCIL GELFTKKPIF QANQELAQLE LISRICGSPC PAVWPDVIKL
PYFNTMKPKK QYRRKLREEF VFIPAAALDL FDYMLALDPS KRCTAEQALQ
CEFLRDVEPS KMPPPDLPLW QDCHELWSKK RRRQKQMGMT DDVSTIKAPR
KDLSLGLDDS RTNTP
His-CycK (aa M1-S300) 
MTSHHHHHHS SMGSRTSLYK KAGSDYDIPT TENLYFQGMK ENKENSSPSV
TSANLDHTKP CWYWDKKDLA HTPSQLEGLD PATEARYRRE GARFIFDVGT
RLGLHYDTLA TGIIYFHRFY MFHSFKQFPR YVTGACCLFL AGKVEETPKK
CKDIIKTARS LLNDVQFGQF GDDPKEEVMV LERILLQTIK FDLQVEHPYQ
FLLKYAKQLK GDKNKIQKLV QMAWTFVNDS LCTTLSLQWE PEIIAVAVMY
LAGRLCKFEI QEWTSKPMYR RWWEQFVQDV PVDVLEDICH QILDLYSQGK
QQMPHHTPHQ LQQPPSLQPT PQVPQVQQSQ PSQSSEPS
CAK1 (aa M1-P368)

MKLDSIDITH CQLVKSTRTA RIYRSDTYAI KCLALDFDIP PHNAKFEVSI
LNKLGNKCKH ILPLLESKAT DNNDLLLLFP FEEMNLYEFM QMHYKRDRRK
KNPYYDLLNP SIPIVADPPV QKYTNQLDVN RYSLSFFRQM VEGIAFLHEN
KIIHRDIKPQ NIMLTNNTST VSPKLYIIDF GISYDMANNS QTSAEPMDSK VTDISTGIYK
APEVLFGVKC YDGGVDVWSL LIIISQWFQR ETSRMGHVPA MIDDGSDDMN
SDGSDFRLIC SIFEKLGIPS IQKWEEVAQH GSVDAFVGMF GADGDGKYVL
DQEKDVQISI VERNMPRLDE IADVKVKQKF INCILGMVSF SPNERWSCQR ILQELEKP

### 1.3 Expression of the CDK12-CycK and CDK13-CycK complex

The Hi-5 insect cells were cultivated in Insect Xpress Medium (Lonza # BE12-730Q) and for co-infection the following baculovirus with multiplicity of infection (MOI) was using for the expression of the complex: CDK12 and CDK13 with MOI 1.0; CycK and CAK1 with MOI 0.5.

The complex formation was performed by co-infection of Hi-5 cells grown in suspension to a density of 2 × 10⁶ cells/mL in 8 L waver for 72 h. The cells were harvested by centrifugation (10 min., 170g, 4 °C) and the cell pellets stored at -80 °C.

### 1.4 Purification of the CDK12 and CDK13 complex

Purification of the His-CDK12/His-CycK/CAK1 or His-CDK13/His-CycK/CAK1 complex was achieved by affinity chromatography using Ni-Sepharose High Performance (GE Healthcare #17-5268-02) or HisTrap^{™}HP (GE Healthcare #17-5247-01/05)

Cell pellets were resuspended in lysis buffer (50 millimol/L Hepes pH 7.5, 500 millimol/L NaCl, 40 millimol/L Imidazol, 10% Glycerol; 0.5% NP40, Benzonase (150 U / 10 g cell pellet), 1 millimol/L DTT and 1 x Complete EDTA-free protease inhibitor cocktail (Roche #1873580)).

The lysate was incubated on ice for 30 minutes and clarified by centrifugation (1h, 4°C, 27500 x g). Proteins were captured overnight at 4 °C using Ni-Sepharose or HisTrap HP material, washed with CDK12/13 wash buffer (50 millimol/L Hepes pH 7.5, 500 millimol/L NaCl, 40 millimol/L imidazole, 10% Glycerol, 1 millimol/L DTT) and eluted with wash buffer by using gradient of imidazole (40-500 millimol/L).

For removal of imidazole the eluted protein complexes were desalted with Zeba^{™} Desalt Spin Columns (Pierce #89893) against CDK12/13 DS buffer (50 millimol/L Hepes pH 7.5, 500 millimol/L NaCl, 10% Glycerol, 1 millimol/L DTT).

The final concentration was calculated densitrometrically using BSA as a standard in a Coomassie stained gel. Elution fractions were aliquoted and shock frozen using liquid nitrogen.

The *in vitro* activity of the compounds of the present invention can be demonstrated in the following assays:

### 2. Biochemical kinase assays

### 2.1 CDK12/CycK low ATP kinase assay

CDK12/CycK -inhibitory activity of compounds of the present invention at 10 micromol/L adenosine-tri-phosphate (ATP) was quantified employing the TR-FRET (TR-FRET = Time Resolved Fluorescence Energy Transfer) based CDK12/CycK activity inhibition assay as described in the following paragraphs.

A complex of human recombinant CDK12 and human recombinant CycK (both N-terminally His-tagged, expression and purification as described above) was used as enzyme. As substrate for the kinase reaction biotinylated peptide biotin-Ahx-KFELLPTPPLSPSRRSGL (C-terminus in amid form) was used which can be purchased e.g. form the company Biosyntan (Berlin-Buch, Germany).

For the assay 50 nanoL of a 100fold concentrated solution of the test compound in DMSO was pipetted into either a black low volume 384well microtiter plate or a black 1536well microtiter plate (both Greiner Bio-One, Frickenhausen, Germany), 2 microL of a solution of CDK12/CycK in aqueous assay buffer [25 millimol/L HEPES pH 7.5, 20 millimol/L MgCl₂, 5 millimol/L β-glycerophosphate, 2 millimol/L EGTA, 1.0 millimol/L dithiothreitol, 0.01% (v/v) Nonidet-P40 (Sigma), 0.01 % (w/v) bovine serum albumin] were added and the mixture was incubated for 15 min at 22°C to allow pre-binding of the test compounds to the enzyme before the start of the kinase reaction. Then the kinase reaction was started by the addition of 3 microL of a solution ATP (16.7 micromol/L => final conc. in the 5 microL assay volume is 10 micromol/L) and substrate (1.67 micromol/L => final conc. in the 5 microL assay volume is 1 micromol/L) in assay buffer and the resulting mixture was incubated for a reaction time of 60 min at 22°C. The concentration of CDK12/CycK was adjusted depending of the activity of the enzyme lot and was chosen appropriate to have the assay in the linear range, typical concentrations were about 2 nanomol/L. The reaction was stopped by the addition of 3 microL of a solution of TR-FRET detection reagents (125 nanomol/L streptavidine-XL665 [Cisbio Bioassays, Codolet, France] and 0.67 nanomol/L anti-Phospho-c-Myc (Ser 62) (E1J4K)-antibody from Cell Signalling [# 13748] and 2 nanomol/L LANCE EU-W1024 labeled anti-rabbit IgG antibody [Perkin-Elmer, product no. 0083]) in an aqueous EDTA-solution (133 millimol/L EDTA, 0.27 % (w/v) bovine serum albumin in 66.7 millimol/L HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar FS (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 micromol/L to 0.07 nanomol/L (20 micromol/L, 5.7 micromol/L, 1.6 micromol/L, 0.47 micromol/L, 0.13 micromol/L, 38 nanomol/L, 11 nanomol/L, 3.1 nanomol/L, 0.9 nanomol/L, 0.25 nanomol/L and 0.07 nanomol/L, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

### 2.2 CDK12/CycK high ATP kinase assay

In the context of the present invention, the term "IC50 CDK12 hATP" refers to the IC₅₀ values obtained according to the assay described in this section (2.2) herein below, i.e. the IC₅₀ values for the inhibition of CDK12 at high (2mM) ATP.

CDK12/CycK -inhibitory activity of compounds of the present invention at 2 millimol/L adenosine-tri-phosphate (ATP) was quantified employing the TR-FRET (TR-FRET = Time Resolved Fluorescence Energy Transfer) based CDK12/CycK activity inhibition assay as described in the following paragraphs.

A complex of human recombinant CDK12 and human recombinant CycK (both N-terminally His-tagged, expression and purification as described above) was used as enzyme. As substrate for the kinase reaction biotinylated peptide biotin-Ahx-KFELLPTPPLSPSRRSGL (C-terminus in amid form) was used which can be purchased e.g. form the company Biosyntan (Berlin-Buch, Germany).

For the assay 50 nanoL of a 100fold concentrated solution of the test compound in DMSO was pipetted into either a black low volume 384well microtiter plate or a black 1536well microtiter plate (both Greiner Bio-One, Frickenhausen, Germany), 2 microL of a solution of CDK12/CycK in aqueous assay buffer [25 millimol/L HEPES pH 7.5, 20 millimol/L MgCl₂, 5 millimol/L β-glycerophosphate, 2 millimol/L EGTA, 1.0 millimol/L dithiothreitol, 0.01% (v/v) Nonidet-P40 (Sigma), 0.01 % (w/v) bovine serum albumin] were added and the mixture was incubated for 15 min at 22°C to allow pre-binding of the test compounds to the enzyme before the start of the kinase reaction. Then the kinase reaction was started by the addition of 3 microL of a solution ATP (3.33 millimol/L => final conc. in the 5 microL assay volume is 2 millimol/L) and substrate (1.67 micromol/L => final conc. in the 5 microL assay volume is 1 micromol/L) in assay buffer and the resulting mixture was incubated for a reaction time of 60 min at 22°C. The concentration of CDK12/CycK was adjusted depending of the activity of the enzyme lot and was chosen appropriate to have the assay in the linear range, typical concentrations were about 0.75 nanomol/L. The reaction was stopped by the addition of 3 microL of a solution of TR-FRET detection reagents (125 nanomol/L streptavidine-XL665 [Cisbio Bioassays, Codolet, France] and 0.67 nanomol/L anti-Phospho-c-Myc (Ser 62) (E1J4K)-antibody from Cell Signalling [# 13748] and 2 nanomol/L LANCE EU-W1024 labeled anti-rabbit IgG antibody [Perkin-Elmer, product no. 0083]) in an aqueous EDTA-solution (133 millimol/L EDTA, 0.27 % (w/v) bovine serum albumin in 66.7 millimol/L HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar FS (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 micromol/L to 0.07 nanomol/L (20 micromol/L, 5.7 micromol/L, 1.6 micromol/L, 0.47 micromol/L, 0.13 micromol/L, 38 nanomol/L, 11 nanomol/L, 3.1 nanomol/L, 0.9 nanomol/L, 0.25 nanomol/L and 0.07 nanomol/L, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

**Table 1. CDK12/CyclinK, 2 mM ATP (high ATP), IC₅₀ - [mol/I] (median)**

| Example No | CDK12/ CyclinK, 2 mM ATP (high ATP), IC50 - [mol/l] (median) |
|---|---|
| 1 | 1.83E-06 |
| 2 | 7.50E-07 |
| 3 | 2.67E-07 |
| 4 | 3.48E-08 |
| 5 | 2.62E-07 |
| 6 | 7.36E-07 |
| 7 | 2.63E-08 |
| 8 | 8.34E-09 |
| 9 | 9.21E-09 |
| 10 | 7.59E-09 |
| 11 | 3.23E-08 |
| 12 | 3.09E-09 |
| 13 | 1.23E-08 |
| 14 | |
| 15 | 5.26E-07 |
| 16 | 7.91E-08 |
| 17 | 5.96E-09 |
| 18 | 4.16E-08 |
| 19 | 4.14E-09 |
| 20 | 1.19E-08 |
| 21 | |
| 22 | 4.89E-07 |
| 23 | 1.60E-07 |
| 24 | 1.51E-08 |

### 2.3 CDK13/CycK low ATP kinase assay

CDK13/CycK -inhibitory activity of compounds of the present invention at 10 micromol/L adenosine-tri-phosphate (ATP) was quantified employing the TR-FRET (TR-FRET = Time Resolved Fluorescence Energy Transfer) based CDK13/CycK activity inhibition assay as described in the following paragraphs.

A complex of human recombinant CDK13 and human recombinant CycK (both N-terminally His-tagged, expression and purification as described above) was used as enzyme. As substrate for the kinase reaction biotinylated peptide biotin-Ahx-KFELLPTPPLSPSRRSGL (C-terminus in amid form) was used which can be purchased e.g. form the company Biosyntan (Berlin-Buch, Germany).

For the assay 50 nanoL of a 100fold concentrated solution of the test compound in DMSO was pipetted into either a black low volume 384well microtiter plate or a black 1536well microtiter plate (both Greiner Bio-One, Frickenhausen, Germany), 2 microL of a solution of CDK13/CycK in aqueous assay buffer [25 millimol/L HEPES pH 7.5, 20 millimol/L MgCl₂, 5 millimol/L β-glycerophosphate, 2 millimol/L EGTA, 1.0 millimol/L dithiothreitol, 0.01% (v/v) Nonidet-P40 (Sigma), 0.01 % (w/v) bovine serum albumin] were added and the mixture was incubated for 15 min at 22°C to allow pre-binding of the test compounds to the enzyme before the start of the kinase reaction. Then the kinase reaction was started by the addition of 3 microL of a solution ATP (16.7 micromol/L => final conc. in the 5 microL assay volume is 10 micromol/L) and substrate (1.67 micromol/L => final conc. in the 5 microL assay volume is 1 micromol/L) in assay buffer and the resulting mixture was incubated for a reaction time of 60 min at 22°C. The concentration of CDK13/CycK was adjusted depending of the activity of the enzyme lot and was chosen appropriate to have the assay in the linear range, typical concentrations were about 5 nanomol/L. The reaction was stopped by the addition of 3 microL of a solution of TR-FRET detection reagents (125 nanomol/L streptavidine-XL665 [Cisbio Bioassays, Codolet, France] and 0.67 nanomol/L anti-Phospho-c-Myc (Ser 62) (E1J4K)-antibody from Cell Signalling [# 13748] and 2 nanomol/L LANCE EU-W1024 labeled anti-rabbit IgG antibody [Perkin-Elmer, product no. 0083]) in an aqueous EDTA-solution (133 millimol/L EDTA, 0.27 % (w/v) bovine serum albumin in 66.7 millimol/L HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar FS (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 micromol/L to 0.07 nanomol/L (20 micromol/L, 5.7 micromol/L, 1.6 micromol/L, 0.47 micromol/L, 0.13 micromol/L, 38 nanomol/L, 11 nanomol/L, 3.1 nanomol/L, 0.9 nanomol/L, 0.25 nanomol/L and 0.07 nanomol/L, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

### 2.4 CDK2/CycE kinase assay

CDK2/CycE -inhibitory activity of compounds of the present invention was quantified employing the CDK2/CycE TR-FRET assay as described in the following paragraphs.

Recombinant fusion proteins of GST and human CDK2 and of GST and human CycE, expressed in insect cells (Sf9) and purified by Glutathion-Sepharose affinity chromatography, were purchased from ProQinase GmbH (Freiburg, Germany). As substrate for the kinase reaction biotinylated peptide biotin-Ttds-YISPLKSPYKISEG (C-terminus in amid form) was used which can be purchased e.g. form the company JERINI peptide technologies (Berlin, Germany).

For the assay 50 nanoL of a 100fold concentrated solution of the test compound in DMSO was pipetted into a black low volume 384well microtiter plate or a black 1536well microtiter plate (both Greiner Bio-One, Frickenhausen, Germany), 2 microL of a solution of CDK2/CycE in aqueous assay buffer [50 millimol/L Tris/HCl pH 8.0, 10 millimol/L MgCl₂, 1.0 millimol/L dithiothreitol, 0.1 millimol/L sodium ortho-vanadate, 0.01% (v/v) Nonidet-P40 (Sigma)] were added and the mixture was incubated for 15 min at 22°C to allow pre-binding of the test compounds to the enzyme before the start of the kinase reaction. Then the kinase reaction was started by the addition of 3 microL of a solution of adenosine-tri-phosphate (ATP, 3.33 millimol/L => final conc. in the 5 microL assay volume is 2 millimol/L) and substrate (1.25 micromol/L => final conc. in the 5 microL assay volume is 0.75 micromol/L) in assay buffer and the resulting mixture was incubated for a reaction time of 25 min at 22°C. The concentration of CDK2/CycE was adjusted depending of the activity of the enzyme lot and was chosen appropriate to have the assay in the linear range, typical concentrations were in the range of 10 ng/ml. The reaction was stopped by the addition of 3 microL of a solution of TR-FRET detection reagents (0.333 micromol/L streptavidine-XL665 [Cisbio Bioassays, Codolet, France] and 1.67 nanomol/L anti-RB(pSer807/pSer811)-antibody from BD Pharmingen [# 558389] and 2 nanomol/L LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (167 millimol/L EDTA, 0.2 % (w/v) bovine serum albumin in 100 millimol/L HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 micromol/L to 0.07 nanomol/L (20 micromol/L, 5.7 micromol/L, 1.6 micromol/L, 0.47 micromol/L, 0.13 micromol/L, 38 nanomol/L, 11 nanomol/L, 3.1 nanomol/L, 0.9 nanomol/L, 0.25 nanomol/L and 0.07 nanomol/L, the dilution series prepared separately before the assay on the level of the 100fold concentrated solutions in DMSO by serial dilutions, exact concentrations may vary depending pipettors used) in duplicate values for each concentration and IC50 values were calculated using Genedata Screener^{™} software.

**Table 2. CDK2/CyclinE, 2mM ATP (high ATP), IC₅₀ - [mol/I]**

| Example No | CDK2/CyclinE, 2mM ATP (high ATP), IC50 - [mol/l] (median) |
|---|---|
| 1 | 9.81E-06 |
| 2 | 1.12E-05 |
| 3 | 7.92E-06 |
| 4 | 1.04E-05 |
| 5 | > 2.00E-5 |
| 6 | > 2.00E-5 |
| 7 | > 2.00E-5 |
| 8 | > 2.00E-5 |
| 9 | > 2.00E-5 |
| 10 | > 2.00E-5 |
| 11 | > 2.00E-5 |
| 12 | > 2.00E-5 |
| 13 | 1.63E-05 |
| 14 | > 2.00E-5 |
| 15 | 3.12E-06 |
| 16 | 4.05E-06 |
| 17 | 6.72E-06 |
| 18 | > 5.71E-6 |
| 19 | > 2.00E-5 |
| 20 | > 5.71E-6 |
| 21 | 6.39E-06 |
| 22 | 3.12E-06 |
| 23 | 2.86E-06 |
| 24 | 7.82E-06 |

### 2.5 CDK9/CycT1 high ATP kinase assay

CDK9/CycT1 -inhibitory activity of compounds of the present invention at a high ATP concentration after preincubation of enzyme and test compounds was quantified employing the CDK9/CycT1 TR-FRET assay as described in the following paragraphs.

Recombinant full-length His-tagged human CDK9 and CycT1, expressed in insect cells and purified by Ni-NTA affinity chromatography, were purchased from Life Technologies (Cat. No PV4131). As substrate for the kinase reaction biotinylated peptide biotin-Ttds-YISPLKSPYKISEG (C-terminus in amide form) was used which can be purchased e.g. form the company JERINI peptide technologies (Berlin, Germany).

For the assay 50 nanoL of a 100fold concentrated solution of the test compound in DMSO was pipetted into either a black low volume 384well microtiter plate or a black 1536well microtiter plate (both Greiner Bio-One, Frickenhausen, Germany), 2 microL of a solution of CDK9/CycT1 in aqueous assay buffer [50 millimol/L Tris/HCl pH 8.0, 10 millimol/L MgCl₂, 1.0 millimol/L dithiothreitol, 0.1 millimol/L sodium ortho-vanadate, 0.01% (v/v) Nonidet-P40 (Sigma)] were added and the mixture was incubated for 15 min at 22°C to allow pre-binding of the test compounds to the enzyme before the start of the kinase reaction. Then the kinase reaction was started by the addition of 3 microL of a solution of adenosine-tri-phosphate (ATP, 3.3 millimol/L => final conc. in the 5 microL assay volume is 2 millimol/L) and substrate (1.25 micromol/L => final conc. in the 5 microL assay volume is 0.75 micromol/L) in assay buffer and the resulting mixture was incubated for a reaction time of 25 min at 22°C. The concentration of CDK9/CycT1 was adjusted depending of the activity of the enzyme lot and was chosen appropriate to have the assay in the linear range, typical concentrations were in the range of 0.5 microg/ml. The reaction was stopped by the addition of 3 microL of a solution of TR-FRET detection reagents (0.33 micromol/L streptavidine-XL665 [Cisbio Bioassays, Codolet, France] and 1.67 nanomol/L anti-RB(pSer807/pSer811)-antibody from BD Pharmingen [# 558389] and 2 nanomol/L LANCE EU-W1024 labeled anti-mouse IgG antibody [Perkin-Elmer, product no. AD0077]) in an aqueous EDTA-solution (167 millimol/L EDTA, 0.2 % (w/v) bovine serum albumin in 100 millimol/L HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 micromol/L to 0.07 nanomol/L (20 micromol/L, 5.7 micromol/L, 1.6 micromol/L, 0.47 micromol/L, 0.13 micromol/L, 38 nanomol/L, 11 nanomol/L, 3.1 nanomol/L, 0.9 nanomol/L, 0.25 nanomol/L and 0.07 nanomol/L, the dilution series prepared separately before the assay on the level of the 100fold concentrated solutions in DMSO by serial dilutions, exact concentrations may vary depending pipettors used) in duplicate values for each concentration and IC₅₀-values were calculated using Genedata Screener^{™} software.

**Table 3. CDK9/CyclinT1, 2mM ATP (high ATP), IC₅₀ - [mol/I]**

| Example No | CDK9/CyclinT1, 2mM ATP (high ATP), IC50 - [mol/l] (median) |
|---|---|
| 1 | 4.58E-06 |
| 2 | 6.35E-06 |
| 3 | 1.54E-05 |
| 4 | > 2.00E-5 |
| 5 | 1.78E-05 |
| 6 | > 2.00E-5 |
| 7 | > 2.00E-5 |
| 8 | > 2.00E-5 |
| 9 | > 1.91E-5 |
| 10 | > 2.00E-5 |
| 11 | > 2.00E-5 |
| 12 | > 2.00E-5 |
| 13 | 2.78E-06 |
| 14 | > 2.00E-5 |
| 15 | |
| 16 | |
| 17 | |
| 18 | 3.40E-07 |
| 19 | 5.06E-07 |
| 20 | 8.47E-07 |
| 21 | 3.05E-06 |
| 22 | 1.89E-06 |
| 23 | 6.24E-07 |
| 24 | 3.56E-06 |

### 3. qRT-PCR assay: BRCA1, ATR, MCL1 in MDA-MB-231, CAL-120

Tissue cultured human MDA-MB-231 human breast cancer cells were plated in 500 microL per well at 200,000 cells/well in a 24well microtiter plate. After 24h, the cells were exposed continuously for 24 h to test substances (substances were added with Tecan HP D300 Dispenser). RNA was prepared using Qiagen RNeasy MiniKit (#74106), RNA was quantified using a NanoDrop Equipment, and 600 nano gamms of RNA was coverted to cDNA using a SuperScript VILO kit (Thermofisher #11755050) followed by qPCR amplification. BRCA1 and ATR gene expression was measured by RT-qPCR and normalised to GAPDH housekeeping gene expression. qPCR primer sets have been purchased from Thermo Fisher Scientific / Applied Biosystems: BRCA1, # Hs 01556193; ATR, # Hs 00992123; GAPDH, # Hs 03929097.

**Table 4. Inhibition of BRCA1 mRNA expression in MDA-MB-231 and CAL-120 cells.**

| Example No | RTqPCR-MDA-MB-231, IC50 [mol/l], BRCA1 (median) | RTqPCR-CAL-120, IC50 [mol/l], BRCA1 (median) |
|---|---|---|
| 1 | 5.14 E-7 | |
| 2 | 2.72 E-7 | |
| 3 | | |
| 4 | 6.94 E-9 | |
| 5 | 1.09 E-7 | |
| 6 | | |
| 7 | | |
| 8 | 3.82 E-8 | |
| 9 | 1.02 E-7 | |
| 10 | 3.73 E-8 | |
| 11 | | |
| 12 | 7.57 E-9 | |
| 13 | 5.71 E-8 | |
| 14 | | |
| 15 | | |
| 16 | | |
| 17 | | |
| 18 | 7.15 E-8 | |
| 19 | 3.45 E-8 | |
| 20 | 5.38 E-8 | |
| 21 | 9.88 E-8 | |
| 22 | | |
| 23 | 1.02 E-7 | |
| 24 | 2.95 E-8 | |

### 4. Proliferation assay: MDA-MB-231, CAL-120

Human tumour cells were originally obtained from the American Type Culture Collection (ATCC), or from the Deutsche Sammlung von Mikroorganismen und Zellkulturen (DSMZ, German Collection of Microorganisms and Cell Cultures). Cultivated tumour cells (CAL-120, human breast adenocarcinoma cells, DSMZ ACC-459; MDA-MB-231, human breast cancer cells, ATCC HTB-26) were plated at a density of 4,000 cells/well in a 96-well multititer plate in 200 microL of their respective growth medium supplemented 10% fetal calf serum. After 24 hours, the cells of one plate (zero-point plate) were stained with crystal violet (see below), while the medium of the other plates was supplemented with the test substances in various concentrations (0 micromol/L, as well as in the range of 0.01-10 micromol/L; the final concentration of the solvent dimethyl sulfoxide was adjusted to 0.1%) using a Tecan HP D300 Digital Dispenser. The cells were incubated for 4 days in the presence of test substances. Cell proliferation was determined by staining the cells with crystal violet: the cells were fixed by adding 20 microL/measuring point of an 11% glutaric aldehyde solution for 15 minutes at room temperature. After three washing cycles of the fixed cells with water, the plates were dried at room temperature. The cells were stained by adding 100 microL/measuring point of a 0.1% crystal violet solution (pH 3.0). After three washing cycles of the stained cells with water, the plates were dried at room temperature. The dye was dissolved by adding 100 microL/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µM) cells (=100%). The IC50 values (inhibitory concentration at 50% of maximal effect) were determined by means of a 4 parameter fit.

**Table 5. Antiproliferative data for the compounds of the present invention.**

| Example No | Prolif-MDA MB-231, IC50 - [mol/l] (median) | Prolif-CAL-120, IC50 - [mol/l] (median) |
|---|---|---|
| 1 | | 5.80E-07 |
| 2 | | 3.70E-07 |
| 3 | | 1.73E-07 |
| 4 | 1.23E-07 | 1.03E-07 |
| 5 | 3.19E-07 | 3.19E-07 |
| 6 | 7.77E-07 | 2.24E-07 |
| 7 | 9.05E-08 | 1.03E-07 |
| 8 | 1.14E-07 | 9.07E-08 |
| 9 | 1.90E-07 | 1.09E-07 |
| 10 | 1.23E-07 | 6.98E-08 |
| 11 | 4.34E-07 | 4.19E-07 |
| 12 | 2.25E-08 | 1.48E-08 |
| 13 | 8.59E-08 | 2.92E-08 |
| 14 | 3.19E-07 | 2.48E-07 |
| 15 | 2.59E-07 | 3.55E-07 |
| 16 | 1.79E-07 | 2.11E-07 |
| 17 | 1.12E-07 | 8.81E-08 |
| 18 | 7.21E-08 | 1.04E-07 |
| 19 | 3.75E-08 | 6.32E-08 |
| 20 | 5.29E-08 | 8.35E-08 |
| 21 | 9.83E-08 | 9.90E-08 |
| 22 | 2.86E-07 | 3.33E-07 |
| 23 | 7.58E-08 | 9.93E-08 |
| 24 | 2.17E-08 | 3.02E-08 |

### 5. Proliferation assay: CDK12 degrader resistant MDA-MB-231 and CAL-120

CDK12-degrader resistant cells were generated by propagation of CAL-120 human breast adenocarcinoma cells (DSMZ ACC-459) and MDA-MB-231 human breast cancer cells (ATCC HTB-26) in presence of increasing concentrations of the CDK12 degrader compound example 4 from WO 2021/116178. Initial concentrations of example 4 were 2.0E-08 M for CAL-120 cells and 4.6E-08 M for MDA-MB-231 cells, corresponding to ca. 2-fold the IC50 of inhibition of the cells in an proliferation assay. After the treated cells recovered and proliferated again the concentration of compound example 4 was increased to 3-fold IC50. This procedure was repeated according to the following concentration steps: 4.5-fold IC50, 6-fold IC50, 7.5-fold IC50, 9-fold IC50, 12-fold IC50, 15-fold IC50, 20-fold IC50, 25-fold IC50, 30-fold IC50 (highest concentration for MDA-MB-231 cells), 50-fold IC50 (highest concentration for CAL-120 cells). This procedure resulted in the generation of a MDA-MB-231 cell pool named 'MDA-MB-231-CDK12d-res' proliferating in presence of 30-fold IC50 concentrations (6.9E-07 M) of compound example 4, and in a CAL-120 cell pool named 'CAL-120-CDK12d-res' proliferating in presence of 50-fold IC50 concentrations (5.0E-07 M) of compound example 4.

To determine the inhibitory effect of test compounds on proliferation of CAL-120-CDK12d-res, MDA-MB-231-CDK12d-res the cells were plated at a density of 4,000 cells/well in a 96-well multititer plate in 200 microL of their respective growth medium supplemented 10% fetal calf serum. After 24 hours, the cells of one plate (zero-point plate) were stained with crystal violet (see below), while the medium of the other plates was supplemented with the test substances in various concentrations (0 micromol/L, as well as in the range of 0.01-10 micromol/L; the final concentration of the solvent dimethyl sulfoxide was adjusted to 0.1%) using a Tecan HP D300 Digital Dispenser. The cells were incubated for 4 days in the presence of test substances. Cell proliferation was determined by staining the cells with crystal violet: the cells were fixed by adding 20 microL/measuring point of an 11% glutaric aldehyde solution for 15 minutes at room temperature. After three washing cycles of the fixed cells with water, the plates were dried at room temperature. The cells were stained by adding 100 microL/measuring point of a 0.1% crystal violet solution (pH 3.0). After three washing cycles of the stained cells with water, the plates were dried at room temperature. The dye was dissolved by adding 100 microL/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µM) cells (=100%). The IC50 values (inhibitory concentration at 50% of maximal effect) were determined by means of a 4 parameter fit.

**Table 6. Antiproliferative data for compounds of the present invention and comparator compounds.**

| Example No | Prolif-MDA MB-231, IC50 - [mol/l] (median) | Prolif-MDA MB-231-CDK12d-res, IC50 - [mol/l] (median) | Ratio | | Prolif-CAL-120, IC50 - [mol/l] (median) | Prolif-CAL-120-CDK12d-res, IC50 - [mol/l] (median) | Ratio |
|---|---|---|---|---|---|---|---|
| Comparator WO 2021/116178, example 4 | 2.53E-08 | 9.36E-07 | 37.0 | | 1.20E-08 | 7.34E-07 | 61.2 |
| Comparator WO 2021/116178, example 273 | 6.00E-09 | 4.27E-07 | 71.1 | | 4.49E-09 | 3.20E-07 | 71.3 |
| Comparator WO 2021/116178, example 274 | 6.74E-09 | 1.52E-07 | 22.6 | | 4.89E-09 | 1.02E-07 | 20.9 |
| 7 | 9.05E-08 | 1.56E-07 | 1.7 | | 1.03E-07 | 1.12E-07 | 1.1 |
| 8 | 1.14E-07 | 1.78E-07 | 1.6 | | 9.07E-08 | 1.73E-07 | 1.9 |
| 9 | 1.90E-07 | 1.50E-07 | 0.8 | | 1.09E-07 | 1.05E-07 | 1.0 |
| 10 | 1.23E-07 | 1.56E-07 | 1.3 | | 6.98E-08 | 1.39E-07 | 2.0 |
| 12 | 2.25E-08 | 3.10E-08 | 1.4 | | 1.48E-08 | 2.21E-08 | 1.5 |
| 17 | 1.12E-07 | 1.56E-07 | 1.4 | | 8.81E-08 | 1.34E-07 | 1.5 |
| 18 | 7.21E-08 | 2.88E-07 | 4.0 | | 1.04E-07 | 2.52E-07 | 2.4 |
| 19 | 3.75E-08 | 1.03E-07 | 2.7 | | 6.32E-08 | 9.71E-08 | 1.5 |
| 20 | 5.29E-08 | 1.55E-07 | 2.9 | | 8.35E-08 | 1.18E-07 | 1.4 |
| 24 | 2.17E-08 | 6.26E-08 | 2.9 | | 3.02E-08 | 4.07E-08 | 1.3 |

The data of Table 6 demonstrate that comparator compounds from WO 2021/116178, which exhibit strong CDK12/CyclinK protein degradation activity, show a 20- to more than 70-fold decreased anti proliferative potency against the resistant cell lines MDA-MB-231-CDK12d-res and CAL-120-CDK12d-res as compared to the parental cell lines MDA-MB-231 and CAL-120. In contrast, the compounds of the present invention are characterized by similar antiproliferative potency against resistant and parental cell lines (ratio below or equal to 4).

### 6. Proliferation assay: cell line panel

Human tumour cells were originally obtained from the American Type Culture Collection (ATCC), from Cell Line Services GmbH (CLS), from the Deutsche Sammlung von Mikroorganismen und Zellkulturen (DSMZ, German Collection of Microorganisms and Cell Cultures), from the Japanese Cancer Research Resources Bank (JCRB), from Public Health England, from the European Collection of Authenticated Cell Cultures (ECACC), from Asterand Biosciences, or from the National Cancer Institute (NCI). Cultivated tumour cells were plated at the density as indicated in Table 6 in 96-well multititer plates in 200 microL of their respective growth medium supplemented 10% fetal calf serum. After 24 hours, the cells of one plate (zero-point plate) were stained with crystal violet (see below), while the medium of the other plates was supplemented with the test substances in various concentrations (0 micromol/L, as well as in the range of 0.003-3 micromol/L; the final concentration of the solvent dimethyl sulfoxide was adjusted to 0.1%) using a Tecan HP D300 Digital Dispenser. The cells were incubated for 4 days in the presence of test substances. Cell proliferation was determined by staining the cells with crystal violet: the cells were fixed by adding 20 microL/measuring point of an 11% glutaric aldehyde solution for 15 minutes at room temperature. After three washing cycles of the fixed cells with water, the plates were dried at room temperature. The cells were stained by adding 100 microL/measuring point of a 0.1% crystal violet solution (pH 3.0). After three washing cycles of the stained cells with water, the plates were dried at room temperature. The dye was dissolved by adding 100 microL/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µM) cells (=100%). The IC50 values (inhibitory concentration at 50% of maximal effect) were determined by means of a 4 parameter fit.

**Table 7. Antiproliferative cell line panel data for examples no 7 and 8 of the present invention (Result category, IC50 (mean), A: ≤ 3.0E-08 [mol/I], B: between 3.0E-08 [mol/l] and 1.0E-07 [mol/l], C: ≥ 1.0E-07 [mol/I])**

| Cell line | Source | Indication | Cell density, cells/well | Result Category Example 7 / 8 |
|---|---|---|---|---|
| AU565 | ATCC CRL-2351 | breast cancer, HER2 positive | 4000 | B / A |
| BT474 | ATCC HTB-20 | breast cancer, HER2 positive | 4000 | B / B |
| EFM192A | DSMZ ACC 258 | breast cancer, HER2 positive | 4000 | C / C |
| HCC1419 | ATCC CRL-2326 | breast cancer, HER2 positive | 4000 | B / B |
| HCC1954 | ATCC CRL-2338 | breast cancer, HER2 positive | 4000 | B / B |
| CAL-120 | DSMZ ACC 549 | breast cancer, triple-negative | 4000 | C / B |
| HCC1143 | DSMZ ACC 517 | breast cancer, triple-negative | 4000 | C / C |
| HCC1806 | ATCC CRL-2335 | breast cancer, triple-negative | 4000 | C / B |
| MDA-MB-231 | DSMZ ACC 732 | breast cancer, triple-negative | 4000 | B / C |
| MDA-MB-468 | NCI | breast cancer, triple-negative | 4000 | B / B |
| HUH-7 | JRBC 0403 | liver cancer | 4000 | B / B |
| JHH-7 | JRBC 1031 | liver cancer | 4000 | C / B |
| NCl-H1299 | ATCC CRL-5803 | lung cancer, non-small cell | 4000 | C / C |
| HT-144 | ATCC HTB-63 | melanoma | 4000 | B / B |
| COV318 | ECACC #7071903 | ovarian cancer | 4000 | C / C |

### 7. CDK12 protein level: Protein Simple (CDK12, CAL-120), Westernblot (CDK9, 12, 13)

CAL-120 human breast cancer cells (DSMZ ACC 459) were seeded at 300,000 cells/well in 6-well plates containing 2 mL of growth medium (DMEM, 10%FCS, glutamine) and incubated for 24h at 37°C in an humidified incubator. Test compounds were added at various concentrations, control wells received solvent (DMSO), and the plates were incubated for another 18h at 37°C. Cells were washed 2 times with PBS and lysed in 75 microL lysis buffer (MSD-Puffer (MSD, #R60TX-2), +1% SDS +PhosSTOP (Roche #04906837001) + complete mini (Roche # 04693159001)) by scraping. The lysates were pushed 2 times through Qiashedders followed by centrifugation at 14,000 rpm for 30-50 sec. The supernatant was stored at -20°C. Proteins were separated by applying 0.4 microgramms of protein lysate to Protein Simple 66 - 440 kDa (Protein Simple #SM-S002) size assay columns on a PEGGY SUE or SALLY SUE equipment according to the supplier's manual. CDK12 and HSP90 (loading control) were detected using anti-human CDK12 antibody (Cell Signaling Technologies (CST) #11793) at 1:25 dilution and anti-human HSP90 antibody (CST #4877) at 1:5,000 dilution. CDK12 and HSP90 peak areas were determined using Protein Simple Compass software. Ratio of CDK12/HSP90 peak areas were calculated for each sample, and DC50 values (degrading concentration to achieve 50% reduction relative to vehicle treated control) were determined by means of a 4 parameter fit.

Westernblot analyses were performed according to standard protocols. 40 microgramms of protein lysates per lane were subjected to polyacrylamide gel electrophoresis using NuPAGE 3-8% tris acetate gels (ThermoFisher) for detection of CDK12 and CDK13 or using NuPAGE 4-12% bis-tris gels (ThermoFisher) for detection of CDK9 followed by protein transfer to nitrocellulose membranes using a BioRad Transblot Turbo equipment. Membranes were probed with rabbit anti CDK12 antibodies (CST # 11793), rabbit anti-CDK13 antibodies (Novus #NB 100-68268), anti-CDK9 antibodies (CST #2316), and anti HSP90 (Becton Dickinson #610419) or anti-GAPDH (Zytomed #RGM2-6C5) antibodies for loading control.

### 8. CDK12 nuclear protein level: immunofluorescence l High Content Analysis

In the context of the present invention, the term "DC50 CDK12" refers to the DC₅₀ values obtained according to the assay described in this section (7) herein below, i.e. the DC₅₀ values for the degradation of CDK12.

CAL-120 human breast cancer cells (DSMZ ACC 459) are seeded in 1536-well microtiter plates (800 cells per well) containing 50 nanoL of compounds in Dose-Response. Control wells received DMSO or Example 4. Plates are then incubated for 24h at 37°C in an humidified incubator and fixed with 4% PFA for 10 min. Then immunofluorescence (IF) against CDK12 (CellSignalling CDK12 Antibody #11973, rabbit, 1:100 dilution) is performed using standard IF protocols. Cells are then stained with Hoechst 33342 (Life Technologies, H-1399, 0.1 microg/ml) and imaged on an automated confocal microscopy system (e.g. Perkin Elmer Opera Phenix). Nuclear and cytoplasmic intensity as well as the nuclear/cytoplasmic intensity ratio is determined by automated image analysis using custom generated scripts (MetaXpress). Data is then transferred to Genedata Screener software, normalized to DMSO and control and DC50 values (degrading concentration to achieve 50% reduction of nuclear CDK12 staining intensity relative to controls) are reported.

**Table 9. Immunofluorescence CDK12 degradation - DC₅₀ [mol/L]**

| Example No | CDK12 degradation IF, DC50 - [mol/l] (median) |
|---|---|
| 1 | 2.38E-06 |
| 2 | > 2.79E-6 |
| 3 | > 2.88E-6 |
| 4 | > 1.00E-6 |
| 5 | 1.00E-06 |
| 6 | 1.00E-06 |
| 7 | 6.71E-07 |
| 8 | 8.93E-07 |
| 9 | 9.50E-07 |
| 10 | > 1.00E-6 |
| 11 | 9.20E-07 |
| 12 | > 1.00E-6 |
| 13 | 1.20E-07 |
| 14 | 6.65E-07 |
| 15 | > 1.00E-6 |
| 16 | > 1.00E-6 |
| 17 | > 1.00E-6 |
| 18 | > 1.00E-6 |
| 19 | |
| 20 | > 1.00E-6 |
| 21 | > 1.00E-6 |
| 22 | > 1.00E-6 |
| 23 | > 1.00E-6 |
| 24 | > 1.00E-6 |

**Table 10. Inhibition IC₅₀ CDK12 high ATP to Degradation DC₅₀ CDK12 ratio**

| Example No | (IC50 CDK12 high ATP) to (Degradation DC50 CDK12) ratio |
|---|---|
| 1 | 0.77 |
| 2 | < 0.27 |
| 3 | < 0.09 |
| 4 | < 0.03 |
| 5 | 0.26 |
| 6 | 0.74 |
| 7 | 0.04 |
| 8 | 9.34E-03 |
| 9 | 9.69E-03 |
| 10 | < 7.59E-3 |
| 11 | 0.04 |
| 12 | < 3.09E-3 |
| 13 | 0.1 |
| 14 | |
| 15 | < 0.53 |
| 16 | < 0.08 |
| 17 | < 5.96E-3 |
| 18 | < 0.04 |
| 19 | |
| 20 | < 0.01 |
| 21 | |
| 22 | < 0.49 |
| 23 | < 0.16 |
| 24 | < 0.02 |

### 9. CYCLIN K nuclear protein level: immunofluorescence / High Content Analysis

CAL-120 human breast cancer cells (DSMZ ACC 459) are seeded in 1536-well microtiter plates (800 cells per well) containing 50 nanoL of compounds in Dose-Response. Control wells received DMSO or Example 4. Plates are then incubated for 24h at 37°C in an humidified incubator and fixed with 4% PFA for 10 min. Then immunofluorescence (IF) against CYCLIN K (ThermoFisher Scientific CCNK Antibody # PA5-85020, rabbit, 1:200 dilution) is performed using standard IF protocols. Cells are then stained with Hoechst 33342 (Life Technologies, H-1399, 0.1 microg/ml) and imaged on an automated confocal microscopy system (e.g. Perkin Elmer Opera Phenix). Nuclear and cytoplasmic intensity as well as the nuclear/cytoplasmic intensity ratio is determined by automated image analysis using custom generated scripts (MetaXpress). Data is then transferred to Genedata Screener software, normalized to DMSO and control and DC50 values (degrading concentration to achieve 50% reduction of nuclear CCNK staining intensity relative to controls) are reported.

**Table 11. Immunofluorescence CYCLIN K degradation - DC₅₀ [mol/L]**

| Example No | CYCLIN K degradation IF, DC50 - [mol/l] (median) |
|---|---|
| 1 | |
| 2 | |
| 4 | |
| 5 | > 1.00E-6 |
| 6 | > 1.00E-6 |
| 8 | > 1.00E-6 |
| 10 | 2.28E-07 |
| 11 | > 1.00E-6 |
| 15 | > 1.00E-6 |
| 17 | > 1.00E-6 |
| 18 | > 1.00E-6 |
| 20 | > 1.00E-6 |
| 23 | 1.24E-07 |
| 24 | > 1.00E-6 |

### 10. In vivo xenotransplantation models

The anti-tumor activity of test compound was examined in murine xenotransplantation models of human cancer. For this purpose, mice were implanted subcutaneously or orthotopically with specific human tumor cells. At a mean tumor size of 20-30 mm² animals were randomized into treatment and control groups (n=10 animals/group) and treatment started with vehicle only or Compound (formulation: 80% PEG400/20% Water; application route: p.o./per os , orally; dose/schedule: 5 mg/kg daily (QD), 5 mg/kg twice daily (2QD) for 2 days on/ 5 days off). The oral application volume was 10 mL/kg. The time interval between two applications per day was 6-7h. The experiment was ended when the untreated control group had tumors of area ≤ 225 mm². The tumor size and the body weight were determined three times weekly. Changes in the body weight were a measure of treatment-related toxicity (> 10% = critical body weight loss and stop of treatment until recovery, > 20% = toxic, termination). The tumor area was detected by means of an electronic caliper gauge [length (mm) x width (mm)]. In vivo anti-tumor efficacy is presented as T/C ratio (Treatment/Control) calculated with tumor areas at study end by the formula [(tumor area of treatment group at day x) - (tumor area of treatment group at day before first treatment)] / [(tumor area of control group at day x) - (tumor area of control group at day before first treatment)]. A compound having a T/C below 0.5 is defined as active (effective). Statistical analysis was assessed using SigmaStat software. A one-way analysis of variance was performed and differences to the control were compared by a pair-wise comparison procedure (Dunn's method).

### 11. CYP inhibition assay

Use of in vitro assays to evaluate the inhibition potential of new drug candidates towards CYP-mediated metabolism has been shown to be effective as part of a strategy to minimize the chances of drug interactions with co-administered drugs.

The inhibitory potency of the test compound towards 5 human cytochrome P450 isoforms (CYP1A2, 2C8, 2C9, 2D6, and 3A4) was determined during the lead optimization phase. In case of CYP3A4 also time dependent inhibitory potential was tested by applying a 30 min pre-incubation time of the test compound in metabolically active incubation system. Human liver microsomes (pooled, >30 male and female donors) were incubated with individual CYP isoform-selective standard probes (phenacetin, amodiaquine, diclofenac, dextromethorphan and midazolam) in the absence and presence of increasing concentrations of the test compound. Furthermore, the inhibitory potency of standard inhibitors was included as positive controls (fluvoxamine for CYP1A2, montelukast for CYP2C8, sulfaphenazole for CYP2C9, fluoxetine for CYP2D6, ketoconazole for CYP3A4 and mibefradil for CYP3A4-preincubation). Incubation conditions (protein and substrate concentration, incubation time) were optimized with regard to linearity and metabolite turnover. Incubation medium consists of 50 millimol/L potassium phosphate buffer (pH 7.4) containing 1 millimol/L EDTA, NADPH regenerating system (1 millimol/L NADP, 5 millimol/L glucose 6-phosphate, glucose 6-phosphate dehydrogenase (1.5 U/mL). Sequential dilutions and incubations were performed on a Freedom Evo Workstation (Tecan, Crailsheim, FRG) in 96-well plates at 37 °C. A final incubation volume of 200 µL was used. Reactions were stopped by addition of 100 µL acetonitrile containing the respective internal standard. After centrifugation the supernatants were analyzed by LC-MS/MS. The LC-MS/MS system for quantification of paracetamol (CYP1A2), desethylamodiaquine (CYP2C8), 4'-hydroxydiclofenac (CYP2C9), dextrorphan (CYP2D6), and 1'-hydroxymidazolam (CYP3A4) comprised a QTRAP 6500^{®} LC-MS/MS system (Applied Biosystems, MDS Sciex, Ontario, Canada) equipped with an electrospray ionization (ESI) interface (Turboionspray^{®} interface) used to generate positive [M+H]+ ions, an Agilent HP 1290 liquid chromatograph (Agilent Technologies, Waldbronn, Germany) and a HTS PAL autosampler (CTC Analytics, Zwingen, Switzerland).

Data analysis: The CYP-mediated activities in the presence of test compounds (inhibitors) were expressed as percentages of the corresponding no inhibitor control samples. A sigmoid-shaped curve was fitted to the data, and the enzyme inhibition parameter IC50 was calculated using a nonlinear least-squares regression analysis of the plot of percent control activity versus concentration of the test inhibitor.

### 12. Investigation of in vitro metabolic stability in rat hepatocytes

Hepatocytes from Han Wistar rats were freshly isolated via a 2-step perfusion method. After perfusion, the liver was carefully removed from the rat: the liver capsule was opened and the hepatocytes were gently shaken out into a Petri dish with ice-cold Williams' medium E (WME). The resulting cell suspension was filtered through sterile gaze in 50 mL falcon tubes and centrifuged at 50 × *g* for 3 min at room temperature. The cell pellet was resuspended in 30 mL WME and centrifuged through a Percoll^{®} gradient for 2 times at 100 × *g*. The hepatocytes were washed again with WME and resuspended in medium containing 5% FCS. Cell viability was determined by trypan blue exclusion.

For the metabolic stability assay liver cells were distributed in WME containing 5% FCS to glass vials at a density of 1.0 × 10⁶ vital cells/ml. The test compound was added to a final concentration of 1 micromol/L. During incubation, the hepatocyte suspensions were continuously shaken at 580 rpm and aliquots were taken at 2, 8, 16, 30, 45 and 90 min, to which equal volumes of cold methanol were immediately added. Samples were frozen at -20° C over night, after subsequently centrifuged for 15 minutes at 3000 rpm and the supernatant was analyzed with an Agilent 1200 HPLC-system with LCMS/MS detection.

The half-life of a test compound was determined from the concentration-time plot. From the half-life the intrinsic clearances were calculated. Together with the additional parameters liver blood flow, amount of liver cells in vivo and in vitro. The hepatic in vivo blood clearance (CLblood) and the maximal oral bioavailability (Fmax) was calculated using the following formulae: CL'intrinsic [ml/(min*kg)] = kel [1/min] / ((cellno / volume of incubation [ml]) * fu,inc) * (cellno / liver weight [g]) * (specific liver weight [g liver /kg body weight]); CLblood well-stirred [L/(h*kg)] = (QH [L/(h*kg)] * fu,blood * CL'intrinsic [L/(h*kg)] ) / (QH [L/(h*kg)] + fu,blood * CL'intrinsic [L/(h*kg)]); Fmax = 1-CLblood / QH and using the following parameter values: Liver blood flow (QH) - 4.2 L/h/kg rat; specific liver weight - 32 g/kg rat body weight; liver cells in vivo- 1.1 x 108 cells/g liver, liver cells in vitro - 1.0 x 106/ml; fu,inc and fu,blood is taken as 1.

### 13. PXR Nuclear Receptor Activation

DPX2 cells (hepatoma cell line stably-cotransfected with a vector for human PXR and a Luciferase reporter gene under the control of two human CYP3A4 promotors, Puracyp, Carlsbad, CA) were cultivated according to manufacturer's instructions with following modifications: Cells were seeded in a 384 well plate and cultivated at 37 °C/ 5 % CO2 in humidified air. 24h prior read-out the cells were treated with compound in a ten point serial dilution of ~1:3 starting at the highest test concentration of 49.8 micromol/L and ending at 2 nanomol/L. Rifampicin was incubated in the same manner as positive control. In addition, for the normalization of the luminescence signal cells were incubated with Rifampicin at a concentration of 16.7 micromol/L corresponding to 100% activation, as well as DMSO for background luminescence corresponding to 0% activation (n=32 wells each). Cells were lyzed and incubated with the Luciferase substrate ONE-Glo^{™} Reagent (Promega, Madison WI, USA) according to manufacturer's instructions and luminescence signal was detected in a plate reader. A concentration-dependent increase of the luciferase activity above 10% of Rifampicin control was classified as PXR transactivation

### 14. In vivo pharmacokinetics in rats

For in vivo pharmacokinetic experiments test compounds were administered to male Wistar rats intravenously at doses of 0.3 to 1 mg/kg and intragastral at doses of 0.5 to 10 mg/kg formulated as solutions using solubilizers such as PEG400 in well-tolerated amounts.

For pharmacokinetics after intravenous administration test compounds were given as i.v. bolus and blood samples were taken at 2 min, 8 min, 15 min, 30 min, 45 min, 1 h, 2 h, 4 h, 6 h, 8 h and 24 h after dosing. Depending on the expected half-life additional samples were taken at later time points (e.g. 48 h, 72 h). For pharmacokinetics after intragastral administration test compounds were given intragastral to fasted rats and blood samples were taken at 5 min, 15 min, 30 min, 45 min, 1 h, 2 h, 4 h, 6 h, 8 h and 24 h after dosing. Depending on the expected half-life additional samples were taken at later time points (e.g. 48 h, 72 h). Blood was collected into Lithium-Heparintubes (Monovetten^{®} , Sarstedt) and centrifuged for 15 min at 3000 rpm. An aliquot of 100 µL from the supernatant (plasma) was taken and precipitated by addition of 400 µL cold acetonitril and frozen at - 20° C over night. Samples were subsequently thawed and centrifuged at 3000 rpm, 4° C for 20 minutes. Aliquots of the supernatants were taken for analytical testing using an Agilent 1200 HPLC-system with LCMS/MS detection. PK parameters were calculated by non-compartmental analysis using a PK calculation software.

PK parameters derived from concentration-time profiles after i.v.: CLplasma: Total plasma clearance of test compound (in L/kg/h); CLblood: Total blood clearance of test compound: CLplasma*Cp/Cb (in L/kg/h) with Cp/Cb being the ratio of concentrations in plasma and blood. PK parameters calculated from concentration time profiles after i.g.: Cmax: Maximal plasma concentration (in mg/L); Cmaxnorm: Cmax divided by the administered dose (in kg/L); Tmax: Time point at which Cmax was observed (in h). Parameters calculated from both, i.v. and i.g. concentration-time profiles: AUCnorm: Area under the concentration-time curve from t=0h to infinity (extrapolated) divided by the administered dose (in kg*h/L); AUC(0-tlast)norm: Area under the concentration-time curve from t=0h to the last time point for which plasma concentrations could be measured divided by the administered dose (in kg*h/L); t1/2: terminal half-life (in h); F: oral bioavailability: AUCnorm after intragastral administration divided by AUCnorm after intravenous administration (in %).

## Claims

1. A compound of formula **(I)** 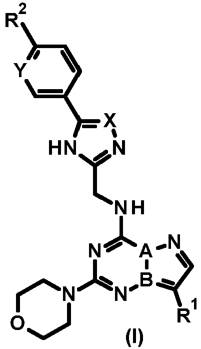 wherein
A and B are independently from each other, selected from a nitrogen atom or a carbon atom, wherein when A is a nitrogen atom, B is a carbon atom and wherein when A is a carbon atom, B is a nitrogen atom;
R¹ is selected from a halogen atom and a C₁-C₃-haloalkyl group;
R² is selected from a C₁-C₃-alkyl group, a C₁-C₃-alkoxy group, a C₁-C₃-haloalkyl group and a C₁-C₃-haloalkoxy group;
X is selected from a nitrogen atom and a CR³ group;
R³ is selected from a hydrogen atom, a C₁-C₃-alkyl group and a C₁-C₃-haloalkyl group;
Y is selected from a nitrogen atom or a carbon atom; or a tautomer, or an N-oxide, or a salt thereof, or a salt of a tautomer, or a salt of an N-oxide, or a mixture of same.

2. The compound according to claim 1, wherein
A and B are independently from each other, selected from a nitrogen atom or a carbon atom, wherein when A is a nitrogen atom, B is a carbon atom and wherein when A is a carbon atom, B is a nitrogen atom;
R¹ is a halogen atom;
R² is selected from a C₁-C₃-alkyl group, a C₁-C₃-alkoxy group, a C₁-C₃-haloalkyl group and a C₁-C₃-haloalkoxy group;
X is selected from a nitrogen atom and a CR³ group;
R³ is selected from a hydrogen atom, a C₁-C₃-alkyl group and a C₁-C₃-haloalkyl group;
Y is selected from a nitrogen atom or a carbon atom; or a tautomer, or an N-oxide, or a salt thereof, or a salt of a tautomer, or a salt of an N-oxide, or a mixture of same.

3. The compound according to claim 1, wherein
A and B are independently from each other, selected from a nitrogen atom or a carbon atom, wherein when A is a nitrogen atom, B is a carbon atom and wherein when A is a carbon atom, B is a nitrogen atom;
R¹ is a C₁-C₃-haloalkyl group;
R² is selected from a C₁-C₃-alkyl group, a C₁-C₃-alkoxy group, a C₁-C₃-haloalkyl group and a C₁-C₃-haloalkoxy group;
X is selected from a nitrogen atom and a CR³ group;
R³ is selected from a hydrogen atom, a C₁-C₃-alkyl group and a C₁-C₃-haloalkyl group;
Y is selected from a nitrogen atom or a carbon atom; or a tautomer, or an N-oxide, or a salt thereof, or a salt of a tautomer, or a salt of an N-oxide, or a mixture of same.

4. The compound according to claim 1 or claim 2, wherein
A and B are independently from each other, selected from a nitrogen atom or a carbon atom, wherein when A is a nitrogen atom, B is a carbon atom and wherein when A is a carbon atom, B is a nitrogen atom;
R¹ is a halogen atom;
R² is selected from a C₁-C₃-haloalkyl group and a C₁-C₃-haloalkoxy group;
X is selected from a nitrogen atom and a CR³ group;
R³ is selected from a hydrogen atom, a C₁-C₃-alkyl group and a C₁-C₃-haloalkyl group;
Y is selected from a nitrogen atom or a carbon atom; or a tautomer, or an N-oxide, or a salt thereof, or a salt of a tautomer, or a salt of an N-oxide, or a mixture of same.

5. The compound according to any one of claims 1 or 3, wherein
A and B are independently from each other, selected from a nitrogen atom or a carbon atom, wherein when A is a nitrogen atom, B is a carbon atom and wherein when A is a carbon atom, B is a nitrogen atom;
R¹ is a C₁-C₃-haloalkyl group;
R² is selected from a C₁-C₃-haloalkyl group and a C₁-C₃-haloalkoxy group;
X is selected from a nitrogen atom and a CR³ group;
R³ is selected from a hydrogen atom, a C₁-C₃-alkyl group and a C₁-C₃-haloalkyl group;
Y is selected from a nitrogen atom or a carbon atom; or a tautomer, or an N-oxide, or a salt thereof, or a salt of a tautomer, or a salt of an N-oxide, or a mixture of same.

6. The compound according to any one of claims 1 to 5, wherein
A is a nitrogen atom,
B is a carbon atom; or a tautomer, or an N-oxide, or a salt thereof, or a salt of a tautomer, or a salt of an N-oxide, or a mixture of same.

7. The compound according to any one of claims 1 to 5, wherein
A is a carbon atom,
B is a nitrogen atom; or a tautomer, or an N-oxide, or a salt thereof, or a salt of a tautomer, or a salt of an N-oxide, or a mixture of same.

8. The compound of formula (I) according to any one of claims 1 to 7 selected from the group consisting of:
8-bromo-2-(morpholin-4-yl)-N-({5-[4-(trifluoromethyl)phenyl]-4H-1,2,4-triazol-3-yl}methyl)pyrazolo[1,5-a][1,3,5]triazin-4-amine,
8-bromo-2-(morpholin-4-yl)-N-({5-[4-(trifluoromethoxy)phenyl]-4H-1,2,4-triazol-3-yl}methyl)pyrazolo[1,5-a][1,3,5]triazin-4-amine,
8-bromo-2-(morpholin-4-yl)-N-({5-[4-(trifluoromethyl)phenyl]-1H-imidazol-2-yl}methyl)pyrazolo[1,5-a][1,3,5]triazin-4-amine,
8-bromo-2-(morpholin-4-yl)-N-({5-[4-(trifluoromethoxy)phenyl]-1H-imidazol-2-yl}methyl)pyrazolo[1,5-a][1,3,5]triazin-4-amine,
2-(morpholin-4-yl)-N-({5-[4-(trifluoromethoxy)phenyl]-4H-1,2,4-triazol-3-yl}methyl)-8-(trifluoromethyl)pyrazolo[1,5-a][1,3,5]triazin-4-amine,
2-(morpholin-4-yl)-8-(trifluoromethyl)-N-({5-[4-(trifluoromethyl)phenyl]-4H-1,2,4-triazol-3-yl}methyl)pyrazolo[1,5-a][1,3,5]triazin-4-amine,
2-(morpholin-4-yl)-7-(trifluoromethyl)-N-({5-[4-(trifluoromethyl)phenyl]-1H-imidazol-2-yl}methyl)imidazo[2,1-f][1,2,4]triazin-4-amine,
2-(morpholin-4-yl)-N-({5-[4-(trifluoromethoxy)phenyl]-1H-imidazol-2-yl}methyl)-7-(trifluoromethyl)imidazo[2,1-f][1,2,4]triazin-4-amine,
2-(morpholin-4-yl)-8-(trifluoromethyl)-N-({5-[4-(trifluoromethyl)phenyl]-1H-imidazol-2-yl}methyl)pyrazolo[1,5-a][1,3,5]triazin-4-amine,
2-(morpholin-4-yl)-N-({5-[4-(trifluoromethoxy)phenyl]-1H-imidazol-2-yl}methyl)-8-(trifluoromethyl)pyrazolo[1,5-a][1,3,5]triazin-4-amine,
2-(morpholin-4-yl)-7-(trifluoromethyl)-N-({5-[4-(trifluoromethyl)phenyl]-4H-1,2,4-triazol-3-yl}methyl)imidazo[2,1-f][1,2,4]triazin-4-amine,
2-(morpholin-4-yl)-7-(trifluoromethyl)-N-({5-[6-(trifluoromethyl)pyridin-3-yl]-1H-imidazol-2-yl}methyl)imidazo[2,1-f][1,2,4]triazin-4-amine,
N-{[5-(4-methoxyphenyl)-1H-imidazol-2-yl]methyl}-2-(morpholin-4-yl)-7-(trifluoromethyl)imidazo[2,1-f][1,2,4]triazin-4-amine,
2-(morpholin-4-yl)-N-({5-[4-(trifluoromethoxy)phenyl]-4H-1,2,4-triazol-3-yl}methyl)-7-(trifluoromethyl)imidazo[2,1-f][1,2,4]triazin-4-amine,
N-{[5-(6-methoxypyridin-3-yl)-4H-1,2,4-triazol-3-yl]methyl}-2-(morpholin-4-yl)-8-(trifluoromethyl)pyrazolo[1,5-a][1,3,5]triazin-4-amine,
2-(morpholin-4-yl)-8-(trifluoromethyl)-N-({5-[6-(trifluoromethyl)pyridin-3-yl]-4H-1,2,4-triazol-3-yl}methyl)pyrazolo[1,5-a][1,3,5]triazin-4-amine,
2-(morpholin-4-yl)-N-({5-[6-(trifluoromethoxy)pyridin-3-yl]-4H-1,2,4-triazol-3-yl}methyl)-8-(trifluoromethyl)pyrazolo[1,5-a][1,3,5]triazin-4-amine,
N-{[5-(6-methoxypyridin-3-yl)-4H-1,2,4-triazol-3-yl]methyl}-2-(morpholin-4-yl)-7-(trifluoromethyl)imidazo[2,1-f][1,2,4]triazin-4-amine,
2-(morpholin-4-yl)-N-({5-[6-(trifluoromethoxy)pyridin-3-yl]-4H-1,2,4-triazol-3-yl}methyl)-7-(trifluoromethyl)imidazo[2,1-f][1,2,4]triazin-4-amine,
2-(morpholin-4-yl)-7-(trifluoromethyl)-N-({5-[6-(trifluoromethyl)pyridin-3-yl]-4H-1,2,4-triazol-3-yl}methyl)imidazo[2,1-f][1,2,4]triazin-4-amine,
N-{[5-(6-methoxypyridin-3-yl)-1H-imidazol-2-yl]methyl}-2-(morpholin-4-yl)-8-(trifluoromethyl)pyrazolo[1,5-a][1,3,5]triazin-4-amine,
N-{[5-(6-methylpyridin-3-yl)-4H-1,2,4-triazol-3-yl]methyl}-2-(morpholin-4-yl)-8-(trifluoromethyl)pyrazolo[1,5-a][1,3,5]triazin-4-amine,
N-{[5-(6-methylpyridin-3-yl)-4H-1,2,4-triazol-3-yl]methyl}-2-(morpholin-4-yl)-7-(trifluoromethyl)imidazo[2,1-f][1,2,4]triazin-4-amine and
2-(morpholin-4-yl)-8-(trifluoromethyl)-N-({5-[6-(trifluoromethyl)pyridin-3-yl]-1H-imidazol-2-yl}methyl)pyrazolo[1,5-a][1,3,5]triazin-4-amine.

9. A compound of formula (I), or a stereoisomer, a tautomer, an N-oxide, a hydrate, a solvate, or a salt thereof, particularly a pharmaceutically acceptable salt thereof, or a mixture of same, according to any one of claims 1-8 for use as a medicament.

10. A compound of formula (I), or a stereoisomer, a tautomer, an N-oxide, a hydrate, a solvate, or a salt thereof, particularly a pharmaceutically acceptable salt thereof, or a mixture of same, according to any one of claims 1-8 for use in the treatment and/or prophylaxis of a disease, preferably wherein the disease is a hyperproliferative disorder.

11. A compound of formula (I), or a stereoisomer, a tautomer, an N-oxide, a hydrate, a solvate, or a salt thereof, particularly a pharmaceutically acceptable salt thereof, or a mixture of same, according to any one of claims 1-8 for use in the treatment of breast cancer, liver cancer, lung cancer, ovarian cancer, endometrial cancer, cervical cancer, colorectal cancer, gastric cancer, esophageal cancer, bladder cancer, prostate cancer, Ewing sarcoma, glioblastoma and acute myeloid leukemia.

12. A compound of formula (I), or a stereoisomer, a tautomer, an N-oxide, a hydrate, a solvate, or a salt thereof, particularly a pharmaceutically acceptable salt thereof, or a mixture of same, according to any one of claims 1-8 for use in the treatment of lung cancer, breast cancer, liver cancer, colorectal cancer, gastric cancer, prostate cancer and leukemia.

13. A pharmaceutical composition comprising a compound of formula (I) or a stereoisomer, a tautomer, an N-oxide, a hydrate, a solvate, or a salt thereof, particularly a pharmaceutically acceptable salt thereof, or a mixture of same, according to any one of claims 1-8, and a pharmaceutically acceptable carrier.

14. The pharmaceutical composition according to claim 13 for use in the treatment and/or prophylaxis of a hyperproliferative disease, preferably wherein the hyperproliferative disease is cancer such as lung cancer, breast cancer, liver cancer, colorectal cancer, gastric cancer, prostate cancer or leukemia.

15. A pharmaceutical combination comprising:
- one or more first active ingredients selected from a compound of general formula (I) according to any one of claims 1 to 8, and
- one or more second active ingredients selected from chemotherapeutic anti-cancer agents.

## Patentansprüche

1. Eine Verbindung mit der Formel **(I)** 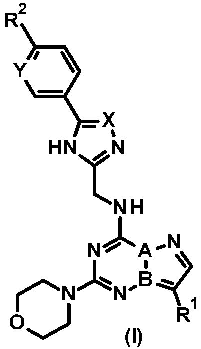 wobei
A und B unabhängig voneinander aus einem Stickstoffatom oder einem Kohlenstoffatom ausgewählt sind, wobei, wenn A ein Stickstoffatom ist, B ein Kohlenstoffatom ist, und wobei, wenn A ein Kohlenstoffatom ist, B ein Stickstoffatom ist;
R¹ aus einem Halogenatom und einer C₁-C₃-Halogenalkylgruppe ausgewählt ist;
R² aus einer C₁-C₃-Alkylgruppe, einer C₁-C₃-Alkoxygruppe, einer C₁-C₃-Halogenalkylgruppe und einer C₁-C₃-Halogenalkoxygruppe ausgewählt ist;
X aus einem Stickstoffatom und einer CR³-Gruppe ausgewählt ist;
R³ aus einem Wasserstoffatom, einer C₁-C₃-Alkylgruppe und einer C₁-C₃-Halogenalkylgruppe ausgewählt ist;
Y aus einem Stickstoffatom oder einem Kohlenstoffatom ausgewählt ist; oder ein Tautomer, oder ein N-Oxid, oder ein Salz davon, oder ein Salz eines Tautomers, oder ein Salz eines N-Oxids, oder eine Mischung davon.

2. Die Verbindung nach Anspruch 1, wobei
A und B unabhängig voneinander aus einem Stickstoffatom oder einem Kohlenstoffatom ausgewählt sind, wobei, wenn A ein Stickstoffatom ist, B ein Kohlenstoffatom ist, und wobei, wenn A ein Kohlenstoffatom ist, B ein Stickstoffatom ist;
R¹ ein Halogenatom ist;
R² aus einer C₁-C₃-Alkylgruppe, einer C₁-C₃-Alkoxygruppe, einer C₁-C₃-Halogenalkylgruppe und einer C₁-C₃-Halogenalkoxygruppe ausgewählt ist;
X aus einem Stickstoffatom und einer CR³-Gruppe ausgewählt ist;
R³ aus einem Wasserstoffatom, einer C₁-C₃-Alkylgruppe und einer C₁-C₃-Halogenalkylgruppe ausgewählt ist;
Y aus einem Stickstoffatom oder einem Kohlenstoffatom ausgewählt ist; oder ein Tautomer, oder ein N-Oxid, oder ein Salz davon, oder ein Salz eines Tautomers, oder ein Salz eines N-Oxids, oder eine Mischung davon.

3. Die Verbindung nach Anspruch 1, wobei
A und B unabhängig voneinander aus einem Stickstoffatom oder einem Kohlenstoffatom ausgewählt sind, wobei, wenn A ein Stickstoffatom ist, B ein Kohlenstoffatom ist, und wobei, wenn A ein Kohlenstoffatom ist, B ein Stickstoffatom ist;
R¹ eine C₁-C₃-Halogenalkylgruppe ist;
R² aus einer C₁-C₃-Alkylgruppe, einer C₁-C₃-Alkoxygruppe, einer C₁-C₃-Halogenalkylgruppe und einer C₁-C₃-Halogenalkoxygruppe ausgewählt ist;
X aus einem Stickstoffatom und einer CR³-Gruppe ausgewählt ist;
R³ aus einem Wasserstoffatom, einer C₁-C₃-Alkylgruppe und einer C₁-C₃-Halogenalkylgruppe ausgewählt ist;
Y aus einem Stickstoffatom oder einem Kohlenstoffatom ausgewählt ist; oder ein Tautomer, oder ein N-Oxid, oder ein Salz davon, oder ein Salz eines Tautomers, oder ein Salz eines N-Oxids, oder eine Mischung davon.

4. Die Verbindung nach Anspruch 1 oder Anspruch 2, wobei
A und B unabhängig voneinander aus einem Stickstoffatom oder einem Kohlenstoffatom ausgewählt sind, wobei, wenn A ein Stickstoffatom ist, B ein Kohlenstoffatom ist, und wobei, wenn A ein Kohlenstoffatom ist, B ein Stickstoffatom ist;
R¹ ein Halogenatom ist;
R² aus einer C₁-C₃-Halogenalkylgruppe und einer C₁-C₃-Halogenalkoxygruppe ausgewählt ist;
X aus einem Stickstoffatom und einer CR³-Gruppe ausgewählt ist;
R³ aus einem Wasserstoffatom, einer C₁-C₃-Alkylgruppe und einer C₁-C₃-Halogenalkylgruppe ausgewählt ist;
Y aus einem Stickstoffatom oder einem Kohlenstoffatom ausgewählt ist; oder ein Tautomer, oder ein N-Oxid, oder ein Salz davon, oder ein Salz eines Tautomers, oder ein Salz eines N-Oxids, oder eine Mischung davon.

5. Die Verbindung gemäß einem der Ansprüche 1 oder 3, wobei
A und B unabhängig voneinander aus einem Stickstoffatom oder einem Kohlenstoffatom ausgewählt sind, wobei, wenn A ein Stickstoffatom ist, B ein Kohlenstoffatom ist, und wobei, wenn A ein Kohlenstoffatom ist, B ein Stickstoffatom ist;
R¹ eine C₁-C₃-Halogenalkylgruppe ist;
R² aus einer C₁-C₃-Halogenalkylgruppe und einer C₁-C₃-Halogenalkoxygruppe ausgewählt ist;
X aus einem Stickstoffatom und einer CR³-Gruppe ausgewählt ist;
R³ aus einem Wasserstoffatom, einer C₁-C₃-Alkylgruppe und einer C₁-C₃-Halogenalkylgruppe ausgewählt ist;
Y aus einem Stickstoffatom oder einem Kohlenstoffatom ausgewählt ist; oder ein Tautomer, oder ein N-Oxid, oder ein Salz davon, oder ein Salz eines Tautomers, oder ein Salz eines N-Oxids, oder eine Mischung davon.

6. Die Verbindung gemäß einem der Ansprüche 1 bis 5, wobei
A ein Stickstoffatom ist,
B ein Kohlenstoffatom ist; oder ein Tautomer, oder ein N-Oxid, oder ein Salz davon, oder ein Salz eines Tautomers, oder ein Salz eines N-Oxids, oder eine Mischung davon.

7. Die Verbindung gemäß einem der Ansprüche 1 bis 5, wobei
A ein Kohlenstoffatom ist,
B ein Stickstoffatom ist; oder ein Tautomer, oder ein N-Oxid, oder ein Salz davon, oder ein Salz eines Tautomers, oder ein Salz eines N-Oxids, oder eine Mischung davon.

8. Die Verbindung mit der Formel (I) gemäß einem der Ansprüche 1 bis 7, ausgewählt aus der Gruppe bestehend aus:
8-Brom-2-(morpholin-4-yl)-N-({5-[4-(trifluormethyl)phenyl]-4H-1,2,4-triazol-3-yl}methyl)pyrazolo[1,5-a][1,3,5]triazin-4-amin,
8-Brom-2-(morpholin-4-yl)-N-({5-[4-(trifluormethoxy)phenyl]-4H-1,2,4-triazol-3-yl}methyl)pyrazolo[1,5-a][1,3,5]triazin-4-amin,
8-Brom-2-(morpholin-4-yl)-N-({5-[4-(trifluormethyl)phenyl]-1H-imidazol-2-yl}methyl)pyrazolo[1,5-a][1,3,5]triazin-4-amin,
8-Brom-2-(morpholin-4-yl)-N-({5-[4-(trifluormethoxy)phenyl]-1H-imidazol-2-yl}methyl)pyrazolo[1,5-a][1,3,5]triazin-4-amin,
2-(Morpholin-4-yl)-N-({5-[4-(trifluormethoxy)phenyl]-4H-1,2,4-triazol-3-yl}methyl)-8-(trifluormethyl)pyrazolo[1,5-a][1,3,5]triazin-4-amin,
2-(Morpholin-4-yl)-8-(trifluormethyl)-N-({5-[4-(trifluormethyl)phenyl]-4H-1,2,4-triazol-3-yl}methyl)pyrazolo[1,5-a][1,3,5]triazin-4-amin,
2-(Morpholin-4-yl)-7-(trifluormethyl)-N-({5-[4-(trifluormethyl)phenyl]-1H-imidazol-2-yl}methyl)imidazo[2,1-f][1,2,4]triazin-4-amin,
2-(Morpholin-4-yl)-N-({5-[4-(trifluormethoxy)phenyl]-1H-imidazol-2-yl}methyl)-7-(trifluormethyl)imidazo[2,1-f][1,2,4]triazin-4-amin,
2-(Morpholin-4-yl)-8-(trifluormethyl)-N-({5-[4-(trifluormethyl)phenyl]-1H-imidazol-2-yl}methyl)pyrazolo[1,5-a][1,3,5]triazin-4-amin,
2-(Morpholin-4-yl)-N-({5-[4-(trifluormethoxy)phenyl]-1H-imidazol-2-yl}methyl)-8-(trifluormethyl)pyrazolo[1,5-a][1,3,5]triazin-4-amin,
2-(Morpholin-4-yl)-7-(trifluormethyl)-N-({5-[4-(trifluormethyl)phenyl]-4H-1,2,4-triazol-3-yl}methyl)imidazo[2,1-f][1,2,4]triazin-4-amin,
2-(Morpholin-4-yl)-7-(trifluormethyl)-N-({5-[6-(trifluormethyl)pyridin-3-yl]-1H-imidazol-2-yl}methyl)imidazo[2,1-f][1,2,4]triazin-4-amin,
N-{[5-(4-Methoxyphenyl)-1H-imidazol-2-yl]methyl}-2-(morpholin-4-yl)-7-(trifluormethyl)imidazo[2,1-f][1,2,4]triazin-4-amin,
2-(Morpholin-4-yl)-N-({5-[4-(trifluormethoxy)phenyl]-4H-1,2,4-triazol-3-yl}methyl)-7-(trifluormethyl)imidazo[2,1-f][1,2,4]triazin-4-amin,
N-{[5-(6-Methoxypyridin-3-yl)-4H-1,2,4-triazol-3-yl]methyl}-2-(morpholin-4-yl)-8-(trifluormethyl)pyrazolo[1,5-a][1,3,5]triazin-4-amin,
2-(Morpholin-4-yl)-8-(trifluormethyl)-N-({5-[6-(trifluormethyl)pyridin-3-yl]-4H-1,2,4-triazol-3-yl}methyl)pyrazolo[1,5-a][1,3,5]triazin-4-amin,
2-(Morpholin-4-yl)-N-({5-[6-(trifluormethoxy)pyridin-3-yl]-4H-1,2,4-triazol-3-yl}methyl)-8-(trifluormethyl)pyrazolo[1,5-a][1,3,5]triazin-4-amin,
N-{[5-(6-Methoxypyridin-3-yl)-4H-1,2,4-triazol-3-yl]methyl}-2-(morpholin-4-yl)-7-(trifluormethyl)imidazo[2,1-f][1,2,4]triazin-4-amin,
2-(Morpholin-4-yl)-N-({5-[6-(trifluormethoxy)pyridin-3-yl]-4H-1,2,4-triazol-3-yl}methyl)-7-(trifluormethyl)imidazo[2,1-f][1,2,4]triazin-4-amin,
2-(Morpholin-4-yl)-7-(trifluormethyl)-N-({5-[6-(trifluormethyl)pyridin-3-yl]-4H-1,2,4-triazol-3-yl}methyl)imidazo[2,1-f][1,2,4]triazin-4-amin,
N-{[5-(6-Methoxypyridin-3-yl)-1H-imidazol-2-yl]methyl}-2-(morpholin-4-yl)-8-(trifluormethyl)pyrazolo[1,5-a][1,3,5]triazin-4-amin,
N-{[5-(6-Methylpyridin-3-yl)-4H-1,2,4-triazol-3-yl]methyl}-2-(morpholin-4-yl)-8-(trifluormethyl)pyrazolo[1,5-a][1,3,5]triazin-4-amin,
N-{[5-(6-Methylpyridin-3-yl)-4H-1,2,4-triazol-3-yl]methyl}-2-(morpholin-4-yl)-7-(trifluormethyl)imidazo[2,1-f][1,2,4]triazin-4-amin und
2-(Morpholin-4-yl)-8-(trifluormethyl)-N-({5-[6-(trifluormethyl)pyridin-3-yl]-1H-imidazol-2-yl}methyl)pyrazolo[1,5-a][1,3,5]triazin-4-amin.

9. Eine Verbindung mit der Formel (I) oder ein Stereoisomer, ein Tautomer, ein N-Oxid, ein Hydrat, ein Solvat oder ein Salz davon, insbesondere ein pharmazeutisch verträgliches Salz davon, oder eine Mischung davon, gemäß einem der Ansprüche 1 bis 8 zur Verwendung als Arzneimittel.

10. Eine Verbindung mit der Formel (I) oder ein Stereoisomer, ein Tautomer, ein N-Oxid, ein Hydrat, ein Solvat oder ein Salz davon, insbesondere ein pharmazeutisch verträgliches Salz davon, oder eine Mischung davon, gemäß einem der Ansprüche 1 bis 8 zur Verwendung bei der Behandlung und/oder Prophylaxe einer Erkrankung, wobei die Erkrankung vorzugsweise eine hyperproliferative Erkrankung ist.

11. Eine Verbindung mit der Formel (I) oder ein Stereoisomer, ein Tautomer, ein N-Oxid, ein Hydrat, ein Solvat oder ein Salz davon, insbesondere ein pharmazeutisch verträgliches Salz davon, oder eine Mischung davon, gemäß einem der Ansprüche 1 bis 8 zur Verwendung bei der Behandlung von Brustkrebs, Leberkrebs, Lungenkrebs, Eierstockkrebs, Gebärmutterschleimhautkrebs, Gebärmutterhalskrebs, Darmkrebs, Magenkrebs, Speiseröhrenkrebs, Blasenkrebs, Prostatakrebs, Ewing-Sarkom, Glioblastom und akuter myeloischer Leukämie.

12. Eine Verbindung mit der Formel (I) oder ein Stereoisomer, ein Tautomer, ein N-Oxid, ein Hydrat, ein Solvat oder ein Salz davon, insbesondere ein pharmazeutisch verträgliches Salz davon, oder eine Mischung davon, gemäß einem der Ansprüche 1 bis 8 zur Verwendung bei der Behandlung von Lungenkrebs, Brustkrebs, Leberkrebs, Darmkrebs, Magenkrebs, Prostatakrebs und Leukämie.

13. Eine Pharmazeutische Zusammensetzung, umfassend eine Verbindung mit der Formel (I) oder ein Stereoisomer, ein Tautomer, ein N-Oxid, ein Hydrat, ein Solvat oder ein Salz davon, insbesondere ein pharmazeutisch verträgliches Salz davon, oder eine Mischung davon gemäß einem der Ansprüche 1 bis 8 sowie einen pharmazeutisch verträglichen Träger.

14. Die pharmazeutische Zusammensetzung gemäß Anspruch 13 zur Verwendung bei der Behandlung und/oder Prophylaxe einer hyperproliferativen Erkrankung, wobei die hyperproliferative Erkrankung vorzugsweise Krebs ist, wie Lungenkrebs, Brustkrebs, Leberkrebs, Darmkrebs, Magenkrebs, Prostatakrebs oder Leukämie.

15. Eine pharmazeutische Kombination, umfassend:
- einen oder mehrere erste Wirkstoffe, ausgewählt aus einer Verbindung mit der allgemeinen Formel (I) gemäß einem der Ansprüche 1 bis 8, und
- einen oder mehrere zweite Wirkstoffe, ausgewählt aus chemotherapeutischen Antikrebsmitteln.

## Revendications

1. Composé de formule **(I)** 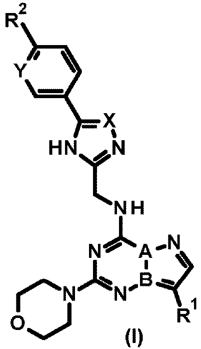 dans laquelle
A et B sont choisis, indépendamment l'un de l'autre, parmi un atome d'azote ou un atome de carbone, dans lequel, lorsque A est un atome d'azote, B est un atome de carbone, et dans lequel, lorsque A est un atome de carbone, B est un atome d'azote ;
R¹ est choisi parmi un atome d'halogène et un groupe haloalkyle en C₁ à C₃ ;
R² est choisi parmi un groupe alkyle en C₁ à C₃, un groupe alcoxy en C₁ à C₃, un groupe haloalkyle en C₁ à C₃ et un groupe haloalcoxy en C₁ à C₃ ;
X est choisi parmi un atome d'azote et un groupe CR³ ;
R³ est choisi parmi un atome d'hydrogène, un groupe alkyle en C₁ à C₃ et un groupe haloalkyle en C₁ à C₃ ;
Y est choisi parmi un atome d'azote ou un atome de carbone ; ou un tautomère, ou un N-oxyde, ou un sel de celui-ci, ou un sel d'un tautomère, ou un sel d'un N-oxyde, ou un mélange de ceux-ci.

2. Composé selon la revendication 1, dans lequel
A et B sont choisis, indépendamment l'un de l'autre, parmi un atome d'azote ou un atome de carbone, dans lequel, lorsque A est un atome d'azote, B est un atome de carbone, et dans lequel, lorsque A est un atome de carbone, B est un atome d'azote ;
R¹ est un atome d'halogène ;
R² est choisi parmi un groupe alkyle en C₁ à C₃, un groupe alcoxy en C₁ à C₃, un groupe haloalkyle en C₁ à C₃ et un groupe haloalcoxy en C₁ à C₃ ;
X est choisi parmi un atome d'azote et un groupe CR³ ;
R³ est choisi parmi un atome d'hydrogène, un groupe alkyle en C₁ à C₃ et un groupe haloalkyle en C₁ à C₃ ;
Y est choisi parmi un atome d'azote ou un atome de carbone ; ou un tautomère, ou un N-oxyde, ou un sel de celui-ci, ou un sel d'un tautomère, ou un sel d'un N-oxyde, ou un mélange de ceux-ci.

3. Composé selon la revendication 1, dans lequel
A et B sont choisis, indépendamment l'un de l'autre, parmi un atome d'azote ou un atome de carbone, dans lequel, lorsque A est un atome d'azote, B est un atome de carbone, et dans lequel, lorsque A est un atome de carbone, B est un atome d'azote ;
R¹ est un groupe haloalkyle en C₁ à C₃ ;
R² est choisi parmi un groupe alkyle en C₁ à C₃, un groupe alcoxy en C₁ à C₃, un groupe haloalkyle en C₁ à C₃ et un groupe haloalcoxy en C₁ à C₃ ;
X est choisi parmi un atome d'azote et un groupe CR³ ;
R³ est choisi parmi un atome d'hydrogène, un groupe alkyle en C₁ à C₃ et un groupe haloalkyle en C₁ à C₃ ;
Y est choisi parmi un atome d'azote ou un atome de carbone ; ou un tautomère, ou un N-oxyde, ou un sel de celui-ci, ou un sel d'un tautomère, ou un sel d'un N-oxyde, ou un mélange de ceux-ci.

4. Composé selon la revendication 1 ou 2, dans lequel
A et B sont choisis, indépendamment l'un de l'autre, parmi un atome d'azote ou un atome de carbone, dans lequel, lorsque A est un atome d'azote, B est un atome de carbone, et dans lequel, lorsque A est un atome de carbone, B est un atome d'azote ;
R¹ est un atome d'halogène ;
R² est choisi parmi un groupe haloalkyle en C₁ à C₃ et un groupe haloalcoxy en C₁ à C₃ ;
X est choisi parmi un atome d'azote et un groupe CR³ ;
R³ est choisi parmi un atome d'hydrogène, un groupe alkyle en C₁ à C₃ et un groupe haloalkyle en C₁ à C₃ ;
Y est choisi parmi un atome d'azote ou un atome de carbone ; ou un tautomère, ou un N-oxyde, ou un sel de celui-ci, ou un sel d'un tautomère, ou un sel d'un N-oxyde, ou un mélange de ceux-ci.

5. Composé selon l'une quelconque des revendications 1 ou 3, dans lequel
A et B sont choisis, indépendamment l'un de l'autre, parmi un atome d'azote ou un atome de carbone, dans lequel, lorsque A est un atome d'azote, B est un atome de carbone, et dans lequel, lorsque A est un atome de carbone, B est un atome d'azote ;
R¹ est un groupe haloalkyle en C₁ à C₃ ;
R² est choisi parmi un groupe haloalkyle en C₁ à C₃ et un groupe haloalcoxy en C₁ à C₃ ;
X est choisi parmi un atome d'azote et un groupe CR³ ;
R³ est choisi parmi un atome d'hydrogène, un groupe alkyle en C₁ à C₃ et un groupe haloalkyle en C₁ à C₃ ;
Y est choisi parmi un atome d'azote ou un atome de carbone ; ou un tautomère, ou un N-oxyde, ou un sel de celui-ci, ou un sel d'un tautomère, ou un sel d'un N-oxyde, ou un mélange de ceux-ci.

6. Composé selon l'une quelconque des revendications 1 à 5, dans lequel
A est un atome d'azote,
B est un atome de carbone ; ou un tautomère, ou un N-oxyde, ou un sel de celui-ci, ou un sel d'un tautomère, ou un sel d'un N-oxyde, ou un mélange de ceux-ci.

7. Composé selon l'une quelconque des revendications 1 à 5, dans lequel
A est un atome de carbone,
B est un atome d'azote ; ou un tautomère, ou un N-oxyde, ou un sel de celui-ci, ou un sel d'un tautomère, ou un sel d'un N-oxyde, ou un mélange de ceux-ci.

8. Composé de formule (I) selon l'une quelconque des revendications 1 à 7, choisi dans le groupe constitué par :
8-bromo-2-(morpholin-4-yl)-N-({5-[4-(trifluorométhyl)phényl]-4H-1,2,4-triazol-3-yl}méthyl)pyrazolo[1,5-a][1,3,5]triazin-4-amine,
8-bromo-2-(morpholin-4-yl)-N-({5-[4-(trifluorométhoxy)phényl]-4H-1,2,4-triazol-3-yl}méthyl)pyrazolo[1,5-a][1,3,5]triazin-4-amine,
8-bromo-2-(morpholin-4-yl)-N-({5-[4-(trifluorométhyl)phényl]-1H-imidazol-2-yl}méthyl)pyrazolo[1,5-a][1,3,5]triazin-4-amine,
8-bromo-2-(morpholin-4-yl)-N-({5-[4-(trifluorométhoxy)phényl]-1H-imidazol-2-yl}méthyl)pyrazolo[1,5-a][1,3,5]triazin-4-amine,
2-(morpholin-4-yl)-N-({5-[4-(trifluorométhoxy)phényl]-4H-1,2,4-triazol-3-yl}méthyl)-8-(trifluorométhyl)pyrazolo[1,5-a][1,3,5]triazin-4-amine,
2-(morpholin-4-yl)-8-(trifluorométhyl)-N-({5-[4-(trifluorométhyl)phényl]-4H-1,2,4-triazol-3-yl}méthyl)pyrazolo[1,5-a][1,3,5]triazin-4-amine,
2-(morpholin-4-yl)-7-(trifluorométhyl)-N-({5-[4-(trifluorométhyl)phényl]-1H-imidazol-2-yl}méthyl)imidazo[2,1-f][1,2,4]triazin-4-amine,
2-(morpholin-4-yl)-N-({5-[4-(trifluorométhoxy)phényl]-1H-imidazol-2-yl}méthyl)-7-(trifluorométhyl)imidazo[2,1-f][1,2,4]triazin-4-amine,
2-(morpholin-4-yl)-8-(trifluorométhyl)-N-({5-[4-(trifluorométhyl)phényl]-1H-imidazol-2-yl}méthyl)pyrazolo[1,5-a][1,3,5]triazin-4-amine,
2-(morpholin-4-yl)-N-({5-[4-(trifluorométhoxy)phényl]-1H-imidazol-2-yl}méthyl)-8-(trifluorométhyl)pyrazolo[1,5-a][1,3,5]triazin-4-amine,
2-(morpholin-4-yl)-7-(trifluorométhyl)-N-({5-[4-(trifluorométhyl)phényl]-4H-1,2,4-triazol-3-yl}méthyl)imidazo[2,1-f][1,2,4]triazin-4-amine,
2-(morpholin-4-yl)-7-(trifluorométhyl)-N-({5-[6-(trifluorométhyl)pyridin-3-yl]-1H-imidazol-2-yl}méthyl)imidazo[2,1-f][1,2,4]triazin-4-amine,
N-{[5-(4-méthoxyphényl)-1H-imidazol-2-yl]méthyl}-2-(morpholin-4-yl)-7-(trifluorométhyl)imidazo[2,1-f][1,2,4]triazin-4-amine,
2-(morpholin-4-yl)-N-({5-[4-(trifluorométhoxy)phényl]-4H-1,2,4-triazol-3-yl}méthyl)-7-(trifluorométhyl)imidazo[2,1-f][1,2,4]triazin-4-amine,
N-{[5-(6-méthoxypyridin-3-yl)-4H-1,2,4-triazol-3-yl]méthyl}-2-(morpholin-4-yl)-8-(trifluorométhyl)pyrazolo[1,5-a][1,3,5]triazin-4-amine,
2-(morpholin-4-yl)-8-(trifluorométhyl)-N-({5-[6-(trifluorométhyl)pyridin-3-yl]-4H-1,2,4-triazol-3-yl}méthyl)pyrazolo[1,5-a][1,3,5]triazin-4-amine,
2-(morpholin-4-yl)-N-({5-[6-(trifluorométhoxy)pyridin-3-yl]-4H-1,2,4-triazol-3-yl}méthyl)-8-(trifluorométhyl)pyrazolo[1,5-a][1,3,5]triazin-4-amine,
N-{[5-(6-méthoxypyridin-3-yl)-4H-1,2,4-triazol-3-yl]méthyl}-2-(morpholin-4-yl)-7-(trifluorométhyl)imidazo[2,1-f][1,2,4]triazin-4-amine,
2-(morpholin-4-yl)-N-({5-[6-(trifluorométhoxy)pyridin-3-yl]-4H-1,2,4-triazol-3-yl}méthyl)-7-(trifluorométhyl)imidazo[2,1-f][1,2,4]triazin-4-amine,
2-(morpholin-4-yl)-7-(trifluorométhyl)-N-({5-[6-(trifluorométhyl)pyridin-3-yl]-4H-1,2,4-triazol-3-yl}méthyl)imidazo[2,1-f][1,2,4]triazin-4-amine,
N-{[5-(6-méthoxypyridin-3-yl)-1H-imidazol-2-yl]méthyl}-2-(morpholin-4-yl)-8-(trifluorométhyl)pyrazolo[1,5-a][1,3,5]triazin-4-amine,
N-{[5-(6-méthylpyridin-3-yl)-4H-1,2,4-triazol-3-yl]méthyl}-2-(morpholin-4-yl)-8-(trifluorométhyl)pyrazolo[1,5-a][1,3,5]triazin-4-amine,
N-{[5-(6-méthylpyridin-3-yl)-4H-1,2,4-triazol-3-yl]méthyl}-2-(morpholin-4-yl)-7-(trifluorométhyl)imidazo[2,1-f][1,2,4]triazin-4-amine, et
2-(morpholin-4-yl)-8-(trifluorométhyl)-N-({5-[6-(trifluorométhyl)pyridin-3-yl]-1H-imidazol-2-yl}méthyl)pyrazolo[1,5-a][1,3,5]triazin-4-amine.

9. Composé de formule (I), ou un de ses stéréoisomères, tautomères, N-oxydes, hydrates, solvates ou sels, en particulier un de ses sels pharmaceutiquement acceptables, ou un mélange de ceux-ci, selon l'une quelconque des revendications 1 à 8, destiné à être utilisé comme médicament.

10. Composé de formule (I), ou un de ses stéréoisomères, tautomères, N-oxydes, hydrates, solvates ou sels, en particulier un de ses sels pharmaceutiquement acceptables, ou un mélange de ceux-ci, selon l'une quelconque des revendications 1 à 8, destiné à être utilisé dans le traitement et/ou la prophylaxie d'une maladie, de préférence dans laquelle la maladie est un trouble hyperprolifératif.

11. Composé de formule (I), ou un de ses stéréoisomères, tautomères, N-oxydes, hydrates, solvates ou sels, en particulier un de ses sels pharmaceutiquement acceptables, ou un mélange de ceux-ci, selon l'une quelconque des revendications 1 à 8, destiné à être utilisé dans le traitement du cancer du sein, du cancer du foie, du cancer du poumon, du cancer de l'ovaire, du cancer de l'endomètre, du cancer du col de l'utérus, du cancer colorectal, du cancer de l'estomac, du cancer de l'œsophage, du cancer de la vessie, du cancer de la prostate, du sarcome d'Ewing, du glioblastome et de la leucémie myéloïde aiguë.

12. Composé de formule (I), ou un de ses stéréoisomères, tautomères, N-oxydes, hydrates, solvates ou sels, en particulier un de ses sels pharmaceutiquement acceptables, ou un mélange de ceux-ci, selon l'une quelconque des revendications 1 à 8, destiné à être utilisé dans le traitement du cancer du poumon, du cancer du sein, du cancer du foie, du cancer colorectal, du cancer de l'estomac, du cancer de la prostate et de la leucémie.

13. Composition pharmaceutique comprenant un composé de formule (I) ou un de ses stéréoisomères, tautomères, N-oxydes, hydrates, solvates ou sels, en particulier un de ses sels pharmaceutiquement acceptables, ou un mélange de ceux-ci, selon l'une quelconque des revendications 1 à 8, et un excipient pharmaceutiquement acceptable.

14. Composition pharmaceutique selon la revendication 13, destinée à être utilisée dans le traitement et/ou la prophylaxie d'une maladie hyperproliférative, de préférence dans laquelle la maladie hyperproliférative est un cancer tel que le cancer du poumon, le cancer du sein, le cancer du foie, le cancer colorectal, le cancer de l'estomac, le cancer de la prostate ou la leucémie.

15. Combinaison pharmaceutique, comprenant :
- un ou plusieurs premiers principes actifs choisis parmi un composé de formule générale (I) selon l'une quelconque des revendications 1 à 8, et
- un ou plusieurs seconds principes actifs choisis parmi des agents anticancéreux chimiothérapeutiques.
